# EUROPEAN PATENT APPLICATION

(11) **EP 4 635 958 A1**
(43) Date of publication of application: **22.10.2025**
(21) Application number: 23902835.0
(22) Date of filing: 15.12.2023
(51) Int. Cl.: C07D 498/22, A61K 31/553, A61P 35/00

(54) **PYRIMIDINE-CONTAINING POLYCYCLIC DERIVATIVE INHIBITOR, AND PREPARATION METHOD THEREFOR AND USE THEREOF**

(30) Priority: 15.12.2022 CN 202211616536; 16.01.2023 CN 202310077472; 14.07.2023 CN 202310870328; 11.09.2023 CN 202311168469; 18.09.2023 CN 202311205445; 02.11.2023 CN 202311451767; 27.11.2023 CN 202311598512
(71) Applicant: Shanghai Hansoh Biomedical Co., Ltd., Shanghai 201203 (CN); Jiangsu Hansoh Pharmaceutical Group Co., Ltd., Lianyungang, Jiangsu 222047 (CN)
(72) Inventor: LIU, Shiqiang, Shanghai 201203 (CN); YU, Mingcheng, Shanghai 201203 (CN); YUAN, Yida, Shanghai 201203 (CN); CEN, Cheng, Shanghai 201203 (CN); YU, Wensheng, Shanghai 201203 (CN); LEE, Chih-Hung, Shanghai 201203 (CN)
(74) Representative: Cabinet Beau de Loménie
(86) International application number: PCT/CN2023/139221
(87) International publication number: WO 2024/125642

(57) **Abstract**

The present invention relates to a pyrimidine-containing polycyclic derivative inhibitor, a preparation method therefor and a use thereof. In particular, the present invention relates to a pyrimidine-containing polycyclic compound or a stereoisomer thereof, a preparation method therefor, a pharmaceutical composition containing the compound, and a use thereof in preparation of a drug for treating cancer and other related diseases.

## Description

### Technical Field

The present invention belongs to the field of drug synthesis, and specifically relates to a pyrimidine-containing polycyclic derivative inhibitor, and a preparation method therefor and the use thereof.

### Background Art

Rat sarcoma (RAS) protein is encoded by proto-oncogenes HRAS, NRAS and KRAS, is divided into four proteins HRAS, NRAS, KRAS4A and KRAS4B, and is a GTP (guanosine triphosphate) binding protein. RAS is located on the inner surface of the cell membrane, and its upstream is receptor tyrosine kinase (RTK). Upon activation, RAS regulates downstream signaling pathways such as PI3K and RAF, thereby regulating cell growth, survival, migration, differentiation and other functions.

RAS has two main states in the body: an inactive state bound to GDP (guanosine diphosphate) and an activated state bound to GTP. Its activity is regulated by two proteins: guanine nucleotide exchange factor (GEF) which promotes the release of GDP from RAS protein, allowing GTP to bind and activate RAS; GTPase activating protein (GAP), which activates the GTPase activity of RAS protein, hydrolyzes GTP bound to RAS protein into GDP and inactivates RAS. Under normal circumstances, RAS protein is in an inactive state. After mutation, its conformation changes, and RAS is in a continuously activated state, and the downstream signaling pathways are also continuously activated, leading to the occurrence of various cancers.

RAS is the first confirmed oncogene and the oncogene with the highest mutation rate, accounting for an average of 25% in human cancers. The most common oncogenic mutation in the RAS family is KRAS (85%), while NRAS (12%) and HRAS (3%) are less common. KRAS mutations are mainly found in a series of cancers such as pancreatic cancer (95%), colorectal cancer (52%), and lung cancer (31%). The most common mutation of KRAS is point mutation, which often occurs at G12 and G13 in the p-loop (aa 10 to 17) and at Q61 in the Switch II region (aa 59 to 76), among which G12 mutation is the most common (83%). Epidemiological studies in European and American countries have shown that KRAS G12D is the most common pathogenic mutation in pancreatic cancer, intestinal cancer, endometrial cancer and lung cancer, with incidence rates of 36%, 12%, 6% and 4%, respectively.

Although there is a huge clinical need, there is no drug directly targeting KRAS on the market so far. Currently, the only clinical treatment for patients with KRAS mutations is chemotherapy. There are two main factors that make the development of KRAS inhibitors difficult. Firstly, the RAS protein has a smooth structure, making it difficult for small molecules to bind to the protein surface; secondly, the affinity of RAS GTPase for GTP is as high as picomolar (pM) level, and the level of endogenous GTP is high, making it difficult for small molecule drugs to block the binding of RAS GTPase and GTP. Currently, no KRAS G12D inhibitors have entered clinical trials.

There are currently no specific targeted drugs for KRAS G12D, and there is great clinical demand. KRAS G12D inhibitors with a higher selectivity, a better activity and a better safety have the potential to treat a variety of cancers and have broad market prospects.

### Summary of the Invention

An object of the present invention is to provide a compound represented by general formula (I), or a stereoisomer or pharmaceutically acceptable salt thereof: 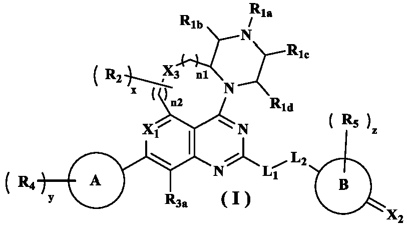
X₁ is selected from N or CR_{3b};
X₂ is selected from N-OR₆ₐ or CR_{6b}R_{6c};
X₃ is selected from O, S or NRₙ₁; preferably O or S;
L₁ is selected from a bond, O, S or NRₙ₂; preferably O or S; more preferably O;
L₂ is selected from C₁-C₄ alkylene, and the C₁-C₄ alkylene is optionally substituted with 1 or 2 Rₐ; preferably C₁-C₄ alkylene or C₁-C₄ deuterated alkylene, wherein the C₁-C₄ alkylene and C₁-C₄ deuterated alkylene are optionally substituted with 1 to 4 Rₐ;
ring A is selected from C₃₋₁₂ cycloalkyl, 3- to 12-membered heterocyclyl, C₆₋₁₄ aryl, or 5- to 14-membered heteroaryl; preferably C₃₋₁₀ cycloalkyl, 3- to 10-membered heterocyclyl, C₆₋₁₂ aryl or 5- to 12-membered heteroaryl;
ring B is selected from C₃₋₁₂ cycloalkyl, 3- to 12-membered heterocyclyl, C₆₋₁₄ aryl, or 5- to 14-membered heteroaryl; preferably C₃₋₁₀ cycloalkyl, 3- to 10-membered heterocyclyl, C₆₋₁₂ aryl or 5- to 12-membered heteroaryl; more preferably 5- to 8-membered saturated or unsaturated heterocyclyl, 7- to 10-membered fused heterocyclyl or 6- to 10-membered bridged heterocyclyl;
R₁ₐ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -C(O)(CH₂)ₙ₃R_{b}, -C(O)O(CH₂)ₙ₃R_{b} or - C(O)[(CH₂NR_{c}C(O))]ₙ₄(CH₂)ₙ₃R_{b}, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl and any CH₂ of (CH₂)ₙ₃ are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl, -C(O)(CH₂)ₙ₃R_{b}, -C(O)O(CH₂)ₙ₃R_{b} or - C(O)[(CH₂NR_{c}C(O))]ₙ₄(CH₂)ₙ₃R_{b}, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl and any CH₂ of (CH₂)ₙ₃ are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl;
R₁ₐ is preferably hydrogen or Pg, wherein the Pg is selected from trityl, benzyl, p-toluenesulfonyl, p-methoxybenzyl, formate, acetyl, benzyloxycarbonyl, tert-butoxycarbonyl, p-methoxyphenyl, -C(O)O(CRₐₐR_{bb})ₙ₉OC(O)(CR_{dd}Rₑₑ)ₙ₁₀R_{b}, - C(O)(CRₐₐR_{bb})ₙ₉R_{b}, -C(O)O(CRₐₐR_{bb})ₙ₉R_{b} or - C(O)[(CRₐₐR_{bb}NR_{cc}C(O))]ₙ₉(CR_{dd}Rₑₑ)ₙ₁₀R_{b};
each of R_{1b}, R_{1c} or R_{1d} is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
or R_{1b} and R_{1c} together with adjacent atoms to which they are attached form 3- to 12-membered heterocyclyl or 5- to 14-membered heteroaryl optionally substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, -(CH₂)ₙ₇-C₁₋₆ alkoxy, -(CH₂)ₙ₇-C₁₋₆ deuterated alkoxy, -(CH₂)ₙ₇-C₁₋₆ haloalkoxy, -(CH₂)ₙ₇-C₂₋₆ alkenyl, -(CH₂)ₙ₇-C₂₋₆ alkynyl, -(CH₂)ₙ₇-C₃₋₁₂ cycloalkyl, -(CH₂)ₙ₇-3- to 12-membered heterocyclyl, -(CH₂)ₙ₇-C₆₋₁₄ aryl or - (CH₂)ₙ₇-5- to 14-membered heteroaryl; preferably form 3- to 12-membered heterocyclyl or 5- to 14-membered heteroaryl optionally substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; more preferably form C₃₋₈ cycloalkyl, 3- to 8-membered heterocyclyl, C₆₋₁₀ aryl or 5- to 10-membered heteroaryl optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₂ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl; more preferably hydrogen, deuterium, methyl, ethyl, deuterated methyl, deuterated ethyl, halomethyl or haloethyl;
or any two R₂ substituents together with adjacent atoms to which they are attached form C₃₋₁₂ cycloalkyl or 3- to 12-membered heterocyclyl optionally substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; more preferably form C₃₋₈ cycloalkyl or 3- to 8-membered heterocyclyl optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl
each of R₃ₐ or R_{3b} is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₄ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ alkyl-C(O)O-, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -OC(O)(CH₂)ₙ₅R_{d}, -O(CH₂)ₙ₅C(O)R_{d}, -O(CH₂)ₙ₅C(O)OR_{d}, - O(CH₂)ₙ₅OC(O)OR_{d}, -OC(O)O(CH₂)ₙ₅R_{d}, -O(CH₂)ₙ₅C(O)NR_{d}Rₑ, - O(CH₂)ₙ₅OC(O)NR_{d}Rₑ or -O(CH₂)ₙ₅P(=O)R_{d}Rₑ, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl and any CH₂ of (CH₂)ₙ₅ are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl; preferably hydrogen, deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₁₋₃ alkyl-C(O)O-, C₂₋₄ alkenyl, C₂₋₄ alkynyl, C₃₋₈ cycloalkyl, 3- to 8-membered heterocyclyl, C₆₋₁₀ aryl, 5-to 10-membered heteroaryl, -OC(O)(CH₂)ₙ₅R_{d}, -O(CH₂)ₙ₅C(O)R_{d}, - O(CH₂)ₙ₅C(O)OR_{d}, -O(CH₂)ₙ₅OC(O)OR_{d}, -OC(O)O(CH₂)ₙ₅R_{d}, - O(CH₂)ₙ₅C(O)NR_{d}Rₑ, -O(CH₂)ₙ₅OC(O)NR_{d}Rₑ or -O(CH₂)ₙ₅P(=O)R_{d}Rₑ, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₁₋₃ alkyl-C(O)O-, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl and any CH₂ of (CH₂)ₙ₅ are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₅ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ alkyl-C(O)O-, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -OC(O)(CH₂)ₙ₆R_{f}, -O(CH₂)ₙ₆C(O)R_{f}, -O(CH₂)ₙ₁C(O)OR_{f}, - OC(O)O(CH₂)ₙ₆R_{f}, -O(CH₂)ₙ₆C(O)NR_{f}R_{g}, -O(CH₂)ₙ₆OC(O)NR_{f}R_{g} or - O(CH₂)ₙ₆P(=O) R_{f}R_{g}, wherein the amino, hydroxyl, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl and any CH₂ of (CH₂)ₙ₆ are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl; preferably hydrogen, deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl, -OC(O)(CH₂)ₙ₆R_{f}, -O(CH₂)ₙ₆C(O)R_{f}, -O(CH₂)ₙ₆C(O)OR_{f}, - OC(O)O(CH₂)ₙ₆R_{f}, -O(CH₂)ₙ₆C(O)NR_{f}R_{g}, -O(CH₂)ₙ₆OC(O)NR_{f}R_{g} or - O(CH₂)ₙ₆P(=O) R_{f}R_{g}, wherein the amino, hydroxyl, C₁₋₃ alkyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl and any CH₂ of (CH₂)ₙ₆ are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
or any two R₅ together with adjacent atoms to which they are attached form C₃₋₁₂ cycloalkyl or 3- to 12-membered heterocyclyl optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl; preferably form C₃₋₈ cycloalkyl or 3- to 8-membered heterocyclyl optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
each of R₆ₐ, R_{6b} or R_{6c} is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
or R_{6b} and R_{6c} together with adjacent atoms to which they are attached form C₃₋₁₂ cycloalkyl or 3- to 12-membered heterocyclyl optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl; preferably form C₃₋₈ cycloalkyl or 3- to 8-membered heterocyclyl optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
each of Rₙ₁ or Rₙ₂ is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
each of Rₐ, R_{b}, R_{c}, R_{d}, Rₑ, R_{f} or R_{g} is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
or two Rₐ attached to the same carbon atom are connected to form C₃₋₁₂ cycloalkyl or 3- to 12-membered heterocyclyl optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl; preferably form C₃₋₈ cycloalkyl or 3- to 8-membered heterocyclyl optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
each of Rₐₐ, R_{bb}, R_{cc}, R_{dd} or Rₑₑ is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
x is selected from 0, 1, 2, 3, 4, 5 or 6;
y is selected from 0, 1, 2, 3, 4, 5 or 6;
z is selected from 0, 1, 2, 3, 4, 5 or 6;
n1 is selected from 0, 1 or 2;
n2 is selected from 0, 1 or 2;
n3 is selected from 0, 1, 2, 3, 4, 5 or 6;
n4 is selected from 0, 1, 2, 3, 4, 5 or 6;
n5 is selected from 0, 1, 2, 3, 4, 5 or 6;
n6 is selected from 0, 1, 2, 3, 4, 5 or 6;
n7 is selected from 0, 1 or 2;
n9 is selected from 0, 1 or 2; and
n10 is selected from 0, 1 or 2.

In a preferred embodiment, the compound, or the stereoisomer or pharmaceutically acceptable salt thereof is characterized in that, R_{1b} and R_{1c} together with adjacent atoms to which they are attached form the following structure 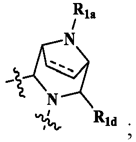 preferably form the following structure 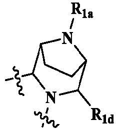 or 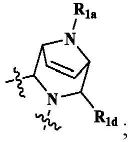 more preferably form the following structure

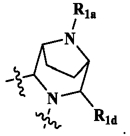

In a preferred embodiment, the compound, or the stereoisomer or pharmaceutically acceptable salt thereof is characterized in that, L₂ is selected from 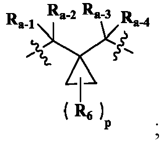 preferably 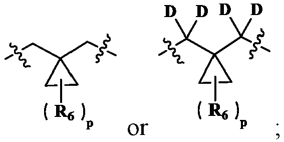
each of Rₐ₋₁, Rₐ₋₂, Rₐ₋₃ and Rₐ₋₄ is independently selected from hydrogen, deuterium, halogen, amino, hydroxyl, cyano, C₁₋₆ alkyl, C₁₋₆ deuterated alkyl, C₁₋₆ haloalkyl or C₁₋₆ hydroxyalkyl; preferably hydrogen, deuterium, halogen, amino, hydroxyl, cyano, C₁₋₃ alkyl, C₁₋₃ deuterated alkyl, C₁₋₃ haloalkyl or C₁₋₃ hydroxyalkyl; more preferably hydrogen or deuterium;
R₆ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl;
and p is selected from 0, 1, 2, 3 or 4.

In a preferred embodiment, the compound, or the stereoisomer or pharmaceutically acceptable salt thereof is characterized in that, ring A is selected from phenyl, pyridyl, naphthyl, benzothienyl, benzopyrazolyl, quinolyl or isoquinolyl.

In a preferred embodiment, the compound, or the stereoisomer or pharmaceutically acceptable salt thereof is characterized in that, the compound is further represented by general formula (II) or (II-A): 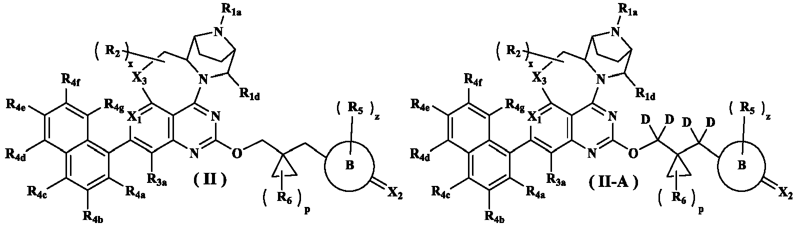
R_{4b} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ alkyl-C(O)O-, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -OC(O)(CH₂)ₙ₅R_{d}, -O(CH₂)ₙ₅C(O)R_{d}, -O(CH₂)ₙ₅C(O)OR_{d}, - O(CH₂)ₙ₅OC(O)OR_{d}, -OC(O)O(CH₂)ₙ₅R_{d}, -O(CH₂)ₙ₅C(O)NR_{d}Rₑ, - O(CH₂)ₙ₅OC(O)NR_{d}Rₑ or -O(CH₂)ₙ₅P(=O)R_{d}Rₑ, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl and any CH₂ of (CH₂)ₙ₅ are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl; preferably selected from hydrogen, deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₁₋₃ alkyl-C(O)O-, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl, -OC(O)(CH₂)ₙ₅R_{d}, -O(CH₂)ₙ₅C(O)R_{d}, - O(CH₂)ₙ₅C(O)OR_{d}, -O(CH₂)ₙ₅OC(O)OR_{d}, -OC(O)O(CH₂)ₙ₅R_{d}, - O(CH₂)ₙ₅C(O)NR_{d}Rₑ, -O(CH₂)ₙ₅OC(O)NR_{d}Rₑ or -O(CH₂)ₙ₅P(=O)R_{d}Rₑ, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₁₋₃ alkyl-C(O)O-, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl and any CH₂ of (CH₂)ₙ₅ are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
each of R₄ₐ, R_{4c}, R_{4d} or R₄ₑ is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{4f} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{4g} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl; more preferably C₂₋₄ alkynyl;
R₆ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
p is selected from 0, 1, 2, 3 or 4;
R₁ₐ, R_{1d}, R₂, R₃ₐ, R_{3b}, R₅, X₁, X₂, X₃, x and z are as defined in any embodiment above.

In a preferred embodiment, the compound is further represented by general formula (III): 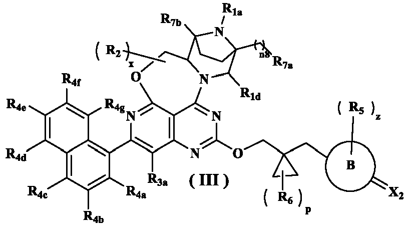
R_{4b} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ alkyl-C(O)O-, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl; preferably hydrogen, deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₁₋₃ alkyl-C(O)O-, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₁₋₃ alkyl-C(O)O-, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
each of R₄ₐ, R_{4c}, R_{4d} or R₄ₑ is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{4f} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{4g} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₆ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₇ₐ is selected from deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ deuterated alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ deuterated alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{7b} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
p is selected from 0, 1, 2, 3 or 4;
n8 is selected from 0, 1 or 2;
R₁ₐ, R_{1d}, R₂, R₃ₐ, R₅, X₂, X₃, x and z are as defined in any embodiment.

In a preferred embodiment, the compound is further represented by general formula (II-B) or (II-C): 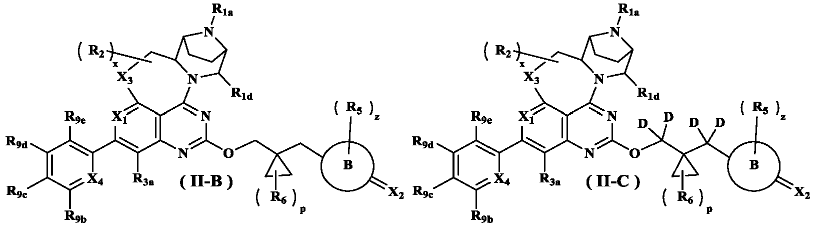
preferably, the compound is further represented by general formula (II-B-1) or (II-C-1): 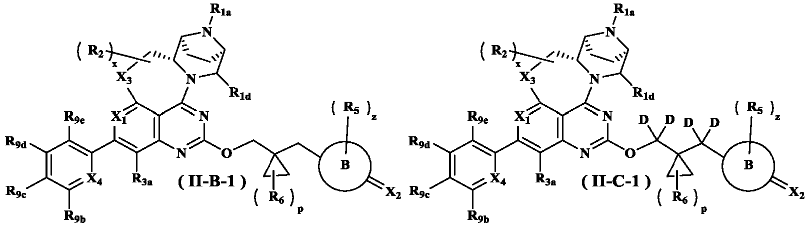
X₂ is selected from N-OR₆ₐ or CR_{6b}R_{6c}; preferably N-OR₆ₐ;
X₄ is selected from N or CR₉ₐ;
R₉ₐ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{9b} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{9c} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{9d} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₉ₑ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₆ is selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
p is selected from 0, 1, 2, 3 or 4;
R₁ₐ, R_{1d}, R₂, R₃ₐ, R_{3b}, R₅, X₁, X₂, X₃, x and z are as defined in any embodiment above.

In a preferred embodiment, the compound is further represented by general formula (V): 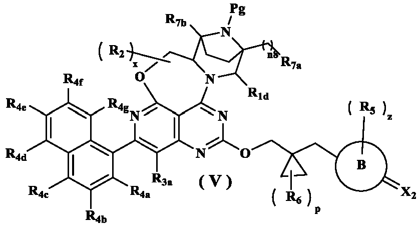
X₂ is selected from N-OR₆ₐ or CR_{6b}R_{6c};
ring B is selected from C₃₋₁₂ cycloalkyl, 3- to 12-membered heterocyclyl, C₆₋₁₄ aryl, or 5- to 14-membered heteroaryl; preferably C₃₋₁₀ cycloalkyl, 3- to 10-membered heterocyclyl, C₆₋₁₂ aryl or 5- to 12-membered heteroaryl; more preferably 5- to 8-membered saturated or unsaturated heterocyclyl, 7- to 10-membered fused heterocyclyl or 6- to 10-membered bridged heterocyclyl;
Pg is selected from trityl, benzyl, p-toluenesulfonyl, p-methoxybenzyl, formate, acetyl, benzyloxycarbonyl, tert-butoxycarbonyl, p-methoxyphenyl, - C(O)O(CRₐₐR_{bb})ₙ₉OC(O)(CR_{dd}Rₑₑ)ₙ₁₀R_{b}, -C(O)(CRₐₐR_{bb})ₙ₉R_{b}, - C(O)O(CRₐₐR_{bb})ₙ₉R_{b} or -C(O)[(CRₐₐR_{bb}NR_{cc}C(O))]ₙ₉(CR_{dd}Rₑₑ)ₙ₁₀R_{b};
R_{b} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
each of Rₐₐ, R_{bb}, R_{cc}, R_{dd} or Rₑₑ is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl; more preferably hydrogen, deuterium, fluorine, chlorine, bromine, amino, nitro, hydroxyl, cyano, mercapto, methyl, ethyl, propyl or isopropyl;
R₂ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₃ₐ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{4b} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ alkyl-C(O)O-, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -OC(O)(CH₂)ₙ₅R_{d}, -O(CH₂)ₙ₅C(O)R_{d}, -O(CH₂)ₙ₅C(O)OR_{d}, - O(CH₂)ₙ₅OC(O)OR_{d}, -OC(O)O(CH₂)ₙ₅R_{d}, -O(CH₂)ₙ₅C(0)NRₐRₑ, - O(CH₂)ₙ₅OC(O)NR_{d}R, or -O(CH₂)ₙ₅P(=O)R_{d}Rₑ, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl and any CH₂ of (CH₂)ₙ₅ are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl; preferably selected from hydrogen, deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₁₋₃ alkyl-C(O)O-, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl, -OC(O)(CH₂)ₙ₅R₄, -O(CH₂)ₙ₅C(O)R_{d}, - O(CH₂)ₙ₅C(O)OR_{d}, -O(CH₂)ₙ₅OC(O)OR_{d}, -OC(O)O(CH₂)ₙ₅R_{d}, - O(CH₂)ₙ₅C(O)NR_{d}Rₑ, -O(CH₂)ₙ₅OC(O)NR_{d}Rₑ or -O(CH₂)ₙ₅P(=O)R_{d}Rₑ, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₁₋₃ alkyl-C(O)O-, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl and any CH₂ of (CH₂)ₙ₅ are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₄ₐ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{4c} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{4d} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₄ₑ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{4f} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{4g} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₅ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ alkyl-C(O)O-, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, hydroxyl, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl; preferably selected from hydrogen, deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, hydroxyl, C₁₋₃ alkyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₆ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₆ₐ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{6b} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{6c} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₇ₐ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ deuterated alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ deuterated alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ deuterated alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{7b} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{d} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
Rₑ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
n5 is selected from 0, 1, 2, 3, 4, 5 or 6;
n8 is selected from 0, 1 or 2;
n9 is selected from 0, 1 or 2;
n10 is selected from 0, 1 or 2;
x is selected from 0, 1, 2, 3, 4, 5 or 6;
z is selected from 0, 1, 2, 3, 4, 5 or 6;
and p is selected from 0, 1, 2, 3 or 4.

In a preferred embodiment, the compound is further represented by general formula (IV): 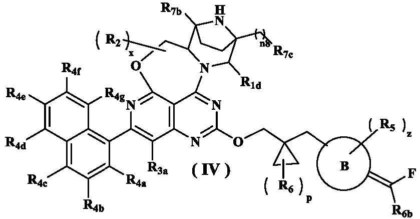
preferably, the compound is further represented by general formula (IV-1): 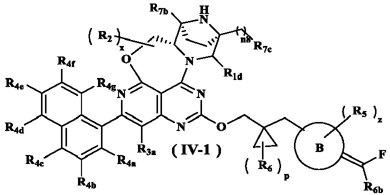
R_{1d} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen;
R₂ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen, deuterium, fluorine, chlorine, bromine, methyl, ethyl or cyclopropyl;
or two R₂ substituents together with adjacent atoms to which they are attached form C₃₋₈ cycloalkyl; preferably form cyclopropyl;
R₃ₐ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl or isopropyl; more preferably hydrogen or fluorine;
R₄ₐ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R_{4b} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, -OC(O) C₁₋₆ alkyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl, ethynyl, acetoxy, propionyloxy, butyryloxy, p-pivaloyloxy or cyclopropyl; more preferably hydrogen, deuterium, fluorine, chlorine, hydroxyl, amino, methyl, acetoxy, propionyloxy, butyryloxy or p-pivaloyloxy;
R_{4c} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R_{4d} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R₄ₑ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R_{4f} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R_{4g} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine, hydroxyl, cyano, methyl, ethyl or ethynyl;
R₅ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen, deuterium, fluorine, chlorine, amino, hydroxyl, cyano, methyl or ethyl;
R_{6b} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen, fluorine, difluoromethyl or trifluoromethyl;
R_{7c} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ deuterated alkoxy, C₁₋₃ haloalkoxy, C₂₋₄ alkenyl, C₂₋₄ haloalkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, deuterated methoxy, deuterated ethoxy, hydroxymethyl, hydroxyethyl, vinyl, halovinyl, or ethynyl; more preferably hydrogen, deuterium, fluorine, chlorine, methyl, ethyl, methoxy, ethoxy, deuterated methoxy, deuterated ethoxy, vinyl, monofluorovinyl or difluorovinyl;
R_{7b} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen or deuterium;
x is selected from 0, 1 or 2;
z is selected from 0, 1, 2, 3, 4, 5 or 6;
p is selected from 0, 1 or 2;
and n8 is selected from 0, 1 or 2.

In a preferred embodiment, the compound, or the stereoisomer or pharmaceutically acceptable salt thereof is characterized in that, ring B is selected from 5- to 8-membered saturated or unsaturated monocyclic heterocyclyl, 5- or 6-membered heterocyclyl fused C₃₋₆ cycloalkyl, 5- or 6-membered heterocyclyl fused 5- or 6-membered heterocyclyl, 5- or 6-membered heterocyclyl fused phenyl, 5- or 6-membered heterocyclyl fused 5- or 6-membered heteroaryl or 6- to 10-membered bridged heterocyclyl;
preferably selected from

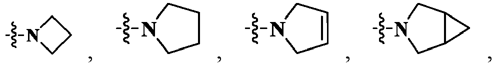

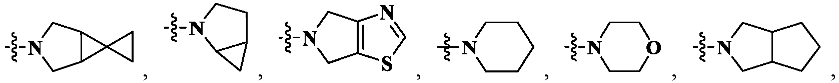

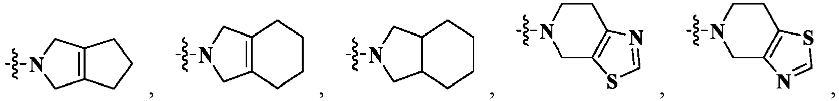

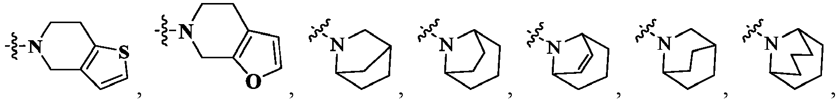

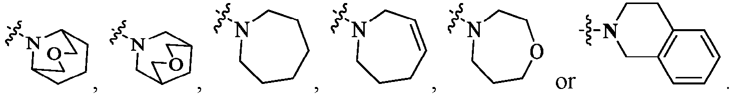

In a preferred embodiment, the compound is further represented by general formula (III-A) or (III-B): 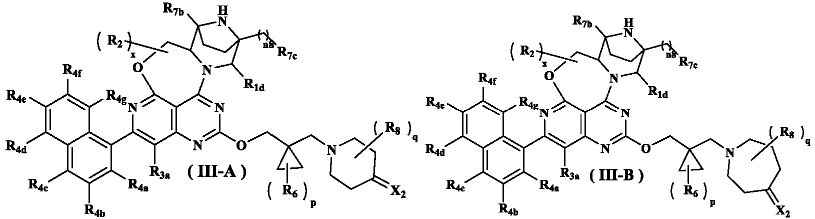
preferably the compound is further represented by general formula (III-A-1) or (III-B-1): 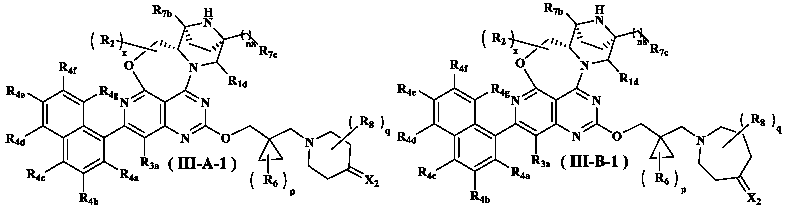
X₂ is selected from N-OR₆ₐ or CR_{6b}R_{6c}; preferably CR_{6b}R_{6c};
R_{1d} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen;
R₂ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen, deuterium, fluorine, chlorine, bromine, methyl, ethyl or cyclopropyl;
or two R₂ substituents together with adjacent atoms to which they are attached form C₃₋₈ cycloalkyl; preferably form cyclopropyl;
R₃ₐ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen or fluorine;
R₄ₐ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R_{4b} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, -OC(O) C₁₋₆ alkyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl, ethynyl, acetoxy, propionyloxy, butyryloxy, p-pivaloyloxy or cyclopropyl; more preferably hydrogen, deuterium, fluorine, chlorine, hydroxyl, amino, methyl, acetoxy, propionyloxy, butyryloxy or p-pivaloyloxy;
R_{4c} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R_{4d} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R₄, is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R_{4f} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R_{4g} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine, hydroxyl, cyano, methyl, ethyl or ethynyl;
R₆ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen;
R₆ₐ is selected from hydrogen, deuterium, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy or C₃₋₈ cycloalkyl; preferably hydrogen, deuterium, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably methyl, ethyl, -CH₂CH₂F, - CH₂CHF₂, -CH₂CF₃ or cyclopropyl;
R_{6b} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen, fluorine, difluoromethyl or trifluoromethyl;
R_{6c} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen or fluorine;
R_{7c} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ deuterated alkoxy, C₁₋₃ haloalkoxy, C₂₋₄ alkenyl, C₂₋₄ haloalkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, deuterated methoxy, deuterated ethoxy, hydroxymethyl, hydroxyethyl, vinyl, halovinyl, or ethynyl; more preferably hydrogen, deuterium, fluorine, chlorine, methyl, ethyl, methoxy, ethoxy, deuterated methoxy, deuterated ethoxy, vinyl, monofluorovinyl or difluorovinyl;
R_{7b} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen or deuterium;
R₈ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen, deuterium, fluorine, chlorine, amino, hydroxyl, cyano, methyl or ethyl;
or any two R₈ substituents together with adjacent atoms to which they are attached form C₃₋₈ cycloalkyl or 3- to 8-membered heterocyclyl; preferably form cyclopropyl, 5-membered nitrogen-containing heterocyclyl or 6-membered nitrogen-containing heterocyclyl;
x is selected from 0, 1 or 2;
p is selected from 0, 1 or 2;
q is selected from 0, 1, 2, 3, 4, 5 or 6;
and n8 is selected from 0, 1 or 2.

The present invention further provides a method for preparing a compound represented by general formula (I), or a stereoisomer or pharmaceutically acceptable salt thereof, comprising the following step: 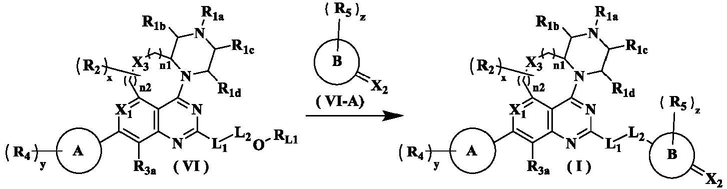
a compound represented by general formula (VI) is reacted with a compound represented by general formula (VI-A) in the presence of a halide salt and a base to obtain the compound represented by general formula (I);
R_{L1} is selected from hydrogen or a hydroxyl protecting group;
ring A, ring B, X₁, X₂, X₃, L₁, L₂, R₁ₐ, R_{1b}, R_{1c}, R_{1d}, R₂, R₃ₐ, R₄, R₅, x, y, z, n1 and n2 are as described in any of the above embodiments.

In a preferred embodiment, the preparation method is a method for preparing a compound represented by general formula (II-D), or a stereoisomer or pharmaceutically acceptable salt thereof, comprising the following step: 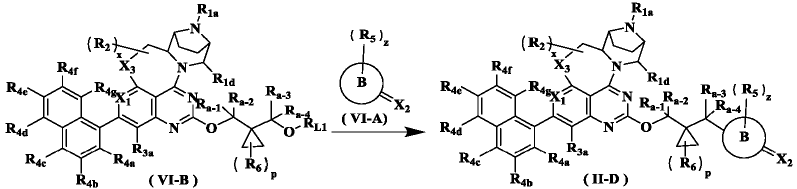
a compound represented by general formula (VI-B) is reacted with a compound represented by general formula (VI-A) in the presence of a halide salt and a base to obtain the compound represented by general formula (II-D);
R_{L1} is selected from hydrogen or a hydroxyl protecting group;
each of Rₐ₋₁, Rₐ₋₂, Rₐ₋₃ and Rₐ₋₄ is independently selected from hydrogen, deuterium, halogen, amino, hydroxyl, cyano, C₁₋₃ alkyl, C₁₋₃ deuterated alkyl, C₁₋₃ haloalkyl or C₁₋₃ hydroxyalkyl; preferably hydrogen or deuterium;
ring B, X₁, X₂, X₃, R₁ₐ, R_{1d}, R₂, R₃ₐ, R₄ₐ, R_{4b}, R_{4c}, R_{4d}, R₄ₑ, R_{4f}, R_{4g}, R₅, R₆, x, p or z are as described in any of the above embodiments;
when R_{4g} is alkynyl, R_{4g} can be optionally further substituted with C₁₋₆ silanyl in addition to the above definition.

In a preferred embodiment, the preparation method is a method for preparing a compound represented by general formula (II- E), or a stereoisomer or pharmaceutically acceptable salt thereof, comprising the following step: 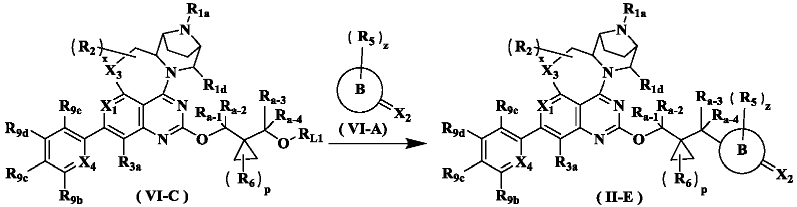
a compound represented by general formula (VI-C) is reacted with the compound represented by general formula (VI-A) in the presence of a halide salt and a base, and a protecting group is further removed to afford the compound represented by general formula (II-E);
R_{L1} is selected from hydrogen or a hydroxyl protecting group;
each of Rₐ₋₁, Rₐ₋₂, Rₐ₋₃ and Rₐ₋₄ is independently selected from hydrogen, deuterium, halogen, amino, hydroxyl, cyano, C₁₋₃ alkyl, C₁₋₃ deuterated alkyl, C₁₋₃ haloalkyl or C₁₋₃ hydroxyalkyl; preferably hydrogen or deuterium;
ring B, X₁, X₂, X₃, X₄, R₁ₐ, R_{1d}, R₂, R₃ₐ, R₉ₐ, R_{9b}, R_{9c}, R_{9d}, R₉ₑ, R₅, R₆, x, p or z are as described in any of the above embodiments.

In a preferred embodiment, the preparation method is a method for preparing a compound represented by general formula (III-A), or a stereoisomer or pharmaceutically acceptable salt thereof, comprising the following step: 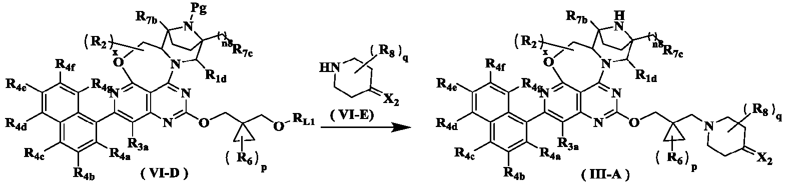
the compound represented by general formula (VI-D) is reacted with the compound represented by general formula (VI-E) in the presence of a halide salt and a base, and the protecting group is further removed to afford the compound represented by general formula (III-A);
R_{L1} is selected from hydrogen or a hydroxyl protecting group;
ring B, X₁, X₂, X₃, R₁ₐ, R_{1d}, R₂, R₃ₐ, R₄ₐ, R_{4b}, R_{4c}, R_{4d}, R₄ₑ, R_{4f}, R_{4g}, R₆, R_{7b}, R_{7c}, R₈, x, p, q, n8 and Pg are as described in any of the above embodiments;
when R_{4g} is alkynyl, R_{4g} can be optionally further substituted with C₁₋₆ silanyl in addition to the above definition.

In a preferred embodiment, the preparation method is a method for preparing a compound represented by general formula (IV), or a stereoisomer or pharmaceutically acceptable salt thereof, comprising the following step: 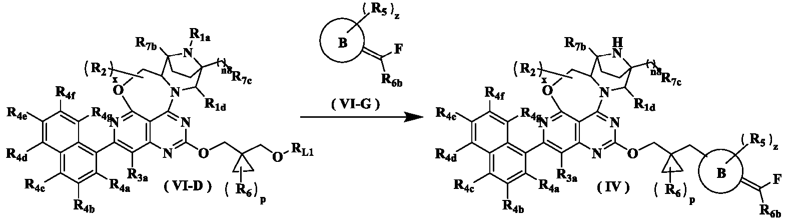
the compound represented by general formula (VI-D) is reacted with the compound represented by general formula (VI-G) in the presence of a halide salt and a base, and the protecting group is further removed to afford the compound represented by general formula (IV);
R_{L1} is selected from hydrogen or a hydroxyl protecting group;
ring B, X₁, X₂, X₃, R₁ₐ, R_{1d}, R₂, R₃ₐ, R₄ₐ, R_{4b}, R_{4c}, R_{4d}, R₄ₑ, R_{4f}, R_{4g}, R₅, R₆, R_{6b}, R_{7b}, R_{7c}, x, p, z, n8 and Pg are as described in any of the above embodiments;
when R_{4g} is alkynyl, R_{4g} can be optionally further substituted with C₁₋₆ silanyl in addition to the above definition.

In a preferred embodiment, the hydroxyl protecting group is selected from methyl, tert-butyl, triphenyl, methylthiomethyl ether, 2-methoxyethoxymethyl ether, methoxymethyl ether, p-methoxybenzyl ether, pivaloyl, benzyl ether group, methoxymethyl, trimethylsilyl, tert-butyldimethylsilyl, acetyl, benzoyl or p-toluenesulfonyl; preferably trimethylsilyl or tert-butyldimethylsilyl;

In a preferred embodiment, the halide salt is selected from potassium iodide, sodium iodide, potassium bromide, sodium bromide, potassium chloride or sodium chloride; preferably potassium iodide or sodium iodide.

In a preferred embodiment, the base is selected from sodium hydroxide, potassium hydroxide, sodium hydride, sodium n-propoxide, sodium tert-butoxide, potassium tert-butoxide, trimethylamine, triethylamine or N,N-diisopropylethylamine; preferably N,N-diisopropylethylamine.

The present invention further provides a compound represented by general formula (VII-F), or a stereoisomer or pharmaceutically acceptable salt thereof: 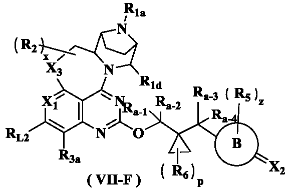
R_{L2} is selected from halogen; preferably chlorine or bromine;
each of Rₐ₋₁, Rₐ₋₂, Rₐ₋₃ and Rₐ₋₄ is independently selected from hydrogen, deuterium, halogen, amino, hydroxyl, cyano, C₁₋₃ alkyl, C₁₋₃ deuterated alkyl, C₁₋₃ haloalkyl or C₁₋₃ hydroxyalkyl; preferably hydrogen or deuterium;
ring B, X₁, X₂, X₃, R₁ₐ, R_{1d}, R₂, R₃ₐ, R₅, R₆, x, p and z are as described in any of the above embodiments.

The present invention further provides a method for preparing a compound represented by general formula (I), or a stereoisomer or pharmaceutically acceptable salt thereof, comprising the following step 1: 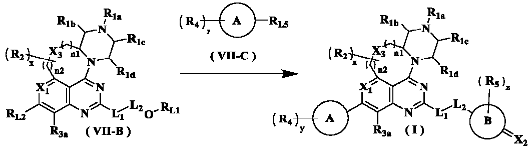
a compound represented by general formula (VII-B) and a compound represented by general formula (VII-C) are subjected to a coupling reaction to afford the compound represented by general formula (I);
optionally, further comprising step 2: 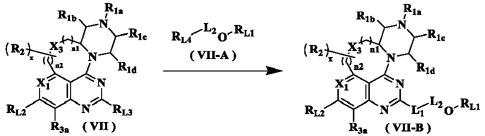
a compound represented by general formula (VII) is reacted with a compound represented by general formula (VII-A) under an alkaline condition to afford the compound represented by general formula (VII-B);
R_{L4} is selected from -OH, or -SH; preferably -OH;
R_{L2} is selected from halogen; preferably chlorine or bromine;
R_{L3} is selected from halogen, hydroxyl or -S(O)ₘ₁-C₁₋₃ alkyl; preferably chlorine, bromine, -S(O)-CH₃ or -S(O)₂-CH₃;
R_{L5} is selected from boronic acid group, borate salt group, chain borate ester group or cyclic borate ester group;
m1 is selected from 0, 1 or 2;
ring A, ring B, X₁, X₂, X₃, L₁, L₂, R₁ₐ, R_{1b}, R_{1c}, R_{1d}, R₂, R₃ₐ, R₄, R₅, x, y, z, n1 and n2 are as described in any of the above embodiments.

In a preferred embodiment, the preparation method is a method for preparing a compound represented by general formula (II-D), or a stereoisomer or pharmaceutically acceptable salt thereof, comprising the following step 1: 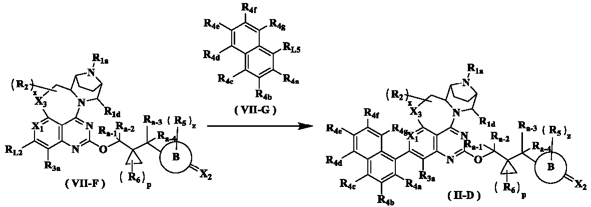
the compound represented by general formula (VII-F) and a compound represented by general formula (VII-G) are subjected to a coupling reaction to afford the compound represented by general formula (II-D);
optionally, further comprising step 2: 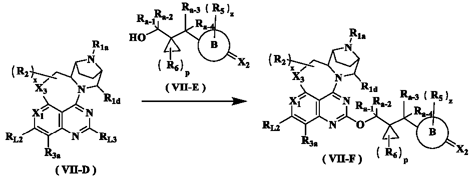
the compound represented by general formula (VII-D) is reacted with a compound represented by general formula (VII-E) under an alkaline condition to afford the compound represented by general formula (VII-F);
each of Rₐ₋₁, Rₐ₋₂, Rₐ₋₃ and Rₐ₋₄ is independently selected from hydrogen, deuterium, halogen, amino, hydroxyl, cyano, C₁₋₃ alkyl, C₁₋₃ deuterated alkyl, C₁₋₃ haloalkyl or C₁₋₃ hydroxyalkyl; preferably hydrogen or deuterium;
R_{L2} is selected from halogen; preferably chlorine or bromine;
R_{L3} is selected from halogen, hydroxyl or -S(O)ₘ₁-C₁₋₃ alkyl; preferably chlorine, bromine, -S(O)-CH₃ or -S(O)₂-CH₃;
R_{L5} is selected from boronic acid group, borate salt group, chain borate ester group or cyclic borate ester group;
m1 is selected from 0, 1 or 2;
ring B, X₁, X₂, X₃, R₁ₐ, R_{1d}, R₂, R₃ₐ, R₄ₐ, R_{4b}, R_{4c}, R_{4d}, R₄ₑ, R_{4f}, R_{4g}, R₅, R₆, x, p or z are as described in any of the above embodiments;
when R_{4g} is alkynyl, R_{4g} can be optionally further substituted with C₁₋₆ silanyl in addition to the above definition.

In a preferred embodiment, the preparation method is a method for preparing a compound represented by general formula (II- E), or a stereoisomer or pharmaceutically acceptable salt thereof, comprising the following step 1: 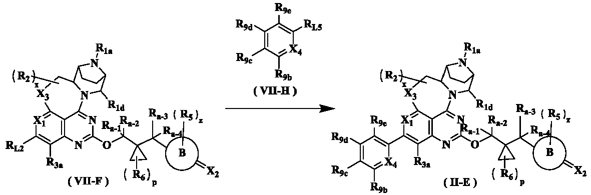
the compound represented by general formula (VII-F) and a compound represented by general formula (VII-H) are subjected to a coupling reaction to afford the compound represented by general formula (II-E);
optionally, further comprising step 2: 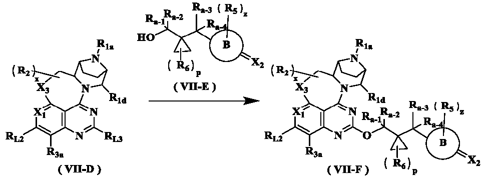
the compound represented by general formula (VII-D) is reacted with the compound represented by general formula (VII-E) under an alkaline condition to afford the compound represented by general formula (VII-F);
each of Rₐ₋₁, Rₐ₋₂, Rₐ₋₃ and Rₐ₋₄ is independently selected from hydrogen, deuterium, halogen, amino, hydroxyl, cyano, C₁₋₃ alkyl, C₁₋₃ deuterated alkyl, C₁₋₃ haloalkyl or C₁₋₃ hydroxyalkyl; preferably hydrogen or deuterium;
R_{L2} is selected from halogen; preferably chlorine or bromine;
R_{L3} is selected from halogen, hydroxyl or -S(O)ₘ₁-C₁₋₃ alkyl; preferably chlorine, bromine, -S(O)-CH₃ or -S(O)₂-CH₃;
R_{L5} is selected from boronic acid group, borate salt group, chain borate ester group or cyclic borate ester group;
m1 is selected from 0, 1 or 2;
ring B, X₁, X₂, X₃, X₄, R₁ₐ, R_{1d}, R₂, R₃ₐ, R₉ₐ, R_{9b}, R_{9c}, R_{9d}, R₉ₑ, R₅, R₆, x, p or z are as described in any of the above embodiments.

In a preferred embodiment, the preparation method is a method for preparing a compound represented by general formula (III-A), or a stereoisomer or pharmaceutically acceptable salt thereof, comprising the following step: 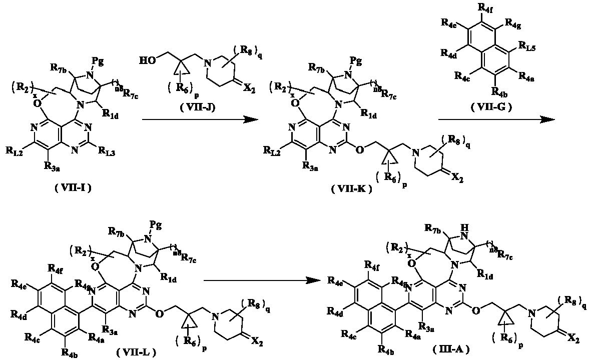
a compound represented by general formula (VII-I) is reacted with a compound represented by general formula (VII-J) under an alkaline condition to afford the compound represented by general formula (VII-K); the compound represented by general formula (VII-K) and the compound represented by general formula (VII-G) are subjected to a coupling reaction to obtain the compound represented by general formula (VII-L), and the protecting group is further removed to obtain the compound represented by general formula (III-A);
R_{L2} is selected from halogen; preferably chlorine or bromine;
R_{L3} is selected from halogen, hydroxyl or -S(O)ₘ₁-C₁₋₃ alkyl; preferably chlorine, bromine, -S(O)-CH₃ or -S(O)₂-CH₃;
R_{L5} is selected from boronic acid group, borate salt group, chain borate ester group or cyclic borate ester group;
m1 is selected from 0, 1 or 2;
ring B, X₁, X₂, R₁ₐ, R_{1d}, R₂, R₃ₐ, R₄ₐ, R_{4b}, R_{4c}, R_{4d}, R₄ₑ, R_{4f}, R_{4g}, R₆, R_{7b}, R_{7c}, R₈, x, p, q, n8 and Pg are as described in any of the above embodiments;
when R_{4g} is alkynyl, R_{4g} can be optionally further substituted with C₁₋₆ silanyl in addition to the above definition; .

In a preferred embodiment, the preparation method is a method for preparing a compound represented by general formula (IV), or a stereoisomer or pharmaceutically acceptable salt thereof, comprising the following step: 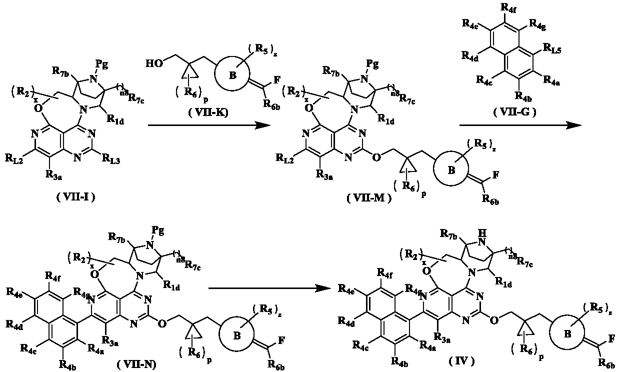
a compound represented by general formula (VII-I) is reacted with a compound represented by general formula (VII-K) under an alkaline condition to afford the compound represented by general formula (VII-M); the compound represented by general formula (VII-M) and the compound represented by general formula (VII-G) are subjected to a coupling reaction to obtain the compound represented by general formula (VII-N), and the protecting group is further removed to obtain the compound represented by general formula (IV);
R_{L2} is selected from halogen; preferably chlorine or bromine;
R_{L3} is selected from halogen, hydroxyl or -S(O)ₘ₁-C₁₋₃ alkyl; preferably chlorine, bromine, -S(O)-CH₃ or -S(O)₂-CH₃;
R_{L5} is selected from boronic acid group, borate salt group, chain borate ester group or cyclic borate ester group;
m1 is selected from 0, 1 or 2;
ring B, X₁, X₂, R₁ₐ, R_{1d}, R₂, R₃ₐ, R₄ₐ, R_{4b}, R_{4c}, R_{4d}, R₄ₑ, R_{4f}, R_{4g}, R₆, R_{6b}, R_{7b}, R_{7c}, x, p, n8 and Pg are as described in any of the above embodiments;
when R_{4g} is alkynyl, R_{4g} can be optionally further substituted with C₁₋₆ silanyl in addition to the above definition.

In a preferred embodiment, R_{L4} is selected from substituted or unsubstituted 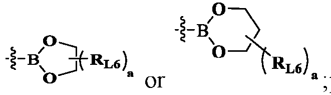 preferably 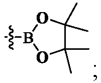
R_{L6} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃alkoxy, C₁₋₃ deuterated alkoxy, C₁₋₃ haloalkoxy, C₂₋₄ alkenyl, C₂₋₄ haloalkenyl, C₂₋₄ alkynyl or C₃₋₈ cycloalkyl;
a is selected from 0, 1, 2, 3, 4, 5 or 6.

In a preferred embodiment, the coupling reaction is performed in the presence of a palladium catalyst.

In a preferred embodiment, the palladium catalyst is selected from palladium carbon, palladium acetate, diphenylphosphino ferrocene palladium dichloride, tetrakis(triphenylphosphine)palladium, bis(triphenylphosphine)palladium dichloride, [1,1'-bis(diphenylphosphino)ferrocene]palladium dichloride, [(di(1-adamantyl)butylphosphino)-2-(2'-amino-1,1'-biphenyl)]palladium(II) methanesulfonate, methanesulfonate (dimethyl-n-butylphosphino)-2'-amino-1,1'-biphenyl-2-yl)palladium(II) dichloromethane adduct or methanesulfonic acid [n-butyldi(1-adamantyl)phosphine](2-amino-1,1'-biphenyl-2-yl)palladium(II).

In a preferred embodiment, the base is selected from sodium hydroxide, potassium hydroxide, lithium hydroxide, sodium hydride, sodium n-propoxide, sodium tert-butoxide, potassium tert-butoxide or lithium bis(trimethylsilyl)amide; preferably sodium hydride or lithium bis(trimethylsilyl)amide.

The present invention further provides a preferred embodiment, which relates to a pharmaceutical composition comprising a therapeutically effective dose of any of the compounds described in the above herein, or the stereoisomer or pharmaceutically acceptable salt or prodrug thereof and one or more pharmaceutically acceptable carriers, diluents or excipients.

The present invention further relates to the use of the compound described in the above herein, or the stereoisomer or pharmaceutically acceptable salt or prodrug thereof, or the pharmaceutical composition in the preparation of a KRAS inhibitor drug; preferably a drug for KRAS G12D, KRAS G12V, or KRAS G13D mutations.

The present invention further provides a preferred embodiment, which further relates to use of the compound, or the stereoisomer or pharmaceutically acceptable salt or prodrug thereof, or the pharmaceutical composition in a method for preventing and/or treating a condition mediated by a KRAS inhibitor. The method comprises administering to the patient a therapeutically effective dose of the above compound, or the stereoisomer or pharmaceutically acceptable salt or prodrug thereof, or the pharmaceutical composition thereof.

The present invention further provides a preferred embodiment, which relates to the use of the compound described herein, or the stereoisomer or pharmaceutically acceptable salt or prodrug thereof, or the pharmaceutical compositions described herein in the preparation of a drug for treating diseases or conditions such as Noonan syndrome, Leopard syndrome, leukemia, neuroblastoma, melanoma, esophageal cancer, head and neck tumors, breast cancer, lung cancer and colon cancer; preferably non-small cell lung cancer, colon cancer, esophageal cancer and head and neck tumors.

The present invention further provides a preferred embodiment, which relates to the use of the compound and composition of the present invention in the treatment of diseases or conditions such as Noonan syndrome, Leopard syndrome, leukemia, neuroblastoma, melanoma, breast cancer, esophageal cancer, head and neck tumors, lung cancer and colon cancer.

In some embodiments, the present invention provides a method for treating a cancer condition, comprising administering a therapeutically effective amount of the compound of the present invention, or the pharmaceutically acceptable salt, ester, prodrug, solvate, hydrate or derivative thereof, to a patient with a cancer condition.

In certain embodiments of the present invention, the weight percent of the compound, or the stereoisomer or pharmaceutically acceptable salt thereof in the pharmaceutical composition is 0.1% to 95%, preferably 90%, 85%, 80%, 75%, 70%, 60%, 50%, based on free base.

In certain embodiments of the present invention, the pharmaceutical composition is selected from a tablet, a capsule, a liquid preparation or an injection, preferably further comprises a filler, optionally further comprises a disintegrant, or further comprises one or more of a glidant or lubricant.

In certain embodiments of the present invention, the pharmaceutical composition is an immediate release preparation or a sustained release preparation.

In certain embodiments of the present invention, the unit dose of the compound, or the stereoisomer or pharmaceutically acceptable salt thereof in the pharmaceutical composition is 1-1000 mg, preferably 1-500 mg, or preferably 1 mg, 2 mg, 3 mg, 5 mg, 10 mg, 20 mg, 40 mg, 50 mg, 60 mg, 80 mg, 100 mg, 200 mg, 300 mg, 400 mg or 500 mg, based on the free base.

In certain embodiments of the present invention, the compound, or the stereoisomer or pharmaceutically acceptable salt thereof can be administered by any convenient method, for example, by oral, parenteral, buccal, sublingual, nasal, rectal, intrathecal or transdermal administration, and the pharmaceutical composition can be adjusted accordingly.

In certain embodiments of the present invention, the compound, or the stereoisomer or pharmaceutically acceptable salt thereof can be formulated into a liquid or solid preparation, such as a syrup, suspension, emulsion, tablet, capsule, powder, granule, or lozenge.

In some embodiments, the cancer treated by the compound or composition of the present invention is Noonan syndrome, Leopard syndrome, leukemia, neuroblastoma, melanoma, breast cancer, esophageal cancer, head and neck tumors, gastric cancer, lung cancer and colon cancer; preferably non-small cell lung cancer, colon cancer, esophageal cancer and head and neck tumors.

### Detailed Description of the Invention

Unless stated to the contrary, all technical and scientific terms used herein generally have the same meanings as commonly understood by one of ordinary skill in the art. Specifically, the terms used in the description and claims have the following meanings.

The term "alkyl" refers to a linear or branched saturated aliphatic hydrocarbon group which may be optionally substituted with one or more substituents. In particular embodiments, the alkyl refers to a linear saturated hydrocarbon group having 1 to 20 (C₁₋₂₀), 1 to 15 (C₁₋₁₅), 1 to 12 (C₁₋₁₂), 1 to 10 (C₁₋₁₀), 1 to 8 (C₁₋₈), 1 to 6 (C₁₋₆), or 1 to 3 (C₁₋₃) carbon atoms, or a branched saturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈) or 3 to 6 (C₃₋₆) carbon atoms. As used herein, linear C₁₋₆ alkyl and branched C₃₋₆ alkyl groups are also referred to as "lower alkyl". For example, C₁₋₆ alkyl refers to a linear saturated monovalent hydrocarbon group having 1 to 6 carbon atoms or a branched saturated monovalent hydrocarbon group having 3 to 6 carbon atoms. In one embodiment, the C₁₋₆ alkyl contains 1 to 6 (e.g., 1, 2, 3, 4, 5, 6) carbon atoms. Non-limiting examples include methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl and various branched chain isomers thereof. In one embodiment, the alkyl is an optionally substituted alkyl as described elsewhere herein.

The term "alkylene" refers to an alkyl in which one hydrogen atom is further substituted, wherein "alkyl" is as defined above. Non-limiting examples of "alkylene" include: methylene (-CH₂-), ethylene (-(CH₂)₂-), propylene (-(CH₂)₃-) or butylene (-(CH₂)₄-). In one embodiment, the alkylene is an optionally substituted alkylene as described elsewhere herein.

The term "alkenyl" refers to a linear or branched unsaturated aliphatic hydrocarbon group containing at least one carbon-carbon double bond and the carbon-carbon double bond may be located at any position within the alkenyl, and the alkenyl may be optionally substituted with one or more substituents. In particular embodiments, the alkenyl is a linear unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆), or 2 to 4 (C₂₋₄) carbon atoms, or a branched unsaturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈) or 3 to 6 (C₃₋₆) carbon atoms. Unless otherwise specified, the term "alkenyl" as used herein includes both linear and branched alkenyl. For example, C₂₋₆ alkenyl refers to a linear unsaturated hydrocarbon group having 2 to 6 carbon atoms or a branched unsaturated hydrocarbon group having 3 to 6 carbon atoms. In one embodiment, the C₂₋₆ alkenyl contains 2 to 6 (for example, 2, 3, 4, 5, 6) carbon atoms. Non-limiting examples of alkenyl include: 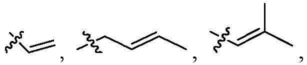 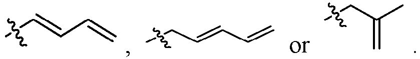 One of ordinary skill in the art can understand that the term "alkenyl" may also include groups having "cis" and "trans" configurations, or alternatively, groups having "E" and "Z" configurations. In one embodiment, the alkenyl is an optionally substituted alkenyl described elsewhere herein.

The term "alkynyl" refers to a linear or branched unsaturated aliphatic hydrocarbon group containing at least one carbon-carbon triple bond and the carbon-carbon triple bond may be located at any position within the alkynyl, and the alkynyl may be optionally substituted with one or more substituents. In particular embodiments, the alkynyl is a linear unsaturated hydrocarbon group having 2 to 20 (C₂₋₂₀), 2 to 15 (C₂₋₁₅), 2 to 12 (C₂₋₁₂), 2 to 10 (C₂₋₁₀), 2 to 8 (C₂₋₈), 2 to 6 (C₂₋₆), or 2 to 4 (C₂₋₄) carbon atoms, or a branched unsaturated hydrocarbon group having 3 to 20 (C₃₋₂₀), 3 to 15 (C₃₋₁₅), 3 to 12 (C₃₋₁₂), 3 to 10 (C₃₋₁₀), 3 to 8 (C₃₋₈) or 3 to 6 (C₃₋₆) carbon atoms. Unless otherwise specified, the term "alkynyl" as used herein includes both linear and branched alkynyl. For example, C₂₋₆ alkynyl refers to a linear unsaturated hydrocarbon group having 2 to 6 carbon atoms or a branched unsaturated hydrocarbon group having 3 to 6 carbon atoms. In one embodiment, the C₂₋₆ alkynyl contains 2 to 6 (for example, 2, 3, 4, 5, 6) carbon atoms. Non-limiting examples of alkynyl include: 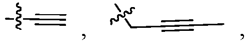 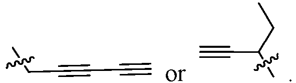 In one embodiment, the alkynyl is an optionally substituted alkynyl described elsewhere herein.

The term "cycloalkyl" refers to a saturated or partially unsaturated aliphatic hydrocarbon monocyclic, polycyclic (two or more rings) cyclic group, which may be optionally substituted with one or more substituents. In particular embodiments, the cycloalkyl ring contains 3 to 20 (C₃₋₂₀), 3 to 12 (C₃₋₁₂), 3 to 8 (C₃₋₈), or 3 to 6 (C₃₋₆) carbon atoms. In one embodiment, the cycloalkyl ring contains 6 to 14 (C₆₋₁₄) or 7 to 10 (C₇₋₁₀) carbon atoms; it may contain one or more double bonds, but does not have a completely conjugated π electron system. Non-limiting examples of monocyclic cycloalkyl include cyclopropyl, cyclobutyl, cyclopentyl, cyclopentenyl, cyclohexyl, cyclohexenyl, cyclohexadienyl, cycloheptyl, cycloheptatrienyl, cyclooctyl, etc.; and the polycyclic cycloalkyl includes spirocycloalkyl, fused cycloalkyl and bridged cycloalkyl. In one embodiment, the cycloalkyl is an optionally substituted cycloalkyl described elsewhere herein or a cycloalkyl optionally fused to a heterocyclyl, aryl or heteroaryl, and non-limiting examples include indanyl, tetrahydronaphthyl, benzocycloheptyl, etc.

The term "heterocyclyl" refers to a saturated or partially unsaturated monocyclic or polycyclic cyclic hydrocarbon group, wherein one or more ring atoms are heteroatoms selected from nitrogen, oxygen, boron, phosphorus or sulfur, wherein the nitrogen, phosphorus or sulfur atom may be optionally oxidized, the nitrogen atom may be optionally quaternized, the ring carbon atoms may be optionally substituted with oxygen, but excluding ring moieties of -O-O-, -O-S-, and the remaining ring atoms are carbons; the heterocyclyl may contain one or more double bonds but do not have a fully conjugated π electron system. In particular embodiments, the heterocyclyl contains 3 to 20, 3 to 12, 3 to 8 or 3 to 6 ring atoms, of which 1 to 4 are heteroatoms. In one embodiment, the heterocyclyl contains 3 to 6, 4 to 6, 3 to 8, 3 to 10, 6 to 10 or 7 to 11 ring atoms. In one embodiment, the heterocyclyl contains 3 to 8 (e.g., 3, 4, 5, 6, 7, or 8) ring atoms. Non-limiting examples of monocyclic heterocyclyl include tetrahydropyrrolyl, azetidinyl, oxetanyl, oxacyclohexyl, tetrahydrofuranyl, tetrahydropyranyl, tetrahydrothiophenyl, dihydroimidazolyl, dihydrofuranyl, dihydropyrazolyl, dihydropyrrolyl, piperidinyl, piperazinyl, morpholinyl, thiomorpholinyl, homopiperazinyl, pyranyl, etc. Polycyclic heterocyclyl includes spiro heterocyclyl, fused heterocyclyl, and bridged heterocyclyl. In one embodiment, the heterocyclyl is an optionally substituted heterocyclyl described elsewhere herein, or a heterocyclyl that is further fused to other cycloalkyl, heterocyclyl, aryl and heteroaryl through any two or more atoms on the ring.

The term "fused heterocyclyl" refers to a polycyclic heterocyclic group with each ring in the system sharing a pair of adjacent atoms with other rings in the system, and one or more rings may contain one or more double bonds, but no ring has a fully conjugated π electron system, wherein one or more ring atoms are heteroatoms selected from nitrogen, oxygen, boron, phosphorus or sulfur, and the remaining ring atoms are carbons. In particular embodiments, the fused heterocyclyl comprises 5 to 20 or 6 to 14 ring atoms, and in one embodiment, it comprises 7 to 10 (e.g., 7, 8, 9, 10) ring atoms; according to the number of constituent rings, it can be divided into a bicyclic, tricyclic, tetracyclic or polycyclic fused heterocyclyl; preferably bicyclic or tricyclic fused heterocyclyl. In one embodiment, the fused heterocyclyl is 5-membered/5-membered or 5-membered/6-membered bicyclic fused heterocyclyl. In one embodiment, the fused heterocyclyl is an optionally substituted fused heterocyclyl described elsewhere herein, or a fused heterocyclyl that can be fused to cycloalkyl, heterocyclyl, aryl or heteroaryl; Non-limiting examples of fused heterocyclyl include:

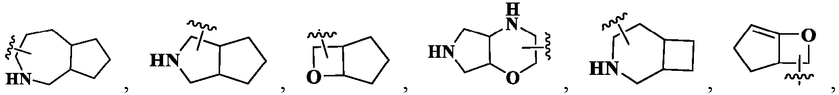

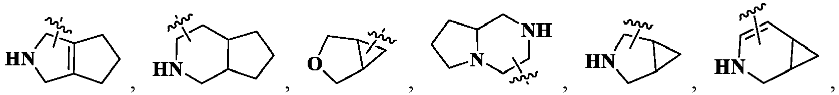

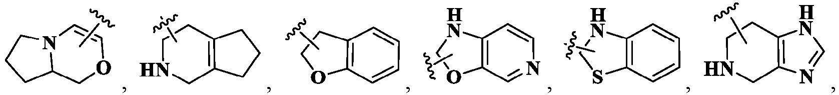

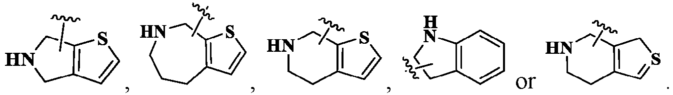

The term "bridged heterocyclyl" refers to a polycyclic heterocyclic group with any two rings sharing two atoms that are not directly connected. It may contain one or more double bonds, but no ring has a fully conjugated π electron system, wherein one or more ring atoms are heteroatoms selected from nitrogen, oxygen, boron, phosphorus or sulfur, and the remaining ring atoms are carbons. In particular embodiments, the bridged heterocyclyl contains 5 to 20 or 6 to 14 ring atoms. In one embodiment, it contains 7 to 10 (e.g., 7, 8, 9, or 10) ring atoms; according to the number of constituent rings, it can be divided into a bicyclic, tricyclic, tetracyclic or polycyclic bridged heterocyclyl; preferably bicyclic, tricyclic or tetracyclic bridged heterocyclyl. In one embodiment, the bridged heterocyclyl is bicyclic or tricyclic. In one embodiment, the bridged heterocyclyl is an optionally substituted bridged heterocyclyl described elsewhere herein. Non-limiting examples of bridged heterocyclyl include: 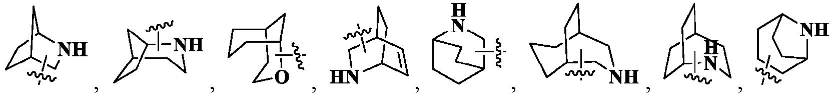 or

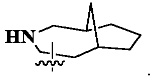

The term "aryl" refers to an all-carbon monocyclic or fused polycyclic (i.e., rings sharing a pair of adjacent carbon atoms) group containing at least one conjugated π electron system, which may be optionally substituted with one or more substituents. In particular embodiments, the aryl contains 6 to 20, 6 to 14, or 6 to 10 ring atoms; In one embodiment, aryl may further refer to a bicyclic, tricyclic or tetracyclic ring system, in which at least one ring is an aromatic ring and the other rings may be saturated or partially unsaturated carbocyclic rings or rings containing one or more heteroatoms independently selected from O, S and N. In one embodiment, the aryl is selected from benzo 5-10 membered heteroaryl, benzo 3- to 10-membered cycloalkyl or benzo 3- to 10-membered heterocyclyl. In one embodiment, the aryl is selected from benzo 5- or 6-membered heteroaryl, benzo 3- to 6-membered cycloalkyl or benzo 3- to 6-membered heterocyclyl, wherein the heterocyclyl is heterocyclyl containing 1 to 3 nitrogen atoms, oxygen atoms or sulfur atoms. Non-limiting examples of aryl include phenyl, naphthyl, fluorenyl, azulenyl, anthryl, phenanthrenyl, pyrenyl, biphenyl, terphenyl, dihydronaphthyl, indenyl, tetrahydronaphthyl (tetralinyl),

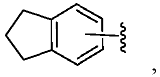

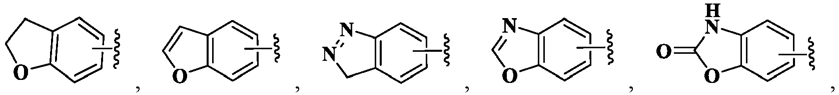

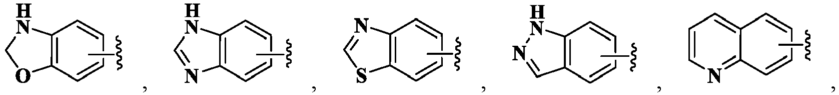

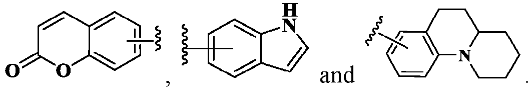

The term "arylene" refers to bivalent aryl formed by further substitution of one hydrogen atom on aryl, wherein the arylene is optionally substituted or unsubstituted, and the aryl is defined as above.

The term "heteroaryl" refers to an optionally substituted monocyclic, polycyclic group or ring system containing at least one aromatic ring having one or more heteroatoms independently selected from O, S and N. In particular embodiments, the heteroaryl contains 5 to 20, 5 to 15, or 5 to 10 ring atoms, of which 1 to 4 are heteroatoms. In one embodiment, the heteroaryl contains 5 or 6 ring atoms. In particular embodiments, heteroaryl may further refer to a bicyclic, tricyclic or tetracyclic ring, in which at least one ring is an aromatic ring having one or more heteroatoms independently selected from O, S and N, and the other rings may be saturated or partially unsaturated carbocyclic rings or rings containing one or more heteroatoms independently selected from O, S and N. In one embodiment, the heteroaryl is selected from heteroaryl fused 6- to 10-membered aryl, heteroaryl fused 3- to 10-membered cycloalkyl or heteroaryl fused 3- to 10-membered heterocyclyl. Further in one embodiment, the heteroaryl is selected from 5- or 6-membered heteroaryl fused 6- to 10-membered aryl, 5- or 6-membered heteroaryl fused 3- to 6-membered cycloalkyl, 5- or 6-membered heteroaryl fused 3- to 6-membered heterocyclyl, wherein the heterocyclyl is heterocyclyl containing 1 to 3 nitrogen atoms, oxygen atoms or sulfur atoms. Non-limiting examples include: furanyl, imidazolyl, isothiazolyl, isoxazolyl, oxadiazolyl, oxazolyl, pyrazinyl, pyrazolyl, pyridazinyl, pyridyl, pyrimidinyl, pyrrolyl, thiadiazolyl, thiazolyl, thienyl, tetrazolyl, triazinyl, triazolyl, benzofuranyl, benzimidazolyl, benzisoxazolyl, benzopyranyl, benzothiadiazolyl, benzothienyl, benzothiophenyl, benzothienyl, benzotriazolyl, imidazopyridyl, imidazothiazolyl, indolizinyl, indolyl, indazolyl, isobenzofuranyl, isobenzothienyl, isoindolyl, isoquinolinyl, naphthyridinyl, oxazolopyridyl, phthalazinyl, pteridinyl, purinyl, pyridopyridyl, pyrrolopyridyl, quinolyl, quinoxalinyl, quinazolinyl, thiadiazolopyrimidinyl, thienopyridyl, acridinyl, benzindolyl, carbazolyl, bibenzofuranyl, phenanthrolinyl, phenanthridinyl, phenarsazinyl, phenazinyl, phenothiazinyl, phenoxazinyl, xanthenyl,

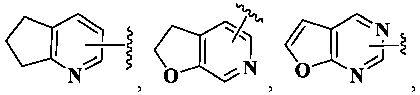

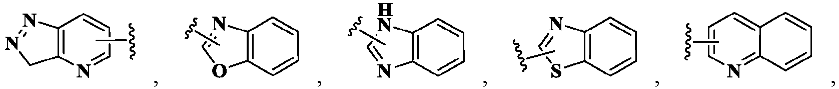

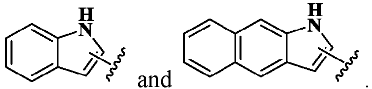

The term "heteroarylene" refers to bivalent heteroaryl formed by further replacing one hydrogen atom of cycloalkyl, wherein the heteroarylene is optionally substituted or unsubstituted, and the heteroaryl is defined as above.

The term "heteroalkyl" refers to a stable linear or branched, or cyclic hydrocarbon group, or a combination thereof, and is composed of the indicated number of carbon atoms and one or more (in one embodiment, one to three) heteroatoms selected from O, N, Si and S, and wherein the nitrogen and sulfur atoms are optionally oxidized and the nitrogen heteroatom may be optionally quaternized. In one embodiment, the heteroatoms O, N and S may be placed at any interior position of the heteroalkyl group. In one embodiment, the heteroatom Si may be placed at any position (e.g., interior or terminal position) of the heteroalkyl group, including the position where the alkyl group is connected to the rest of the molecule. Non-limiting examples include: -CH₂-CH₂-O-CH₃, -CH₂-CH₂-NH-CH₃, -CH₂-CH₂-N(CH₃)-CH₃, -CH₂-S-CH₂-CH₃, -CH₂-CH₂-S(O)-CH₃, -CH₂-CH₂-S(O)₂-CH₃, -CH = CH-O-CH₃, -Si(CH₃)₃, -CH₂-CH = N-OCH₃ and -CH = CH-N(CH₃)-CH₃. Up to two heteroatoms can be consecutive, for example, -CH₂-NH-O-CH₃ and -CH₂-O-Si(CH₃)₃. In particular embodiments, the heteroalkyl is optionally substituted heteroalkyl described elsewhere herein.

The term "alkoxy" refers to -O-(alkyl) and -O-(unsubstituted cycloalkyl), wherein the alkyl or cycloalkyl is as defined above. Non-limiting examples of alkoxy include: methoxy, ethoxy, propoxy, butoxy, cyclopropoxy, cyclobutoxy, cyclopentyloxy or cyclohexyloxy. In one embodiment, the alkoxy is optionally substituted alkoxy as described elsewhere herein.

The term "alkylacyl" refers to -C(O)-alkyl, wherein the alkyl is as defined above.

The term "haloalkyl" refers to alkyl substituted with one or more halogens, wherein the alkyl is as defined above. Non-limiting examples of haloalkyl include: trifluoromethyl, -CH₂CF₃,

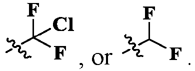

The term "haloalkoxy" refers to alkoxy substituted with one or more halogens, wherein the alkoxy is as defined above.

The term "hydroxyalkyl" refers to alkyl substituted with hydroxyl, wherein the alkyl is as defined above.

The term "alkylthio" refers to -S-(alkyl) and -S-(unsubstituted cycloalkyl), wherein the alkyl or cycloalkyl is as defined above. Non-limiting examples of alkylthio include: methylthio, ethylthio, propylthio, butylthio, cyclopropylthio, cyclobutylthio, cyclopentylthio or cyclohexylthio. In one embodiment, the alkylthio is optionally substituted alkylthio as described elsewhere herein.

The term "haloalkylthio" refers to alkylthio substituted with one or more halogens, wherein the alkylthio is as defined above.

The term "alkenylcarbonyl" refers to -C(O)-(alkenyl), wherein the alkenyl is as defined above. Non-limiting examples of alkenylcarbonyl include: ethenylcarbonyl, propenylcarbonyl, or butenylcarbonyl. In one embodiment, the alkenylcarbonyl is optionally substituted alkenylcarbonyl as described elsewhere herein.

The term "aminocarbonyl" refers to NH₂-C(O)-.

The term "alkylaminocarbonyl" refers to aminocarbonyl (NH₂-C(O)-) in which one or both of two hydrogens is substituted with alkyl, wherein the alkyl is as described above.

The term "alkylamino" refers to amino in which one or both of two hydrogens is substituted with alkyl, wherein the alkyl is as described above.

The term "carbonyl" refers to a -C(O)-, -(CO)- or -C(=O)- group. All the symbols are used interchangeably in the description.

The term "hydroxy" refers to -OH group.

The term "halogen" refers to fluorine, chlorine, bromine or iodine.

The term "acetyl" refers to -C(O)CH₃.

The term "oxo" or "side oxygen" refers to =O.

The term "hydrogen" includes proton (¹H), deuterium (²H), tritium (³H) and/or a mixture thereof. In certain embodiments, one or more hydrogen-occupied positions in the compound may be enriched with deuterium and/or tritium. Such isotopically enriched analogs may be prepared from suitable isotopically labeled starting materials obtained from commercial sources or by known literature procedures.

The alkyl, alkylene, alkenyl, alkynyl, cycloalkyl, heterocyclyl, aryl, arylene, heteroaryl, heteroarylene, heteroalkyl, alkoxy, alkylthio, hydroxyalkyl, alkenylcarbonyl, aminocarbonyl, alkylaminocarbonyl, alkylamino, alkylacyl may be substituted or unsubstituted. In one embodiment, the substituent is selected from one or more of the following groups: alkyl, alkenyl, alkynyl, alkoxy, alkylthio, alkylamino, alkylacyl, halogen, mercapto, hydroxyl, nitro, cyano, azidyl, oximido, phosphate group, oxo, thio, carboxyl, carboxylate group, cycloalkyl, heterocyclyl, aryl, heteroaryl, heterocycloalkoxy, cycloalkylthio or heterocycloalkylthio.

Different terms such as "X is selected from A, B, or C", "X is selected from A, B and C", "X is A, B or C", and "X is A, B and C" all express the same meaning, i.e., X can be any one or more of A, B, and C.

"Optional" or "optionally" means that the event or circumstance subsequently described may but need not to occur, and the description includes the occasion where the event or circumstance occurs or does not occur. For example, "heterocyclic group optionally substituted with alkyl" means the alkyl may but need not be present, and the description includes the case where the heterocyclic group is substituted with alkyl and the case where the heterocyclic group is not substituted with alkyl.

In various parts of the present invention, linking substituents are described. When it is clear that a linking group is required for the structure, the markush variables listed for that group should be understood as referring to the linking group. For example, if a linking group is required for the structure and the markush group definition for that variable lists "alkyl" or "aryl," it should be understood that the "alkyl" or "aryl" respectively represents the attached alkylene group or arylene group.

The term "substituted" means that any one or more hydrogen atoms on the designated atom are substituted with a substituent, provided that the valence state of the designated atom is normal, and the substituted compound is stable. When the substituent is oxo (i.e., =O), it means that two hydrogen atoms are substituted. The term "optionally substituted" means that a group may or may not be substituted. Unless otherwise specified, the type and number of substituents may be arbitrary on the basis of chemical practicability. It goes without saying, the substituents may be only in their possible chemical positions, a person skilled in the art can determine the possible or impossible substitutions (by experiment or theory) without paying too much effort. For example, the amino group having a free hydrogen or a hydroxyl group may be unstable when combined the carbon atoms having an unsaturated (e.g., olefinic) bond.

Unless stated to the contrary, the indefinite articles "a" and "an" and the definite article "the" in the present description and claims include plural as well as singular forms.

"Pharmaceutical composition" denotes a mixture containing one or more compounds described herein or physiologically/pharmaceutically acceptable salts or prodrug thereof and other chemical components, as well as other components, such as physiologically/pharmaceutically acceptable carriers and excipients. The purpose of pharmaceutical compositions is to facilitate administration to living organisms and facilitate the absorption of active ingredients to exert biological activity.

"Pharmaceutically acceptable salts" refer to salts of the compounds of the present invention, which are safe and effective when used in mammals, and have appropriate biological activity.

"Stereoisomer" comprises all enantiomerically/diastereomerically/ stereoisomerically pure and enantiomerically/diastereomerically/stereoisomerically enriched compounds of the present invention.

"Stereoisomerically pure" refers to a composition that comprises one stereoisomer of a compound and is substantially free of another stereoisomer of the compound. For example, a stereoisomerically pure composition of a compound having one chiral center will be substantially free of the opposite enantiomer of the compound. A stereoisomerically pure composition of a compound having two chiral centers will be substantially free of other diastereomers of the compound. A typical stereoisomerically pure compound comprises greater than about 80% by weight of one stereoisomer of the compound and less than about 20% by weight of another stereoisomer of the compound, greater than about 90% by weight of one stereoisomer of the compound and less than about 10% by weight of another stereoisomer of the compound, greater than about 95% by weight of one stereoisomer of the compound and less than about 5% by weight of another stereoisomer of the compound, greater than about 97% by weight of one stereoisomer of the compound and less than about 3% by weight of another stereoisomer of the compound, or greater than about 99% by weight of one stereoisomer of the compound and less than about 1% by weight of another stereoisomer of the compound.

"Stereoisomerically enriched" refers to a composition comprising greater than about 55% by weight, greater than about 60% by weight, greater than about 70% by weight, or greater than about 80% by weight of one stereoisomer of a compound.

"Enantiomerically pure" refers to a stereoisomerically pure composition of a compound having one chiral center. Similarly, the term "enantiomerically enriched" refers to a stereomerically enriched composition of a compound having one chiral center.

"Optical activity" and "enantiomeric activity" refer to a combination of molecules having an enantiomeric or diastereomeric excess of no less than about 50%, no less than about 70%, no less than about 80%, no less than about 90%, no less than about 91%, no less than about 92%, no less than about 93% , no less than about 94%, no less than about 95%, no less than about 96%, no less than about 97%, no less than about 98%, no less than about 99%, no less than about 99.5%, or no less than about 99.8%. In particular embodiments, the compound comprises about 95% or more of the desired enantiomer or diastereomer and about 5% or less of the less preferred enantiomer or diastereomer, based on the total weight of the racemate. When describing optically active compounds, the prefixes R and S are used to indicate the absolute configuration of the molecule relative to its chiral center. (+) and (-) are used to represent the optical rotation of the compound, that is, the direction of the plane of polarized light rotated by the optically active compound. The prefix (-) indicates that the compound is levorotatory, that is, the compound rotates the plane of polarized light to the left or counterclockwise. The prefix (+) indicates that the compound is dextrorotatory, that is, the compound rotates the plane of polarized light to the right or clockwise. However, the signs of optical rotation (+) and (-) are independent of the absolute configurations R and S of the molecule.

### Detailed Description of Embodiments

The present invention will be further described below in conjunction with examples, but these examples are not meant to limit the scope of the present invention.

### Example

The structures of the compounds of the present invention are determined by nuclear magnetic resonance (NMR) or/and mass spectrometry (MS). The NMR shift (δ) is given in the unit of 10⁻⁶ (ppm). NMR was determined using a Bruker AVANCE-400 nuclear magnetic instrument. The solvents for determination were deuterated dimethyl sulfoxide (DMSO-*d₆*), deuterated chloroform (CDCl₃), and deuterated methanol (CD₃OD), and the internal standard was tetramethylsilane (TMS).

MS was measured using a FINNIGAN LCQAd (ESI) mass spectrometer (manufacturer: Thermo, model: Finnigan LCQ advantage MAX).

HPLC determination used Agilent 1200DAD high-pressure liquid chromatograph instrument (Sunfire C₁₈ 150 × 4.6 mm chromatographic column) and Waters 2695-2996 high-pressure liquid chromatograph instrument (Gimini C₁₈ 150 × 4.6 mm chromatographic column).

The average kinase inhibition rate and IC₅₀ value were measured using NovoStar microplate reader (BMG Company, Germany).

Yantai Huanghai HSGF254 or Qingdao GF254 silica gel plate is used as a thin layer chromatography silica plate, and the silica gel plate for the thin layer chromatography (TLC) is of the specification of 0.15 mm-0.2 mm, and the specification when separating and purifying a product by thin layer chromatography is 0.4 mm-0.5 mm.

For the column chromatography, Yantai Huanghai silica gel of 200-300 mesh silica gel was generally used as a carrier.

The known starting materials of the present invention can be synthesized by methods known in the art, or can be purchased from ABCR GmbH & Co. KG, Acros Organics, Aldrich Chemical Company, Accela ChemBio Inc., Chembee Chemicals Inc. and other companies.

If there is no special instruction in the examples, reactions can be carried out under an argon atmosphere or a nitrogen atmosphere.

Argon atmosphere or nitrogen atmosphere means that the reaction bottle is connected to an argon or nitrogen balloon with a volume of about 1 L.

The hydrogen atmosphere means that the reaction bottle is connected to a hydrogen balloon with a volume of about 1 L.

The pressurized hydrogenation reaction uses Parr 3916EKX hydrogenator and Qinglan QL-500 hydrogen generator or HC2-SS hydrogenator.

The hydrogenation reaction is usually performed under the condition that vacuumizing and filling hydrogen is repeated three times.

The microwave reaction uses CEM Discover-S 908860 microwave reactor.

If there is no special instruction in the examples, the solution refers to an aqueous solution.

If there is no special instruction in the examples, the reaction temperature is room temperature, which is 20°C to 30°C.

The progress of the reaction in the examples is monitored by thin layer chromatography (TLC). The developing agent system used in the reaction is: A: dichloromethane and methanol system, B: n-hexane and ethyl acetate system, and C: petroleum ether and ethyl acetate system, D: acetone, the volume ratio of the solvents is adjusted according to the polarity of the compounds.

The eluent system of column chromatography and the developing agent system of thin layer chromatography used to purify compounds include: A: n-hexane and ethyl acetate system, B: n-hexane and tetrahydrofuran system, the volume ratio of the solvents is adjusted according to the polarity of the compounds, and a small amount of alkaline or acidic reagents such as triethylamine and acetic acid can also be added for adjustment.

### Intermediate 1

### Preparation of 4-(difluoromethylene)piperidine trifluoroacetate

### Step 1: tert-butyl 4-(difluoromethylene)piperidine-1-carboxylate

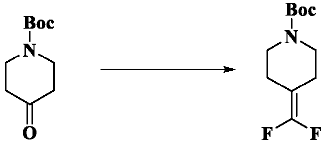

THF (300 mL) was added to a three-necked flask, the obtained mixture was subjected to nitrogen replacement, then cooled to 0°C, and dibromodifluoromethane (42.12 g, 200.76 mmol) was added, the obtained mixture was stirred for 10 min, then HMPT (32.76 g, 200.76 mmol) was added, stirring was continued for 1 h, tert-butyl 4-carbonylpiperidine-1-carboxylate (10 g, 50.19 mmol) was then added, and the obtained mixture was gradually warmed to room temperature and stirred for 1 h. Then zinc powder (12.85 g, 200.76 mmol) and HMPT (2 mL) were added, and the obtained mixture was heated to 70°C and refluxed for 3 h. The reaction liquid was cooled to room temperature. Water was added to quench the reaction, and the obtained mixture was extracted with ethyl acetate. The organic phases were combined, washed with saturated sodium chloride aqueous solution, dried over anhydrous sodium sulfate, concentrated, and purified by column chromatography to obtain tert-butyl 4-(difluoromethylene)piperidine-1-carboxylate (4.5 g, yield 38.4%).

### Step 2: Preparation of 4-(difluoromethylene)piperidine trifluoroacetate

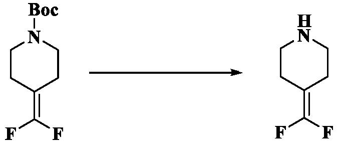

Tert-butyl 4-(difluoromethylene)piperidine-1-carboxylate (380 mg, 1.63 mol) was added to dichloromethane (20 mL), and TFA (3 mL) was added dropwise under ice bath. After the reaction liquid was evenly mixed, reacted at room temperature for 1 h, and concentrated under reduced pressure to obtain crude compound 4-(difluoromethylene)piperidine trifluoroacetate (400 mg, TF salt, yield 99%).

MS m/z (ESI):134 [M+H].

### Intermediate 2

### Step 1: tert-butyl 4-(methoxyimino)piperidine-1-carboxylate

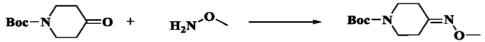

Tert-butyl 4-carbonylpiperidine-1-carboxylate (1.59 g, 8 mmol) and O-methylhydroxylamine hydrochloride (801.77 mg, 9.60 mmol) was dissolved in EtOH (20 mL). Under nitrogen protection by nitrogen replacement, K₂CO₃ (3.32 g, 24.00 mmol) was added, and the obtained mixture was heated to reflux for 3 h. The reaction liquid was concentrated, extracted with ethyl acetate. The organic phases were combined, dried over anhydrous sodium sulfate, filtered, and the organic phase was concentrated to obtain tert-butyl 4-(methoxyimino)piperidine-1-carboxylate (1.2 g, 65.71%).

MS m/z (ESI): 229[M+H].

### Step 2: N-methoxypiperidine-4-imine trifluoroacetate

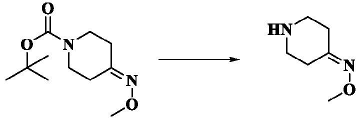

Trifluoroacetic acid (10 mL) was added to a solution of tert-butyl 4-methoxyiminopiperidine-1-carboxylate (3 g, 13.1 mmol) in dichloromethane (30 mL) at room temperature, and the obtained mixture was stirred at room temperature for 2 h after the addition was completed. The reaction liquid was concentrated to obtain N-methoxypiperidine-4-imine trifluoroacetate (3.18 g, yield 100%).

MS m/z (ESI): 129 [M+H].

### Intermediate 3

### Step 1: Preparation of tert-butyl 3-(fluoromethylene)pyrrolidine-1-carboxylate

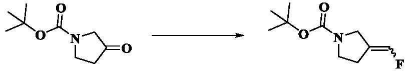

The substrate (fluoromethyl)triphenylphosphonium tetrafluoroborate (14.91 g, 39.02 mmol) was dissolved in THF (100 mL) under nitrogen protection by nitrogen replacement. The obtained mixture was cooled to -25°C, NaHMDS (7.16 g, 39.02 mmol) was added dropwise. After the dropwise addition was completed, the obtained mixture was stirred for 10 min. Then, a solution of tert-butyl 3-carbonylpyrrolidine-1-carboxylate (5.56 g, 30.02 mmol) in THF (60 mL) was added dropwise. After the dropwise addition was completed, the obtained mixture was stirred for 10 min, then warmed to room temperature and stirred for 3 h. The reaction liquid was slowly added to saturated aqueous NH₄Cl solution to quench the reaction, and the obtained mixture was extracted with ethyl acetate, and the organic phase was washed with saturated aqueous sodium chloride solution, dried over anhydrous sodium sulfate, concentrated, and then purified by silica gel column chromatography to obtain the target compound 3-(fluoromethylene)pyrrolidine-1-carboxylate (3.1 g, yield 51.3%).

MS m/z (ESI): 202 [M+H].

### Step 2: Preparation of 3-(fluoromethylene)pyrrolidine trifluoroacetate

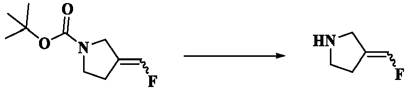

The substrate 3-(fluoromethylene)pyrrolidine-1-carboxylate (150 mg, 745.39 µmol) was dissolved in anhydrous dichloromethane (2 mL), TFA (0.5 mL) was added, and the obtained mixture was stirred at room temperature for 1 h. Concentration was performed to obtain the crude target compound 3-(fluoromethylene)pyrrolidine trifluoroacetate (140 mg, crude), which was directly used in the next reaction.

MS m/z (ESI): 102 [M+H].

### Intermediate 4

### Step 1: Preparation of tert-butyl 4-(fluoromethylene)azacyclohexane-1-carboxylate

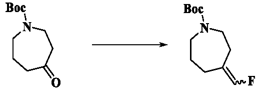

(Fluoromethyl)triphenylphosphonium tetrafluoroborate (23.29 g, 60.95 mmol) was added to tetrahydrofuran (300 mL), and a solution of 18-crown-6 (2.48 g, 9.38 mmol) and 1.0 M potassium tert-butoxide in tetrahydrofuran (56.27 mL, 56.27 mmol) were added, the obtained mixture was cooled to 0°C, tert-butyl 4-oxoazacyclopenta-1-carboxylate (10 g, 46.89 mmol) was added, the obtained mixture was maintained at 0°C for 20 min, and heated to room temperature overnight. Water was added, the obtained mixture was extracted with ethyl acetate. The organic phases were combined, washed with saturated sodium chloride aqueous solution, dried over anhydrous sodium sulfate, concentrated under reduced pressure, and purified by silica gel column chromatography to obtain compound tert-butyl 4-(fluoromethylene)azacyclohexane-1-carboxylate (9.58 g, yield 89.1%).

MS m/z (ESI): 230 [M+H].

### Step 2: Preparation of 4-(fluoromethylene)azacyclohexane hydrochloride

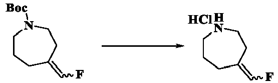

Tert-butyl 4-(fluoromethylene)azacyclohexane-1-carboxylate (9.58 g, 41.78 mmol) was added to dichloromethane (50 mL), a solution of 4.0 M hydrochloric acid in ethyl acetate (70 mL) was added, and the obtained mixture was stirred at room temperature for 1 h. Concentration was performed under reduced pressure to obtain compound 4-(fluoromethylene)azacyclohexane hydrochloride (3.8 g, yield 99.7%).

MS m/z (ESI):130 [M+H].

### Step 3: Preparation of methyl 1-(4-(fluoromethylene)azacyclohexane-1-carbonyl)cyclopropane-1-carboxylate

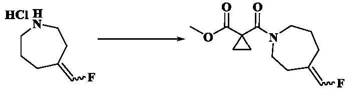

1-methoxycarbonylcyclopropanecarboxylic acid (7.31 g, 50.71 mmol) was added to dichloromethane (70 mL), N,N-diisopropylethylamine (16.39 g, 126.78 mmol) was added, and N,N,N' ,N' ,-tetramethyl-O-(7-azabenzotriazol-1-yl)uronium hexafluorophosphate (17.54 g, 46.49 mmol) was added in portions at room temperature, 4-(fluoromethylene)azacyclohexane hydrochloride (7 g, 42.26 mmol) was added in portions at room temperature, and the obtained mixture was stirred at room temperature for 2 h. Water was added, the obtained mixture was extracted with ethyl acetate. The organic phases were combined, washed with saturated sodium chloride aqueous solution, dried over anhydrous sodium sulfate, concentrated under reduced pressure, and purified by silica gel column chromatography to obtain methyl 1-(4-(fluoromethylene)azacyclohexane-1-carbonyl)cyclopropane-1-carboxylate (4.8 g, yield 44.5%).

MS m/z (ESI):256[M+H].

### Step 4: Preparation of (1-((4-(fluoromethylene)azacyclohexane-1-yl)methyl)cyclopropyl)methanol

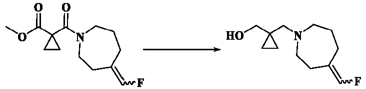

Methyl 1-(4-(fluoromethylene)azacyclohexane -1-carbonyl)cyclopropane-1-carboxylate (4.8 g, 18.80 mmol) was added to tetrahydrofuran (50 mL). Under nitrogen protection, a solution of 2.5 M lithium aluminum hydride (15.47 mL, 21.45 mmol) in tetrahydrofuran was added dropwise at 0-10°C, and the obtained mixture was reacted at room temperature for 2 h. Sodium sulfate decahydrate (9.6 g, 29.80 mmol) was added, and the obtained mixture was stirred for 1 h, filtered, and the filtrate was concentrated to obtain compound (1-((4-(fluoromethylene)azacyclohexane-1-yl)methyl)cyclopropyl)methanol (3.4 g, yield 86.6%).

MS m/z (ESI):214 [M+H].

### Intermediate 5

### Step 1: Preparation of 1-tert-butyl 4-methyl-5-oxo-azacyclohexane-1,4-dicarboxylate

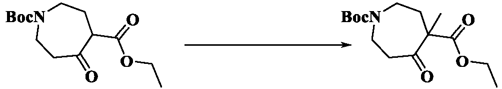

NaH (1.55 g, 64.59 mmol) was dissolved in DMF (60 mL), and the obtained mixture was stirred evenly, cooled to 0°C under nitrogen protection by nitrogen replacement, and a solution of ethyl 1-Boc-5-oxoazepane-carboxylate (10 g, 35.05 mmol) in DMF (40 mL) was added dropwise. After the dropwise addition was completed, the obtained mixture was stirred at room temperature for 1 h, then cooled to 0°C, methyl iodide (12.5 g, 88.03 mmol) was slowly added dropwise, and after the dropwise addition was completed, the obtained mixture was stirred at room temperature for 4 h. 200 mL of water was slowly added to the reaction liquid to quench the reaction, and the obtained mixture was extracted with ethyl acetate. The organic phases were combined, washed with saturated aqueous sodium chloride solution, dried over anhydrous sodium sulfate, and concentrated under reduced pressure to obtain 1-tert-butyl 4-methyl-5-oxo-azacyclohexane-1,4-dicarboxylate (10.7 g, crude product), which was directly used in the next reaction.

MS m/z (ESI): 300[M+H].

### Step 2: Preparation of tert-butyl 4-methyl-5-oxo-azacyclopentane-1-carboxylate

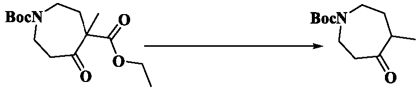

The substrate 1-tert-butyl 4-methyl-5-oxo-azacyclohexane-1,4-dicarboxylate (8.0 g, 26.72 mmol) was dissolved in dioxane (80 mL). A solution of KOH (7.5 g, 133.68 mmol) in water (56 mL) was added, the obtained mixture was heated to 100°C, and stirred overnight. The mixture was diluted with 300 mL of water, extracted with ethyl acetate. The organic phases were combined, washed with saturated aqueous sodium chloride solution, dried over anhydrous sodium sulfate, concentrated under reduced pressure, and purified by silica gel column chromatography to obtain the target compound tert-butyl 4-methyl-5-oxo-azacyclopentane-1-carboxylate (3.8 g).

MS m/z (ESI): 228 [M+H].

### Step 3: Preparation of tert-butyl 4-(difluoromethylene)-5-methylazacyclopentane-1-carboxylate

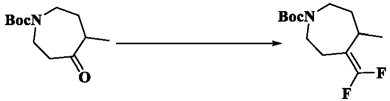

Tert-butyl 4-methyl-5-oxo-azacyclopentane-1-carboxylate (1.0 g, 4.4 mmol) was dissolved in THF (20 mL), the obtained mixture was cooled to 0°C under nitrogen protection by nitrogen replacement, HMPT (2.87 g, 17.61 mmol) and CF₂Br₂ (3.7 g, 17.63 mmol) were added, the reaction liquid was warmed to room temperature, zinc powder (1.15 g, 17.69 mmol) and HMPT (10 mg, 0.06 mmol) were added, the obtained mixture was heated to 70°C, and stirred for 3 h. The reaction liquid was cooled to room temperature, filtered and rinsed with dichloromethane (50 mL) to obtain the filtrate. The filtrate was concentrated and purified by silica gel column chromatography to obtain tert-butyl 4-(difluoromethylene)-5-methyl-azacyclopentane-1-carboxylate (350 mg, yield 30.4%).

MS m/z (ESI): 262[M+H].

### Step 4: Preparation of 4-(difluoromethylene)-5-methylazacyclohexane hydrochloride

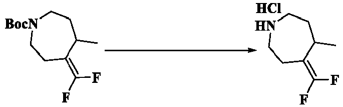

Tert-butyl 4-(difluoromethylene)-5-methyl-azacyclopentane-1-carboxylate (250 mg, 0.96 mmol) was dissolved in DCM (5 mL), and HCl/EtOAc (4 M, 5 mL) was added dropwise. The reaction liquid was stirred at room temperature for 3 h, and concentrated to obtain 4-(difluoromethylene)-5-methylazacyclohexane hydrochloride (240 mg, crude product), which was directly used in the next reaction.

MS m/z (ESI): 162 [M+H].

### Intermediate 6

### Step 1: Preparation of tert-butyl 5-(fluoromethylene)hexahydrocyclopenta [c]pyrrole-2(1H)-carboxylate

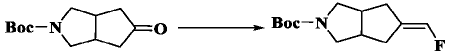

(Fluoromethyl)triphenylphosphonium tetrafluoroborate (52.9 g, 138.49 mmol) and 18-crown ether-6 (6.06 g, 22.93 mmol) were added to tetrahydrofuran (500 mL), and potassium tert-butoxide solution (128 mL, 1 M in THF) was added under nitrogen protection. The reaction was cooled to 0°C, and a solution of tert-butyl 5-oxohexahydrocyclopenta[c]pyrrole-2(1H)-carboxylate (24 g, 106.5 mmol) was added dropwise at 0°C. The reaction was gradually returned to room temperature and stirred overnight. After the end of the reaction, saturated ammonium chloride solution was added to quench the reaction, and the obtained mixture was extracted with ethyl acetate. The organic phases were combined, dried over anhydrous sodium sulfate, concentrated and separated and purified by silica gel column chromatography to obtain tert-butyl 5-(fluoromethylene)hexahydrocyclopenta [c]pyrrole-2(1H)-carboxylate (17 g, yield 66.1%).

MS m/z (ESI):242 [M+H].

### Step 2: Preparation of 5-(fluoromethylene)octahydrocyclopentadiene

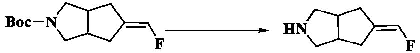

Tert-butyl 5-(fluoromethylene)hexahydrocyclopenta[c]pyrrole-2(1H)-carboxylate (17 g, 70.45 mmol) was added to 10 mL of ethyl acetate solution, and a solution of hydrogen chloride in ethyl acetate (141 mL, 563 mmol, 4 M in EtOAc) was added to the reaction flask. After reacting for 1 h, concentration was performed under reduced pressure, saturated sodium bicarbonate was added to adjust to neutrality, and the obtained mixture was extracted with ethyl acetate. The organic phases were combined, dried over anhydrous sodium sulfate, filtered, and concentrated to obtain 5-(fluoromethylene)octahydrocyclopentadiene (9.9 g, yield 99%).

MS m/z (ESI): 142 [M+H].

### Step 3: Preparation of methyl 1-(5-(fluoromethylene)octahydrocyclopenta [c]pyrrole-2-carbonyl)cyclopropane-1-carboxylate

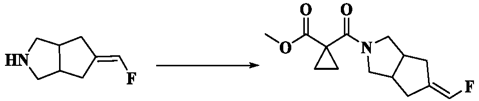

The raw materials 1-methoxycarbonylcyclopropanecarboxylic acid (12 g, 85 mmol), diisopropylethylamine (28 g, 216 mmol), 2-(7-azabenzotriazole)-N,N,N',N'-tetramethyluronium hexafluorophosphate (29 g, 77 mmol) were added to 170 mL of dichloromethane solution. Stirring was performed at room temperature for 5 min, the raw material 5-(fluoromethylene)octahydrocyclopentadiene (9.9 g, 70 mmol) was dissolved in the dichloromethane solution, slowly added dropwise into the reaction flask at 0°C, the obtained mixture was slowly returned to room temperature and stirred for 2 h. After the end of the reaction, water was added to quench the reaction and the obtained mixture was extracted with dichloromethane. The organic phases were combined, dried over anhydrous sodium sulfate, filtered, concentrated, and purified by silica gel column chromatography to obtain methyl 1-(5-(fluoromethylene)octahydrocyclopenta[c]pyrrole-2-carbonyl)cyclopropane-1-carboxylate (17 g, yield 89.8%).

MS m/z (ESI):268 [M+H].

### Step 4: Preparation of (1-((5-(fluoromethylene)hexahydrocyclopenta [c]pyrrole-2(1H)-yl)methyl)cyclopropyl)methanol

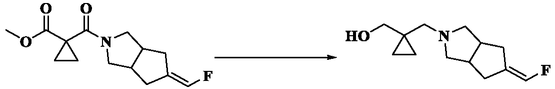

Lithium aluminum hydride (51 mL, 126 mmol, 2.5 M in THF) was added to 160 mL of tetrahydrofuran solution, the obtained mixture was subjected to nitrogen replacement, and cooled to 0°C. Methyl 1-(5-(fluoromethylene)octahydrocyclopenta[c]pyrrole-2-carbonyl)cyclopropane-1-carboxylate (16 g, 60 mmol) was slowly added to the reaction system, the obtained mixture was stirred for 5 min, returned to room temperature, and further stirred for 1 h. After the reaction was completed, 32 g of sodium tartrate was added in portions in small amount to the reaction system for quenching. The reaction system was filtered through diatomaceous earth and washed with ethyl acetate. The organic phase was concentrated under reduced pressure to obtain (1-((5-(fluoromethylene)hexahydrocyclopenta[c]pyrrole-2(1H)-yl)methyl)cyclopropyl)methanol (12.7 g, yield 94.2%).

MS m/z (ESI):226 [M+H].

### Intermediate 7

### Step 1: Preparation of tert-butyl (1S,2S,5R)-3-benzyl-2-(hydroxymethyl)-3,8-diazabicyclo[3.2.1]octane-8-carboxylate

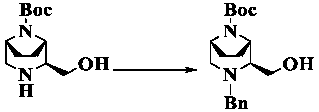

Tert-butyl (1S,2S,5R)-2-(hydroxymethyl)-3,8-diazabicyclo[3.2.1]octane-8-carboxylate (354 g, 1.46 mol) was added to acetonitrile (2100 mL) and water (525 mL), potassium carbonate (404.03 g, 2.92 mol) and benzyl bromide (275.01 g, 1.61 mol) were added, and the obtained mixture was stirred at room temperature for 1 h, layer separation was performed, and the mixture was concentrated. Ethyl acetate was added for extraction twice. The organic phases were combined, dried over anhydrous sodium sulfate, concentrated under reduced pressure, and purified by silica gel column chromatography to obtain compound tert-butyl (1S,2S,5R)-3-benzyl-2-(hydroxymethyl)-3,8-diazabicyclo[3.2.1]octane-8-carboxylate (410 g, yield 84.4%).

MS m/z (ESI):333[M+H].

### Step 2: Preparation of tert-butyl (1S,2S,5R)-3-benzyl-2-((methoxymethoxy) methyl)-3,8-diazabicyclo[3.2.1]octane-8-carboxylate

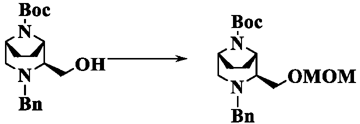

Tert-butyl (1S,2S,SR)-3-benzyl-2-(hydroxymethyl)-3,8-diazabicyclo[3.2.1] octane-8-carboxylate (13 g, 39.11 mmol) and N,N-diisopropylethylamine (22.74 g, 175.97 mmol) were added to dichloromethane (130 mL), bromomethyl methyl ether (19.55 g, 156.42 mmol) was added at 0-10°C, and the obtained mixture was stirred at room temperature for 16 h, water was added, the aqueous layer was extracted with dichloromethane. The organic phases were combined, washed with saturated sodium chloride aqueous solution, dried over anhydrous sodium sulfate, and purified by silica gel column chromatography to obtain compound tert-butyl (1S,2S,5R)-3-benzyl-2-((methoxymethoxy)methyl)-3,8-diazabicyclo[3.2.1]octane-8-carboxylate (9.7 g, yield 65.9%).

MS m/z (ESI):377[M+H].

### Step 3: Preparation of tert-butyl (1R,4S,55)-3-benzyl-1-formyl-4-((methoxymethoxy)methyl)-3, 8-diazabicyclo[3.2.1]octane-8-carboxylate

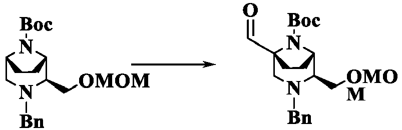

Tert-butyl (1S,2S,5R)-3-benzyl-2-((methoxymethoxy)methyl)-3,8-diazabicyclo[3.2.1]octane-8-carboxylate (9.7 g, 25.76 mmol) and N,N,N',N'-tetramethylethylenediamine (14.97 g, 128.82 mmol) were added to tetrahydrofuran (100 mL), and sec-butyllithium (1.3 M in cyclohexane, 39.64 mL) was added dropwise at -40°C under nitrogen protection. The obtained mixture was stirred at - 40°C for 2 h. Methyl formate (7.74 g, 128.82 mmol) was added dropwise to the reaction mixture at -40°C under nitrogen protection, and the obtained mixture was stirred at room temperature for 3 h. Saturated ammonium chloride solution was added dropwise to quench the reaction, and the separated aqueous layer was extracted with ethyl acetate. The organic layers were combined, dried over anhydrous sodium sulfate, concentrated under reduced pressure, and purified by silica gel column chromatography to obtain compound tert-butyl (1R,4S,5S)-3-benzyl-1-formyl-4-((methoxymethoxy)methyl)-3,8-diazabicyclo[3.2.1]octane-8-carboxylate (5.7 g, yield 54.7%).

MS m/z (ESI):405 [M+H].

### Step 4: Preparation of tert-butyl (1R,4S,5S)-3-benzyl-1-(hydroxymethyl)-4-(methoxymethoxy)methyl)-3,8-diazabicyclo[3.2.1]octane-8-carboxylate

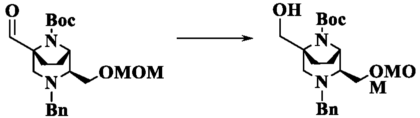

Tert-butyl (1R,4S,5S)-3-benzyl-1-formyl-4-((methoxymethoxy)methyl)-3,8-diazabicyclo[3.2.1]octane-8-carboxylate (5.7 g, 14.09 mmol) was added to methanol (57 mL), and NaBH₄ (1.07 g, 28.18 mmol) was added at 0-10°C, and the obtained mixture was stirred at room temperature for 1 h. Water was added and the mixture was concentrated to remove methanol to obtain crude product. Ethyl acetate was added, and the separated aqueous layer was extracted with ethyl acetate. The organic layers were combined, dried over anhydrous sodium sulfate, concentrated under reduced pressure, and purified by silica gel column chromatography to obtain compound tert-butyl (1R,4S,5S)-3-benzyl-1-(hydroxymethyl)-4-(methoxymethoxy) methyl)-3,8-diazabicyclo[3.2.1]octane-8-carboxylate (4.7 g, yield 82.1%).

MS m/z (ESI):407 [M+H].

### Step 5: Preparation of tert-butyl (1R,4S,5S)-3-benzyl-4-((methoxymethoxy)methyl)-1-(methoxymethyl)-3,8-diazabicyclo[3.2.1]octane-8-carboxylate

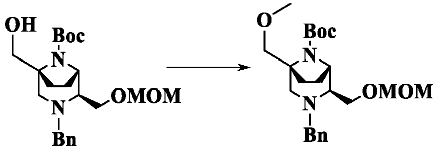

Tert-butyl (1R,4S,5S)-3-benzyl-1-(hydroxymethyl)-4-(methoxymethoxy) methyl)-3,8-diazabicyclo[3.2.1]octane-8-carboxylate (4.7 g, 11.56 mmol) was added to tetrahydrofuran (47 mL), and 60% sodium hydride (1.83 g, 76.31 mmol) was added under ice bath, and the obtained mixture was reacted at room temperature for 1 h. Methyl iodide (49.25 g, 346.85 mmol) was added, and the obtained mixture was stirred at room temperature for 16 h. Water and ethyl acetate were added, layer separation was performed, and the aqueous phase was extracted with ethyl acetate. The organic layers were combined, dried over anhydrous sodium sulfate, concentrated under reduced pressure, and purified by silica gel column chromatography to obtain compound tert-butyl (1R,4S,5S)-3-benzyl-4-((methoxymethoxy)methyl)-1-(methoxymethyl)-3,8-diazabicyclo[3.2.1]octane-8-carboxylate (3.4 g, yield 69.9%).

MS m/z (ESI):421 [M+H].

### Step 6: Preparation of tert-butyl (1R,4S,5S)-3-benzyl-4-(hydroxymethyl)-1-(methoxymethyl)-3,8-diazabicyclo[3.2.1]octane-8-carboxylate

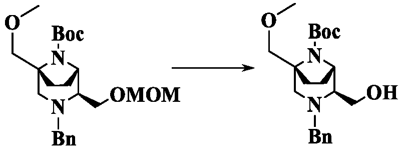

Tert-butyl (1R,4S,5S)-3-benzyl-4-((methoxymethoxy)methyl)-1-(methoxymethyl) -3,8-diazabicyclo[3.2.1]octane-8-carboxylate (3.4 g, 8.09 mmol) was added to ethyl acetate (20 mL), and a solution of 4.0 M hydrochloric acid in ethyl acetate (31.36 mL, 125.44 mmol) was added, and the obtained mixture was stirred at room temperature for 1 h. Concentration was performed under reduced pressure, and dichloromethane (30 mL) was added, N,N-diisopropylethylamine (5.40 g, 41.82 mmol) was added, and di-tert-butyl dicarbonate (2.74 g, 12.55 mmol) was added, and the obtained mixture was stirred at room temperature for 1 h. The obtained mixture was concentrated under reduced pressure, and purified by silica gel column chromatography to obtain compound tert-butyl (1R,4S,5S)-3-benzyl-4-(hydroxymethyl)-1-(methoxymethyl)-3,8-diazabicyclo[3.2.1]octane-8-carboxylate (2.5 g, yield 82.0%).

MS m/z (ESI):377 [M+H].

### Step 7: Preparation of tert-butyl (1R,4S,5S)-4-(hydroxymethyl)-1-(methoxymethyl)-3,8-diazabicyclo[3.2.1]octane-8-carboxylate

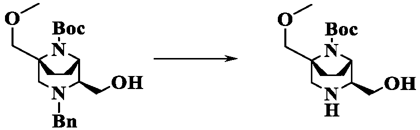

Tert-butyl (1R,4S,5S)-3-benzyl-4-(hydroxymethyl)-1-(methoxymethyl)-3,8-diazabicyclo[3.2.1]octane-8-carboxylate (2.5 g, 6.64 mmol) was added to methanol (20 mL), and 10% palladium hydroxide (1.30 g, 9.30 mmol) was added, and the obtained mixture was stirred at room temperature for 16 h under a 0.3-0.4 MPa hydrogen atmosphere. The obtained mixture was heated to 80°C and stirred for 8 h. The obtained mixture was filtered through a celite pad, concentrated under reduced pressure, and purified by silica gel column chromatography to obtain compound tert-butyl (1R,48S,5S)-4-(hydroxymethyl)-1-(methoxymethyl)-3,8-diazabicyclo[3.2.1] octane-8-carboxylate (1.8 g, yield 94.7%).

MS m/z (ESI):287 [M+H].

### Step 8: Preparation of tert-butyl (1R,4S,4S,5S)-4-[(7-chloro-8-fluoro-4-hydroxy-2-methylsulfanyl-pyrido[4,3-d]pyrimidin-5-yl)oxymethyl]-1-(methoxymethyl)-3,8-diazabicyclo[3.2.1]octane-8-carboxylate

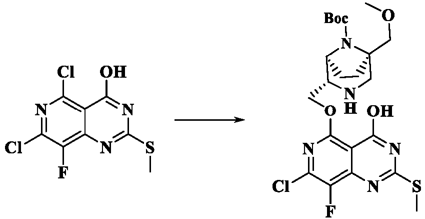

Tert-butyl (1R,4S,5S)-4-(hydroxymethyl)-1-(methoxymethyl)-3,8-diazabicyclo [3.2.1]octane-8-carboxylate (1.88 g, 6.57 mmol) was added to tetrahydrofuran (25 mL), and 60% sodium hydride (1.13 g, 28.23 mmol) was added under ice bath, and the obtained mixture was reacted under ice bath for 1 h. 5,7-dichloro-8-fluoro-2-methylsulfanyl-pyrido[4,3-d]pyrimidin-4-ol (2.21 g, 7.88 mmol) was added in portions at 0-10°C within 2 min, and the obtained mixture was stirred at 65°C for 1 h. Water and ethyl acetate were added for extraction, layer separation was performed, and the aqueous phase was extracted with ethyl acetate, washed with saturated sodium chloride aqueous solution, dried over anhydrous sodium sulfate, and concentrated under reduced pressure to obtain compound tert-butyl (1R,4S,4S,5S)-4-[(7-chloro-8-fluoro-4-hydroxy-2-methylsulfanyl-pyrido[4,3-d]pyrimidin-5-yl)oxymethyl]-1-(methoxymethyl)-3,8-diazabicyclo[3.2.1]octane-8-carboxylate (3.48 g, yield 100%).

MS m/z (ESI):530 [M+H].

### Step 9: Preparation of tert-butyl (5aS,6S,9R)-2-chloro-1-fluoro-9-(methoxymethyl)-12-(methylthio)-5a,6,7,8,9,10-hexahydro-5H-4-oxa-3,10a,11,13,14-pentaaza-6,9-methylnaphtho[1,8-ab]heptene-4-carboxylate

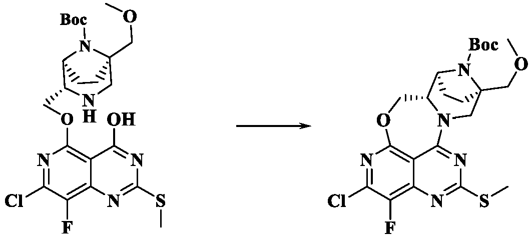

Tert-butyl (1R,4S,4S,SS)-4-[(7-chloro-8-fluoro-4-hydroxy-2-methylsulfanylpyrido[4,3-d]pyrimidin-5-yl)oxymethyl]-1-(methoxymethyl)-3,8-diazabicyclo[3.2.1]octane-8-carboxylate (3.48 g, 6.57 mmol) and N,N-diisopropylethylamine (4.24 g, 32.83 mmol) were added to chloroform (40 mL), bis(2-oxo'-3-oxazolidinyl)phosphinic chloride (3.34 g, 13.13 mmol) was added, and the obtained mixture was stirred at 65°C for 1 h. The obtained mixture was cooled to room temperature, water and dichloromethane were added, the aqueous phase was extracted with dichloromethane, dried over anhydrous sodium sulfate, concentrated under reduced pressure, and purified by silica gel column chromatography to obtain compound tert-butyl (5aS,6S,9R)-2-chloro-1-fluoro-9-(methoxymethyl)-12-(methylthio)-5a,6,7,8,9,10-hexahydro-5H-4-oxa-3,10a,11,13,14-pentaaza-6,9-methylnaphthaleno[1,8-ab]heptene-4-carboxylate (2.17 g, yield 64.6%).

MS m/z (ESI):512 [M+H].

### Intermediate 8

### Step 1: Preparation of tert-butyl (1S,2S,SR)-3-benzyl-2-formyl-3,8-diazabicyclo[3.2.1]octane-8-carboxylate

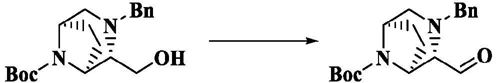

Tert-butyl (1S,2S,SR)-3-benzyl-2-(hydroxymethyl)-3,8-diazabicyclo[3.2.1] octane-8-carboxylate (28 g, 83.97 mmol) was dissolved in dichloromethane (280 mL) and water (280 mL). 2,2,6,6-tetramethylpiperidine oxide (1.26 g, 8.08 mmol) and sodium bicarbonate (33.95 g, 404.08 mmol) were added. Aqueous sodium hypochlorite solution (42.11 g, 565.72 mmol) was added under ice bath, and the obtained mixture was reacted under ice bath for 20 min. Water was added to quench the reaction, and the obtained mixture was extracted with dichloromethane, and the organic phases were combined, washed with saturated sodium chloride aqueous solution, dried over anhydrous sodium sulfate, concentrated under reduced pressure, and purified by silica gel column chromatography to obtain compound tert-butyl (1S,2S,5R)-3-benzyl-2-formyl-3,8-diazabicyclo[3.2.1]octane-8-carboxylate (10.5 g, yield 37.7%).

MS m/z (ESI):331[M+H].

### Step 2: Preparation of tert-butyl (1S,2S,5R)-3-benzyl-2-(1-hydroxyethyl)-3,8-diazabicyclo[3.2.1]octane-8-carboxylate

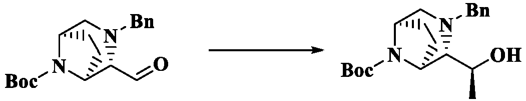

Tert-butyl (1S,2S,5R)-3-benzyl-2-formyl-3,8-diazabicyclo[3.2.1]octane-8-carboxylate (10.5 g, 31.78 mmol) was dissolved in tetrahydrofuran (100 mL), the obtained mixture was subjected to nitrogen replacement, cooled to -60°C, and methylmagnesium bromide (4.73 g, 39.63 mmol) was added dropwise. The obtained mixture was slowly warmed to 0°C and reacted for 2 h, saturated ammonium chloride aqueous solution was added to quench the reaction. The obtained mixture was extracted with ethyl acetate. The organic phases were combined, washed with saturated sodium chloride aqueous solution, dried over anhydrous sodium sulfate, concentrated under reduced pressure, and purified by silica gel column chromatography to obtain compound tert-butyl (1S,2S,5R)-3-benzyl-2-(1-hydroxyethyl)-3,8-diazabicyclo[3.2.1]octane-8-carboxylate (9.64 g, yield 87.7%).

MS m/z (ESI):347[M+H].

### Step 3: Preparation of tert-butyl (1S,2S,5R)-2-acetyl-3-benzyl-3,8-diazabicyclo[3.2.1]octane-8-carboxylate

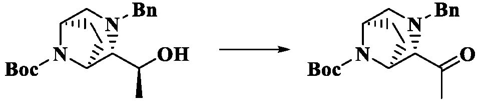

Tert-butyl (1S,2S,SR)-3-benzyl-2-(1-hydroxyethyl)-3,8-diazabicyclo[3.2.1]octane-8-carboxylate (9.64 g, 27.81 mmol) was dissolved in dichloromethane (100 mL), and Dess-Martin oxidant (17.01 g, 40.11 mmol) was added in portions under ice bath. After the addition was completed, the obtained mixture was warmed to room temperature and reacted for 2 h, and water was added to quench the reaction. The obtained mixture was extracted with dichloromethane. The organic phases were combined, washed with saturated sodium chloride aqueous solution, dried over anhydrous sodium sulfate, concentrated under reduced pressure and purified by silica gel column chromatography to obtain compound tert-butyl (1S,2S,SR)-2-acetyl-3-benzyl-3,8-diazabicyclo[3.2.1]octane-8-carboxylate (9.1 g, yield 94.9%).

MS m/z (ESI):345[M+H].

### Step 4: Preparation of tert-butyl (1S,2S,5R)-3-benzyl-2-(2-hydroxypropan-2-yl)-3,8-diazabicyclo[3.2.1]octane-8-carboxylate

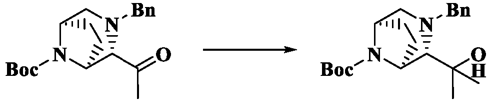

Tert-butyl (1S,2S,SR)-2-acetyl-3-benzyl-3,8-diazabicyclo[3.2.1]octane-8-carboxylate (9.10 g, 26.42 mmol) was dissolved in tetrahydrofuran (100 mL), the obtained mixture was subjected to nitrogen replacement, cooled to -60°C, and methylmagnesium bromide (3.94 g, 33.00 mmol) was added dropwise. The obtained mixture was slowly warmed to 0°C and reacted for 2 h, saturated ammonium chloride aqueous solution was added to quench the reaction. The obtained mixture was extracted with ethyl acetate. The organic phases were combined, washed with saturated sodium chloride aqueous solution, dried over anhydrous sodium sulfate, concentrated under reduced pressure, and purified by silica gel column chromatography to obtain compound tert-butyl (1S,2S,5R)-3-benzyl-2-(2-hydroxypropan-2-yl)-3,8-diazabicyclo[3.2.1]octane-8-carboxylate (2 g, yield 21.0%).

MS m/z (ESI):361[M+H].

### Step 5: Preparation of tert-butyl (1S,2S,5R)-2-(2-hydroxypropan-2-yl)-3,8-diazabicyclo[3.2.1]octane-8-carboxylate

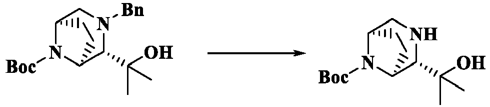

Tert-butyl (1S,2S,5R)-3-benzyl-2-(2-hydroxyprop-2-yl)-3,8-diazabicyclo[3.2.1] octane-8-carboxylate (2 g, 5.55 mmol) was dissolved in methanol (20 mL), and palladium hydroxide (1 g, 10%) was added. The obtained mixture was subjected to nitrogen replacement, reacted at room temperature for 16 h, and filtered, the filtrate was concentrated under reduced pressure, and purified by silica gel column chromatography to obtain compound tert-butyl (1S,2S,5R)-2-(2-hydroxyprop-2-yl)-3,8-diazabicyclo[3.2.1]octane-8-carboxylate (903 mg, yield 60.2%).

MS m/z (ESI):271[M+H].

### Intermediate 9

### Step 1: Preparation of tert-butyl (1S,2S,SR)-3-benzyl-2-((S)-cyclopropyl (hydroxy)methyl)-3,8-diazabicyclo[3.2.1]octane-8-carboxylate

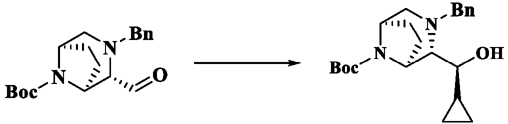

Tert-butyl (1R,2R,5S)-3-benzyl-2-formyl-3,8-diazabicyclo[3.2.1]octane-8-carboxylate (1.4 g, 4.24 mmol) was dissolved in tetrahydrofuran (30 mL), the obtained mixture was subjected to nitrogen replacement and cooled to 0°C, cyclopropyl magnesium bromide solution (8.5 mL, 8.47 mmol, 1 M in THF) was slowly added dropwise to the system. The reaction was kept warm for half an hour, then slowly warmed to room temperature and the stirring was continued for 3 h. After the reaction was completed, water was added to quench the reaction, and the obtained mixture was extracted with ethyl acetate, and washed with saturated sodium chloride aqueous solution. The organic phase was dried over anhydrous sodium sulfate, concentrated under reduced pressure and separated by silica gel column chromatography to obtain tert-butyl (1S,2S,SR)-3-benzyl-2-((S)-cyclopropyl(hydroxy)methyl)-3,8-diazabicyclo[3.2.1]octane-8-carboxylate (1.2 g, yield 76.0%).

MS m/z (ESI):373 [M+H].

### Step 2: Preparation of tert-butyl (1S,2S,5R)-2-((S)-cyclopropyl (hydroxy)methyl)-3,8-diazabicyclo[3.2.1]octane-8-carboxylate

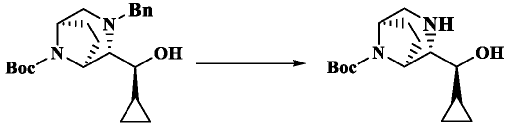

The raw material tert-butyl (1S,2S,SR)-3-benzyl-2-((S)-cyclopropyl(hydroxy)methyl)-3,8-diazabicyclo[3.2.1]octane-8-carboxylate (1.18 g, 3.17 mmol) was dissolved in 20 mL of methanol, and 600 mg of palladium hydroxide/carbon (10%wt) were weighed and added to the reaction liquid. The reaction flask was placed in a hydrogen gas tank with the pressure set to 65 psi, and hydrogen replacement was performed 3 times. After reacting at room temperature for 4 h, the reaction liquid was filtered through diatomaceous earth and washed with methanol. The filtrate was concentrated under reduced pressure and separated by silica gel column chromatography to obtain tert-butyl (1S,2S,5R)-2-((S)-cyclopropyl(hydroxy)methyl)-3,8-diazabicyclo[3.2.1]octane-8-carboxylate (810 mg, yield 90.6%).

MS m/z (ESI):283 [M+H].

### Intermediate 10

### Step 1: Preparation of 1-(tert-butyl)2-methyl(R)-4-(difluoromethylene) pyrrolidine-1,2-dicarboxylate

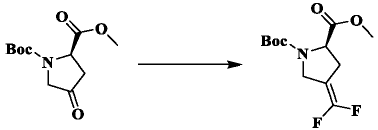

Hexamethylphosphoramide (8.83 g, 49.3 mmol, 10.6 mL) was added to a solution of dibromo(difluoro)methane (10.35 g, 49.3 mmol, 4.5 mL) in tetrahydrofuran (300 mL) at 0°C, and the obtained mixture was stirred at 0°C for 1 h after the addition was completed. A solution of 1-(tert-butyl)2-methyl(R)-4-oxopyrrolidine-1,2-dicarboxylate (3 g, 12.3 mmol) in tetrahydrofuran (200 mL) was added dropwise at 0°C. After the addition was completed, the obtained mixture was stirred at room temperature for 1 h. Zinc powder (3.23 g, 49.3 mmol) was added, then hexamethylphosphoramide (550 mg, 3.07 mmol) was added. After the addition was completed, the obtained mixture was stirred at 70°C for 4 h. Water (300 mL) was added, and the obtained mixture was extracted with ethyl acetate, and washed with saturated sodium chloride aqueous solution. The organic phase was dried over anhydrous sodium sulfate, concentrated under reduced pressure, and purified by silica gel column chromatography to obtain 1-(tert-butyl)2-methyl(R)-4-(difluoromethylene)pyrrolidine-1,2-dicarboxylate (2 g, yield: 58%).

MS m/z (ESI): 278 [M+H].

### Step 2: Preparation of tert-butyl (2R)-4-(difluoromethylene)-2-(hydroxymethyl)pyrrolidine-1-carboxylate

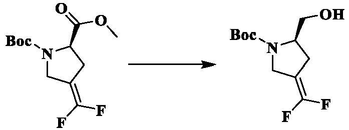

Lithium borohydride (1.43 g, 65.66 mmol) was added to a solution of 1-(tert-butyl)2-methyl(R)-4-(difluoromethylene)pyrrolidine-1,2-dicarboxylate (2 g, 7.21 mmol) in tetrahydrofuran (20 mL) at 0°C, and the obtained mixture was stirred at room temperature for 2 h after the addition was completed. Ammonium chloride aqueous solution (50 mL) was added dropwise to the reaction liquid at 0°C, the obtained mixture was stirred for another 1 h, extracted with ethyl acetate, and washed with saturated sodium chloride aqueous solution. The organic phase was dried over anhydrous sodium sulfate, filtered, and concentrated to obtain tert-butyl (2R)-4-(difluoromethylene)-2-(hydroxymethyl)pyrrolidine-1-carboxylate (1.7 g, yield 95%).

MS m/z (ESI): 250 [M+H].

### Step 3: Preparation of [(2R)-4-(difluoromethylene)pyrrolidin-2-yl]methanol trifluoroacetate

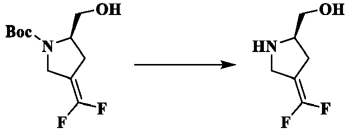

Trifluoroacetic acid (2 mL) was added to a solution of tert-butyl (2R)-4-(difluoromethylene)-2-(hydroxymethyl)pyrrolidine-1-carboxylate (200 mg, 0.8 mmol) in dichloromethane (6 mL) at room temperature, and the obtained mixture was stirred at room temperature for 1 h after the addition was completed. The reaction liquid was concentrated to obtain [(2R)-4-(difluoromethylene)pyrrolidin-2-yl]methanol trifluoroacetate (211 mg, yield 100%).

MS m/z (ESI): 150 [M+H].

### Intermediate 11

### Step 1: Preparation of (1S,4S)-2-(4-methoxyphenyl)-2-azabicyclo[2.2.2]octan-5-one

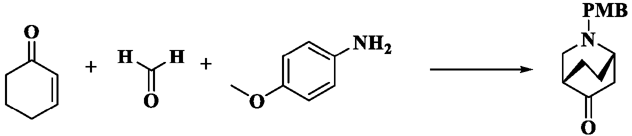

In an eggplant-shaped flask, 4-methoxyaniline (12.68 g, 102.99 mmol) and (S)-proline (3.23 g, 28.09 mmol) were dissolved in 240 mL of DMSO, the obtained mixture was subjected to nitrogen replacement, and paraformaldehyde (13.16 M, 7.11 mL) and 2-cyclohexene-1-one (18 g, 187.25 mmol) were quickly added, then the obtained mixture was reacted in an oil bath at 50°C for 24 h. After the addition was completed, the mixture was diluted with ethyl acetate, washed with water and saturated sodium chloride solution. The organic phase was dried over anhydrous sodium sulfate, filtered, concentrated, and purified by silica gel column chromatography to obtain (1S,4S)-2-(4-methoxyphenyl)-2-azabicyclo[2.2.2]oct-5-one (2.3 g, yield 10.6%).

MS m/z (ESI): 246 [M+H].

### Step 2: Preparation of (1S,4R)-5-(difluoromethylene)-2-(4-methoxyphenyl)-2-azabicyclo[2.2.2] octane

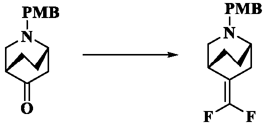

In a 250 mL three-necked flask, THF (60 mL) was added, the obtained mixture was subjected to nitrogen replacement, and cooled under ice bath for 5 min. After cooling, dibromo(difluoro)methane (10.43 g, 49.72 mmol) was added. Another syringe was taken and tri(dimethylamino)phosphine (8.11 g, 49.72 mmol) was slowly injected into the reaction liquid. The obtained mixture was reacted under ice bath for 1 h. (1S,4S)-2-(4-methoxyphenyl)-2-azabicyclo[2.2.2]oct-5-one (2.30 g, 9.94 mmol) was dissolved in 20 mL of tetrahydrofuran and the obtained mixture was slowly added to the reaction. After the addition was completed, the reaction was moved to room temperature and stirred for 1 h, zinc powder (3.25 g, 49.72 mmol) was added and additional 0.5 mL of tri(dimethylamino)phosphine was added, and the obtained mixture was reacted overnight in an oil bath at 75°C. The obtained mixture was cooled to room temperature, water was added to the reaction liquid to quench the reaction. the obtained mixture was filtered to obtain the filtrate, and extraction was performed with ethyl acetate. The organic phase was washed with saturated sodium chloride solution, dried over anhydrous sodium sulfate, filtered, concentrated, and purified by silica gel column chromatography to obtain (1S,4R)-5-(difluoromethylene)-2-(4-methoxyphenyl)-2-azabicyclo[2.2.2]octane (900 mg, yield: 34.1%).

MS m/z (ESI): 280 [M+H].

### Step 3: Preparation of (1S,4R)-5-(difluoromethylene)-2-azabicyclo[2.2.2]octane

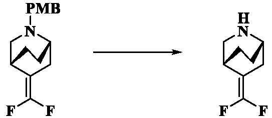

In an eggplant-shaped flask, (1S,4R)-5-(difluoromethylene)-2-(4-methoxyphenyl)-2-azabicyclo[2.2.2]octane (945 mg, 3.39 mmol) was dissolved in 20 mL of acetonitrile and 10 mL of water. Another EP tube was taken, and cerium ammonium nitrate (3.60 g, 6.78 mmol) was dissolved in 1 mL of water. The obtained mixture was added to the reaction liquid under ice bath and placed under ice bath for 30 min. After the addition was completed, saturated sodium sulfite solution was added to the reaction liquid to quench the reaction, and the filtrate was directly lyophilized. Ethyl acetate was added to the obtained solid for dilution, the obtained mixture was filtered, and the organic phase was concentrated to obtain (1S,4R)-5-(difluoromethylene)-2-azabicyclo[2.2.2]octane (530 mg, yield 98.2%).

MS m/z (ESI): 160 [M+H].

### Intermediate 12

### Step 1: Preparation of tert-butyl 4-(2,2,2-trifluoroethylene)piperidine-1-carboxylate

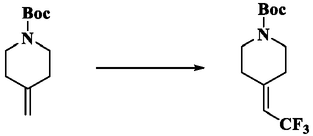

Tert-butyl 4-ethylenepiperidine-1-carboxylate (500 mg, 2.53 mmol), tris(2-phenylpyridine)iridium (49.79 mg, 76.04 µmol), S-(trifluoromethyl) dibenzothiophenium tetrafluoroborate (1.03 g, 3.04 mmol) and 25 mL of N,N-dimethylformamide were added into the Schlenk tube. The obtained mixture was subjected to nitrogen replacement and placed in a photoreactor to react overnight. After the addition was completed, the obtained mixture was diluted with water and ethyl acetate, and washed with saturated sodium chloride solution. The organic phase was dried over anhydrous sodium sulfate, filtered, concentrated, and purified by silica gel column chromatography to obtain tert-butyl 4-(2,2,2-trifluoroethylene)piperidine-1-carboxylate (359 mg, yield 53.4%).

MS m/z(ESI): 210 [M-tBu].

### Step 2: Preparation of 4-(2,2,2-trifluoroethylene)piperidine

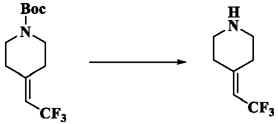

In an eggplant-shaped flask, 4 mL of a mixed solution of dichloromethane/trifluoroacetic acid = 3/1 was added to tert-butyl 4-(2,2,2-trifluoroethylene)piperidine-1-carboxylate (150 mg, 565.46 µmol), and the obtained mixture was reacted at room temperature for 1 h. The reaction liquid was concentrated to obtain the crude 4-(2,2,2-trifluoroethylene)piperidine (93.39 mg), which was directly used in the next reaction.

MS m/z(ESI): 166 [M+H].

### Intermediate 13

### Step 1: Preparation of tert-butyl 4-(2,2,2-trifluoroethylene)piperidine-1-carboxylate

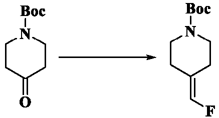

NaHMDS (1 M, 112.93 mL) was added dropwise to a suspension of (fluoromethyl)triphenylphosphonium tetrafluoroborate (43.15 g, 112.93 mmol) in THF (120 mL) at -20°C. After the addition was completed, the reaction was continued at -20°C for 20 min. In addition, tert-butyl 4-carbonylpiperidine-1-carboxylate (15 g, 75.28 mmol) was dissolved in THF (10 mL) and added dropwise to the reaction liquid. After the addition was completed, the obtained mixture was transferred to room temperature and reacted for 40 min. Saturated ammonium chloride solution was added to the reaction liquid to quench the reaction, and the obtained mixture was extracted with ethyl acetate. The organic phase was dried over anhydrous sodium sulfate, filtered, concentrated, and purified by flash silica gel column chromatography to obtain tert-butyl 4-(difluoromethylene)piperidine-1-carboxylate (7.5 g, yield 46.3%).

MS m/z (ESI):216 [M+H].

### Step 2: Preparation of 4-(fluoromethylene)piperidine trifluoroacetate

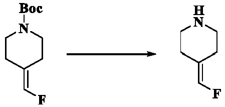

Tert-butyl 4-(fluoromethylene)piperidine-1-carboxylate (7.2 g, 33.47 mol) was added to dichloromethane (50 mL), and TFA (10 mL) was added dropwise under ice bath. After the reaction liquid was evenly mixed, reacted at room temperature for 1 h, and concentrated under reduced pressure to obtain crude compound 4-(difluoromethylene)piperidine trifluoroacetate (7.1 g, TF salt, yield 100%).

MS m/z (ESI):116 [M+H].

### Intermediate 14

### Step 1: Preparation of (1-(((tert-butyldiphenylsilyl)oxo)methyl) cyclopropyl)methanol

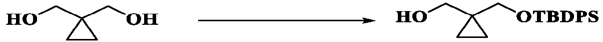

[1-(hydroxymethyl)cyclopropyl]methanol (29.73 g, 291.06 mmol) and TEA (44.10 g, 436.59 mmol) were added to dichloromethane (400 mL). After mixing evenly, tert-butyldiphenylsilyl chloride (40 g, 145.53 mmol) was added dropwise to the reaction liquid at 25°C, and the obtained mixture was reacted at 25°C for 16 h. Saturated ammonium chloride solution was added to the reaction liquid, and the obtained mixture was extracted with ethyl acetate. The organic phase was dried over anhydrous sodium sulfate, filtered, concentrated, and purified by flash silica gel column chromatography to obtain (1-(((tert-butyldiphenylsilyl)oxo)methyl) cyclopropyl)methanol (45 g, yield 45.4%).

### Intermediate 15

### Step 1: Preparation of 3-chloro-5-(4,4,5,5-tetramethyl-1,3,2-dioxaborolan-2-yl)-4-(trifluoromethyl)aniline

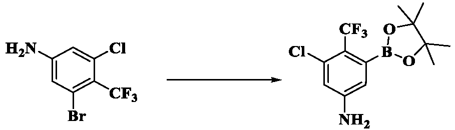

Under nitrogen protection, potassium acetate (5.18 g, 52.83 mmol) and Pd(dppf)Cl₂.DCM (871.69 mg, 1.06 mmol) were added to a solution of 3-bromo-5-chloro-4-(trifluoromethyl)aniline (2.9 g, 10.57 mmol) and 4,4,5,5-tetramethyl-2-(4,4,5,5-tetramethyl-1,3,2-dioxaborolan-2-yl)-1,3,2-dioxaborolane (6.71 g, 26.41 mmol) in dioxane (30 mL), and the obtained mixture was stirred at 120°C for 12 h. The reaction liquid was concentrated, and saturated NaCl aqueous solution was added, and the obtained mixture was extracted with ethyl acetate. The organic phase was dried over anhydrous sodium sulfate, filtered, then concentrated, and purified by silica gel column chromatography to obtain 3-chloro-5-(4,4,5,5-tetramethyl-1,3,2-dioxaborolan-2-yl)-4-(trifluoromethyl)aniline (2.6 g, yield 76.5%).

MS m/z (ESI):322 [M+H].

### Example 1-1

### Step 1: methyl (R)-5-methoxy-3,4-dihydro-2H-pyrrole-2-carboxylate

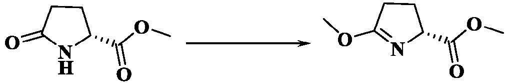

Methyl (R)-5-carbonylpyrrolidine-2-carboxylate (5 g, 34.93 mmol) and dimethyl sulfate (6.6 g, 52.33 mmol) were added to the round-bottom flask. Under nitrogen protection, the obtained mixture was heated to 60°C and stirred for 16 h. The obtained mixture was cooled, added dropwise to a solution of MTBE/TEA (100 mL/20 mL) at 0°C, and saturated aqueous potassium carbonate solution was added to quench the reaction, and then the obtained mixture was extracted with MTBE. The organic phases were combined, washed with saturated sodium chloride aqueous solution, dried over anhydrous sodium sulfate, and concentrated to obtain methyl (R)-5-methoxy-3,4-dihydro-2H-pyrrole-2-carboxylate (4.3 g, yield 78.2%).

MS m/z (ESI):158 [M+H].

### Step 2: Preparation of methyl (R,E)-5-(2-ethoxy-1-nitro-2-carbonylethylene)pyrrolidine-2-carboxylate

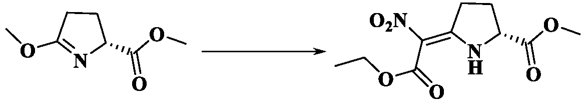

Methyl (R)-5-methoxy-3,4-dihydro-2H-pyrrole-2-carboxylate (4.0 g, 25.45 mmol) was added to ethyl nitroacetate (15 mL), and the obtained mixture was heated to 60°C and stirred for 16 h. The obtained mixture was concentrated under reduced pressure and purified by silica gel column chromatography to obtain compound methyl (R,E)-5-(2-ethoxy-1-nitro-2-carbonylethylene)pyrrolidine-2-carboxylate (2.1 g, yield 31.9%).

MS m/z (ESI):259 [M+H].

### Step 3: Preparation of ethyl (1S,5R)-4-carbonyl-3,8-diazabicyclo[3.2.1]octane-2-carboxylate

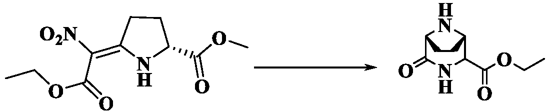

Methyl (R,E)-5-(2-ethoxy-1-nitro-2-carbonylethylene)pyrrolidine-2-carboxylate (2 g, 7.75 mmol) was added to ethanol (30 mL), and Pd/C (2 g) was added, the obtained mixture was reacted for 48 h under a hydrogen atmosphere, filtered, washed with ethanol. The organic phase was concentrated under reduced pressure, and purified by silica gel column chromatography to obtain compound ethyl (1S,5R)-4-carbonyl-3,8-diazabicyclo[3.2.1]octane-2-carboxylate (1.3 g, yield 84.4%).

MS m/z (ESI):199 [M+H].

### Step 4: Preparation of 8-(tert-butyl) 2-ethyl (1S,2S,5R)-4-carbonyl-3,8-diazabicyclo[3.2.1]octane-2,8-dicarboxylate

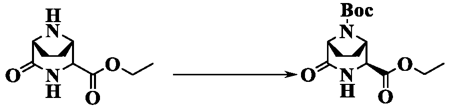

Ethyl (1S,5R)-4-carbonyl-3,8-diazabicyclo[3.2.1]octane-2-carboxylate (1.2 g, 6.05 mmol) was added to dichloromethane (20 mL), triethylamine (1.84 g, 18.2 mmol) and di-tert-butyl dicarbonate (1.58 g, 7.24 mmol) were added, the reaction liquid was evenly mixed, and reacted at room temperature for 16 h, water was added to quench the reaction, and the obtained mixture was extracted with dichloromethane. The organic phases were combined, washed with saturated sodium chloride aqueous solution, dried over anhydrous sodium sulfate, concentrated under reduced pressure and purified by silica gel column chromatography to obtain compound 8-(tert-butyl) 2-ethyl (1S,2S,5R)-4-carbonyl-3,8-diazabicyclo[3.2.1]octane-2,8-dicarboxylate (920 mg, yield 50.9%).

MS m/z (ESI):299 [M+H].

### Step 5: Preparation of 8-(tert-butyl) 2-ethyl (1S,2S,5R)-3,8-diazabicyclo[3.2.1]octane-2,8-dicarboxylate

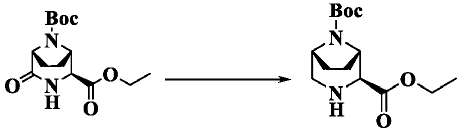

8-(tert-butyl) 2-ethyl (1S,2S,SR)-4-carbonyl-3,8-diazabicyclo[3.2.1]octane-2,8-dicarboxylate (900 mg, 3.02 mmol) was added to the borane dimethyl sulfide solution (2 M in THF, 10 mL), and the obtained mixture was reacted at room temperature for 16 h under nitrogen protection. Methanol was added to quench the reaction, and the obtained mixture was heated to 60°C and stirred for 16 h. The obtained mixture was concentrated under reduced pressure and purified by silica gel column chromatography to obtain compound 8-(tert-butyl) 2-ethyl (1S,2S,5R)-3,8-diazabicyclo[3.2.1]octane-2,8-dicarboxylate (570 mg, yield 66.2%).

MS m/z (ESI):285[M+H].

### Step 6: Preparation of tert-butyl (1S,2S,5R)-2-(hydroxymethyl)-3,8-diazabicyclo[3.2.1]octane-8-carboxylate

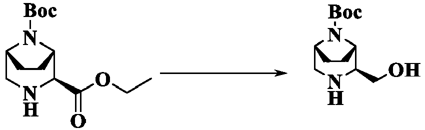

8-(tert-butyl) 2-ethyl (1S,2S,5R)-3,8-diazabicyclo[3.2.1]octane-2,8-dicarboxylate (530 mg, 1.86 mmol) was added to THF (20 mL), and LiAlH₄ (1 M in THF, 3 mL) was added dropwise under nitrogen protection under ice bath. The obtained mixture was warmed to room temperature and reacted for 3 h. Water was added to quench the reaction, the obtained mixture was filtered, and the filtrate was concentrated under reduced pressure, purified by silica gel column chromatography to obtain compound tert-butyl (1S,2S,5R)-2-(hydroxymethyl)-3,8-diazabicyclo[3.2.1]octane-8-carboxylate (380 mg, yield 84.3%).

MS m/z (ESI):243 [M+H].

### Step 7: Preparation of tert-butyl (1S,2S,5R)-2-(((tert-butyldiphenylsilyl) oxo)methyl)-3,8-diazabicyclo[3.2.1]octane-8-carboxylate

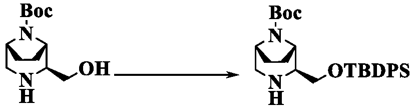

Tert-butyl (1S,2S,5R)-2-(hydroxymethyl)-3,8-diazabicyclo[3.2.1]octane-8-carboxylate (350 mg, 1.44 mmol) was added to dichloromethane (20 mL), DIPEA (930 mg, 7.19 mmol) and TBDPSCl (480 mg, 1.75 mmol) were added. The reaction liquid was evenly mixed, then stirred at 25°C for 3 h, and water was added, and the obtained mixture was extracted with dichloromethane. The organic phases were combined, washed with saturated sodium chloride aqueous solution, dried over anhydrous sodium sulfate, concentrated under reduced pressure and purified by silica gel column chromatography to obtain compound tert-butyl (1S,2S,5R)-2-(((tert-butyldiphenylsilyl)oxo)methyl)-3,8-diazabicyclo[3.2.1]octane-8-carboxylate (630 mg, yield 91.0%).

### Step 8: Preparation of 2,6-dichloro-3-fluoropyridine-4-amine

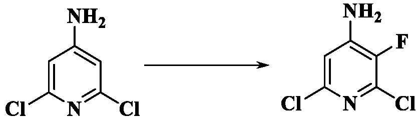

2,6-dichloropyridin-4-amine (10 g, 61.35 mmol) was added to acetonitrile (100 mL), Selectfluoro (26.1 g, 73.67 mmol) was added, and the obtained mixture was heated to 80°C and stirred for 8 h. The mixture was cooled to room temperature, and water was added, and the obtained mixture was extracted with dichloromethane. The organic phases were combined, washed with saturated sodium chloride aqueous solution, dried over anhydrous sodium sulfate, concentrated under reduced pressure, and purified by silica gel column chromatography to obtain compound 2,6-dichloro-3-fluoropyridin-4-amine (2.5 g, yield 22.5%).

MS m/z (ESI): 181 [M+H].

### Step 9: Preparation of 2,6-dichloro-3-fluoro-5-iodopyridin-4-amine

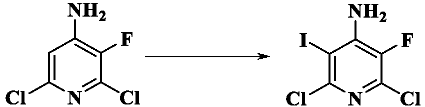

2,6-dichloro-3-fluoropyridin-4-amine (2.4 g, 13.26 mmol) was added to acetonitrile (50 mL), NIS (3.6 g, 16.00 mmol) and p-toluenesulfonic acid hydrate (120 mg, 0.63 mmol) were added, and the obtained mixture was heated to 70°C and stirred for 6 h under nitrogen protection. Water was added, and the obtained mixture was extracted with ethyl acetate. The organic phases were combined, washed with saturated sodium chloride aqueous solution, dried over anhydrous sodium sulfate, concentrated under reduced pressure, and purified by silica gel column chromatography to obtain compound 2,6-dichloro-3-fluoro-5-iodopyridin-4-amine (3.8 g, yield 93.4%).

MS m/z (ESI):307 [M+H].

### Step 10: Preparation of ethyl 4-amino-2,6-dichloro-5-fluoronicotinate

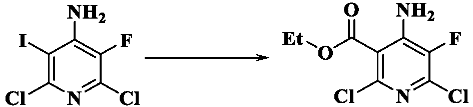

2,6-dichloro-3-fluoro-5-iodopyridin-4-amine (3.5 g, 11.40 mmol) was added to ethanol (20 mL), triethylamine (4.6 g, 45.46 mmol) and Pd(PPh₃)₂Cl₂ (1.6 g, 2.28 mmol) were added, and the obtained mixture was heated to 80°C and stirred for 16 h under carbon monoxide atmosphere (1.5 MPa). The mixture was cooled to room temperature, filtered, washed with ethanol, concentrated under reduced pressure, and purified by silica gel column chromatography to obtain compound ethyl 4-amino-2,6-dichloro-5-fluoronicotinate (2.1 g, yield 73.0%).

MS m/z (ESI):253[M+H].

### Step 11: Preparation of ethyl 2,6-dichloro-5-fluoro-4-(3-(2,2,2-trichloroacetyl) ureido)nicotinate

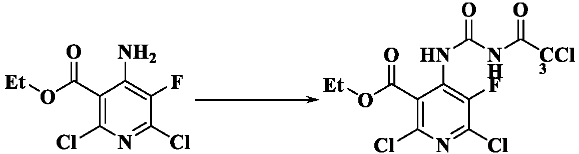

Ethyl 4-amino-2,6-dichloro-5-fluoronicotinate (2 g, 7.90 mmol) was added to THF (50 mL), and a solution of trichloroacetyl isocyanate (1.8 g, 9.55 mmol) in THF (10 mL) was added dropwise under ice bath. The obtained mixture was reacted at room temperature for 2 h, concentrated, and recrystallized to obtain compound ethyl 2,6-dichloro-5-fluoro-4-(3-(2,2,2-trichloroacetyl)ureido)nicotinate (2.9 g, yield 83.1%).

MS m/z (ESI):442 [M+H].

### Step 12: Preparation of 5,7-dichloro-8-fluoropyrido[4,3-d]pyrimidine-2,4-diphenol

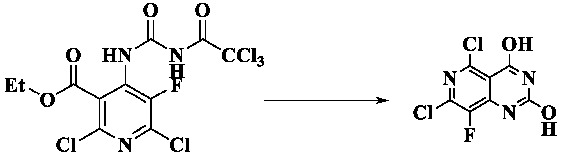

Ethyl 2,6-dichloro-5-fluoro-4-(3-(2,2,2-trichloroacetyl)ureido)nicotinate (2.7 g, 6.12 mmol) was added to methanol (10 mL), and a solution of ammonia in methanol (7 M, 10 mL) was added. The obtained mixture was reacted at room temperature for 1 h, concentrated under reduced pressure, washed with water, and slurried to obtain compound 5,7-dichloro-8-fluoropyrido[4,3-d]pyrimidine-2,4-diphenol (1.2 g, yield 78.4%).

MS m/z (ESI):250 [M+H].

### Step 13: Preparation of 2,4,5,7-tetrachloro-8-fluoropyrido[4,3-d]pyrimidine

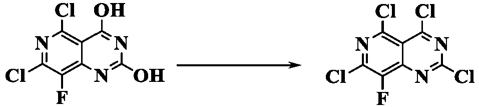

5,7-dichloro-8-fluoropyrido[4,3-d]pyrimidine-2,4-diphenol (1.1 g, 4.40 mmol) was added to POCl₃ (20 mL), the obtained mixture was heated to reflux for 16 h, and concentrated under reduced pressure to obtain a crude compound 2,4,5,7-tetrachloro-8-fluoropyrido[4,3-d]pyrimidine (1.4 g).

MS m/z (ESI):288 [M+H].

### Step 14: Preparation of tert-butyl (1S,2S,5R)-2-(((tert-butyldiphenylsilyl) oxo)methyl)-3-(2,5,7-trichloro-8-fluoropyrido[4,3-d]pyrimidin-4-yl)-3,8-diazabicyclo[3.2.1]octane-8-carboxylate

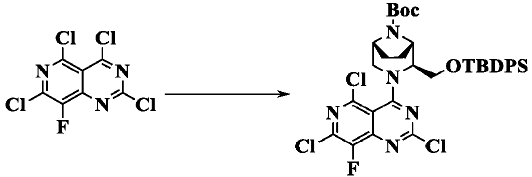

2,4,5,7-tetrachloro-8-fluoropyrido[4,3-d]pyrimidine (1.3 g) was added to dichloromethane (20 mL), and tert-butyl (1S,2S,5R)-2-(((tert-butyldiphenylsilyl)oxo)methyl)-3,8-diazabicyclo[3.2.1]octane-8-carboxylate (2.4 g, 4.99 mmol) and triethylamine (1.4 g, 13.84 mmol) were added. The obtained mixture was reacted at room temperature for 3 h. Water was added and the obtained mixture was extracted with dichloromethane. The organic phases were combined, washed with saturated sodium chloride aqueous solution, dried over anhydrous sodium sulfate, concentrated under reduced pressure, and purified by silica gel column chromatography to obtain compound tert-butyl (1S,2S,5R)-2-(((tert-butyldiphenylsilyl)oxo)methyl)-3-(2,5,7-trichloro-8-fluoropyrido[4,3-d]pyrimidin-4-yl)-3,8-diazabicyclo[3.2.1]octane-8-carboxylate (2.8 g).

### Step 15: Preparation of tert-butyl (1S,2S,5R)-2-(((tert-butyldiphenylsilyl)oxo)methyl)-3-(2-((1-(((tert-butyldiphenylsilyl)oxo)methyl) cyclopropyl)methoxy)-5,7-dichloro-8-fluoropyrido[4,3-d]pyrimidin-4-yl)-3,8-diazabicyclo[3.2.1]octane-8-carboxylate

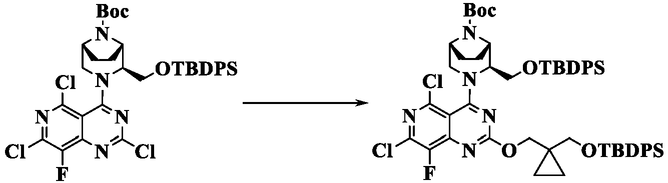

NaH (27.68 mg, 692.05 µmol, 60% purity) was added to a solution of tert-butyl (1S,2S,SR)-2-(((tert-butyldiphenylsilyl)oxo)methyl)-3-(2,5,7-trichloro-8-fluoropyrido[4,3-d]pyrimidin-4-yl)-3,8-diazabicyclo[3.2.1]octane-8-carboxylate and [1-[[tert-butyl(diphenyl)silyl]oxymethyl]cyclopropyl]methanol (235.67 mg, 692.05 µmol) in THF (15 mL) at 0°C, and the obtained mixture was warmed to room temperature and stirred for 15 h after the addition was completed. The reaction was quenched with ammonium chloride aqueous solution, and the obtained mixture was extracted with ethyl acetate. The organic phase was washed with saturated sodium chloride aqueous solution, dried over anhydrous sodium sulfate, and concentrated to obtain tert-butyl (1S,2S,5R)-2-(((tert-butyldiphenylsilyl)oxo)methyl)-3-(2-((1-(((tert-butyldiphenylsilyl)oxo)methyl)cyclopropyl)methoxy)-5,7-dichloro-8-fluoropyrido[4,3-d]pyrimidin-4-yl)-3,8-diazabicyclo[3.2.1]octane-8-carboxylate (622 mg, yield 99.8%).

### Step 16: Preparation of tert-butyl (5aS,6S,9R)-2-chloro-1-fluoro-12-((1-(hydroxymethyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate

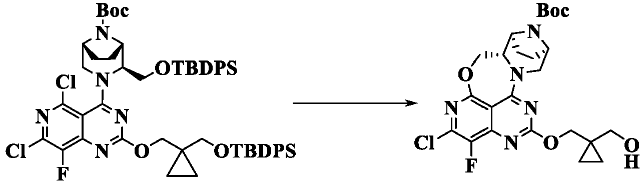

TBAF (1 M, 3.61 mL) was added to a solution of tert-butyl (1S,2S,5R)-2-(((tert-butyldiphenylsilyl)oxo)methyl)-3-(2-((1-(((tert-butyldiphenylsilyl) oxo)methyl)cyclopropyl)methoxy)-5,7-dichloro-8-fluoropyrido[4,3-d]pyrimidin-4-yl)-3,8-diazabicyclo[3.2.1]octane-8-carboxylate (622 mg, 600.83 µmol) in THF (20 mL) at room temperature, and the obtained mixture was stirred at room temperature for 1 h. Water was added, and the obtained mixture was extracted with ethyl acetate. The organic phase was washed with saturated sodium chloride aqueous solution, dried over anhydrous sodium sulfate, concentrated, and then purified by silica gel column chromatography to obtain tert-butyl (5aS,6S,9R)-2-chloro-1-fluoro-12-((1-(hydroxymethyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate (210 mg, yield 66.9%).

MS m/z (ESI):522 [M+H].

### Step 17: Preparation of tert-butyl (5aS,6S,9R)-1-fluoro-2-(7-fluoro-3-(methoxymethoxy)-8-((triisopropylsilyl)ethynyl)naphthalen-1-yl)-12-((1-(hydroxymethyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate

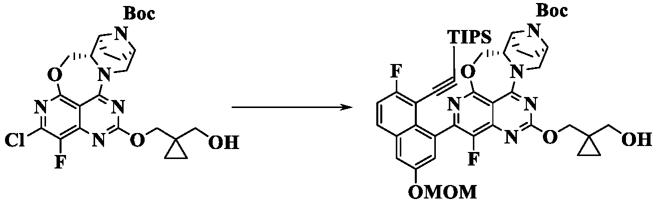

Tert-butyl (5aS,6S,9R)-2-chloro-1-fluoro-12-((1-(hydroxymethyl)cyclopropyl) methoxy)-5a,6,7,8,9, 10-hexahydro-5H-4-oxa-3, 10a, 11, 13, 14-pentaaza-6,9-methylenenaphtho[1,8-ab]heptalene-14-carboxylate (210 mg, 402.32 µmol), 2-[2-fluoro-6-(methoxymethoxy)-8-(4,4,5,5-tetramethyl-1,3,2-dioxaborolan-2-yl)-1-naphthyl]ethynyl-triisopropyl-silane (14.65 mg, 20.12 µmol) and Cs₂CO₃ (262.17 mg, 804.65 µmol) were added to dioxane (5 mL), and the obtained mixture was heated to 100°C and stirred for 13 h under nitrogen protection. Water was added, and the obtained mixture was extracted with ethyl acetate. The organic phase was washed with saturated sodium chloride aqueous solution, dried over anhydrous sodium sulfate, and concentrated to obtain tert-butyl (5aS,6S,9R)-1-fluoro-2-(7-fluoro-3-(methoxymethoxy)-8-((triisopropylsilyl)ethynyl)naphthalen-1-yl)-12-((1-(hydroxymethyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate (350 mg, yield 99.8%).

### Step 18: Preparation of tert-butyl (5aS,6S,9R)-2-(8-ethynyl-7-fluoro-3-(methoxymethoxy)naphthalen-1-yl)-1-fluoro-12-((1-(hydroxymethyl)cyclopropyl) methoxy)-5a,6,7,8,9, 10-hexahydro-5H-4-oxa-3, 10a, 11, 13, 14-pentaaza-6,9-methylenenaphtho[1,8-ab]heptalene-14-carboxylate

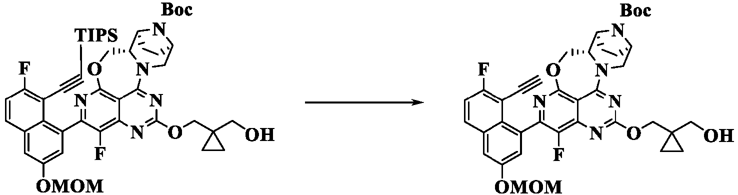

TBAF (1 M, 1.20 mL) was added to a solution of tert-butyl (5aS,6S,9R)-1-fluoro-2-(7-fluoro-3-(methoxymethoxy)-8-((triisopropylsilyl)ethynyl)naphthalen-1-yl)-12-((1-(hydroxymethyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate (350 mg, 401.34 µmol) in THF (10 mL) at room temperature, and the obtained mixture was stirred at room temperature for 1 h after the addition was completed. Water was added, and the obtained mixture was extracted with ethyl acetate. The organic phase was washed with saturated sodium chloride aqueous solution, dried over anhydrous sodium sulfate, concentrated, and then purified by preparative silica gel plate to obtain tert-butyl (5aS,6S,9R)-2-(8-ethynyl-7-fluoro-3-(methoxymethoxy)naphthalen-1-yl)-1-fluoro-12-((1-(hydroxymethyl)cyclopropyl) methoxy)-5a,6,7,8,9,10-hexahydro-5H-4-oxa-3,10a,11,13,14-pentaaza-6,9-methylenenaphtho[1,8-ab]heptalene-14-carboxylate (120 mg, yield 41.8%).

MS m/z (ESI): 716 [M+H].

### Step 19: Preparation of tert-butyl (5aS,6S,9R)-2-(8-ethynyl-7-fluoro-3-(methoxymethoxy)naphthyl-1-yl)-1-fluoro-12-((1-(((methylsulfonyl)oxo)methyl)cyclopropyl)methoxy) -5a,6,7,8,9,10-hexahydro-5H-4-oxa-3,10a,11,13,14-pentaaza-6,9-methylenenaphtho[1,8-ab]heptalene-14-carboxylate

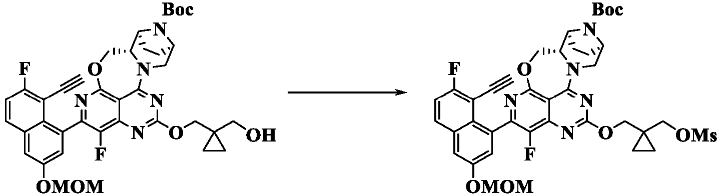

Triethylamine (42.41 mg, 419.15 µmol, 58.46 µL) was added to a solution of tert-butyl (5aS,6S,9R)-2-(8-ethynyl-7-fluoro-3-(methoxymethoxy)naphthalen-1-yl)-1-fluoro-12-((1-(hydroxymethyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate (100 mg, 139.72 µmol) in dichloromethane (15 mL) at 0°C, then methanesulfonyl chloride (32.01 mg, 279.43 µmol) was added, and the obtained mixture was stirred at 0°C for 30 min. Aqueous sodium bicarbonate solution was added, and the obtained mixture was extracted with dichloromethane. The organic phase was washed with ammonium chloride aqueous solution, then washed with saturated sodium chloride aqueous solution, dried over anhydrous sodium sulfate, and concentrated to obtain tert-butyl (5aS,6S,9R)-2-(8-ethynyl-7-fluoro-3-(methoxymethoxy)naphthyl-1-yl)-1-fluoro-12-((1-(((methylsulfonyl)oxo) methyl)cyclopropyl)methoxy) -5a,6,7,8,9,10-hexahydro-5H-4-oxa-3,10a,11,13,14-pentaaza-6,9-methylenenaphtho[1,8-ab]heptalene-14-carboxylate (110 mg, yield 99.2%)

MS m/z (ESI):794 [M+H].

### Step 20: Preparation of tert-butyl (5aS,6S,9R)-12-((1-((4-(difluoromethylene) piperidin-1-yl)methyl)cyclopropyl)methoxy)-2-(8-ethynyl-7-fluoro-3-(methoxymethoxy)naphthalen-1-yl)-1-fluoro-5a,6,7,8,9,10-hexahydro-5H-4-oxa-3,10a,11,13,14-pentaaza-6,9-methylenenaphtho[1,8-ab]heptalene-14-carboxylate

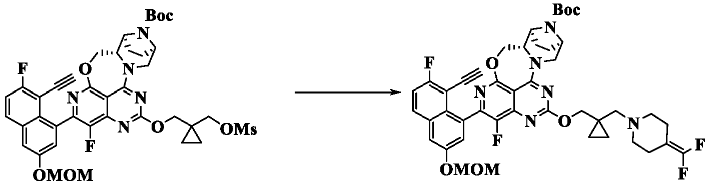

Tert-butyl (5aS,6S,9R)-2-(8-ethynyl-7-fluoro-3-(methoxymethoxy)naphthyl-1-yl)-1-fluoro-12-((1-(((methylsulfonyl)oxo)methyl)cyclopropyl)methoxy) - 5a,6,7,8,9,10-hexahydro-5H-4-oxa-3,10a,11,13,14-pentaaza-6,9-methylenenaphtho[1,8-ab]heptalene-14-carboxylate (110 mg, 138.57 µmol), 4-(difluoromethylene)piperidine (55.35 mg, 415.71 µmol), DIPEA (179.09 mg, 1.39 mmol) and NaI (62.31 mg, 415.71 µmol) were added to acetonitrile (8 mL), and the obtained mixture was heated to 50°C and stirred for 13 h. Ammonium chloride aqueous solution was added to the reaction liquid, and the obtained mixture was extracted with ethyl acetate. The organic phase was washed with saturated sodium chloride aqueous solution, dried over anhydrous sodium sulfate, concentrated, and then purified by preparative silica gel plate to obtain tert-butyl (5aS,6S,9R)-12-((1-((4-(difluoromethylene)piperidin-1-yl)methyl)cyclopropyl)methoxy)-2-(8-ethynyl-7-fluoro-3-(methoxymethoxy)naphthalen-1-yl)-1-fluoro-5a,6,7,8,9,10-hexahydro-5H-4-oxa-3,10a,11,13,14-pentaaza-6,9-methylenenaphtho[1,8-ab]heptalene-14-carboxylate (70 mg, yield 60.8%) as yellow solid.

MS m/z (ESI): 831 [M+H].

### Step 21: Preparation of 4-((5aS,6S,9R)-12-((1-((4-(difluoromethylene) piperidin-1-yl)methyl)cyclopropyl)methoxy)-1-fluoro-5a,6,7,8,9,10-hexahydro-5H-4-oxa-3,10a,11,13,14-pentaaza-6,9-methylenenaphtho[1,8-ab]heptalene-2-yl)-5-ethynyl-6-fluoronaphthalen-2-ol

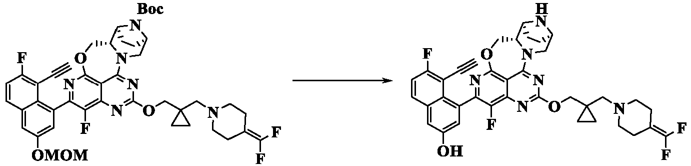

HCl (3.07 mg, 84.25 µmol, 3 mL) was added to a solution of tert-butyl (SaS,6S,9R)-12-((1-((4-(difluoromethylene)piperidin-1-yl)methyl)cyclopropyl) methoxy)-2-(8-ethynyl-7-fluoro-3-(methoxymethoxy)naphthalen-1-yl)-1-fluoro-5a,6,7,8,9,10-hexahydro-5H-4-oxa-3,10a,11,13,14-pentaaza-6,9-methylenenaphtho[1,8-ab]heptalene-14-carboxylate (70 mg, 84.25 µmol) in dichloromethane (3 mL) and methanol (0.5 mL) at 0°C. After the addition was completed, the obtained mixture was stirred at room temperature for 30 min, and the reaction liquid was slowly added to an sodium bicarbonate aqueous solution, and the obtained mixture was extracted with dichloromethane/methanol, and the organic phase was washed with saturated sodium chloride aqueous solution, dried over anhydrous sodium sulfate, concentrated, and purified by preparative HPLC to obtain 4-((5aS,6S,9R)-12-((1-((4-(difluoromethylene)piperidin-1-yl)methyl)cyclopropyl) methoxy)-1-fluoro-5a,6,7,8,9,10-hexahydro-5H-4-oxa-3,10a,11,13,14-pentaaza-6,9-methylenenaphtho[1,8-ab]heptalen-2-yl)-5-ethynyl-6-fluoronaphthalen-2-ol (30 mg, yield 51.8%).

MS m/z (ESI): 687 [M+H].

**The examples in the following table refer to the synthesis of example 1-1:**

| **Example compounds and stereoisomer structures thereof** | **MS m/z (ESI)** |
|---|---|
| 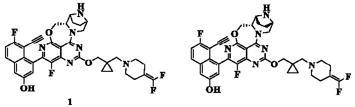 | 687 [M+H] |
| 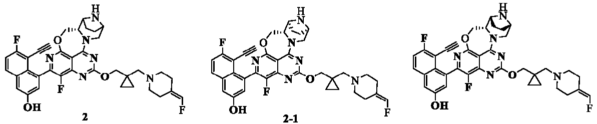 | 669 [M+H] |
| **Example 2-1:** ¹H NMR (400 MHz, MeOD) δ 7.85 (dt, *J =* 9.2, 4.2 Hz, 1H), 7.35 - 7.27 (m, 2H), 7.27 - 7.17 (m, 1H), 6.46 (d, *J* = 82.4 Hz, 1H), 5.05 (t, *J =* 12.1 Hz, 1H), 4.67 - 4.32 (m, 5H), 4.15 (s, 1H), 3.83 - 3.68 (m, 2H), 3.05 (s, 3H), 2.95 - 2.76 (m, 4H), 2.37 (s, 2H), 2.08 - 1.79 (m, 6H), 0.86 - 0.65 (m, 4H). | |
| 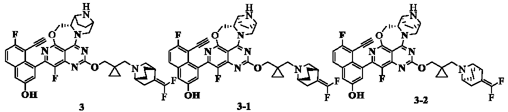 | 713 [M+H] |
| 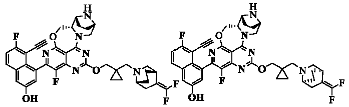 | |
| **Example 3-1:** ¹H NMR(400 MHz, MeOD) δ 7.85(dt, *J* = 9.2, 4.9 Hz, 1H), 7.36 - 7.26(m, 2H), 7.25 - 7.14(m, 1H), 5.03(t, *J =* 11.5 Hz, 1H), 4.69 - 4.30(m, 4H), 4.16(d, *J* = 7.0 Hz, 1H), 3.76(d, *J =* 23.7 Hz, 2H), 3.19(d, *J =* 46.7 Hz, 4H), 2.90(s, 2H), 2.71(s, 1H), 2.49(d, *J* = 18.1 Hz, 1H), 2.24 - 1.53(m, 10H), 0.95 - 0.74(m, 4H). | |
| 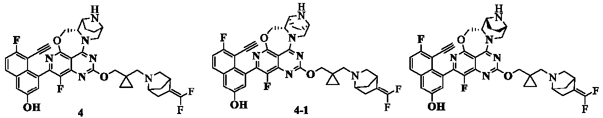 | 699 [M+H] |
| **Example 4-1:** ¹H NMR (400 MHz, MeOD) δ 7.87 - 7.83 (m, 1H), 7.37 - 7.28 (m, 2H), 7.24 - 7.14 (m, 1H), 5.03 (t, J = 11.5 Hz, 1H), 4.69 - 4.30 (m, 4H), 4.16 (d, J = 7.0 Hz, 1H), 3.76 (d, J = 23.7 Hz, 2H), 3.19 (d, J = 46.7 Hz, 4H), 2.88 (s, 2H), 2.75 (s, 1H), 2.50 - 2.46 (m, 1H), 2.24 - 1.53 (m, 8H), 0.95 - 0.74 (m, 4H). | |
| 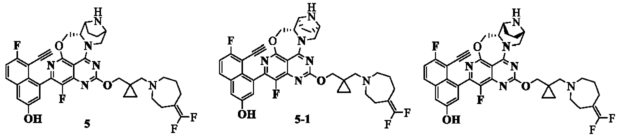 | 701 [M+H] |
| **Example 5-1:** ¹H NMR (400 MHz, MeOD) δ 7.84 (dt, *J =* 9.2, 4.9 Hz, 1H), 7.36 - 7.27 (m, 2H), 7.27 - 7.14 (m, 1H), 5.10 - 5.04 (m, 1H), 4.65 - 4.05 (m, 6H), 3.98 (d, *J* = 5.7 Hz, 1H), 3.91 - 3.86 (m, 1H), 3.81 - 3.65 (m, 3H), 2.99 - 2.82 (m, 4H), 2.39 - 1.68 (m, 10H), 0.87 - 0.63 (m, 4H). | |
| 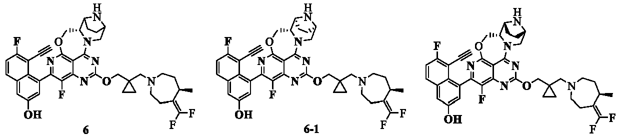 | 715 [M+H] |
| **Example 6-1:** ¹H NMR (400 MHz, MeOD) δ 7.85 - 7.77 (m, 1H), 7.36 - 7.27 (m, 2H), 7.26 - 7.15 (m, 1H), 5.10 - 5.04 (m, 1H), 4.65 - 4.05 (m, 6H), 3.98 (d, *J =* 5.7 Hz, 1H), 3.91 - 3.86 (m, 1H), 3.81 - 3.65 (m, 3H), 2.99 - 2.82 (m, 4H), 2.37 - 1.72 (m, 9H), 0.90 - 0.64 (m, 7H). | |
| 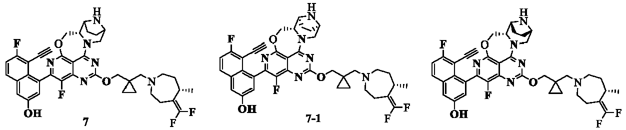 | 715 [M+H] |
| **Example 7-1:** ¹H NMR (400 MHz, MeOD) δ 7.84 - 7.76 (m, 1H), 7.35 - 7.26 (m, 2H), 7.26 - 7.15 (m, 1H), 5.03 (t, *J =* 11.9 Hz, 1H), 4.65 - 4.05 (m, 6H), 3.98 (d, *J* = 5.7 Hz, 1H), 3.91 - 3.86 (m, 1H), 3.81 - 3.65 (m, 3H), 3.02 - 2.85 (m, 4H), 2.35 - 1.64 (m, 9H), 0.87 - 0.60 (m, 7H). | |
| 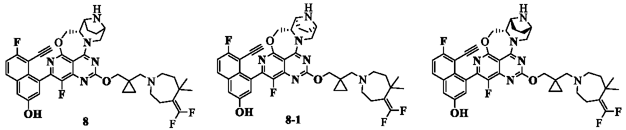 | 729 [M+H] |
| **Example 8-1:** ¹H NMR (400 MHz, MeOD) δ 7.82 - 7.77 (m, 1H), 7.36 - 7.27 (m, 2H), 7.26 - 7.15 (m, 1H), 5.02 (t, *J =* 12.0 Hz, 1H), 4.65 - 4.05 (m, 6H), 3.98 (d, *J* = 5.7 Hz, 1H), 3.91 - 3.86 (m, 1H), 3.81 - 3.65 (m, 3H), 3.02 - 2.85 (m, 4H), 2.35 - 1.64 (m, 8H), 1.02 (s, 6H), 0.90 - 0.64 (m, 4H). | |
| 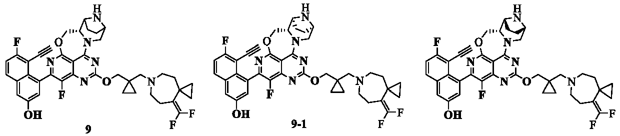 | 727 [M+H] |
| **Example 9-1:** ¹H NMR (400 MHz, MeOD) δ7.83 (dt, *J* = 9.2, 4.9 Hz, 1H), 7.35 - 7.26 (m, 2H), 7.25 - 7.16 (m, 1H), 5.06 - 5.00 (m, 1H), 4.60 - 4.07 (m, 6H), 3.98 - 3.81 (m, 2H), 3.81 - 3.65 (m, 3H), 3.02 - 2.85 (m, 4H), 2.35 - 1.64 (m, 8H), 0.88 - 0.66 (m, 8H). | |
| 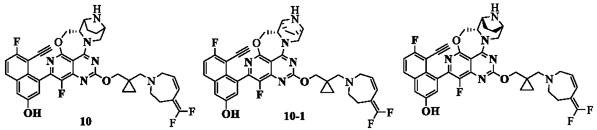 | 699 [M+H] |
| **Example 10-1:** ¹H NMR (400 MHz, MeOD) δ 7.88 - 7.76 (m, 1H), 7.37 - 7.26 (m, 2H), 7.25 - 7.15 (m, 1H), 6.16 (d, *J* = 11.9 Hz, 1H), 5.70 - 5.57 (m, 1H) 5.05 (t, *J =* 12.1 Hz, 1H), 4.65 - 4.05 (m, 6H), 3.98 (d, *J=* 5.7 Hz, 1H), 3.91 - 3.86 (m, 1H), 3.81 - 3.65 (m, 3H), 2.99 - 2.82 (m, 4H), 2.39 - 1.68 (m, 6H), 0.87 - 0.63 (m, 4H). | |
| 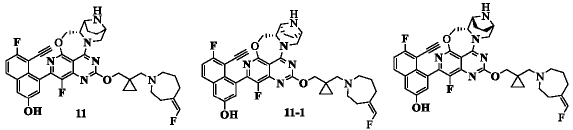 | 683 [M+H] |
| **Example 11-1:** ¹H NMR (400 MHz, MeOD) δ 7.82 (dt, *J* = 9.2, 4.7 Hz, 1H), 7.38 - 7.28 (m, 2H), 7.26 - 7.15 (m, 1H), 6.29 (d, *J* = 76.8 Hz, 1H), 5.10 - 5.04 (m, 1H), 4.65 - 4.06 (m, 6H), 3.98 (d, *J* = 5.7 Hz, 1H), 3.91 - 3.86 (m, 1H), 3.81 - 3.65 (m, 3H), 3.02 - 2.82 (m, 4H), 2.41 - 1.69 (m, 10H), 0.88 - 0.62 (m, 4H). | |
| 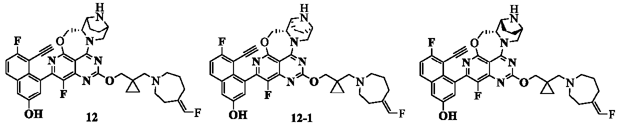 | 683 [M+H] |
| **Example 12-1:** ¹H NMR (400 MHz, MeOD) δ 7.83 (dt, *J =* 9.2, 4.7 Hz, 1H), 7.39 - 7.27 (m, 2H), 7.24 - 7.13 (m, 1H), 6.30 (d, *J =* 78.4 Hz, 1H), 5.11 - 5.05 (m, 1H), 4.66 - 4.04 (m, 6H), 3.97 (d, *J* = 5.6 Hz, 1H), 3.92 - 3.86 (m, 1H), 3.82 - 3.65 (m, 3H), 3.03 - 2.82 (m, 4H), 2.40 - 1.69 (m, 10H), 0.89 - 0.63 (m, 4H). | |
| 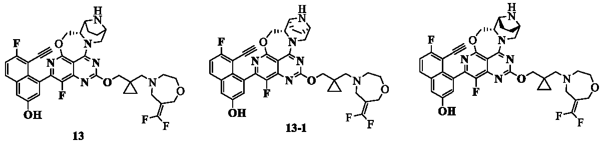 | 703 [M+H] |
| **Example 13-1:** ¹H NMR (400 MHz, MeOD) δ 7.86 - 7.77 (m, 1H), 7.38 - 7.26 (m, 2H), 7.26 - 7.14 (m, 1H), 5.12 - 5.05 (m, 1H), 4.87 - 4.20 (m, 6H), 3.92 - 3.78 (m, 2H), 3.57 - 2.99 (m, 7H), 2.82 - 1.69 (m, 8H), 0.88 - 0.62 (m, 4H). | |
| 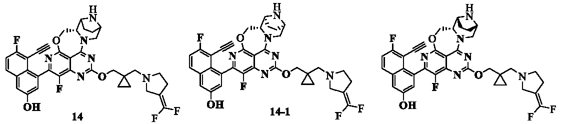 | 673 [M+H] |
| **Example 14-1:** ¹H NMR (400 MHz, MeOD) δ 7.75 (ddd, *J* = 9.1, 5.7, 3.5 Hz, 1H), 7.28 - 7.12 (m, 3H), 5.00 (ddd, *J* = 13.9, 6.9, 2.4 Hz, 1H), 4.58 - 4.50 (m, 1H), 4.43 - 4.25 (m, 3H), 4.08 (dt, *J* = 7.3, 2.3 Hz, 1H), 3.80 - 3.67 (m, 2H), 3.44 (d, *J* = 35.5 Hz, 1H), 3.27 (s, 1H), 3.18 (d, *J* = 3.3 Hz, 1H), 2.80 - 2.53 (m, 4H), 2.38 (d, *J* = 7.9 Hz, 2H), 1.84 (ddq, *J* = 22.4, 14.9, 8.9 Hz, 4H), 1.22 (d, *J =* 17.2 Hz, 1H), 0.66 (d, *J =* 2.0 Hz, 2H), 0.50 (s, 2H). | |
| 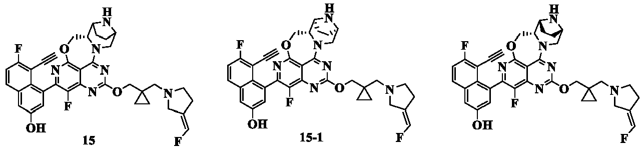 | 655 [M+H] |
| **Example 15-1:** ¹H NMR (500 MHz, Chloroform-d) δ 7.76 (ddd, *J* = 8.1, 3.8, 2.0 Hz, 1H), 7.68 (s, 1H), 7.43 (d, *J* = 2.2 Hz, 1H), 7.40 (t, *J* = 2.2 Hz, 1H), 7.31 - 7.15 (m, 2H), 4.46 (dd, *J* = 12.7, 5.8 Hz, 1H), 4.17 (dd, *J* = 12.8, 6.4 Hz, 1H), 4.14 - 4.05 (m, 4H), 3.77 (td, *J* = 6.2, 4.9 Hz, 1H), 3.48 - 3.43 (m, 1H), 3.35 - 3.29 (m, 2H), 3.23 - 3.19 (m, 1H), 2.97 (dd, *J* = 2.9, 0.8 Hz, 2H), 2.80 (t, *J =* 2.5 Hz, 2H), 2.67 (s, 2H), 2.43 (qd, *J =* 2.8, 0.9 Hz, 2H), 2.26 - 2.19 (m, 2H), 2.00 - 1.95 (m, 2H), 1.94 - 1.88 (m, 2H), 1.73-1.58 (m, 2H). | |
| 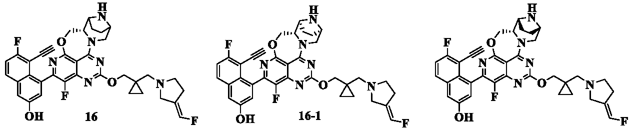 | 655 [M+H] |
| **Example 16-1:** ¹H NMR (400 MHz, MeOD) δ 7.88 - 7.76 (m, 1H), 7.36 - 7.29 (m, 2H), 7.13 (d, *J* = 2.6 Hz, 1H), 6.41 (d, *J* = 78.2 Hz, 1H), 5.34 (d, *J* = 13.2 Hz, 1H), 4.42 - 4.35 (m, 3H), 4.02 - 3.92 (m, 2H), 3.72 - 3.67 (m, 1H), 3.63 - 3.57 (m, 1H), 3.21 - 3.13 (m, 2H), 2.92 - 2.66 (m, 4H), 2.62 - 2.36 (m, 4H), 2.27 - 2.12 (m, 2H), 1.97 - 1.79 (m, 2H), 0.78 - 0.68 (m, 4H). | |
| 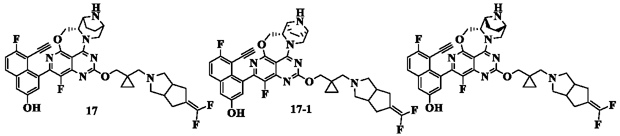 | 713 [M+H] |
| **Example 17-1:** ¹H NMR (400 MHz, MeOD) δ 7.86 - 7.79 (m, 1H), 7.34 - 7.26 (m, 2H), 7.18 (d, J = 2.6 Hz, 1H), 5.41 (d, J = 13.2 Hz, 1H), 4.56 - 4.35 (m, 3H), 4.02 (d, J = 8.8 Hz, 1H), 3.71 - 3.65 (m, 2H), 3.57 (d, J = 4.8 Hz, 1H), 3.24 - 3.16 (m, 1H), 2.89 - 2.62 (m, 4H), 2.58 - 2.37 (m, 4H), 2.32 - 2.21 (m, 2H), 2.15 - 2.04 (m, 3H), 1.86 - 1.79 (m, 2H), 1.33 - 1.27 (m, 2H), 0.72 - 0.51 (m, 4H). | |
| 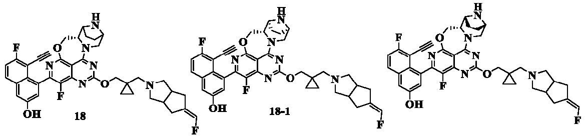 | 695 [M+H] |
| **Example 18-1:** ¹H NMR (400 MHz, MeOD) δ 7.86 - 7.79 (m, 1H), 7.34 - 7.26 (m, 2H), 7.18 (d, *J* = 2.6 Hz, 1H), 6.29 (d, *J* = 78.2 Hz, 1H), 5.41 (d, *J =* 13.2 Hz, 1H), 4.56 - 4.35 (m, 3H), 4.02 (d, *J =* 8.8 Hz, 1H), 3.71 - 3.65 (m, 2H), 3.57 (d, *J =* 4.8 Hz, 1H), 3.24 - 3.16 (m, 1H), 2.89 - 2.62 (m, 4H), 2.58 - 2.37 (m, 4H), 2.32 - 2.21 (m, 2H), 2.15 - 2.04 (m, 3H), 1.86 - 1.79 (m, 2H), 1.33 - 1.27 (m, 2H), 0.72 - 0.51 (m, 4H). | |
| 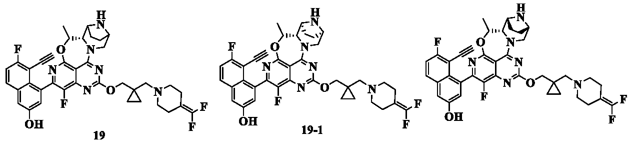 | 701 [M+H] |
| 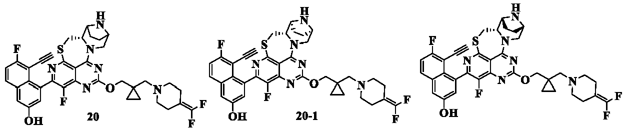 | 703 [M+H] |
| 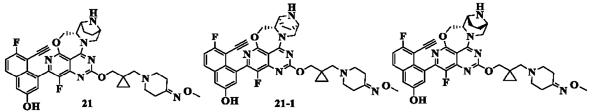 | 682 [M+H] |

**The following synthesis methods can also be adopted for the following examples:**

### Example 19-1

### Step 1: Preparation of 3-benzyl 8-tert-butyl (1S,2S,5R)-2-(hydroxymethyl)-3,8-diazabicyclo[3.2.1]octane-3,8-dicarboxylate

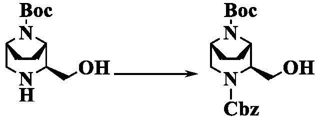

Tert-butyl (1S,2S,5R)-2-(hydroxymethyl)-3,8-diazabicyclo[3.2.1]octane-8-carboxylate (25 g, 103.17 mmol) was added to ethyl acetate (100 mL) and water (100 mL), and sodium bicarbonate (17.33 g, 206.34 mmol) and benzyl chloroformate (22.88 g, 134.12 mmol) were added. The mixture was stirred at room temperature for 4 h, layer separation was performed, and the obtained mixture was extracted with ethyl acetate, and the organic phases were combined, dried over anhydrous sodium sulfate, filtered, concentrated under reduced pressure, and purified by silica gel column chromatography to obtain compound 3-benzyl 8-tert-butyl (1S,2S,5R)-2-(hydroxymethyl)-3,8-diazabicyclo[3.2.1]octane-3,8-dicarboxylate (26 g, yield 66.9%).

MS m/z (ESI):377[M+H].

### Step 2: Preparation of 3-benzyl 8-tert-butyl (1S,2S,5R)-2-formyl-3,8-diazabicyclo[3.2.1]octane-3,8-dicarboxylate

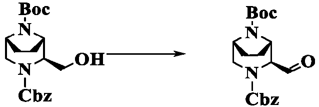

3-benzyl 8-tert-butyl (1S,2S,SR)-2-(hydroxymethyl)-3,8-diazabicyclo [3.2.1]octane-3,8-dicarboxylate (24.3 g, 64.55 mmol) and 2,2,6,6-tetramethylpiperidine oxide (1.0 g, 6.46 mmol) were added to dichloromethane (240 mL) and water (240 mL), sodium bicarbonate (21.69 g, 258.20 mmol) was added, and the obtained mixture was cooled to 0°C. Sodium hypochlorite solution (1.3 M, 148.96 mL, 258.20 mmol) was added dropwise at 0-10°C, and the obtained mixture was stirred at 0-10°C for 10 min. Layer separation was performed, and the aqueous phase was extracted with dichloromethane. The organic phases were combined, washed with saturated sodium chloride aqueous solution, dried over anhydrous sodium sulfate, filtered, concentrated under reduced pressure, and purified by silica gel column chromatography to obtain compound 3-benzyl 8-tert-butyl (1S,2S,SR)-2-formyl-3,8-diazabicyclo[3.2.1]octane-3,8-dicarboxylate (19 g, yield 78.6%).

MS m/z (ESI):375[M+H].

### Step 3: Preparation of 3-benzyl 8-tert-butyl (1S,2S,5R)-2-[(1S)-1-hydroxyethyl]-3,8-diazabicyclo[3.2.1]octane-3,8-dicarboxylate

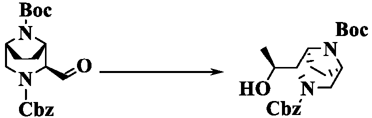

3-benzyl 8-tert-butyl (1S,2S,5R)-2-formyl-3,8-diazabicyclo[3.2.1]octane-3,8-dicarboxylate (19 g, 50.74 mmol) was added to tetrahydrofuran (190 mL), and methylmagnesium bromide (3 M solution in tetrahydrofuran, 21.99 mL) was added dropwise at -70°C under nitrogen protection. The obtained mixture was stirred at - 70°C for 2 h. Saturated ammonium chloride solution was added dropwise, and the separated aqueous layer was extracted with ethyl acetate. The organic layers were combined, dried over anhydrous sodium sulfate, filtered, concentrated under reduced pressure, and purified by silica gel column chromatography to obtain compound 3-benzyl 8-tert-butyl (1S,2S,5R)-2-[(15)-1-hydroxyethyl]-3,8-diazabicyclo[3.2.1]octane-3,8-dicarboxylate (16 g, yield 80.8%).

MS m/z (ESI):391 [M+H].

### Step 4: Preparation of tert-butyl (1S,2S,5R)-2-[(1S)-1-hydroxyethyl]-3,8-diazabicyclo[3.2.1]octane-8-carboxylate

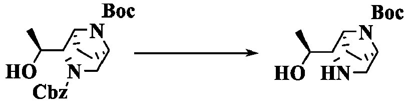

3-benzyl 8-tert-butyl (1S,2S,5R)-2-[(1S)-1-hydroxyethyl]-3,8-diazabicyclo [3.2.1]octane-3,8-dicarboxylate (60 g, 153.66 mmol) and 10% palladium on carbon (6.5 g, 61.46 mmol) were added to methanol (480 mL), and the obtained mixture was stirred for 4 h under a hydrogen atmosphere (0.34 MPa). The obtained mixture was filtered, washed with methanol, concentrated under reduced pressure, and purified by silica gel column chromatography to obtain compound tert-butyl (1S,2S,5R)-2-[(1S)-1-hydroxyethyl]-3,8-diazabicyclo[3.2.1]octane-8-carboxylate (36.6 g, yield 92.9%).

MS m/z (ESI):257[M+H].

### Step 5: Preparation of tert-butyl (1S,2S,5R)-2-[(S)-1-[tert-butyl(dimethyl) silyl]oxyethyl]-3,8-diazabicyclo[3.2.1]octane-8-carboxylate

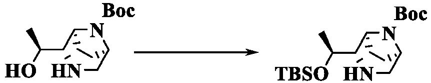

Tert-butyl (1S,2S,SR)-2-[(1S)-1-hydroxyethyl]-3,8-diazabicyclo[3.2.1]octane-8-carboxylate (40 g, 156.04 mmol) and imidazole (21.25 g, 312.09 mmol) were added to N,N-dimethylformamide (320 mL), and tert-butyldimethylchlorosilane (58.80 g, 390.11 mmol) was added under ice bath. The obtained mixture was reacted at room temperature for 40 h. Water and ethyl acetate were added, layer separation was performed, the aqueous phase was extracted with ethyl acetate, the organic layers were combined, dried over anhydrous sodium sulfate, concentrated under reduced pressure, and purified by silica gel column chromatography to obtain compound tert-butyl (1S,2S,5R)-2-[(S)-1-[tert-butyl(dimethyl)silyl]oxyethyl]-3,8-diazabicyclo[3.2.1]octane-8-carboxylate (50.7 g, yield 87.7%).

MS m/z (ESI):371 [M+H].

### Step 6: Preparation of tert-butyl (1S,2S,SR)-2-((S)-1-((tert-butyldimethylsilyl) oxy)ethyl)-3-(2,5,7-trichloro-8-fluoropyrido[4,3-d]pyrimidin-4-yl)-3,8-diazabicyclo[3.2.1]octane-8-carboxylate

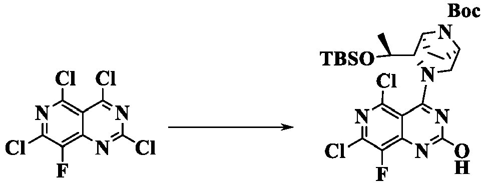

2,4,5,7-tetrachloro-8-fluoropyrido[4,3-d]pyrimidine (36.9 g, 128.61 mmol) and N,N-diisopropylethylamine (24.93 g, 192.92 mmol) were added to DCM (300 mL); a solution of tert-butyl (1S,2S,SR)-2-[(S)-1-[tert-butyl(dimethyl)silyl]oxyethyl]-3,8-diazabicyclo[3.2.1]octane-8-carboxylate (30.98 g, 83.60 mmol) in DCM (70 mL) was added dropwise at -65°C under nitrogen protection. Then the obtained mixture was stirred at room temperature for 16 h. Water was added, layer separation was performed, and the obtained mixture was extracted with dichloromethane, and the organic phases were combined, washed with saturated sodium chloride aqueous solution, dried over anhydrous sodium sulfate, filtered, concentrated under reduced pressure, and purified by silica gel column chromatography to obtain compound tert-butyl (1S,2S,SR)-2-((R)-1-((tertbutyldimethylsilyl)oxy)ethyl)-3-(2,5,7-trichloro-8-fluoropyrido[4,3-d]pyrimidin-4-yl)-3,8-diazabicyclo[3.2.1]octane-8-carboxylate (49.3 g, yield 61.7%).

MS m/z (ESI):602 [M+H].

### Step 7: Preparation of tert-butyl (5S,5aS,6S,9R)-2-chloro-1-fluoro-12-hydroxy-5-methyl-5a,6,7,8,9, 10-hexahydro-5H-4-oxa-3, 10a, 11, 13, 14-pentaaza-6,9-methylnaphtho[1,8-ab]heptene-4-carboxylate

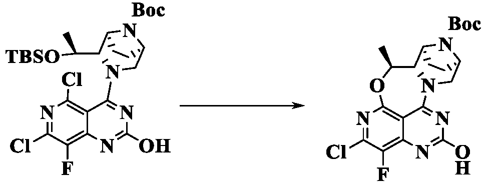

Tert-butyl (1S,2S,SR)-2-((R)-1-((tert-butyldimethylsilyl)oxy)ethyl)-3-(2,5,7-trichloro-8-fluoropyrido[4,3-d]pyrimidin-4-yl)-3,8-diazabicyclo[3.2.1]octane-8-carboxylate (53.4 g, 88.76 mmol) and cesium carbonate (168.08 g, 515.90 mmol) were added to N,N-dimethylformamide (250 mL), 1 M tetrabutylammonium fluoride solution in tetrahydrofuran (515.90 mL, 515.90 mmol) was added, and the obtained mixture was warmed to 65°C and stirred for 2 h after the addition was completed. The reaction was brought to room temperature, water was added, and the obtained mixture was extracted with ethyl acetate. The organic phase was washed with saturated sodium chloride aqueous solution, dried over anhydrous sodium sulfate, filtered, concentrated under reduced pressure, and purified by silica gel column chromatography to obtain tert-butyl (5S,5aS,6S,9R)-2-chloro-1-fluoro-12-hydroxy-5-Methyl-5a,6,7,8,9,10-hexahydro-5H-4-oxa-3,10a,11,13,14-pentaaza-6,9-methylnaphtho[1,8-ab]heptene-4-carboxylate (29.5 g, yield 73.6%).

MS m/z (ESI): 452 [M+H].

### Step 8: Preparation of tert-butyl (5S,5aS,6S,9R)-2,12-dichloro-1-fluoro-5-methyl-5a,6, 7,8,9, 10-hexahydro-5H-4-oxo-3, 10a, 11, 13, 14-pentaaza-6,9-methylnaphtho[1,8-ab]heptene-4-carboxylate

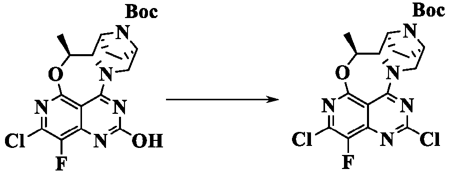

Tert-butyl (5S,5aS,6S,9R)-2-chloro-1-fluoro-12-hydroxy-5-methyl-5a,6,7,8,9,10-hexahydro-5H-4-oxa-3,10a,11,13,14-pentaaza-6,9-methylnaphtho [1,8-ab]heptene-4-carboxylate (29.5 g, 65.28 mmol) was dissolved in phosphorus oxychloride (180.18 g, 1.18 mol) at room temperature. Under nitrogen protection, N,N-diisopropylethylamine (16.87 g, 130.57 mmol) was added at 10-25°C, and then the obtained mixture was stirred at 100°C for 0.5 h. Concentration was performed to obtain a crude product, which was dissolved in ethyl acetate, the obtained mixture was added to saturated sodium bicarbonate to quench the reaction, and kept at pH = 8-9. Methanol and di-tert-butyl dicarbonate (28.50 g, 130.57 mmol) were added, and the obtained mixture was stirred at room temperature for 5 h. The layer separation was performed, and the obtained mixture was extracted with ethyl acetate. The organic phase was washed with saturated sodium chloride aqueous solution, dried over anhydrous sodium sulfate, filtered, concentrated, and then purified by silica gel column chromatography to obtain tert-butyl (5S,5aS,6S,9R)-2,12-dichloro-1-fluoro-5-methyl-5a,6,7,8,9,10-hexahydro-5H-4-oxo-3,10a,11,13,14-pentaaza-6,9-methylnaphtho[1,8-ab]heptene-4-carboxylate (20.5 g, yield 66.8%).

MS m/z (ESI):470 [M+H].

### Step 9: Preparation of tert-butyl (5S,5aS,6S,9R)-2-chloro-12-((1-((4-(difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methylnaphtho[1,8-ab]heptene-4-carboxylate

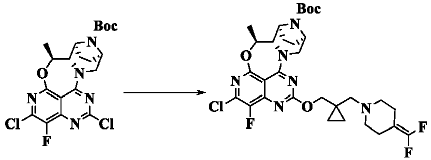

[1-[4-(difluoromethylene)cyclohexyloxy]cyclopropyl]methanol (16.17 g, 74.10 mmol) was added to tetrahydrofuran (100 mL), 60% NaH (3.35 g, 139.48 mmol) was added at 0-10°C under nitrogen protection, and the obtained mixture was stirred at 0-10°C for 0.5 h. A solution of tert-butyl (5S,5aS,6S,9R)-2,12-dichloro-1-fluoro-5-methyl-5a,6,7,8,9,10-hexahydro-5H-4-oxo-3,10a,11,13,14-pentaaza-6,9-methylnaphtho[1,8-ab]heptene-4-carboxylic acid (20.5 g, 43.59 mmol) in tetrahydrofuran (50 mL) was added, and then the obtained mixture was stirred at room temperature for 2 h. Filtration was performed, and tert-butyl alcohol was added to the filtrate to quench the reaction, and the obtained mixture was concentrated, and then purified by silica gel column chromatography to obtain tert-butyl (5S,5aS,6S,9R)-2-chloro-12-((1-((4-(difluoromethylene)piperidin-1-yl)methyl)cyclopropyl)methoxy)-1-fluoro-5-methyl-5a,6,7,8,9,10-hexahydro-5H-4-oxa-3, 10a, 11, 13,14-pentaaza-6,9-methylnaphtho[1,8-ab]heptene-4-carboxylate (22 g, yield 77.4%).

MS m/z (ESI):651 [M+H].

### Step 10: Preparation of tert-butyl (5S,5aS,6S,9R)-12-((1-((4-(difluoromethylene)piperidin-1-yl)methyl)cyclopropyl)methoxy)-1-fluoro-2-(7-fluoro-3-(methoxymethoxy]-8-(triisopropylsilyl)ethynyl)naphthalen-1-yl)-5-methyl-5a,6,7,8,9,10-hexahydro-5H-4-oxa-3,10a,11,13,14-pentaaza-6,9-methylnaphtho[1,8-ab]heptene-4-carboxylate

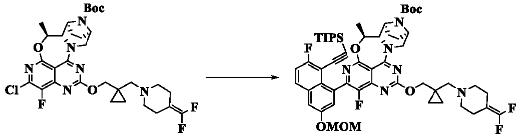

At room temperature, tetrahydrofuran (13 mL) and water (3 mL) were added to tert-butyl (5S,5aS,6S,9R)-2-chloro-12-((1-((4-(difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methylnaphtho[1,8-ab]heptene-4-carboxylate (1.35 g, 2.07 mmol), and potassium phosphate (2.20 g, 10.37 mmol) and methanesulfonic acid [n-butyldi(1-adamantyl)phosphine](2-amino-1,1'-biphenyl-2-yl)palladium(II) (301.99 mg, 414.67 µmol) were added, and the obtained mixture was stirred at 100°C under nitrogen protection under microwave for 30 min. Ethyl acetate was added for extraction, and the obtained mixture was washed with saturated sodium chloride aqueous solution, dried over anhydrous sodium sulfate, filtered, concentrated, and then purified by silica gel column chromatography to obtain tert-butyl (5S,5aS,6S,9R)-12-((1-((4-(difluoromethylene)piperidin-1-yl)methyl)cyclopropyl)methoxy)-1-fluoro-2-(7-fluoro-3-(methoxymethoxy]-8-(triisopropylsilyl)ethynyl)naphthalen-1-yl)-5-methyl-5a,6,7,8,9,10-hexahydro-5H-4-oxa-3,10a,11,13,14-pentaaza-6,9-methylnaphtho[1,8-ab]heptene-4-carboxylate (1.8 mg, yield 86.7%).

MS m/z (ESI): 1001 [M+H].

### Step 11: Preparation of tert-butyl (5S,5aS,6S,9R)-12-((1-((4-(difluoromethylene)piperidin-1-yl)methyl)cyclopropyl)methoxy)-2-(8-ethynyl-7-fluoro-3-(methoxymethoxynaphthalen-1-yl)-1-fluoro-5-methyl-5a,6,7,8,9,10-hexahydro-5H-4-oxa-3,10a,11,13,14-pentaaza-6,9-methylnaphtho[1,8-ab]heptene-4-carboxylate

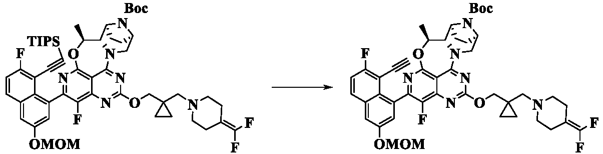

Tert-butyl (5S,5aS,6S,9R)-12-((1-((4-(difluoromethylene)piperidin-1-yl)methyl)cyclopropyl)methoxy)-1-fluoro-2-(7-fluoro-3-(methoxymethoxy]-8-(triisopropylsilyl)ethynyl)naphthalen-1-yl)-5-methyl-5a,6,7,8,9,10-hexahydro-5H-4-oxa-3, 10a, 11, 13,14-pentaaza-6,9-methylnaphtho[1,8-ab]heptene-4-carboxylate (1.8 g, 1.80 mmol) was added to tetrahydrofuran (18 mL), 1 M tetrabutylammonium fluoride (8.99 ml, 8.99 mmol) was added, and the obtained mixture was stirred at room temperature for 1 h. The mixture was concentrated, water was added, and the obtained mixture was extracted with ethyl acetate 3 times. The organic phase was washed with saturated sodium chloride aqueous solution, dried over anhydrous sodium sulfate, concentrated, and purified by preparative chromatography to obtain tert-butyl (5S,5aS,6S,9R)-12-((1-((4-(difluoromethylene)piperidin-1-yl)methyl) cyclopropyl)methoxy)-2-(8-ethynyl-7-fluoro-3-(methoxymethoxynaphthalen-1-yl)-1-fluoro-5-methyl-5a,6,7,8,9,10-hexahydro-5H-4-oxa-3,10a,11,13,14-pentaaza-6,9-methylnaphtho[1,8-ab]heptene-4-carboxylate (1.2 g, yield 79%).

MS m/z (ESI):845 [M+H].

### Step 12: Preparation of 4-((5S,5aS,6S,9R)-12-((1-((4-(difluoromethylene) piperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalene-2-ol

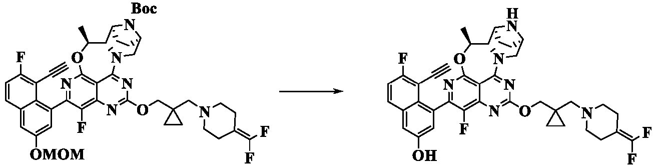

Tert-butyl (5S,5aS,6S,9R)-12-((1-((4-(difluoromethylene)piperidin-1-yl)methyl)cyclopropyl)methoxy)-2-(8-ethynyl-7-fluoro-3-(methoxymethoxynaphthalen-1-yl)-1-fluoro-5-methyl-5a,6,7,8,9,10-hexahydro-5H-4-oxa-3, 10a, 11, 13,14-pentaaza-6,9-methylnaphtho[1,8-ab]heptene-4-carboxylate (1.2 g, 1.42 mmol) was added to dichloromethane (12 mL), a solution of 4.0 M hydrochloric acid in ethyl acetate (8.88 mL, 65.52 mmol) was added, and the obtained mixture was stirred at room temperature for 1 h. The obtained mixture was concentrated, and then purified by preparative column chromatography to obtain 4-((5S,5aS,6S,9R)-12-((1-((4-(difluoromethylene)piperidin-1-yl)methyl) cyclopropyl)methoxy)-1-fluoro-5-methyl-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalen-2-ol (0.46 g, yield 46.2%).

MS m/z (ESI): 701 [M+H].

### Example 21-1

### Step 1: Preparation of tert-butyl 4-methoxyiminopiperidine-1-carboxylate

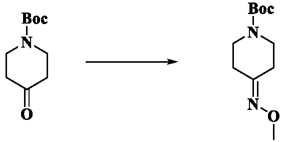

Tert-butyl 4-oxopiperidine-1-carboxylate (500 mg, 2.51 mmol), methoxyamine hydrochloride (272.46 mg, 3.26 mmol), potassium carbonate (1.04 g, 7.53 mmol) and 10 mL ethanol were added to an eggplant-shaped flask, and the obtained mixture was reacted at room temperature overnight. Filtration was performed, the filter cake was washed with ethanol, and the filtrate was concentrated to obtain the product tert-butyl 4-methoxyiminopiperidine-1-carboxylate (573 mg, yield 99.7%).

MS m/z(ESI): 229 [M+H].

### Step 2: Preparation of N-methoxypiperidin-4-imine

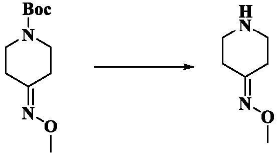

Tert-butyl 4-methoxyiminopiperidine-1-carboxylate (720 mg, 3.15 mmol) and 9 mL of a mixed solution of dichloromethane/trifluoroacetic acid = 2/1 were added to an eggplant-shaped flask, and the obtained mixture was reacted at room temperature for 1 h. 2 mL of N,N-diisopropylethylamine was added to the crude product under ice bath to quench the reaction, and the reaction liquid was concentrated to obtain the product N-methoxypiperidine-4-imine (404 mg, yield 100%).

MS m/z(ESI): 129 [M+H].

### Step 3: Preparation of tert-butyl (5aS,6S,9R)-2-(8-ethynyl-7-fluoro-3-(methoxymethoxy)naphthalen-1-yl)-1-fluoro-12-(1-((4-(methoxyimino)piperidin-1-yl)methyl)cyclopropyl)methoxy)-5a,6,7,8,9,10-hexahydro-5H-4-oxa-3,10a,11,13,14-pentaaza-6,9-methylnaphtho[1,8-ab]heptene-4-carboxylate

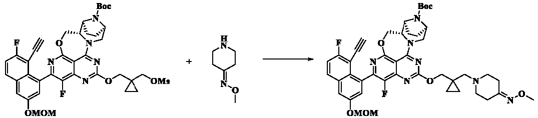

Tert-butyl (5aS,6S,9R)-2-(8-ethynyl-7-fluoro-3-(methoxy-methoxy)naphthalen-1-yl)-1-fluoro-12-((1-(methylsulfonyl)oxy)methyl)cyclopropyl)methoxy-5a,6,7,8,9,10-hexahydro-5H-4-oxa-3,10a,11,13,14-pentaaza-6,9-methylnaphtho[1,8-ab]heptene-4-carboxylate (998.2 mg, 1.26 mmol), sodium iodide (565.4 mg, 3.77 mmol), N,N-diisopropylethylamine (812.6 mg, 6.29 mmol) and 30 mL of acetonitrile were added to an eggplant-shaped flask. Nitrogen replacement was performed and the reaction was carried out overnight in an oil bath at 55°C. The mixture was cooled to room temperature, and saturated ammonium chloride solution was added, and the obtained mixture was extracted with ethyl acetate, dried over anhydrous sodium sulfate, filtered, concentrated, and then purified by preparative column chromatography to obtain tert-butyl (5a8S,6S,9R)-2-(8-ethynyl-7-fluoro-3-(methoxymethoxy)naphthalen-1-yl)-1-fluoro-12-(1-((4-(methoxyimino)piperidin-1-yl)methyl)cyclopropyl)methoxy)-Sa,6,7,8,9, 10-hexahydro-SH-4-oxa-3, 10a, 11, 13, 14-pentaaza-6,9-methylnaphtho[1,8-ab]heptene-4-carboxylate (190 mg, yield 18.3%).

MS m/z(ESI): 826 [M+H].

### Step 4: Preparation of 1-((1-((((5aS,6S,9R)-2-(8-ethynyl-7-fluoro-3-hydroxylnaphthalen-1-yl)-1-fluoro-5a,6,7,8,9,10-hexahydro-5H-4-oxa-3,10a,11,13,14-pentaaza-6,9-methylnaphtho[1,8-ab]hept-12-yl)oxy)methyl)cyclopropyl)methyl)piperidine-4-one-oxo-methyloxime

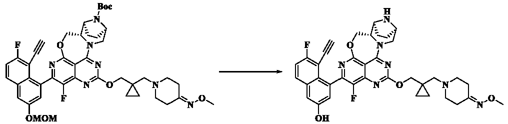

In an eggplant-shaped flask, tert-butyl (5aS,6S,9R)-2-(8-ethynyl-7-fluoro-3-(methoxymethoxy)naphthalen-1-yl)-1-fluoro-12-(1-((4-(methoxyimino)piperidin-1-yl)methyl)cyclopropyl)methoxy)-5a,6,7,8,9,10-hexahydro-5H-4-oxa-3,10a,11,13,14-pentaaza-6,9-methylnaphtho[1,8-ab]heptene-4-carboxylate (190 mg, 230.05 µmol) was dissolved in a mixed solvent of 5 mL of dichloromethane and 1.5 mL of methanol. 5 mL of a solution of 4N hydrochloric acid in dioxane was slowly added to the reaction liquid under ice bath, and the obtained mixture was transferred to room temperature and reacted for 30 min. Saturated sodium bicarbonate solution was added to the reaction liquid under ice bath to quench the reaction, the obtained mixture was extracted with a mixed solvent of ethyl acetate/methanol. The organic phase was dried over anhydrous sodium sulfate, filtered, concentrated, and then purified by preparative column chromatography to obtain 1-((1-((((5aS,6S,9R)-2-(8-ethynyl-7-fluoro-3-hydroxynaphthalen-1-yl)-1-fluoro-5a,6,7,8,9,10-hexahydro-5H-4-oxa-3,10a,11,13,14-pentaaza-6,9-methylnaphtho[1,8-ab]hept-12-yl)oxy)methyl)cyclopropyl)methyl)piperidin-4-one-oxo-methyloxime (124 mg, yield: 79.2%).

MS m/z(ESI): 682[M+H].

¹H NMR(400 MHz, MeOD) δ 7.87 - 7.79(m, 1H), 7.36 - 7.27(m, 2H), 7.25 - 7.14(m, 1H), 5.12(dd, *J =* 12.2, 7.5 Hz, 1H), 4.69 - 4.21(m, 6H), 3.91(d, *J =* 26.7 Hz, 2H), 3.77(s, 3H), 2.78(d, *J* = 26.3 Hz, 4H), 2.64(d, *J =* 13.2 Hz, 4H), 2.41 - 2.30(m, 2H), 1.98(d, *J* = 33.7 Hz, 5H), 0.90(t, *J* = 6.6 Hz, 2H), 0.57(s, 2H).

**The examples in the following table refer to the synthesis of example 1-1 or example 21-1:**

| **Example compounds and stereoisomer structures thereof** | **MS m/z (ESI)** |
|---|---|
| 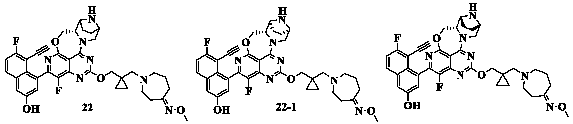 | 696 [M+H] |
| **Example 22-1:** ¹H NMR (400 MHz, MeOD) δ 7.88 - 7.80 (m, 1H), 7.35 - 7.27 (m, 2H), 7.25 - 7.13 (m, 1H), 5.12 - 5.00 (m, 1H), 4.69 - 4.21 (m, 6H), 3.92 - 3.83 (m, 2H), 3.75(s, 3H), 2.77(d, *J* = 26.3 Hz, 4H), 2.64(d, *J* = 13.2 Hz, 4H), 2.41 - 2.30(m, 2H), 1.98 - 1.58 (m, 7H), 0.90 (t, *J =* 6.6 Hz, 2H), 0.57 (s, 2H). | |
| 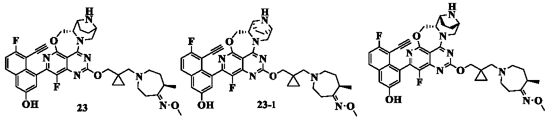 | 710 [M+H] |
| 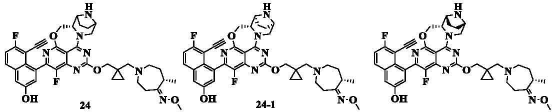 | 710 [M+H] |
| 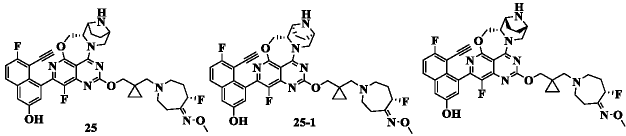 | 714 [M+H] |
| 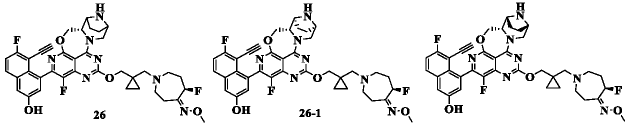 | 714 [M+H] |
| 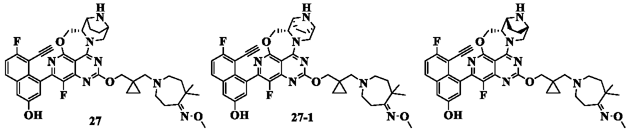 | 724 [M+H] |
| 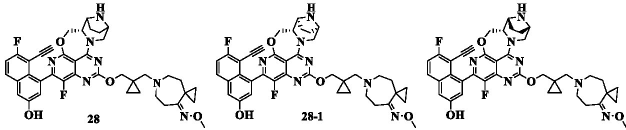 | 722 [M+H] |
| 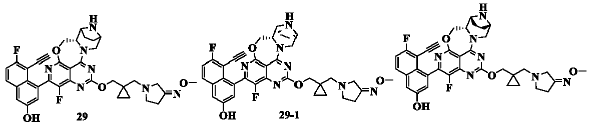 | 668 [M+H] |
| **Example 29-1:** ¹H NMR (400 MHz, MeOD) δ 7.84 - 7.76 (m, 1H), 7.36 - 7.28 (m, 2H), 7.25 - 7.19 (m, 0.5H), 7.17 - 7.08 (m, 0.5H), 5.12 - 5.00 (m, 1H), 4.55 - 4.42 (m, 7H), 4.42 - 4.24 (m, 1H), 4.20 - 4.12 (m, 1H), 3.87 - 3.68 (m, 2H), 3.27 - 3.05 (m, 6H), 3.04 - 2.47 (m, 4H), 2.46 - 2.24 (m, 2H), 2.10 - 1.72 (m, 1H), 0.85 - 0.72 (m, 2H), 0.69 - 0.45 (m, 2H). | |
| 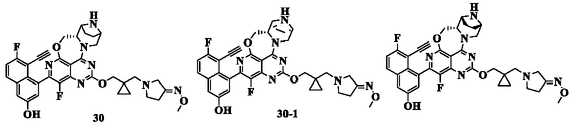 | 668 [M+H] |
| **Example 30-1:** ¹H NMR (400 MHz, MeOD) δ 7.84 - 7.76 (m, 1H), 7.36 - 7.28 (m, 2H), 7.25 - 7.19 (m, 0.5H), 7.17 - 7.08 (m, 0.5H), 5.12 - 5.00 (m, 1H), 4.55 - 4.42 (m, 7H), 4.41 - 4.24 (m, 1H), 4.20 - 4.12 (m, 1H), 3.87 - 3.68 (m, 2H), 3.27 - 3.05 (m, 6H), 3.04 - 2.47 (m, 4H), 2.46 - 2.24 (m, 2H), 2.10 - 1.72 (m, 1H), 0.85 - 0.72 (m, 2H), 0.69 - 0.45 (m, 2H). | |
| 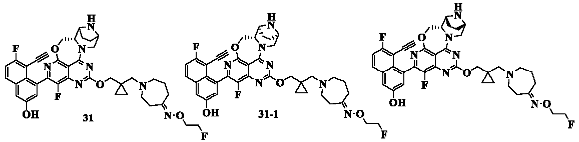 | 728 [M+H] |
| 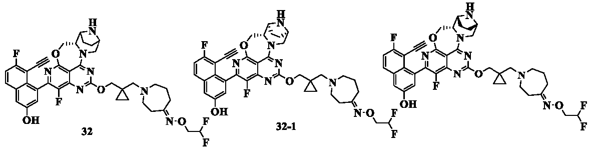 | 746 [M+H] |
| 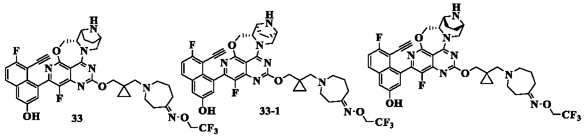 | 764 [M+H] |
| 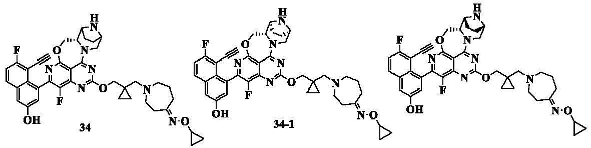 | 722 [M+H] |
| 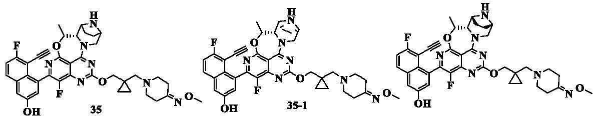 | 696 [M+H] |
| 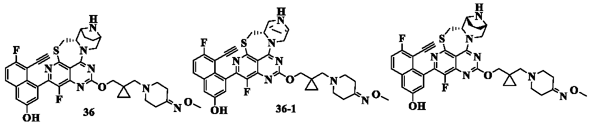 | 698 [M+H] |
| 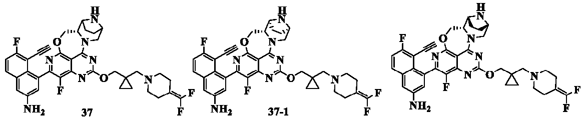 | 686 [M+H] |

**The following synthesis methods can also be adopted for the following examples:**

### Example 35-1

### Step 1: Preparation of (5S,5aS,6S,9R)-2-(8-ethynyl-7-fluoro-3-(methoxymethoxy)naphthalen-1-yl)-1-fluoro-5-methyl-12-((1-(((methylsulfonyl) oxo)methyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate

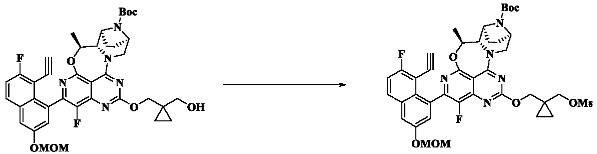

Tert-butyl (5S,5aS,6S,9R)-2-(8-ethynyl-7-fluoro-3-(methoxymethoxy) naphthalen-1-yl)-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methylenenaphtho[1,8-ab]heptalene-14-carboxylate (2 g, 2.74 mmol), DIPEA (1 g, 8.23 mmol) were added to dichloromethane (20 mL), and after mixing evenly, MsCl (470 mg, 4.11 mmol) was added dropwise at 0°C. After the addition was completed, the reaction was continued for 0.5 h, and water was added to quench the reaction, and the obtained mixture was extracted with ethyl acetate, dried over anhydrous sodium sulfate, filtered, and concentrated to obtain the crude product (5S,5aS,6S,9R)-2-(8-ethynyl-7-fluoro-3-(methoxymethoxy)naphthalen-1-yl)-1-fluoro-5-methyl-12-((1-(((methylsulfonyl)oxo)methyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate (2 g, yield 90.3%).

### Step 2: Preparation of tert-butyl (5S,5aS,6S,9R)-2-(8-ethynyl-7-fluoro-3-(methoxymethoxy)naphthalen-1-yl)-1-fluoro-12-((1-((4-(methoxyimino)piperidin-1-yl)methyl)cyclopropyl)methoxy)-5-methyl-5a,6,7,8,9,10-hexahydro-5H-4-oxa-3,10a,11,13,14-pentaaza-6,9-methylenenaphtho[1,8-ab]heptalene-14-carboxylate

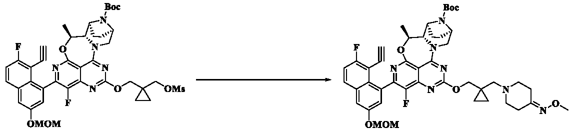

(5 S, 5 aS, 6 S, 9R)-2-(8-ethynyl-7-fluoro-3 -(methoxymethoxy)naphthalen-1-yl)-1-fluoro-5-methyl-12-((1-(((methylsulfonyl)oxo)methyl)cyclopropyl)methoxy)-Sa,6,7,8,9, 10-hexahydro-SH-4-oxa-3, 10a, 11, 13, 14-pentaaza-6,9-methylenenaphtho[1,8-ab]heptalene-14-carboxylate (2 g, 2.47 mmol), piperidin-4-one O-methyloxime (950 mg, 7.41 mmol), DIPEA (1.6 g, 12.35 mmol), NaI (1.1 g, 7.41 mmol) were added to acetonitrile (20 mL). After mixing evenly, the obtained mixture was reacted at 50°C for 16 h, and water was added, and the obtained mixture was extracted with ethyl acetate, dried over anhydrous sodium sulfate, filtered, concentrated, and separated and purified by silica gel column chromatography to obtain the target compound tert-butyl (SS,SaS,6S,9R)-2-(8-ethynyl-7-fluoro-3-(methoxymethoxy)naphthalen-1-yl)-1-fluoro-12-((1-((4-(methoxyimino)piperidin-1-yl)methyl)cyclopropyl)methoxy)-5-methyl-5a,6,7,8,9,10-hexahydro-5H-4-oxa-3,10a,11,13,14-pentaaza-6,9-methylenenaphtho[1,8-ab]heptalene-14-carboxylate (2 g, yield 96.2%).

MS m/z (ESI):840 [M+H].

### Step 3: Preparation of 1-((1-((((5S,5aS,6S,9R)-2-(8-ethynyl-7-fluoro-3-hydroxynaphthalen-1-yl)-1-fluoro-5-methyl-5a,6,7,8,9,10-hexahydro-5H-4-oxa-3,10a,11,13,14-pentaaza-6,9-methylenenaphtho[1,8-ab]heptalen-12-yl)oxo)methyl)cyclopropyl)methyl)piperidin-4-one O-methyloxime formate

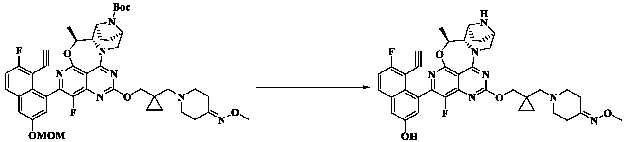

tert-butyl (5S,5aS,6S,9R)-2-(8-ethynyl-7-fluoro-3-(methoxymethoxy)naphthalen-1-yl)-1-fluoro-12-((1-((4-(methoxyimino)piperidin-1-yl)methyl)cyclopropyl) methoxy)-5-methyl-5a,6,7,8,9,10-hexahydro-5H-4-oxa-3,10a,11,13,14-pentaaza-6,9-methylenenaphtho[1,8-ab]heptalene-14-carboxylate (2 g, 2.38 mmol) was added to the dichloromethane/methanol solution (20 mL, volume ratio of 4:1), and after mixing evenly, a solution of hydrochloric acid in dioxane (20 mL, 4 M) was added at 0°C. After the addition was completed, the temperature was warmed to room temperature, and the reaction was continued for 40 min. The mixture was neutralized with the saturated aqueous NaHCO₃ solution, extracted with ethyl acetate, dried over anhydrous sodium sulfate, filtered, concentrated, and separated and purified by preparative column chromatography to obtain 1-((1-((((5S,5aS,6S,9R)-2-(8-ethynyl-7-fluoro-3-hydroxylnaphthalen-1-yl)-1-fluoro-5-methyl-5a,6,7,8,9,10-hexahydro-5H-4-oxa-3,10a,11,13,14-pentaaza-6,9-methylenenaphtho[1,8-ab]heptalene-12-yl)oxo)methyl)cyclopropyl)methyl)piperidine-4-one O-methyloxime formate (600 mg, yield 34%).

MS m/z (ESI):696 [M+H].

¹H NMR (400 MHz, DMSO) δ 10.10 (s, 1H), 8.13 (s, 1H), 7.95 (dt, *J =* 9.6, 5.7 Hz, 1H), 7.51 - 7.39 (m, 1H), 7.37 (d, *J =* 2.5 Hz, 1H), 7.12 (dd, *J =* 50.6, 2.5 Hz, 1H), 5.20 (d, *J =* 13.1 Hz, 1H), 4.62 - 4.43 (m, 1H), 4.37 - 4.22 (m, 2H), 4.22 - 3.90 (m, 2H), 3.83 - 3.59 (m, 4H), 3.21 - 3.05 (m, 2H), 2.55 (s, 2H), 2.48 - 2.30 (m, 6H), 2.20 (d, *J =* 5.6 Hz, 2H), 1.98 - 1.59 (m, 4H), 1.45 (t, *J =* 6.9 Hz, 3H), 0.73 - 0.59 (m, 2H), 0.49 - 0.25 (m, 2H).

### Example 37-1

### Step 1: Preparation of tert-butyl (5aS,6S,9R)-12-((1-((4-(difluoromethylene) piperidin-1-yl)methyl)cyclopropyl)methoxy)-1-fluoro-2-(7-fluoro-3-hydroxy-8-((triisopropylsilyl)ethynyl)naphthalen-1-yl)-5a,6,7,8,9, 10-hexahydro-5H-4-oxa-3,10a,11,13,14-pentaaza-6,9-methylnaphtho[1,8-ab]heptene-14carboxylate

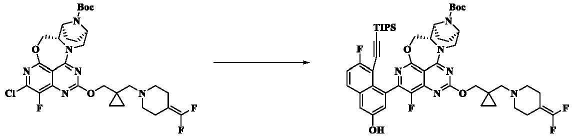

tert-butyl (5aS,6S,9R)-2-chloro-12-((1-((4-(difluoromethylene)piperidin-1-yl)methyl)cyclopropyl)methoxy)-1-fluoro-5a,6,7,8,9,10-hexahydro-5H-4-oxa-3,10a,11,13,14-pentaaza-6,9-methylnaphtho[1,8-ab]heptene-4-carboxylate (100 mg, 156.96 µmol), 6-fluoro-4-(4,4,5,5-tetramethyl-1,3,2-dioxaboran-2-yl)-5-(2-triisopropylsilylethynyl)naphthalen-2-ol (95.59 mg, 204.05 µmol), methanesulfonic acid [n-butyldi(1-adamantyl)phosphine](2-amino-1,1'-biphenyl-2-yl)palladium(II) (11.43 mg, 15.70 µmol), 2N sodium hydroxide aqueous solution (156.96 µL, 313.92 µmol) and 2 mL of tetrahydrofuran were added to a microwave tube. The obtained mixture was subjected to nitrogen replacement, and reacted under microwave at 100°C for 1 h. The obtained mixture was cooled to room temperature, the reaction liquid was concentrated, and purified by silica gel column chromatography to obtain tert-butyl (5aS,6S,9R)-12-((1-((4-(difluoromethylene)piperidin-1-yl)methyl) cyclopropyl)methoxy)-1-fluoro-2-(7-fluoro-3-hydroxy-8-((triisopropylsilyl) ethynyl)naphthalen-1-yl)-5a,6,7,8,9,10-hexahydro-5H-4-oxa-3,10a,11,13,14-pentaaza-6,9-methylnaphtho[1,8-ab]heptene-14carboxylate (110 mg, yield 74.3%).

MS m/z (ESI): 943 [M+H].

### Step 2: Preparation of tert-butyl (5aS,6S,9R)-12-((1-((4-(difluoromethylene)piperidin-1-yl)methyl)cyclopropyl)methoxy)-1-fluoro-2-(7-fluoro-3-((trifluoromethyl)sulfonyl)oxy)-8-((triisopropylsilyl)ethynyl)naphthalen-1-yl)-5a,6,7,8,9,10-hexahydro-5H-4-oxa-3,10a,11,13,14-pentaaza-6,9-methylnaphtho[1,8-ab]heptene-4-carboxylate

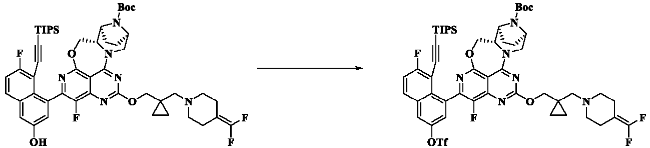

tert-butyl (5aS,6R,9S)-12-((1-((4-(difluoromethylene)piperidin-1-yl)methyl) cyclopropyl)methoxy)-1-fluoro-2-(7-fluoro-3-hydroxy-8-((triisopropylsilyl) ethynyl)naphthalen-1-yl)-5a,6,7,8,9,10-hexahydro-5H-4-oxa-3,10a,11,13,14-pentaaza-6,9-methylnaphtho[1,8-ab]heptene-14carboxylate (110 mg, 116.63 µmol), N,N-diisopropylethylamine (60.29 mg, 466.52 µmol) and 2.5 mL of dichloromethane were added to an eggplant-shaped flask. After cooling under ice bath, trifluoromethanesulfonic anhydride (49.36 mg, 174.95 µmol) was added dropwise, and the reaction was continued under ice bath for 30 min. After the addition was completed, saturated NaHCO₃ was added to the reaction liquid to quench the reaction, and the obtained mixture was extracted with dichloromethane, washed with saturated ammonium chloride solution, and the organic phase was dried and concentrated to obtain tert-butyl (5aS,6S,9R)-12-((1-((4-(difluoromethylene)piperidin-1-yl)methyl)cyclopropyl)methoxy)-1-fluoro-2-(7-fluoro-3-((trifluoromethyl)sulfonyl)oxy)-8-((triisopropylsilyl)ethynyl)naphthalen-1-yl)-5a,6,7,8,9,10-hexahydro-5H-4-oxa-3,10a,11,13,14-pentaaza-6,9-methylnaphtho[1,8-ab]heptene-4-carboxylate (125.4 mg, 100.00% yield).

MS m/z (ESI): 1075 [M+H].

### Step 3: Preparation of tert-butyl (SaS,6S,9R)-2-(3-((tert-butoxycarbonyl)amino)-7-fluoro-8-((triisopropylsilyl)ethynyl)naphthalen-1-yl)-12-(1-((4-(difluoromethylene)piperidin-1-yl)methyl)cyclopropyl)methoxy-1-fluoro-5a,6,7,8,9,10-hexahydro-5H-4-oxa-3,10a,11,13,14-pentaaza-6,9-methylnaphtho[1,8-ab]heptene-4-carboxylate

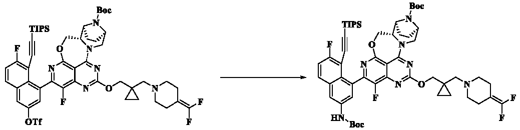

tert-butyl (5aS,68S,9R)-12-((1-((4-(difluoromethylene)piperidin-1-yl)methyl) cyclopropyl)methoxy)-1-fluoro-2-(7-fluoro-3-((trifluoromethyl)sulfonyl)oxy)-8-((triisopropylsilyl)ethynyl)naphthalen-1-yl)-5a,6,7,8,9,10-hexahydro-5H-4-oxa-3,10a,11,13,14-pentaaza-6,9-methylnaphtho[1,8-ab]heptene-4-carboxylate (125.4 mg, 116.63 µmol), tris(dibenzylideneacetone)dipalladium (21.36 mg, 23.33 µmol), 4,5-bisdiphenylphosphine-9,9-dimethylxanthene (33.74 mg, 58.31 µmol), cesium carbonate (114.00 mg, 349.88 µmol), and 4 mL of toluene were added to an eggplant-shaped flask. Nitrogen replacement was performed and the reaction was carried out in an oil bath at 100°C for 8 h. The obtained mixture was cooled to room temperature, and the reaction liquid was concentrated, diluted with ethyl acetate, and the organic phase was washed with water. The organic phase was dried and concentrated, and separated by column chromatography (mobile phase: ethyl acetate/dichloromethane = 2/1) to obtain tert-butyl (SaS,6S,9R)-2-(3-((tert-butoxycarbonyl)amino)-7-fluoro-8-((triisopropylsilyl)ethynyl)naphthalen-1-yl)-12-(1-((4-(difluoromethylene)piperidin-1-yl)methyl)cyclopropyl)methoxy-1-fluoro-5a,6,7,8,9,10-hexahydro-5H-4-oxa-3,10a,11,13,14-pentaaza-6,9-methylnaphtho[1,8-ab]heptene-4-carboxylate (70 mg, yield 57.6%).

MS m/z (ESI): 1042 [M+H].

### Step 4: Preparation of tert-butyl (5aS,6S,9R)-2-(3-((tert-butoxycarbonyl)amino)-8-ethynyl-7-fluoronaphthalen-1-yl)-12-((1-((4-(difluoromethylene)piperidin-1-yl)methyl)cyclopropyl)methoxy)-1-fluoro-5a,6,7,8,9,10-hexahydro-5H-4-oxa-3,10a,11,13,14-pentaaza-6,9-methylnaphtho[1,8-ab]heptene-14carboxylate

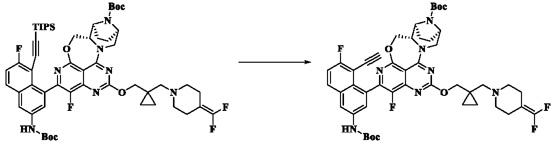

In an eggplant-shaped flask, tert-butyl (SaS,6S,9R)-2-(3-((tert-butoxycarbonyl)amino)-7-fluoro-8-((triisopropylsilyl)ethynyl)naphthalen-1-yl)-12-(1-((4-(difluoromethylene)piperidin-1-yl)methyl)cyclopropyl)methoxy-1-fluoro-Sa,6,7,8,9, 10-hexahydro-SH-4-oxa-3, 10a, 11, 13, 14-pentaaza-6,9-methylnaphtho[1,8-ab]heptene-4-carboxylate (70 mg, 67.16 µmol) was dissolved in 2 mL of tetrahydrofuran, 1N tetrabutylammonium fluoride (134.32 µL, 134.32 µmol) was added, and the obtained mixture was reacted at room temperature for 2 h. The obtained mixture was extracted with ethyl acetate. The organic phase was dried and concentrated to obtain tert-butyl (SaS,6S,9R)-2-(3-((tert-butoxycarbonyl)amino)-8-ethynyl-7-fluoronaphthalen-1-yl)-12-((1-((4-(difluoromethylene)piperidin-1-yl)methyl)cyclopropyl)methoxy)-1-fluoro-Sa,6,7,8,9, 10-hexahydro-SH-4-oxa-3, 10a, 11, 13, 14-pentaaza-6,9-methylnaphtho[1,8-ab]heptene-14carboxylate (59.5 mg).

MS m/z (ESI): 886 [M+H].

### Step 5: Preparation of tert-butyl (5aS,6S,9R)-2-(3-amino-8-ethynyl-7-fluoronaphthalen-1-yl)-12-((1-((4-(difluoromethylene)piperidin-1-yl)methyl)cyclopropyl)methoxy)-1-fluoro-5a,6,7,8,9,10-hexahydro-5H-4-oxa-3,10a,11,13,14-pentaaza-6,9-methylnaphtho[1,8-ab]heptene-14carboxylate

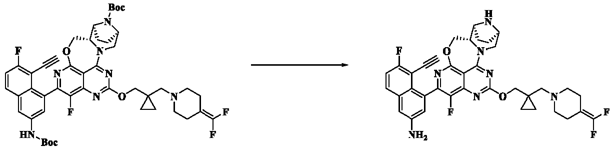

In an eggplant-shaped flask, 4 mL of a mixed solution of dichloromethane/trifluoroacetic acid = 3/1 was added to tert-butyl (SaS,6S,9R)-2-(3-((tert-butoxycarbonyl)amino)-8-ethynyl-7-fluoronaphthalen-1-yl)-12-((1-((4-(difluoromethylene)piperidin-1-yl)methyl)cyclopropyl)methoxy)-1-fluoro-5a,6,7,8,9,10-hexahydro-5H-4-oxa-3,10a,11,13,14-pentaaza-6,9-methylnaphtho[1,8-ab]heptene-14carboxylate (59.5 mg, 67.16 µmol) under ice bath, and the obtained mixture was reacted at room temperature for 30 min. After the addition was completed, the reaction liquid was concentrated and purified by preparative column chromatography to obtain tert-butyl (5aS,6S,9R)-2-(3-amino-8-ethynyl-7-fluoronaphthalen-1-yl)-12-((1-((4-(difluoromethylene)piperidin-1-yl)methyl)cyclopropyl)methoxy)-1-fluoro-5a,6,7,8,9,10-hexahydro-5H-4-oxa-3,10a,11,13,14-pentaaza-6,9-methylnaphtho[1,8-ab]heptene-14carboxylate (13.8 mg, yield 25.7%).

MS m/z (ESI): 686 [M+H].

¹H NMR(400 MHz, CDCl₃) δ 7.73(d, *J* = 7.7 Hz, 1H), 7.28 - 7.17(m, 2H), 7.12(d, *J* = 21.7 Hz, 1H), 5.25 - 5.17(m, 1H), 4.62(d, *J* = 35.3 Hz, 4H), 4.49 - 4.25(m, 4H), 4.20 - 4.05(m, 2H), 2.48(s, 4H), 2.03(s, 5H), 1.60(s, 2H), 0.90(t, *J* = 6.6 Hz, 4H), 0.80(s, 2H).

**Examples 38 to 50 and 70 to 73 refer to the synthesis of example 1-1 or example 37-1; Examples 51 to 69 refer to the synthesis of example 1-1:**

| **Example compounds and stereoisomer structures thereof** | **MS m/z (ESI)** |
|---|---|
| 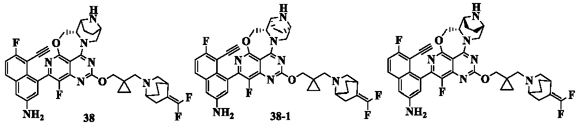 | 712 |
| | [M+H] |
| **Example 38-1:** ¹H NMR (400 MHz, MeOD) δ 7.72 (s, 1H), 7.27 - 7.15 (m, 2H), 7.15 - 7.05 (m, 1H), 5.02 (t, *J =* 14.6 Hz, 1H), 4.74 - 4.29 (m, 6H), 4.13 (s, 1H), 3.78 - 3.62 (m, 2H), 3.02 - 2.57 (m, 6H), 1.97 - 1.53 (m, 10H), 0.85 - 0.54 (m, 4H). | |
| 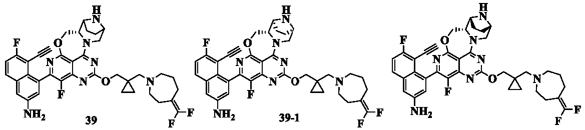 | 700 |
| | [M+H] |
| **Example 39-1:** ¹H NMR (400 MHz, MeOD) δ 7.85 - 7.76 (m, 1H), 7.36 - 7.28 (m, 2H), 7.27 - 7.19 (m, 0.5H), 7.17 - 7.08 (m, 0.5H), 5.12 - 5.02 (m, 1H), 4.55 - 4.42 (m, 4H), 4.42 - 4.24 (m, 1H), 4.20 - 4.12 (m, 1H), 3.87 - 3.68 (m, 2H), 3.27 - 3.06 (m, 4H), 3.04 - 2.47 (m, 2H), 2.46 - 2.23 (m, 4H), 2.11 - 1.72 (m, 5H), 1.50 - 1.35 (m, 2H), 0.84 - 0.70 (m, 2H), 0.69 - 0.44 (m, 2H). | |
| 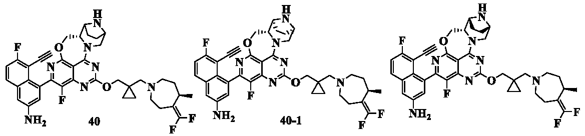 | 714 |
| | [M+H] |
| 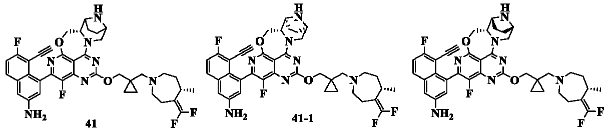 | 714 |
| | [M+H] |
| 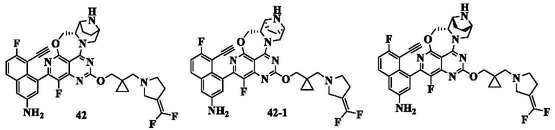 | 672 |
| | [M+H] |
| **Example 42-1:** ¹H NMR (400 MHz, MeOD) δ 7.83 - 7.76 (m, 1H), 7.36 - 7.28 (m, 2H), 7.25 - 7.19 (m, 0.5H), 7.17 - 7.08 (m, 0.5H), 5.12 - 5.00 (m, 1H), 4.55 - 4.42 (m, 4H), 4.42 - 4.24 (m, 1H), 4.20 - 4.12 (m, 1H), 3.87 - 3.68 (m, 2H), 3.27 - 3.05 (m, 4H), 3.04 - 2.47 (m, 4H), 2.46 - 2.23 (m, 2H), 2.10 - 1.72 (m, 3H), 0.84 - 0.70 (m, 2H), 0.69 - 0.45 (m, 2H). | |
| 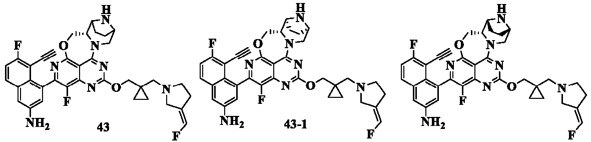 | 654 |
| | [M+H] |
| 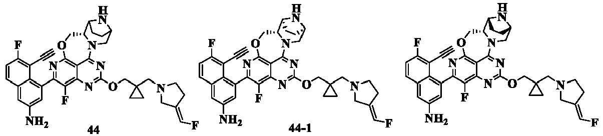 | 654 |
| | [M+H] |
| 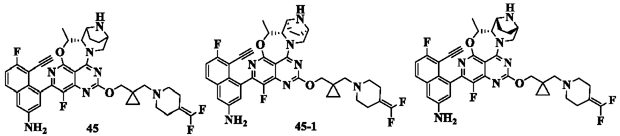 | 699 |
| | [M+H] |
| **Example 45-1:** ¹H NMR(400 MHz, CDCl₃) δ 7.73(d, *J* = 8 Hz, 1H), 7.28 - 7.17(m, 2H), 7.12(d, *J* = 24 Hz, 1H), 5.25 - 5.17(m, 1H), 4.62(d, *J* = 36 Hz, 4H), 4.49 - 4.25(m, 4H), 4.20 - 4.05(m, 1H), 2.48(s, 4H), 2.03(s, 5H), 1.63-1.55(m, 5H), 0.90(t, *J* =4 Hz, 4H), 0.80(s, 2H). | |
| 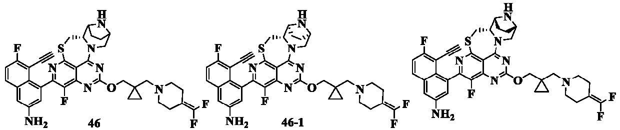 | 702 |
| | [M+H] |
| 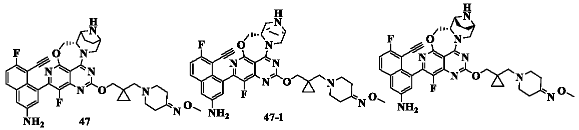 | 681 |
| | [M+H] |
| 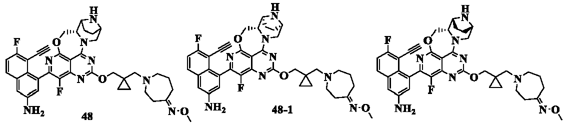 | 695 |
| | [M+H] |
| 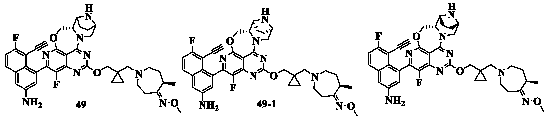 | 709 |
| | **[**M+H] |
| 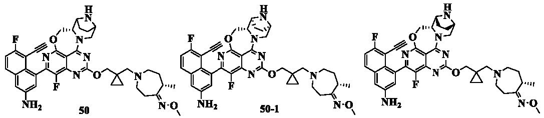 | 709 |
| | [M+H] |
| 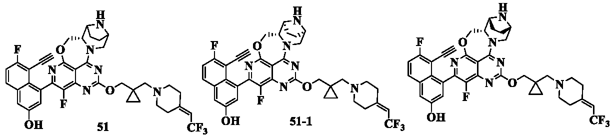 | 719 |
| | [M+H] |
| **Example 51-1:** ¹H NMR(400 MHz, MeOD) δ 7.84(dt, *J* = 9.2, 4.2 Hz, 1H), 7.35 - 7.27(m, 2H), 7.27 - 7.17(m, 1H), 5.72(s, 1H), 5.05(t, *J =* 12.1 Hz, 1H), 4.67 - 4.32(m, 5H), 4.15(s, 1H), 3.83 - 3.68(m, 2H), 3.05(s, 3H), 2.95 - 2.76(m, 4H), 2.37(s, 2H), 2.08 - 1.79(m, 6H), 0.86 - 0.65(m, 4H). | |
| 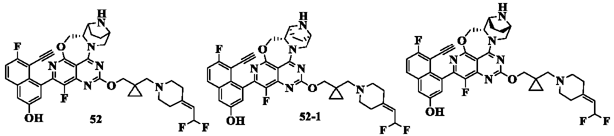 | 701 |
| | [M+H] |
| 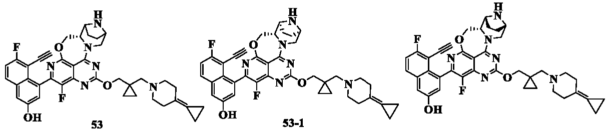 | 677 |
| | [M+H] |
| **Example 53-1:** ¹H NMR (400 MHz, MeOD) δ 7.85 - 7.75 (m, 1H), 7.35 - 7.28 (m, 2H), 7.26 - 7.19 (m, 0.5H), 7.16 - 7.08 (m, 0.5H), 5.11 - 5.02 (m, 1H), 4.55 - 4.43 (m, 4H), 4.42 - 4.24 (m, 1H), 4.20 - 4.12 (m, 1H), 3.87 - 3.69 (m, 2H), 3.27 - 3.06 (m, 4H), 3.04 - 2.47 (m, 2H), 2.46 - 2.23 (m, 4H), 2.13 - 1.72 (m, 9H), 0.84 - 0.71 (m, 2H), 0.69 - 0.45 (m, 2H). | |
| 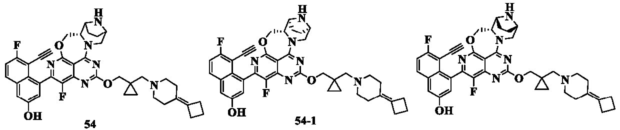 | 691 |
| | [M+H] |
| **Example 54-1:** ¹H NMR (400 MHz, MeOD) δ 7.86 - 7.75 (m, 1H), 7.35 - 7.28 (m, 2H), 7.26 - 7.18 (m, 0.5H), 7.16 - 7.09 (m, 0.5H), 5.12 - 5.02 (m, 1H), 4.56 - 4.43 (m, 4H), 4.40 - 4.24 (m, 1H), 4.21 - 4.12 (m, 1H), 3.88 - 3.69 (m, 2H), 3.27 - 3.06 (m, 4H), 3.04 - 2.47 (m, 2H), 2.46 - 2.23 (m, 4H), 2.14 - 1.72 (m, 9H), 1.40 - 1.22 (m, 2H),0.84 - 0.71 (m, 2H), 0.68 - 0.46 (m, 2H). | |
| 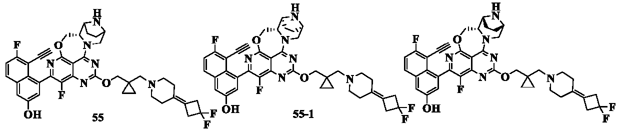 | 727 |
| | [M+H] |
| 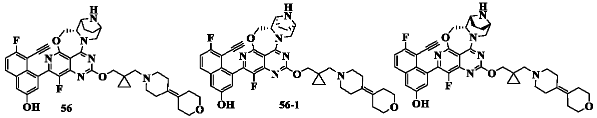 | 721 |
| | [M+H] |
| 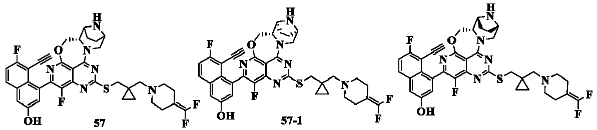 | 703 |
| | [M+H] |
| 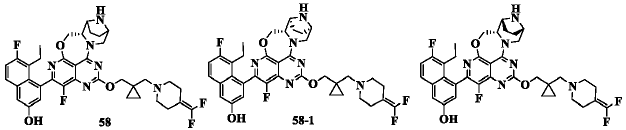 | 691 |
| | [M+H] |
| **Example 58-1:** ¹H NMR (400 MHz, MeOD) δ 7.69 - 7.60 (m, 1H), 7.31 - 7.17 (m, 2H), 7.13 - 7.06 (m, 0.5H), 7.03 - 6.95 (m, 0.5H), 5.21 - 5.02 (m, 1H), 4.68 - 4.54 (m, 3H), 4.53 - 4.33 (m, 3H), 4.26 - 4.11 (m, 1H), 3.92 - 3.71 (m, 2H), 2.86 - 2.64 (m, 4H), 2.62 - 2.36 (m, 2H), 2.30 (s, 3H), 2.24 - 2.12 (m, 1H), 2.07 - 1.76 (m, 5H), 0.89 (t, *J =* 8 Hz, 2H), 0.85 - 0.73 (m, 3H), 0.65 - 0.46 (m, 2H). | |
| 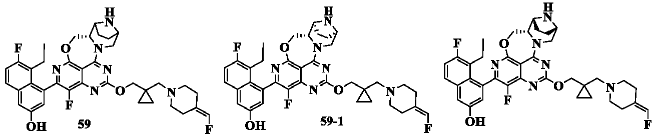 | 673 |
| | [M+H] |
| 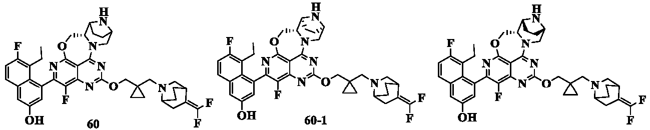 | 717 |
| | [M+H] |
| 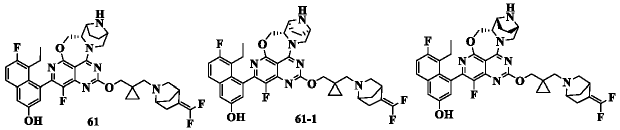 | 703 |
| | [M+H] |
| 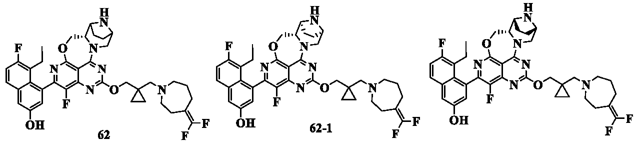 | 705 |
| | [M+H] |
| 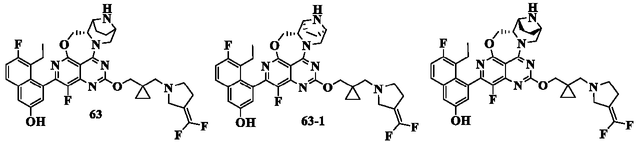 | 677 |
| | [M+H] |
| 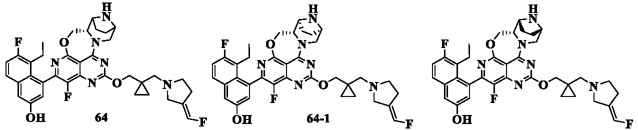 | 659 |
| | [M+H] |
| 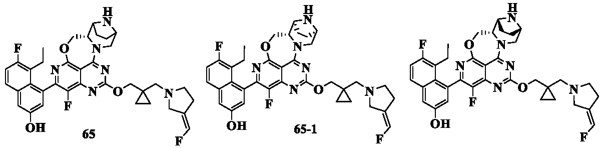 | 659 |
| | [M+H] |
| 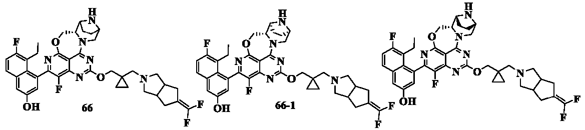 | 717 |
| | [M+H] |
| 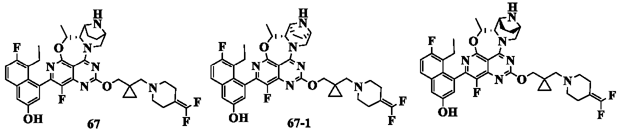 | 705 |
| | [M+H] |
| **Example 67-1:** ¹H NMR (400 MHz, MeOD) δ 7.65 - 7.60 (m, 1H), 7.34 - 7.17 (m, 2H), 7.14 - 7.06 (m, 0.5H), 7.04 - 6.95 (m, 0.5H), 5.22 - 5.02 (m, 1H), 4.68 - 4.54 (m, 3H), 4.54 - 4.35 (m, 2H), 4.26 - 4.11 (m, 1H), 3.92 - 3.72 (m, 2H), 2.86 - 2.64 (m, 4H), 2.62 - 2.36 (m, 2H), 2.30 (s, 3H), 2.24 - 2.13 (m, 1H), 2.08 - 1.75 (m, 5H), 1.59 (t, J = 8 Hz, 3H), 0.89 (t, *J =* 8 Hz, 2H), 0.86 - 0.72 (m, 3H), 0.66 - 0.47 (m, 2H). | |
| 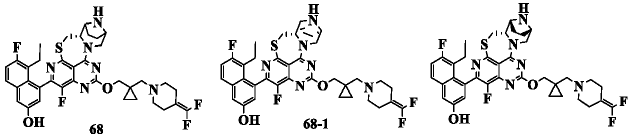 | 707 |
| | [M+H] |
| **Example 68-1:** ¹H NMR (400 MHz, MeOD) δ 7.67 - 7.60 (m, 1H), 7.31 - 7.18 (m, 2H), 7.13 - 7.05 (m, 0.5H), 7.04 - 6.96 (m, 0.5H), 5.21 - 5.04 (m, 1H), 4.68 - 4.55 (m, 1H), 4.53 - 4.33 (m, 3H), 4.27 - 4.11 (m, 1H), 3.92 - 3.72 (m, 2H), 2.98 - 2.64 (m, 6H), 2.63 - 2.36 (m, 2H), 2.30 (s, 3H), 2.24 - 2.12 (m, 1H), 2.07 - 1.76 (m, 5H), 0.89 (t, *J =* 8 Hz, 2H), 0.85 - 0.73 (m, 3H), 0.65 - 0.46 (m, 2H). | |
| 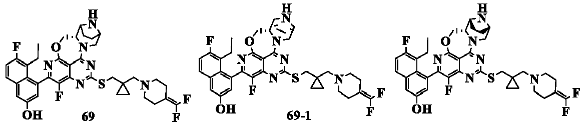 | 707 |
| | [M+H] |
| **Example 69-1:** ¹H NMR (400 MHz, MeOD) δ 7.67 - 7.61 (m, 1H), 7.33 - 7.17 (m, 2H), 7.13 - 7.05 (m, 0.5H), 7.03 - 6.95 (m, 0.5H), 5.21 - 5.02 (m, 1H), 4.68 - 4.54 (m, 3H), 4.53 - 4.33 (m, 3H), 4.26 - 4.11 (m, 1H), 2.86 - 2.64 (m, 6H), 2.62 - 2.36 (m, 2H), 2.30 (s, 3H), 2.25 - 2.12 (m, 1H), 2.08 - 1.76 (m, 5H), 0.89 (t, *J* = 8 Hz, 2H), 0.85 - 0.73 (m, 3H), 0.64 - 0.46 (m, 2H). | |
| 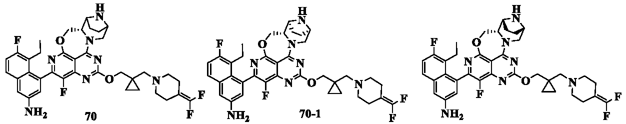 | 690 |
| | [M+H] |
| **Example 70-1:** ¹H NMR (400 MHz, MeOD) δ 7.68 - 7.60 (m, 1H), 7.32 - 7.17 (m, 2H), 7.14 - 7.06 (m, 0.5H), 7.04 - 6.95 (m, 0.5H), 5.21 - 5.02 (m, 1H), 4.68 - 4.54 (m, 3H), 4.54 - 4.33 (m, 3H), 4.26 - 4.11 (m, 1H), 3.92 - 3.71 (m, 2H), 2.86 - 2.64 (m, 4H), 2.62 - 2.36 (m, 2H), 2.30 (s, 3H), 2.24 - 2.11 (m, 1H), 2.08 - 1.76 (m, 5H), 0.89 (t, *J =* 8 Hz, 2H), 0.85 - 0.72 (m, 3H), 0.65 - 0.46 (m, 2H). | |
| 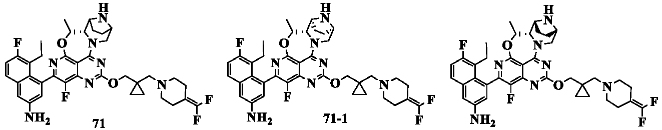 | 704 |
| | [M+H] |
| **Example 71-1:** ¹H NMR (400 MHz, MeOD) δ 7.66 - 7.60 (m, 1H), 7.34 - 7.17 (m, 2H), 7.13 - 7.06 (m, 0.5H), 7.04 - 6.96 (m, 0.5H), 5.22 - 5.02 (m, 1H), 4.68 - 4.54 (m, 3H), 4.54 - 4.35 (m, 2H), 4.26 - 4.11 (m, 1H), 3.92 - 3.72 (m, 2H), 2.86 - 2.64 (m, 4H), 2.62 - 2.36 (m, 2H), 2.30 (s, 3H), 2.24 - 2.12 (m, 1H), 2.08 - 1.77 (m, 5H), 1.59 (t, J = 8 Hz, 3H), 0.88 (t, *J=* 8 Hz, 2H), 0.85 - 0.72 (m, 3H), 0.65 - 0.47 (m, 2H). | |
| 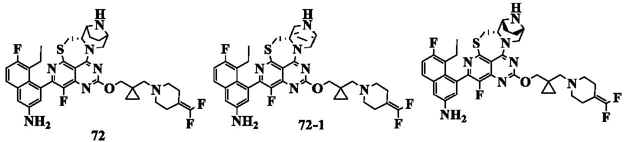 | 706 |
| | [M+H] |
| 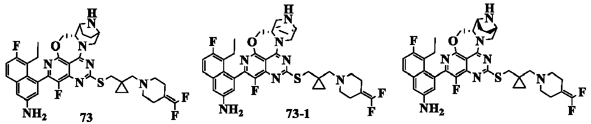 | 706 |
| | [M+H] |

**The following synthesis methods can also be adopted for the following examples:**

### Example 57-1

### Step 1: Preparation of 1-((1-(((tert-butyldiphenylsilyl)oxy)methyl) cyclopropyl)methyl)-4-(difluoromethylene)piperidine

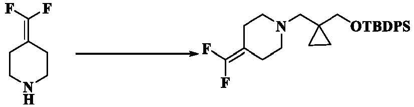

4-(difluoromethylene)piperidine hydrochloride (3.2 g, 18.87 mmol) and [1-[[tert-butyl(diphenyl)silyl]oxymethyl]cyclopropyl]methyl methanesulfonate (7.90 g, 18.87 mmol) were dissolved in acetonitrile (30 mL), and N,N-diisopropylacetamide (12.19 g, 94.34 mmol, 16.43 mL) and sodium iodide (14.14 g, 94.34 mmol) were added. The obtained mixture was warmed to 60°C and reacted for 3 h, and water was added to quench the reaction, and the obtained mixture was extracted with ethyl acetate. The organic phases were combined, washed with saturated sodium chloride aqueous solution, dried over anhydrous sodium sulfate, filtered, concentrated, and purified by silica gel column chromatography to obtain compound 1-((1-(((tert-butyldiphenylsilyl)oxy)methyl)cyclopropyl)methyl)-4-(difluoromethylene)piperidine (6.9 g, yield 80.2%).

MS m/z (ESI):456 [M+H].

### Step 2: Preparation of (1-((4-(difluoromethylene)piperidin-1-yl)methyl)cyclopropyl)methanol

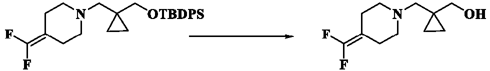

1-((1-(((tert-butyldiphenylsilyl)oxy)methyl)cyclopropyl)methyl)-4-(difluoromethylene)piperidine (6.9 g, 15.14 mmol) was dissolved in a solution of hydrogen chloride in methanol (2 M, 30 mL), and the obtained mixture was reacted at room temperature for 16 h. The reaction liquid was concentrated under reduced pressure and purified by silica gel column chromatography to obtain compound (1-((4-(difluoromethylene)piperidin-1-yl)methyl)cyclopropyl)methanol (2.1 g, yield 63.8%).

MS m/z (ESI):218[M+H].

### Step 3: Preparation of 1-((1-(chloromethyl)cyclopropyl)methyl)-4-(difluoromethylene)piperidine

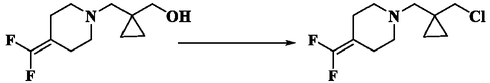

(1-((4-(difluoromethylene)piperidin-1-yl)methyl)cyclopropyl)methanol (2.1 g, 9.67 mmol) was dissolved in dichloromethane (20 mL), and thionyl chloride (4.60 g, 38.66 mmol) was added dropwise under ice bath. After the addition was completed, the obtained mixture was reacted at room temperature for 3 h, concentrated under reduced pressure, and purified by silica gel column chromatography to obtain compound 1-((1-(chloromethyl)cyclopropyl)methyl)-4-(difluoromethylene)piperidine (1.1 g, yield 48.3%).

MS m/z (ESI):236[M+H].

### Step 4: Preparation of S-((1-((4-(difluoromethylene)piperidin-1-yl)methyl)cyclopropyl)methyl)ethanethiol

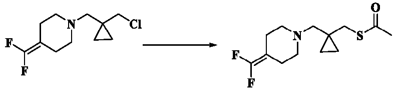

1-((1-(chloromethyl)cyclopropyl)methyl)-4-(difluoromethylene)piperidine (1.1 g, 4.67 mmol) was dissolved in acetonitrile (10 mL), cesium carbonate (15.21 g, 46.67 mmol) and potassium thioacetate (5.33 g, 46.67 mmol) were added. The obtained mixture was warmed to 30°C and reacted for 16 h, and water was added to quench the reaction, and the obtained mixture was extracted with ethyl acetate. The organic phases were combined, washed with saturated sodium chloride aqueous solution, dried over anhydrous sodium sulfate, filtered, concentrated, and purified by silica gel column chromatography to obtain compound S-((1-((4-(difluoromethylene)piperidin-1-yl)methyl)cyclopropyl)methyl)ethanethiol (1 g, yield 77.8%).

MS m/z (ESI):276[M+H].

### Step 5: Preparation of (1-((4-(difluoromethylene)piperidin-1-yl)methyl) cyclopropyl)methanethiol

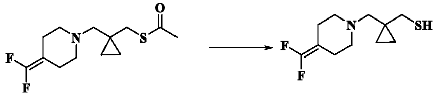

S-((1-((4-(difluoromethylene)piperidin-1-yl)methyl)cyclopropyl)methyl) ethanethiol (400 mg, 1.45 mmol) was dissolved in methanol (4 mL), sodium methoxide (392.36 mg, 7.26 mmol) was added. The obtained mixture was reacted at room temperature for 3 h, concentrated under reduced pressure, and purified by silica gel column chromatography to obtain compound (1-((4-(difluoromethylene)piperidin-1-yl)methyl)cyclopropyl)methanethiol (320 mg, yield 94.4%).

MS m/z (ESI):234[M+H].

### Step 6: Preparation of tert-butyl (5aS,6S,9R)-2-chloro-12-(((1-((4-(difluoromethylene)piperidin-1-yl)methyl)cyclopropyl)methyl)thio)-1-fluoro-5a,6,7,8,9,10-hexahydro-SH-4-oxa-3,10a,11,13,14-pentaaza-6,9-methylnaphtho[1,8-ab]heptene-4-carboxylate

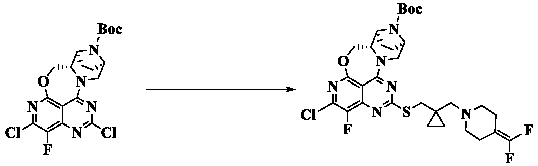

Tert-butyl (1-((4-(difluoromethylene)piperidin-1-yl)methyl)cyclopropyl) methanethiol (300 mg, 1.29 mmol) and (5aS,6S,9R)-2,12-dichloro-1-fluoro-5a,6,7,8,9,10-hexahydro-5H-4-oxo-3,10a,11,13,14-pentaaza-6,9-methylnaphtho[1,8-ab]heptene-4-carboxylate (586.70 mg, 1.29 mmol) were dissolved in ultra-dry tetrahydrofuran (10 mL), and sodium hydride (102.86 mg, 2.57 mmol, purity 60%) was added under ice bath. After the addition was completed, the obtained mixture was reacted at room temperature for 16 h, and tert-butanol was added to quench the reaction, and the obtained mixture was concentrated under reduced pressure, and purified by silica gel column chromatography to obtain compound tert-butyl (5aS,6S,9R)-2-chloro-12-(((1-((4-(difluoromethylene) piperidin-1-yl)methyl)cyclopropyl)methyl)thio)-1-fluoro-5a,6,7,8,9,10-hexahydro-5H-4-oxa-3,10a,11,13,14-pentaaza-6,9-methylnaphtho[1,8-ab]heptene-4-carboxylate (710 mg, yield 84.5%).

MS m/z (ESI):654[M+H].

### Step 7: Preparation of tert-butyl (SaS,6S,9R)-12-(((1-((4-(difluoromethyl)piperidin-1-yl)methyl)cyclopropyl)methyl)thio)-1-fluoro-2-(7-fluoro-3-(methoxymethoxy)-8-((triisopropylsilyl)ethyl)naphthalen-1-yl)-5a,6,7,8,9,10-hexahydro-SH-4-oxy-3,10a,11,13,14-pentaaza-6,9-methylaminonaphtho[1,8-ab]heptene-14-carboxylate

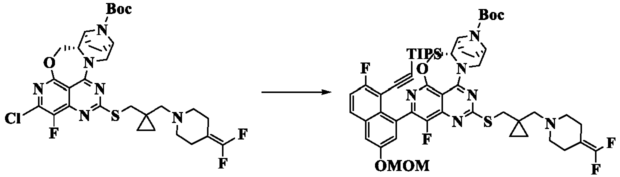

Tert-butyl (5aS,6S,9R)-2-chloro-12-(((1-((4-(difluoromethylene)piperidin-1-yl)methyl)cyclopropyl)methyl)thio)-1-fluoro-5a,6,7,8,9,10-hexahydro-5H-4-oxa-3,10a,11,13,14-pentaaza-6,9-methylnaphtho[1,8 -ab]heptene-4-carboxylate (710 mg, 1.09 mmol) and 2-[2-fluoro-6-(methoxymethoxy)-8-(4,4,5,5-tetramethyl-1,3,2-dioxyboran-2-yl)-1-naphthyl]ethynyltriisopropylsilane (835.71 mg, 1.63 mmol) were dissolved in tetrahydrofuran (10 mL) and water (3 mL). Potassium phosphate (2.31 g, 10.87 mmol) and methanesulfonic acid [n-butyldi(1-adamantyl)phosphine](2-amino-1,1'-biphenyl-2-yl)palladium(II) (158.33 mg, 217.41 µmol) were added. The obtained mixture was subjected to nitrogen replacement, warmed to 80°C, reacted for 2 h, concentrated under reduced pressure, and purified by silica gel column chromatography to obtain compound tert-butyl (SaS,6S,9R)-12-(((1-((4-(difluoromethyl)piperidin-1-yl)methyl)cyclopropyl) methyl)thio)-1-fluoro-2-(7-fluoro-3-(methoxymethoxy)-8-((triisopropylsilyl)ethyl) naphthalen-1-yl)-5a,6,7,8,9, 10-hexahydro-5H-4-oxy-3, 10a, 11, 13,14-pentaaza-6,9-methylaminonaphtho[1,8-ab]heptene-14-carboxylate (820 mg, yield 75.2%).

MS m/z (ESI):1004[M+H].

### Step 8: Preparation of tert-butyl (SaS,6S,9R)-12-(((1-((4-(difluoromethylene)piperidin-1-yl)methyl)cyclopropyl)methyl)thio)-2-(8-ethynyl-7-fluoro-3-(methoxymethoxy)naphthalen-1-yl)-1-fluoro-5a,6,7,8,9,10-hexahydro-5H-4-oxa-3,10a,11,13,14-pentaaza-6,9-methylnaphthol[1,8-ab]heptene-14-carboxylate

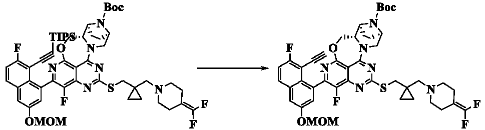

Tert-butyl (5aS,6S,9R)-12-(((1-((4-(difluoromethyl)piperidin-1-yl)methyl) cyclopropyl)methyl)thio)-1-fluoro-2-(7-fluoro-3-(methoxymethoxy)-8-((triisopropylsilyl)ethyl)naphthalen-l-yl)-5a,6,7,8,9,10-hexahydro-5H-4-oxy-3,10a,11,13,14-pentaaza-6,9-methylaminonaphtho[1,8-ab]heptene-14-carboxylate (820 mg, 817.33 µmol) was dissolved in DMF (10 mL), and cesium fluoride (1.24 g, 8.17 mmol) was added. The obtained mixture was warmed to 90°C, reacted for 3 h, concentrated under reduced pressure, and purified by silica gel column chromatography to obtain compound tert-butyl (5aS,6S,9R)-12-(((1-((4-(difluoromethylene)piperidin-1-yl)methyl)cyclopropyl)methyl)thio)-2-(8-ethynyl-7-fluoro-3-(methoxymethoxy)naphthalen-1-yl)-1-fluoro-5a,6,7,8,9,10-hexahydro-5H-4-oxa-3,10a,11,13,14-pentaaza-6,9-methylnaphthol[1,8-ab]heptene-14-carboxylate (610 mg, yield 88.1%).

MS m/z (ESI):848[M+H].

### Step 9: Preparation of 4-((5aS,6S,9R)-12-(((1-((4-(difluoromethylene) piperidin-1-yl)methyl)cyclopropyl)methyl)thio)-1-fluoro-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalen-2-ol

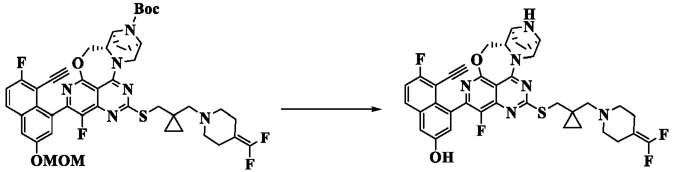

Tert-butyl (5aS,6S,9R)-12-(((1-((4-(difluoromethylene)piperidin-1-yl)methyl) cyclopropyl)methyl)thio)-2-(8-ethynyl-7-fluoro-3-(methoxymethoxy)naphthalen-1-yl)-1-fluoro-5a,6,7,8,9,10-hexahydro-5H-4-oxa-3,10a,11,13,14-pentaaza-6,9-methylnaphthol[1,8-ab]heptene-14-carboxylate (200 mg, 235.84 µmol) was dissolved in dichloromethane (2 mL), and trifluoroacetic acid (0.4 mL) was added dropwise. The obtained mixture was reacted at room temperature for 2 h, concentrated under reduced pressure, and purified by preparative liquid chromatogram to obtain compound 4-((SaS,6S,9R)-12-(((1-((4-(difluoromethylene)piperidin-1-yl)methyl)cyclopropyl)methyl)thio)-1-fluoro-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalen-2-ol (51 mg, yield 30.7%).

MS m/z (ESI):704[M+H].

### Example 58-1

### Step 1: Preparation of tert-butyl (5aS,6R,9S)-2-chloro-12-((1-((4-(difluoromethylene)piperidin-1-yl)methyl)cyclopropyl)methoxy)-1-fluoro-Sa,6,7,8,9, 10-hexahydro-SH-4-oxa-3, 10a, 11, 13, 14-pentaaza-6,9-methylnaphtho[1,8-ab]heptene-4-carboxylate

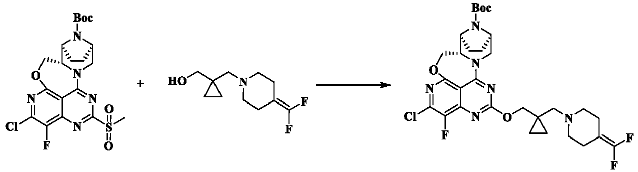

Lithium bis(trimethylsilyl)amide (1 M, 0.85 mL) was added dropwise to a solution of tert-butyl (5aS,6R,9S)-2-chloro-1-fluoro-12-(methylsulfonyl)-5a,6,7,8,9,10-hexahydro-5H-4-oxo-3,10a,11,13,14-pentaaza-6,9-methylnaphtho[1,8-ab]heptene-4-carboxylate (140 mg, 0.28 mmol) and (1-((4-(difluoromethylene)piperidin-1-yl)methyl)cyclopropyl)methanol (79 mg, 0.36 mmol) in tetrahydrofuran (10 mL) at 0°C, and the obtained mixture was stirred at 0°C for 5 min after the addition was completed. The reaction liquid was poured into an ammonium chloride aqueous solution and the obtained mixture was extracted with ethyl acetate. The organic phase was washed with saturated sodium chloride aqueous solution, dried over anhydrous sodium sulfate, filtered, and concentrated to obtain tert-butyl (5aS,6R,9S)-2-chloro-12-((1-((4-(difluoromethylene)piperidin-1-yl)methyl)cyclopropyl)methoxy)-1-fluoro-5a,6,7,8,9,10-hexahydro-5H-4-oxa-3,10a,11,13,14-pentaaza-6,9-methylnaphtho[1,8-ab]heptene-4-carboxylate (178 mg, yield 100%).

MS m/z (ESI): 637 [M+H].

### Step 2: Preparation of tert-butyl (SaS,6R,9S)-12-((1-((4-(difluoromethylene)piperidin-1-yl)methyl)cyclopropyl)methoxy)-2-(8-ethyl-7-fluoro-3-(methoxymethoxynaphthalen-1-yl)-1-fluoro-5a,6,7,8,9,10-hexahydro-5H-4-oxa-3,10a,11,13,14-pentaaza-6,9-methylnaphtho[1,8-ab]heptene-14carboxylate

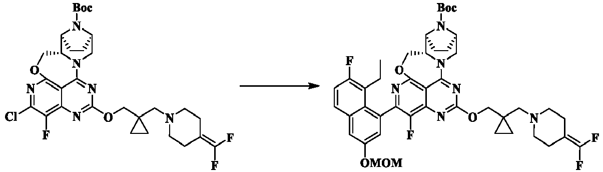

Tert-butyl (5aS,6R,9S)-2-chloro-12-((1-((4-(difluoromethylene)piperidin-1-yl)methyl)cyclopropyl)methoxy)-1-fluoro-5a,6,7,8,9,10-hexahydro-5H-4-oxa-3,10a,11,13,14-pentaaza-6,9-methylnaphtho[1,8-ab]heptene-4-carboxylate (178 mg, 0.28 mmol), 2-(8-ethyl-7-fluoro-3-(methoxymethoxy)naphthalen-1-yl)-4,4,5,5-tetramethyl-1,3,2-dioxaborane (420 mg, 0.42 mmol), methanesulfonate (dimethyl-n-butylphosphino)-2'-amino-1,1'-biphenyl-2-yl)palladium(II) dichloromethane adduct (20 mg, 0.028 mmol), cesium carbonate (228 mg, 0.7 mmol) were stirred in dioxane (10 mL) and water (2 mL) at 90°C for 1 h. Water was added to the reaction liquid, and the obtained mixture was extracted with ethyl acetate. The organic phase was washed with saturated sodium chloride aqueous solution, dried over anhydrous sodium sulfate, filtered, concentrated, and purified by silica gel column chromatography to obtain tert-butyl (SaS,6R,9S)-12-((1-((4-(difluoromethylene)piperidin-1-yl)methyl)cyclopropyl)methoxy)-2-(8-ethyl-7-fluoro-3-(methoxymethoxynaphthalen-1-yl)-1-fluoro-5a,6,7,8,9,10-hexahydro-5H-4-oxa-3, 10a, 11, 13,14-pentaaza-6,9-methylnaphtho[1,8-ab]heptene-14carboxylate (173 mg, yield 74%).

MS m/z (ESI): 835 [M+H].

### Step 3: Preparation of 4-((SaS,6R,9S)-12-((1-((4-(difluoromethylene) piperidin-1-yl)methyl)cyclopropyl)methoxy)-1-fluoro-5a,6,7,8,9,10-hexahydro-5H-4-oxa-3,10a,11,13,14-pentaaza-6,9-methylnaphtho[1,8-ab]hept-2-yl)-5-ethyl-6-fluoronaphthalen-2-ol

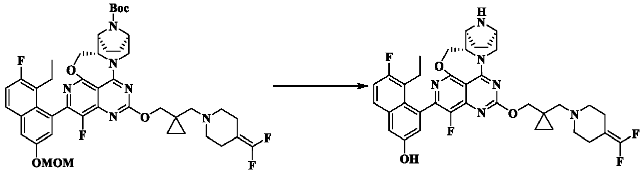

A solution of hydrochloric acid in dioxane (4 M, 4 mL) (< 10°C) was added dropwise to a solution of tert-butyl (SaS,6R,9S)-12-((1-((4-(difluoromethylene) piperidin-1-yl)methyl)cyclopropyl)methoxy)-2-(8-ethyl-7-fluoro-3-(methoxymethoxynaphthalen-1-yl)-1-fluoro-5a,6,7,8,9,10-hexahydro-5H-4-oxa-3,10a,11,13,14-pentaaza-6,9-methylnaphtho[1,8-ab]heptene-14carboxylate (173 mg, 0.21 mmol) in dichloromethane (4 mL) and methanol (0.8 mL) at 5°C, and the obtained mixture was stirred at room temperature for 0.5 h after the addition was completed. The reaction liquid was slowly added to an sodium bicarbonate aqueous solution (80 mL), the obtained mixture was extracted with dichloromethane/methanol = 10/1, and the organic phase was washed with saturated sodium chloride aqueous solution, dried over anhydrous sodium sulfate, filtered, concentrated, and separated and purified by high performance liquid chromatography to obtain 4-((5aS,6R,9S)-12-((1-((4-(difluoromethylene)piperidin-1-yl)methyl)cyclopropyl)methoxy)-1-fluoro-5a,6,7,8,9,10-hexahydro-5H-4-oxa-3,10a,11,13,14-pentaaza-6,9-methylnaphtho[1,8-ab]hept-2-yl)-5-ethyl-6-fluoronaphthalen-2-ol (50 mg, yield 35%).

¹H NMR (400 MHz, MeOD) δ 7.69 - 7.60 (m, 1H), 7.31 - 7.17 (m, 2H), 7.13 - 7.06 (m, 0.5H), 7.03 - 6.95 (m, 0.5H), 5.21 - 5.02 (m, 1H), 4.68 - 4.54 (m, 3H), 4.53 - 4.33 (m, 3H), 4.26 - 4.11 (m, 1H), 3.92 - 3.71 (m, 2H), 2.86 - 2.64 (m, 4H), 2.62 - 2.36 (m, 2H), 2.30 (s, 3H), 2.24 - 2.12 (m, 1H), 2.07 - 1.76 (m, 5H), 0.89 (t, *J* = 8 Hz, 2H), 0.85 - 0.73 (m, 3H), 0.65 - 0.46 (m, 2H).

MS m/z (ESI): 691 [M+H].

### Example 74

### Step 1: Preparation of 1-chloroethyl (4-nitrophenyl)carbonate

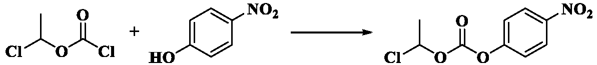

Triethylamine (4.60 g, 45 mmol) and 1-chloroethyl chloroformate (5 g, 35 mmol) were added to a solution of 4-nitrophenol (5.35 g, 38 mmol) in dichloromethane (100 mL) at 0°C, and the obtained mixture was stirred at room temperature for 1 h after the addition was completed. Water (100 mL) was added to the reaction liquid, and the obtained mixture was extracted with dichloromethane, and the organic phase was washed with a saturated aqueous sodium chloride solution, dried over anhydrous sodium sulfate, filtered, concentrated, and purified by silica gel column chromatography to obtain 1-chloroethyl(4-nitrophenyl)carbonate (7.7 g, yield 90%).

### Step 2: Preparation of 1-iodoethyl (4-nitrophenyl)carbonate

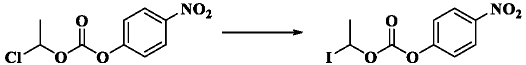

1-chloroethyl(4-nitrophenyl)carbonate (6 g, 24 mmol) and sodium iodide (14.7 g, 97.7 mmol) were stirred in acetone (60 mL) at 50°C for 40 h. The reaction liquid was filtered, and the filtrate was concentrated. Ethyl acetate was added, and then the obtained mixture was filtered. The filtrate was concentrated, and then purified by silica gel column chromatography to obtain 1-iodoethyl(4-nitrophenyl)carbonate (2 g, yield 24%).

### Step 3: Preparation of silver isobutyrate

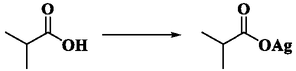

Isobutyric acid (1.5 g, 17 mmol) and silver oxide (2.37 g, 10 mmol) were stirred in acetonitrile (15 mL) and water (7.5 mL) at room temperature for 13 h. The reaction liquid was filtered and the filtrate was concentrated to obtain silver isobutyrate (1.3 g, yield 39%).

### Step 4: Preparation of 1-(4-nitrophenoxy)carbonyloxyethyl 2-methylpropanoate

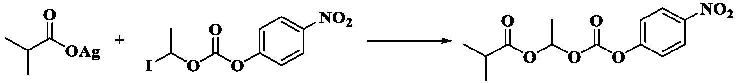

1-iodoethyl(4-nitrophenyl)carbonate (500 mg, 1.5 mmol) and silver isobutyrate (1.2 g, 5.9 mmol) were stirred in toluene (10 mL) at 50°C for 13 h. The reaction liquid was concentrated and purified by silica gel column chromatography to obtain 1-(4-nitrophenoxy)carbonyloxyethyl 2-methylpropanoate (200 mg, yield 45%).

### Step 5: Preparation of 1-(isobutyryloxy)ethyl (5aS,6S,9R)-12-((1-((4-(difluoromethylene)piperidin-1-yl)methyl)cyclopropyl)methoxy)-2-(8-ethynyl-7-fluoro-3-(methoxymethoxy)naphthalen-1-yl)-1-fluoro-5a,6,7,8,9,10-hexahydro-5H-4-oxa-3,10a,11,13,14-pentaaza-6,9-methylenenaphtho[1,8-ab]heptalene-14-carboxylate

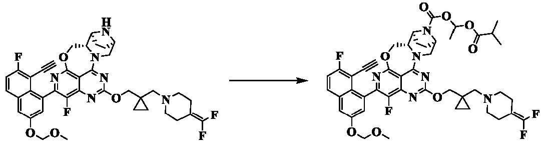

Triethylamine (70 mg, 0.70 mmol) and 4-dimethylaminopyridine (12 mg, 0.1 mmol) were added to a solution of (5aS,6S,9R)-12-((1-((4-(difluoromethylene)piperidin-1-yl)methyl)cyclopropyl)methoxy)-2-(8-ethynyl-7-fluoro-3-(methoxymethoxy)naphthalen-1-yl)-1-fluoro-5a,6,7,8,9,10-hexahydro-5H-4-oxa-3,10a,11,13,14-pentaaza-6,9-methylenenaphtho[1,8-ab]heptalene (100 mg, 0.14 mmol) and 1-(4-nitrophenoxy)carbonyloxyethyl 2-methylpropanoate (80 mg, 0.27 mmol) in dichloromethane (15 mL) at room temperature. After the addition was completed, the obtained mixture was raised to 40°C and stirred for 16 h. Concentration and then purification by silica gel column chromatography gave 1-(isobutyryloxy)ethyl (5aS,6S,9R)-12-((1-((4-(difluoromethylene)piperidin-1-yl)methyl)cyclopropyl)methoxy)-2-(8-ethynyl-7-fluoro-3-(methoxymethoxy)naphthalen-1-yl)-1-fluoro-5a,6,7,8,9,10-hexahydro-5H-4-oxa-3,10a,11,13,14-pentaaza-6,9-methylenenaphtho[1,8-ab]heptalene-14-carboxylate (95 mg, yield 78%).

MS m/z (ESI): 889 [M+H].

### Step 6: Preparation of 1-(isobutyryloxy)ethyl (5aS,6S,9R)-12-((1-((4-(difluoromethylene)piperidin-1-yl)methyl)cyclopropyl)methoxy)-2-(8-ethynyl-7-fluoro-3-hydroxynaphthalen-1-yl)-1-fluoro-5a,6,7,8,9,10-hexahydro-5H-4-oxa-3,10a,11,13,14-pentaaza-6,9-methylenenaphtho[1,8-ab]heptalene-14-carboxylate

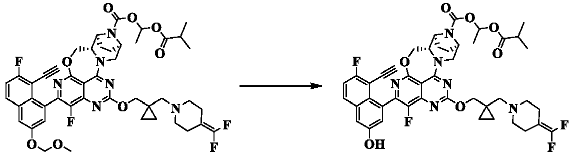

Trifluoroacetic acid (1 mL) was added to a solution of 1-(isobutyryloxy)ethyl (SaS,6S,9R)-12-((1-((4-(difluoromethylene)piperidin-1-yl)methyl)cyclopropyl) methoxy)-2-(8-ethynyl-7-fluoro-3-(methoxymethoxy)naphthalen-1-yl)-1-fluoro-5a,6,7,8,9,10-hexahydro-5H-4-oxa-3,10a,11,13,14-pentaaza-6,9-methylenenaphtho [1,8-ab]heptalene-14-carboxylate (95 mg, 0.11 mmol) in dichloromethane (3 mL) at 0°C. After the addition was completed, the obtained mixture was stirred at room temperature for 1.5 h. The reaction liquid was concentrated and redissolved in dichloromethane, and the obtained mixture was washed with sodium bicarbonate aqueous solution, dried over anhydrous sodium sulfate, filtered, concentrated and purified by preparative HPLC to obtain 1-(isobutyryloxy)ethyl (5aS,6S,9R)-12-((1-((4-(difluoromethylene)piperidin-1-yl)methyl)cyclopropyl)methoxy)-2-(8-ethynyl-7-fluoro-3-hydroxylnaphthalen-1-yl)-1-fluoro-5a,6,7,8,9,10-hexahydro-5H-4-oxa-3,10a,11,13,14-pentaaza-6,9-methylenenaphtho[1,8-ab]heptalene-14-carboxylate (36 mg, yield 39.9%).

MS m/z (ESI): 845 [M+H].

### Examples 75 to 79 refer to the synthesis of example 74:

| **Serial No.** | **Example structure** | **MS m/z (ESI)** | **Serial No.** | **Example structure** | **MS m/z (ESI)** |
|---|---|---|---|---|---|
| **75** | 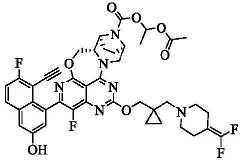 | 817 | 76 | 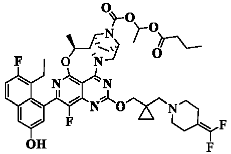 | 863 |
| | | [M+H] | | | [M+H] |
| **Example 75:** ¹H NMR (500 MHz, Chloroform-d) δ 7.76 (ddd, *J =* 8.1, 3.8, 2.0 Hz, 1H), 7.68 (s, 1H), 7.44 - 7.38 (m, 2H), 7.26 (dd, *J =* 10.1, 8.1 Hz, 1H), 4.51 (dd, *J =* 11.9, 6.4 Hz, 1H), 4.42 (tdd, *J =* 5.3, 3.5, 1.8 Hz, 1H), 4.34 (dd, *J* = 11.7, 3.5 Hz, 1H), 4.26 - 4.15 (m, 4H), 4.06 (s, 2H), 3.33 (s, 1H), 2.75 (t*, J* = 3.3 Hz, 4H), 2.64 - 2.54 (m, 3H), 2.37 - 2.26 (m, 4H), 2.25 - 2.20 (m, 2H), 2.20 - 2.14 (m, 1H), 2.05 (dtd, *J =* 10.6, 3.6, 1.8 Hz, 1H), 2.01 - 1.95 (m, 2H), 1.92 - 1.77 (m, 2H), 1.56 (d*, J* = 5.3 Hz, 3H), 1.19 (dd, *J =* 7.0, 3.7 Hz, 3H). | | | | | |
| **77** | 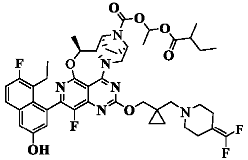 | 877 | **78** | 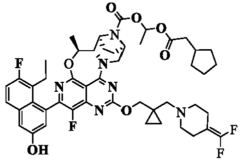 | 903 |
| | | [M+H] | | | [M+H] |
| **79** | 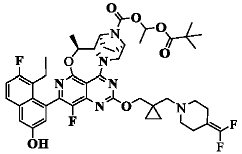 | 877 | | | |
| | | [M+H] | | | |

**Examples 80 to 87 refer to the synthesis of example 1-1 or example 74:**

| **Serial No.** | **Example structure** | **MS m/z (ESI)** | **Serial No.** | **Example structure** | **MS m/z (ESI)** |
|---|---|---|---|---|---|
| **80** | 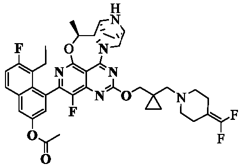 | 747 | **81** | 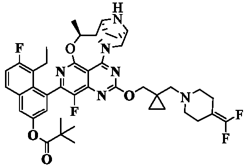 | 789 |
| | | [M+H] | | | [M+H] |
| **82** | 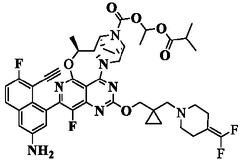 | 858 | **83** | 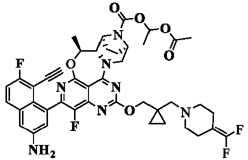 | 830 |
| | | [M+H] | | | [M+H] |
| **84** | 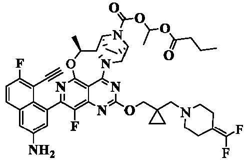 | 858 | **85** | 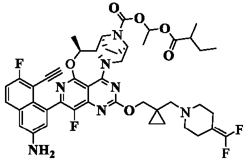 | 872 |
| | | [M+H] | | | [M+H] |
| **86** | 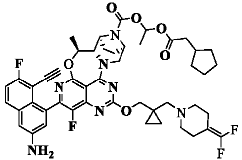 | 898 | **87** | 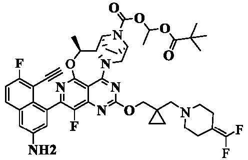 | 872 |
| | | [M+H] | | | [M+H] |

**Examples 88 to 89 refer to the synthesis of example 1-1:**

| **Serial No.** | **Example structure** | **MS m/z (ESI)** | **Serial No.** | **Example structure** | **MS m/z (ESI)** |
|---|---|---|---|---|---|
| **88** | 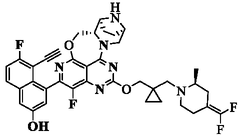 | 701 [M+H ] | 89 | 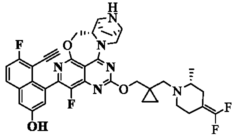 | 701 [M+H ] |
| **Example 88:** ¹H NMR (400 MHz, MeOD) δ 7.81 - 7.67 (m, 1H), 7.27 - 7.02 (m, 3H), 5.87 (s, 1H), 4.97 (ddd, *J* = 13.3, 10.3, 2.3 Hz, 1H), 4.59 - 4.29 (m, 5H), 4.28 - 4.11 (m, 1H), 4.11 - 3.99 (m, 1H), 3.65 (dd, *J* = 32.5, 5.7 Hz, 2H), 3.52 - 3.33 (m, 1H), 3.19 - 3.11 (m, 2H), 3.09 - 2.95 (m, 2H), 2.74 (s, 1H), 2.43 (s, 1H), 2.32 - 2.03 (m, 4H), 0.98 (d, *J* = 6.4 Hz, 3H), 0.76 - 0.56 (m, 2H), 0.55 - 0.37 (m, 2H). | | | | | |

**The following synthesis method can also be adopted for example 89:**

### Example 89

### Step 1: Preparation of tert-butyl (2R)-4-(difluoromethylene)-2-methylpiperidine-1-carboxylate

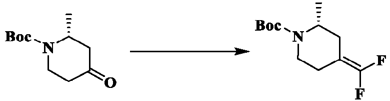

Difluoromethyl-2-pyridyl sulfone (2.27 g, 11.8 mmol) was added to a solution of tert-butyl (2R)-2-methyl-4-oxopiperidine-1-carboxylate (3 g, 14.1 mmol) in N,N-dimethylformamide (30 mL) at -50°C. After the addition was completed, potassium tert-butoxide (2.37 g, 21.1 mmol) was added in portions at -40 to -50°C. After the addition was completed, the obtained mixture was stirred at -40 to 50°C for 1 h. Ammonium chloride aqueous solution (50 mL) was added to the reaction liquid, then the obtained mixture was adjusted to pH 5 with hydrochloric acid aqueous solution (3 M), extracted with ethyl acetate, and the organic phase was washed with sodium bicarbonate aqueous solution, then washed with saturated brine. The organic phase was dried over anhydrous sodium sulfate, filtered, concentrated, and purified by silica gel column chromatography to obtain tert-butyl (2R)-4-(difluoromethylene)-2-methylpiperidine-1-carboxylate (2.7 g, yield 78%).

MS m/z (ESI): 248 [M+H].

### Step 2: Preparation of (2R)-4-(difluoromethylene)-2-methylpiperidine trifluoroacetate

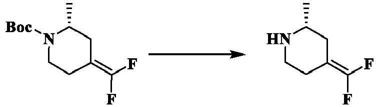

Trifluoroacetic acid (7 mL) was added to a solution of tert-butyl (2R)-4-(difluoromethylene)-2-methylpiperidine-1-carboxylate (2.7 g, 10.9 mmol) in dichloromethane (21 mL) at room temperature, and the obtained mixture was stirred at room temperature for 2 h after the addition was completed. The reaction liquid was concentrated to obtain (2R)-4-(difluoromethylene)-2-methylpiperidine trifluoroacetate (2.85 g, yield 100%).

MS m/z (ESI): 148 [M+H].

### Step 3: Preparation of methyl 1-chlorocarbonylcyclopropanecarboxylate

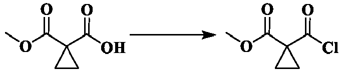

Oxalyl chloride (1.7 g, 13.3 mmol) was added to a solution of 1-methoxycarbonylcyclopropanecarboxylic acid (1.6 g, 11.1 mmol) and N,N-dimethylformamide (162 mg, 2.22 mmol) in dichloromethane (30 mL) at 0°C, and the obtained mixture was stirred at room temperature for 3 h after the addition was completed. The reaction liquid was concentrated to obtain methyl 1-chlorocarbonylcyclopropanecarboxylate (1.8 g, yield 100%).

### Step 4: Preparation of methyl 1-[(2R)-4-(difluoromethylene)-2-methylpiperidine-1-carbonyl]cyclopropanecarboxylate

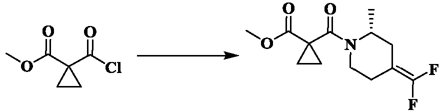

Triethylamine (5.45 g, 53.8 mmol) was added to a solution of (2R)-4-(difluoromethylene)-2-methylpiperidine trifluoroacetate (2.81 g, 10.8 mmol) in dichloromethane (50 mL) at 0°C, and then a solution of methyl 1-chlorocarbonylcyclopropanecarboxylate (1.75 g, 10.8 mmol) in dichloromethane (10 mL) was added, and the obtained mixture was slowly warmed to room temperature and stirred for 13 h. Water (100 mL) was added to the reaction liquid, and the obtained mixture was extracted with dichloromethane. The organic phase was washed with an ammonium chloride aqueous solution and then with saturated sodium chloride aqueous solution. The organic phase was dried over anhydrous sodium sulfate, filtered, concentrated, and then purified by silica gel column chromatography to obtain methyl 1-[(2R)-4-(difluoromethylene)-2-methylpiperidine-1-carbonyl]cyclopropanecarboxylate (2.5 g, yield 85%).

MS m/z (ESI): 274 [M+H].

### Step 5: Preparation of [1-[[(2R)-4-(difluoromethylene)-2-methyl-1-piperidinyl]methyl]cyclopropyl]methanol

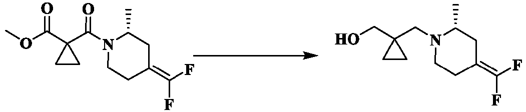

Lithium aluminum tetrahydride (833 mg, 22 mmol) was added in portions to a solution of methyl 1-[(2R)-4-(difluoromethylene)-2-methylpiperidine-1-carbonyl]cyclopropanecarboxylate (2.5 g, 9.15 mmol) in tetrahydrofuran (30 mL) at 0°C, and the obtained mixture was stirred at room temperature for 30 min after the addition was completed. Additional tetrahydrofuran (50 mL) was added to the reaction liquid, and the reaction liquid was quenched with sodium sulfate decahydrate at 0°C, stirred for 20 min, filtered through diatomaceous earth. The filtrate was concentrated, and then purified by silica gel column chromatography to obtain [1-[[(2R)-4-(difluoromethylene)-2-methyl-1-piperidyl]methyl]cyclopropyl] methanol (1.8 g, yield 85%).

MS m/z (ESI): 232 [M+H].

### Step 6: Preparation of tert-butyl (1S,2S,5R)-2-[(1S)-1-(7-chloro-8-fluoro-4-hydroxy-2-methylsulfanyl-pyrido[4,3-d]pyrimidin-5-yl)oxyethyl]-3,8-diazabicyclo[3.2.1]octane-8-carboxylate

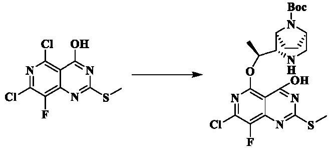

Sodium hydride (702 mg, 17.55 mmol, 60% purity) was added to a solution of 5,7-dichloro-8-fluoro-2-methylsulfanyl-pyrido[4,3-d]pyrimidin-4-ol (1.08 g, 3.86 mmol) and tert-butyl (1S,2S,5R)-2-[(1S)-1-hydroxyethyl]-3,8-diazabicyclo [3.2.1]octane-8-carboxylate (900 mg, 3.51 mmol) in tetrahydrofuran (30 mL) at 0°C, and the obtained mixture was stirred at 60°C for 1 h after the addition was completed. The reaction liquid was poured into ammonium chloride aqueous solution (100 mL) to quench the reaction, and ethyl acetate (100 mL) was added, a large amount of solid was insoluble, filtered, and dried to obtain tert-butyl (1S,2S,5R)-2-[(1S)-1-(7-chloro-8-fluoro-4-hydroxy-2-methylsulfanyl-pyrido[4,3-d]pyrimidin-5-yl)oxyethyl]-3,8-diazabicyclo[3.2.1]octane-8-carboxylate (1.4 g, yield 73%).

MS m/z (ESI): 500 [M+H].

### Step 7: Preparation of tert-butyl (5S,5aS,6R,9S)-2-chloro-1-fluoro-5-methyl-12-(methylthio)-5a,6,7,8,9,10-hexahydro-5H-4-oxa-3,10a,11,13,14-pentaaza-6,9-methylnaphtho[1,8-ab]heptene-4-carboxylate

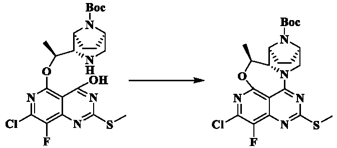

Tert-butyl (1S,2S,SR)-2-[(1S)-1-(7-chloro-8-fluoro-4-hydroxy-2-methylsulfanylpyrido[4,3-d]pyrimidin-5-yl)oxyethyl]-3,8-diazabicyclo[3.2.1]octane-8-carboxylate (1.4 g, 2.80 mmol), N,N-diisopropylethylamine (3.62 g, 28 mmol) and phosphorus oxychloride (1.72 g, 11.2 mmol) were stirred in chloroform (30 mL) at 70°C for 1 h. The reaction liquid was poured into sodium bicarbonate aqueous solution (60 mL), the obtained mixture was extracted with dichloromethane. The organic phase was washed with saturated sodium chloride aqueous solution, dried over anhydrous sodium sulfate, filtered, concentrated and purified by silica gel column chromatography to obtain tert-butyl (5S,5aS,6R,9S)-2-chloro-1-fluoro-5-methyl-12-(methylthio)-5a,6,7,8,9,10-hexahydro-5H-4-oxa-3,10a,11,13,14-pentaaza-6,9-methylnaphtho[1,8-ab]heptene-4-carboxylate (1.1 g, yield 82%).

MS m/z (ESI): 482 [M+H].

### Step 8: Preparation of tert-butyl (5S,5aS,6R,9S)-1-fluoro-2-(7-fluoro-3-(methoxymethoxy)-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methylnaphtho[1,8-ab]heptene-4-carboxylate

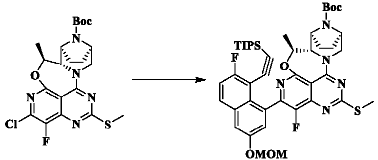

Tert-butyl (5S,5aS,6R,9S)-2-chloro-1-fluoro-5-methyl-12-(methylthio)-5a,6,7,8,9, 10-hexahydro-5H-4-oxa-3, 10a, 11, 13,14-pentaaza-6,9-methylnaphtho[1,8-ab]heptene-4-carboxylate (550 mg, 1.14 mmol), 2-[2-fluoro-6-(methoxymethoxy)-8-(4,4,5,5-tetramethyl-1,3,2-dioxyboran-2-yl)-1-naphthyl]ethynyltriisopropylsilane (877 mg, 1.71 mmol), methanesulfonic acid (dimethyl-n-butylphosphino)-2'-amino-1,1'-biphenyl-2-yl)palladium(II) dichloromethane adduct (125 mg, 0.17 mol), sodium hydroxide aqueous solution (2 M, 2.85 mL) were stirred in tetrahydrofuran (8 mL) under microwave at 100°C for 50 min. An ammonium chloride aqueous solution (50 mL) was added, and the obtained mixture was extracted with ethyl acetate. The organic phase was washed with saturated sodium chloride aqueous solution, dried over anhydrous sodium sulfate, filtered, concentrated and purified by silica gel chromatography to obtain tert-butyl (5S,5aS,6R,9S)-1-fluoro-2-(7-fluoro-3-(methoxymethoxy)-8-((triisopropylsilyl) ethynyl)naphthalen-1-yl)-5-methyl-12-(methylthio)-5a,6,7,8,9,10-hexahydro-5H-4-oxa-3,10a,11,13,14-pentaaza-6,9-methylnaphtho[1,8-ab]heptene-4-carboxylate (0.9 g, yield 94%).

MS m/z (ESI): 832 [M+H].

### Step 9: Preparation of tert-butyl (5S,5aS,6R,9S)-1-fluoro-2-(7-fluoro-3-(methoxymethoxy)-8-((triisopropylsilyl)ethynyl)naphthalen-1-yl)-5-methyl-12-(methylsulfonyl)-5a,6,7,8,9,10-hexahydro-5H-4-oxa-3,10a,11,13,14-pentaaza-6,9-methylnaphtho[1,8-ab]heptene-4-carboxylate

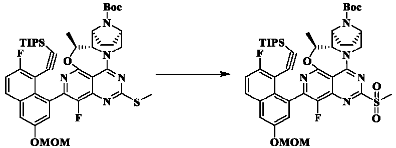

M-chloroperoxybenzoic acid (1.1 g, 5.41 mmol, 85% purity) was added to a solution of tert-butyl (5S,5aS,6R,9S)-1-fluoro-2-(7-fluoro-3-(methoxymethoxy)-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methylnaphtho[1,8-ab]heptene-4-carboxylate (1.8 g, 2.16 mmol) in dichloromethane (30 mL) at 0°C, and the obtained mixture was stirred at room temperature for 30 min after the addition was completed. The reaction liquid was directly concentrated and purified by silica gel column chromatography to obtain tert-butyl (5S,5aS,6R,9S)-1-fluoro-2-(7-fluoro-3-(methoxymethoxy)-8-((triisopropylsilyl)ethynyl)naphthalen-1-yl)-5-methyl-12-(methylsulfonyl)-5a,6,7,8,9,10-hexahydro-5H-4-oxa-3,10a,11,13,14-pentaaza-6,9-methylnaphtho[1,8-ab]heptene-4-carboxylate (740 mg, yield 40%).

MS m/z (ESI): 864 [M+H].

### Step 10: Preparation of tert-butyl (5S,5aS,6R,9S)-12-((1-((((R)-4-(difluoromethylene)-2-methylpiperidin-1-yl)methyl)cyclopropyl)methoxy)-1-fluoro-2-(7-fluoro-3-(methoxymethoxy)-8-((triisopropylsilyl)ethynyl)naphthalen-1-yl)-5-methyl-5a,6,7,8,9,10-hexahydro-5H-4-oxa-3,10a,11,13,14-pentaaza-6,9-methylnaphtho[1,8-ab]heptene-4-carboxylate

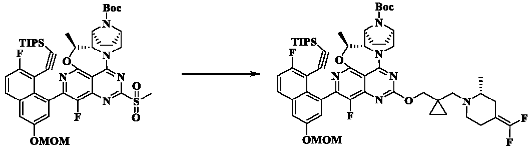

Sodium hydride (171 mg, 4.28 mmol, 60% purity) was added to a solution of tert-butyl (5S,5aS,6R,9S)-1-fluoro-2-(7-fluoro-3-(methoxymethoxy)-8-((triisopropylsilyl) ethynyl)naphthalen-1-yl)-5-methyl-12-(methylsulfonyl)-5a,6,7,8,9,10-hexahydro-5H-4-oxa-3,10a,11,13,14-pentaaza-6,9-methylnaphtho[1,8-ab]heptene-4-carboxylate (740 mg, 0.86 mmol) and [1-[[(2R)-4-(difluoromethylene)-2-methyl-1-piperidyl]methyl]cyclopropyl]methanol (792 mg, 3.43 mmol) in tetrahydrofuran (20 mL) at 0°C, and the obtained mixture was stirred at room temperature for 1 h after the addition was completed. An ammonium chloride aqueous solution (40 mL) was added, and the obtained mixture was extracted with ethyl acetate. The organic phase was washed with saturated sodium chloride aqueous solution, dried over anhydrous sodium sulfate, filtered, and concentrated to obtain tert-butyl (5S,5aS,6R,9S)-12-((1-((((R)-4-(difluoromethylene)-2-methylpiperidin-1-yl)methyl)cyclopropyl) methoxy)-1-fluoro-2-(7-fluoro-3-(methoxymethoxy)-8-((triisopropylsilyl) ethynyl)naphthalen-1-yl)-5-methyl-5a,6,7,8,9,10-hexahydro-5H-4-oxa-3,10a,11,13,14-pentaaza-6,9-methylnaphtho[1,8-ab]heptene-4-carboxylate (868.5 mg, yield 100%).

MS m/z (ESI): 1015 [M+H].

### Step 11: Preparation of tert-butyl (5S,5aS,6R,9S)-12-((1-((((R)-4-(difluoromethylene)-2-methylpiperidin-1-yl)methyl)cyclopropyl)methoxy)-2-(8-ethynyl-7-fluoro-3-(methoxymethoxynaphthalen-1-yl)-1-fluoro-5-methyl-5a,6,7,8,9,10-hexahydro-5H-4-oxa-3,10a,11,13,14-pentaaza-6,9-methylnaphtho[1,8-ab]heptene-4-carboxylate

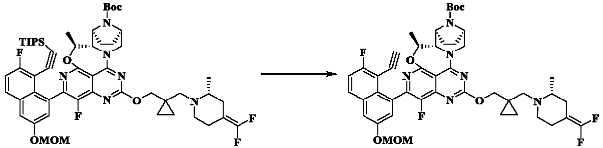

A solution of tetrabutylammonium fluoride in tetrahydrofuran (1 M, 1.7 mL) was added to a solution of tert-butyl (5S,5aS,6R,9S)-12-((1-((((R)-4-(difluoromethylene)-2-methylpiperidin-1-yl)methyl)cyclopropyl)methoxy)-1-fluoro-2-(7-fluoro-3-(methoxymethoxy)-8-((triisopropylsilyl)ethynyl)naphthalen-1-yl)-5-methyl-5a,6,7,8,9,10-hexahydro-5H-4-oxa-3,10a,11,13,14-pentaaza-6,9-methylnaphtho[1,8-ab]heptene-4-carboxylate (868.5 mg, 0.86 mmol) in tetrahydrofuran (8 mL) at room temperature, and the obtained mixture was stirred at room temperature for 30 min after addition was completed. Water (50 mL) was added, and the obtained mixture was extracted with ethyl acetate. The organic phase was washed with water, dried over anhydrous sodium sulfate, filtered, concentrated, and purified by silica gel chromatography to obtain tert-butyl (5S,5aS,6R,9S)-12-((1-((((R)-4-(difluoromethylene)-2-methylpiperidin-1-yl)methyl)cyclopropyl) methoxy)-2-(8-ethynyl-7-fluoro-3-(methoxymethoxynaphthalen-1-yl)-1-fluoro-5-methyl-5a,6,7,8,9,10-hexahydro-5H-4-oxa-3,10a,11,13,14-pentaaza-6,9-methylnaphtho[1,8-ab]heptene-4-carboxylate (600 mg, yield 82%).

MS m/z (ESI): 859 [M+H].

### Step 12: Preparation of 4-((5S,5aS,6R,9S)-12-((1-((((R)-4-(difluoromethylene)-2-methylpiperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalen-2-ol

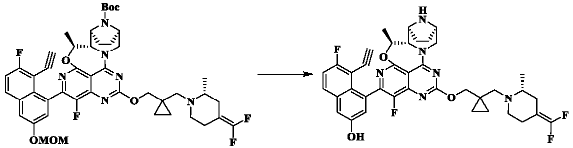

A solution of hydrochloric acid in dioxane (4 M, 8 mL) was added to tert-butyl (5S,5aS,6R,9S)-12-((1-((((R)-4-(difluoromethylene)-2-methylpiperidin-1-yl)methyl)cyclopropyl)methoxy)-2-(8-ethynyl-7-fluoro-3-(methoxymethoxynaphthalen-1-yl)-1-fluoro-5-methyl-5a,6,7,8,9,10-hexahydro-5H-4-oxa-3,10a,11,13,14-pentaaza-6,9-methylnaphtho[1,8-ab]heptene-4-carboxylate (600 mg, 0.7 mmol) in dichloromethane (8 mL) and methanol (1.5 mL) at 0°C, and the obtained mixture was stirred at room temperature for 1 h after addition was completed. The reaction liquid was slowly added to a sodium bicarbonate aqueous solution (50 mL), and the obtained mixture was extracted with dichloromethane/methanol = 10/1, and then extracted with ethyl acetate/methanol = 10/1. The organic phase was washed with saturated sodium chloride aqueous solution, dried over anhydrous sodium sulfate, filtered, concentrated, and purified by silica gel column chromatography to obtain 4-((5S,5aS,6R,9S)-12-((1-((((R)-4-(difluoromethylene)-2-methylpiperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalen-2-ol (380 mg, yield 68%).

MS m/z (ESI): 715 [M+H].

¹H NMR (400 MHz, MeOD) δ 7.89 - 7.77 (m, 1H), 7.36 - 7.26 (m, 2H), 7.25 - 7.19 (m, 0.5H), 7.12 (m, 0.5H), 5.45 - 5.29 (m, 1H), 4.57 - 4.39 (m, 3H), 4.37 - 4.20 (m, 1H), 4.09 (d, *J* = 8 Hz, 1H), 3.77 - 3.58 (m, 2H), 3.25 - 2.92 (m, 4H), 2.80 - 2.62 (m, 1H), 2.50 - 2.33 (m, 1H), 2.34 - 2.13 (m, 3H), 2.13 - 1.99 (m, 2H), 1.98 - 1.70 (m, 3H), 1.59 (t, *J* = 8 Hz, 3H), 1.04 (d, *J* = 4 Hz, 3H), 0.79 - 0.63 (m, 2H), 0.61 - 0.42 (m, 2H).

**Examples 90 to 95 refer to the synthesis of example 1-1 or example 19-1:**

| **Serial No.** | **Example structure** | **MS m/z (ESI)** | **Serial No.** | **Example structure** | **MS m/z (ESI)** |
|---|---|---|---|---|---|
| **90** | 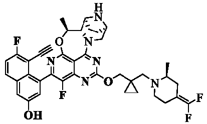 | 715 [M+H] | **91** | 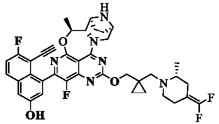 | 715 [M+H] |
| **Example 90:** ¹H NMR (400 MHz, MeOD) δ 7.90 - 7.76 (m, 1H), 7.37 - 7.25 (m, 2H), 7.24 - 7.12 (m, 1H), 5.45 - 5.30 (m, 1H), 4.58 - 4.39 (m, 3H), 4.37 - 4.21 (m, 1H), 4.10 (d, *J =* 8 Hz, 1H), 3.76 - 3.59 (m, 2H), 3.25 - 2.91 (m, 4H), 2.81 - 2.63 (m, 1H), 2.49 - 2.33 (m, 1H), 2.33 - 2.13 (m, 3H), 2.13 - 1.97 (m, 2H), 1.98 - 1.69 (m, 3H), 1.59 (t, *J =* 8 Hz, 3H), 1.03 (d, *J =* 4 Hz, 3H), 0.79 - 0.63 (m, 2H), 0.61 - 0.42 (m, 2H). | | | | | |
| **Example 91:** ¹H NMR (500 MHz, Chloroform-d) δ 7.76 (ddd, *J* = 8.1, 3.8, 2.0 Hz, 1H), 7.68 (s, 1H), 7.43 (d*, J* = 2.2 Hz, 1H), 7.40 (t, *J* = 2.2 Hz, 1H), 7.29 - 7.24 (m, 1H), 4.97 (p, *J* = 7.2 Hz, 1H), 4.07 (s, 2H), 4.03 - 3.96 (m, 2H), 3.73 (dd, *J =* 7.7, 4.9 Hz, 1H), 3.33 (s, 1H), 3.33 - 3.26 (m, 2H), 3.01 - 2.89 (m, 3H), 2.80 (ddd, *J* = 11.5, 4.6, 2.4 Hz, 1H), 2.55 (s, 2H), 2.51 - 2.33 (m, 3H), 2.27 - 2.19 (m, 3H), 2.03 - 1.91 (m, 4H), 1.74 - 1.62 (m, 2H), 1.35 (s, 3H), 1.10 (d, *J* = 7.5 Hz, 3H). | | | | | |
| **92** | 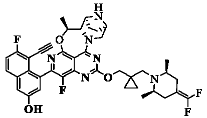 | 729 [M+H] | **93** | 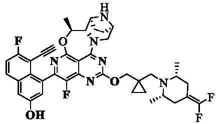 | 729 [M+H] |
| **94** | 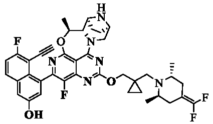 | 729 [M+H] | **95** | 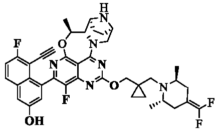 | 729 [M+H] |
| **Example 94:** ¹H NMR (500 MHz, Chloroform-d) δ 7.76 (ddd, *J* = 8.1, 3.8, 2.0 Hz, 1H), 7.68 (s, 1H), 7.44 - 7.38 (m, 2H), 7.28 - 7.23 (m, 1H), 4.97 (p, *J =* 7.2 Hz, 1H), 4.07 (s, 2H), 4.03 - 3.94 (m, 2H), 3.73 (dd, *J* = 7.7, 4.9 Hz, 1H), 3.33 (s, 1H), 3.33 - 3.26 (m, 2H), 3.18 (dqd, *J* = 8.3, 7.4, 5.5 Hz, 2H), 2.93 (dd, *J* = 6.1, 5.2 Hz, 1H), 2.66 (s, 2H), 2.45 (ddt, *J =* 13.6, 5.5, 4.2 Hz, 2H), 2.28 - 2.18 (m, 4H), 2.03 - 1.98 (m, 2H), 1.97 - 1.92 (m, 2H), 1.73 - 1.63 (m, 2H), 1.35 (s, 3H), 1.11 (d, *J =* 7.4 Hz, 6H). | | | | | |

**The following synthesis method can also be adopted for example 91:**

### Example 91

### Step 1: Preparation of tert-butyl (2R)-4-(difluoromethylene)-2-methylpiperidine-1-carboxylate

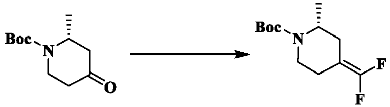

Difluoromethyl-2-pyridyl sulfone (2.27 g, 11.8 mmol) was added to a solution of tert-butyl (2R)-2-methyl-4-oxopiperidine-1-carboxylate (3 g, 14.1 mmol) in N,N-dimethylformamide (30 mL) at -50°C. After the addition was completed, potassium tert-butoxide (2.37 g, 21.1 mmol) was added in portions at -40 to -50°C. After the addition was completed, the obtained mixture was stirred at -40 to 50°C for 1 h. Ammonium chloride aqueous solution (50 mL) was added to the reaction liquid, then the obtained mixture was adjusted to pH 5 with hydrochloric acid aqueous solution (3 M), extracted with ethyl acetate, and the organic phase was washed with sodium bicarbonate aqueous solution, then washed with saturated brine. The organic phase was dried over anhydrous sodium sulfate, filtered, concentrated, and purified by silica gel column chromatography to obtain tert-butyl (2R)-4-(difluoromethylene)-2-methylpiperidine-1-carboxylate (2.7 g, yield 78%).

MS m/z (ESI): 248 [M+H].

### Step 2: Preparation of (2R)-4-(difluoromethylene)-2-methylpiperidine trifluoroacetate

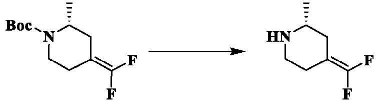

Trifluoroacetic acid (7 mL) was added to a solution of tert-butyl (2R)-4-(difluoromethylene)-2-methylpiperidine-1-carboxylate (2.7 g, 10.9 mmol) in dichloromethane (21 mL) at room temperature, and the obtained mixture was stirred at room temperature for 2 h after the addition was completed. The reaction liquid was concentrated to obtain (2R)-4-(difluoromethylene)-2-methylpiperidine trifluoroacetate (2.85 g, yield 100%).

MS m/z (ESI): 148 [M+H].

### Step 3: Preparation of methyl 1-chlorocarbonylcyclopropanecarboxylate

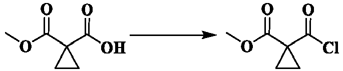

Oxalyl chloride (1.7 g, 13.3 mmol) was added to a solution of 1-methoxycarbonylcyclopropanecarboxylic acid (1.6 g, 11.1 mmol) and N,N-dimethylformamide (162 mg, 2.22 mmol) in dichloromethane (30 mL) at 0°C, and the obtained mixture was stirred at room temperature for 3 h after the addition was completed. The reaction liquid was concentrated to obtain methyl 1-chlorocarbonylcyclopropanecarboxylate (1.8 g, yield 100%).

### Step 4: Preparation of methyl 1-[(2R)-4-(difluoromethylene)-2-methylpiperidine-1-carbonyl]cyclopropanecarboxylate

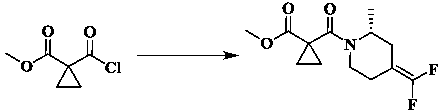

Triethylamine (5.45 g, 53.8 mmol) was added to a solution of (2R)-4-(difluoromethylene)-2-methylpiperidine trifluoroacetate (2.81 g, 10.8 mmol) in dichloromethane (50 mL) at 0°C, and then a solution of methyl 1-chlorocarbonylcyclopropanecarboxylate (1.75 g, 10.8 mmol) in dichloromethane (10 mL) was added, and the obtained mixture was slowly warmed to room temperature and stirred for 13 h. Water (100 mL) was added to the reaction liquid, and the obtained mixture was extracted with dichloromethane. The organic phase was washed with an ammonium chloride aqueous solution and then with saturated sodium chloride aqueous solution. The organic phase was dried over anhydrous sodium sulfate, filtered, concentrated, and then purified by silica gel column chromatography to obtain methyl 1-[(2R)-4-(difluoromethylene)-2-methylpiperidine-1-carbonyl]cyclopropanecarboxylate (2.5 g, yield 85%).

MS m/z (ESI): 274 [M+H].

### Step 5: Preparation of [1-[[(2R)-4-(difluoromethylene)-2-methyl-1-piperidinyl]methyl]cyclopropyl]methanol

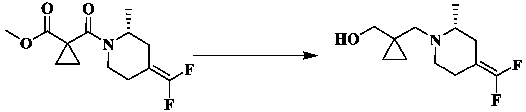

Lithium aluminum tetrahydride (833 mg, 22 mmol) was added in portions to a solution of methyl 1-[(2R)-4-(difluoromethylene)-2-methylpiperidine-1-carbonyl]cyclopropanecarboxylate (2.5 g, 9.15 mmol) in tetrahydrofuran (30 mL) at 0°C, and the obtained mixture was stirred at room temperature for 30 min after the addition was completed. Additional tetrahydrofuran (50 mL) was added to the reaction liquid, and the reaction liquid was quenched with sodium sulfate decahydrate at 0°C, stirred for 20 min, filtered through diatomaceous earth. The filtrate was concentrated, and then purified by silica gel column chromatography to obtain [1-[[(2R)-4-(difluoromethylene)-2-methyl-1-piperidyl]methyl]cyclopropyl] methanol (1.8 g, yield 85%).

MS m/z (ESI): 232 [M+H].

### Step 6: Preparation of tert-butyl (1S,2S,5R)-2-[(1S)-1-(7-chloro-8-fluoro-4-hydroxy-2-methylsulfanyl-pyrido[4,3-d]pyrimidin-5-yl)oxyethyl]-3,8-diazabicyclo[3.2.1]octane-8-carboxylate

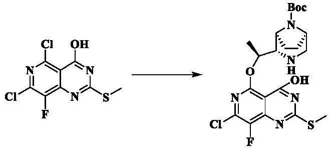

Sodium hydride (702 mg, 17.55 mmol, 60% purity) was added to a solution of 5,7-dichloro-8-fluoro-2-methylsulfanyl-pyrido[4,3-d]pyrimidin-4-ol (1.08 g, 3.86 mmol) and tert-butyl (1S,2S,5R)-2-[(1S)-1-hydroxyethyl]-3,8-diazabicyclo[3.2.1]octane-8-carboxylate (900 mg, 3.51 mmol) in tetrahydrofuran (30 mL) at 0°C, and the obtained mixture was stirred at 60°C for 1 h after the addition was completed. The reaction liquid was poured into ammonium chloride aqueous solution (100 mL) to quench the reaction, and ethyl acetate (100 mL) was added, a large amount of solid was insoluble, filtered, and dried to obtain tert-butyl (1S,2S,5R)-2-[(1S)-1-(7-chloro-8-fluoro-4-hydroxy-2-methylsulfanyl-pyrido[4,3-d]pyrimidin-5-yl)oxyethyl]-3,8-diazabicyclo[3.2.1]octane-8-carboxylate (1.4 g, yield 73%).

MS m/z (ESI): 500 [M+H].

### Step 7: Preparation of tert-butyl (5S,5aS,6R,9S)-2-chloro-1-fluoro-5-methyl-12-(methylthio)-5a,6,7,8,9,10-hexahydro-5H-4-oxa-3,10a,11,13,14-pentaaza-6,9-methylnaphtho[1,8-ab]heptene-4-carboxylate

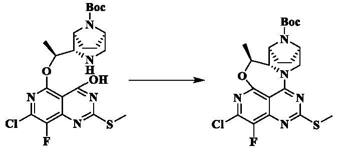

Tert-butyl (1S,2S,SR)-2-[(1S)-1-(7-chloro-8-fluoro-4-hydroxy-2-methylsulfanylpyrido[4,3-d]pyrimidin-5-yl)oxyethyl]-3,8-diazabicyclo[3.2.1]octane-8-carboxylate (1.4 g, 2.80 mmol), N,N-diisopropylethylamine (3.62 g, 28 mmol) and phosphorus oxychloride (1.72 g, 11.2 mmol) were stirred in chloroform (30 mL) at 70°C for 1 h. The reaction liquid was poured into sodium bicarbonate aqueous solution (60 mL), the obtained mixture was extracted with dichloromethane. The organic phase was washed with saturated sodium chloride aqueous solution, dried over anhydrous sodium sulfate, filtered, concentrated and purified by silica gel column chromatography to obtain tert-butyl (5S,5aS,6R,9S)-2-chloro-1-fluoro-5-methyl-12-(methylthio)-5a,6,7,8,9,10-hexahydro-5H-4-oxa-3,10a,11,13,14-pentaaza-6,9-methylnaphtho[1,8-ab]heptene-4-carboxylate (1.1 g, yield 82%).

MS m/z (ESI): 482 [M+H].

### Step 8: Preparation of tert-butyl (5S,5aS,6R,9S)-1-fluoro-2-(7-fluoro-3-(methoxymethoxy)-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methylnaphtho[1,8-ab]heptene-4-carboxylate

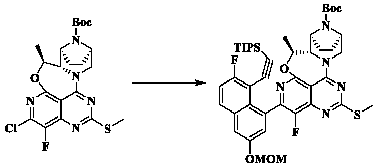

Tert-butyl (5S,5aS,6R,9S)-2-chloro-1-fluoro-5-methyl-12-(methylthio)-5a,6,7,8,9, 10-hexahydro-5H-4-oxa-3, 10a, 11, 13,14-pentaaza-6,9-methylnaphtho[1,8-ab]heptene-4-carboxylate (550 mg, 1.14 mmol), 2-[2-fluoro-6-(methoxymethoxy)-8-(4,4,5,5-tetramethyl-1,3,2-dioxyboran-2-yl)-1-naphthyl]ethynyltriisopropylsilane (877 mg, 1.71 mmol), methanesulfonic acid (dimethyl-n-butylphosphino)-2'-amino-1,1'-biphenyl-2-yl)palladium(II) dichloromethane adduct (125 mg, 0.17 mol), sodium hydroxide aqueous solution (2 M, 2.85 mL) were stirred in tetrahydrofuran (8 mL) under microwave at 100°C for 50 min. An ammonium chloride aqueous solution (50 mL) was added, and the obtained mixture was extracted with ethyl acetate. The organic phase was washed with saturated sodium chloride aqueous solution, dried over anhydrous sodium sulfate, filtered, concentrated and purified by silica gel chromatography to obtain tert-butyl (5S,5aS,6R,9S)-1-fluoro-2-(7-fluoro-3-(methoxymethoxy)-8-((triisopropylsilyl) ethynyl)naphthalen-1-yl)-5-methyl-12-(methylthio)-5a,6,7,8,9,10-hexahydro-5H-4-oxa-3,10a,11,13,14-pentaaza-6,9-methylnaphtho[1,8-ab]heptene-4-carboxylate (0.9 g, yield 94%).

MS m/z (ESI): 832 [M+H].

### Step 9: Preparation of tert-butyl (5S,5aS,6R,9S)-1-fluoro-2-(7-fluoro-3-(methoxymethoxy)-8-((triisopropylsilyl)ethynyl)naphthalen-1-yl)-5-methyl-12-(methylsulfonyl)-5a,6,7,8,9,10-hexahydro-5H-4-oxa-3,10a,11,13,14-pentaaza-6,9-methylnaphtho[1,8-ab]heptene-4-carboxylate

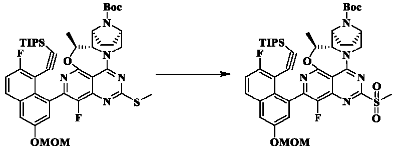

M-chloroperoxybenzoic acid (1.1 g, 5.41 mmol, 85% purity) was added to a solution of tert-butyl (5S,5aS,6R,9S)-1-fluoro-2-(7-fluoro-3-(methoxymethoxy)-8-((triisopropylsilyl)ethynyl)naphthalen-1-yl)-5-methyl-12-(methylthio)-5a,6,7,8,9,10-hexahydro-5H-4-oxa-3,10a,11,13,14-pentaaza-6,9-methylnaphtho [1,8-ab]heptene-4-carboxylate (1.8 g, 2.16 mmol) in dichloromethane (30 mL) at 0°C, and the obtained mixture was stirred at room temperature for 30 min after the addition was completed. The reaction liquid was directly concentrated and purified by silica gel column chromatography to obtain tert-butyl (5S,5aS,6R,9S)-1-fluoro-2-(7-fluoro-3-(methoxymethoxy)-8-((triisopropylsilyl)ethynyl)naphthalen-1-yl)-5-methyl-12-(methylsulfonyl)-5a,6,7,8,9,10-hexahydro-5H-4-oxa-3,10a, 11, 13,14-pentaaza-6,9-methylnaphtho[1,8-ab]heptene-4-carboxylate (740 mg, yield 40%).

MS m/z (ESI): 864 [M+H].

### Step 10: Preparation of tert-butyl (5S,5aS,6R,9S)-12-((1-((((R)-4-(difluoromethylene)-2-methylpiperidin-1-yl)methyl)cyclopropyl)methoxy)-1-fluoro-2-(7-fluoro-3-(methoxymethoxy)-8-((triisopropylsilyl)ethynyl)naphthalen-1-yl)-5-methyl-5a,6,7,8,9,10-hexahydro-5H-4-oxa-3,10a,11,13,14-pentaaza-6,9-methylnaphtho[1,8-ab]heptene-4-carboxylate

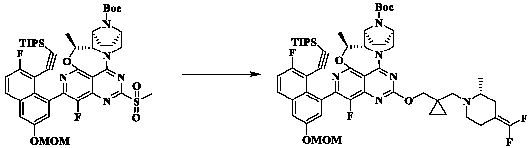

Sodium hydride (171 mg, 4.28 mmol, 60% purity) was added to a solution of tert-butyl (5S,5aS,6R,9S)-1-fluoro-2-(7-fluoro-3-(methoxymethoxy)-8-((triisopropylsilyl) ethynyl)naphthalen-1-yl)-5-methyl-12-(methylsulfonyl)-5a,6,7,8,9,10-hexahydro-5H-4-oxa-3,10a,11,13,14-pentaaza-6,9-methylnaphtho[1,8-ab]heptene-4-carboxylate (740 mg, 0.86 mmol) and [1-[[(2R)-4-(difluoromethylene)-2-methyl-1-piperidyl]methyl]cyclopropyl]methanol (792 mg, 3.43 mmol) in tetrahydrofuran (20 mL) at 0°C, and the obtained mixture was stirred at room temperature for 1 h after the addition was completed. An ammonium chloride aqueous solution (40 mL) was added, and the obtained mixture was extracted with ethyl acetate. The organic phase was washed with saturated sodium chloride aqueous solution, dried over anhydrous sodium sulfate, filtered, and concentrated to obtain tert-butyl (5S,5aS,6R,9S)-12-((1-((((R)-4-(difluoromethylene)-2-methylpiperidin-1-yl)methyl)cyclopropyl)methoxy) -1-fluoro-2-(7-fluoro-3-(methoxymethoxy)-8-((triisopropylsilyl)ethynyl) naphthalen-1-yl)-5-methyl-5a,6,7,8,9,10-hexahydro-5H-4-oxa-3,10a,11,13,14-pentaaza-6,9-methylnaphtho[1,8-ab]heptene-4-carboxylate (868.5 mg, yield 100%).

MS m/z (ESI): 1015 [M+H].

### Step 11: Preparation of tert-butyl (5S,5aS,6R,9S)-12-((1-((((R)-4-(difluoromethylene)-2-methylpiperidin-1-yl)methyl)cyclopropyl)methoxy)-2-(8-ethynyl-7-fluoro-3-(methoxymethoxynaphthalen-1-yl)-1-fluoro-5-methyl-5a,6,7,8,9,10-hexahydro-5H-4-oxa-3,10a,11,13,14-pentaaza-6,9-methylnaphtho[1,8-ab]heptene-4-carboxylate

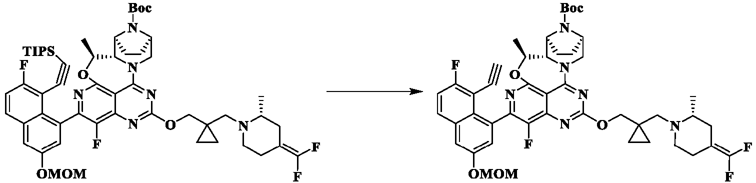

A solution of tetrabutylammonium fluoride in tetrahydrofuran (1 M, 1.7 mL) was added to a solution of tert-butyl (5S,5aS,6R,9S)-12-((1-((((R)-4-(difluoromethylene)-2-methylpiperidin-1-yl)methyl)cyclopropyl)methoxy)-1-fluoro-2-(7-fluoro-3-(methoxymethoxy)-8-((triisopropylsilyl)ethynyl)naphthalen-1-yl)-5-methyl-5a,6,7,8,9,10-hexahydro-5H-4-oxa-3,10a,11,13,14-pentaaza-6,9-methylnaphtho[1,8-ab]heptene-4-carboxylate (868.5 mg, 0.86 mmol) in tetrahydrofuran (8 mL) at room temperature, and the obtained mixture was stirred at room temperature for 30 min after addition was completed. Water (50 mL) was added, and the obtained mixture was extracted with ethyl acetate. The organic phase was washed with water, dried over anhydrous sodium sulfate, filtered, concentrated, and purified by silica gel chromatography to obtain tert-butyl (5S,5aS,6R,9S)-12-((1-((((R)-4-(difluoromethylene)-2-methylpiperidin-1-yl)methyl)cyclopropyl) methoxy)-2-(8-ethynyl-7-fluoro-3-(methoxymethoxynaphthalen-1-yl)-1-fluoro-5-methyl-5a,6,7,8,9,10-hexahydro-5H-4-oxa-3,10a,11,13,14-pentaaza-6,9-methylnaphtho[1,8-ab]heptene-4-carboxylate (600 mg, yield 82%).

MS m/z (ESI): 859 [M+H].

### Step 12: Preparation of 4-((5S,5aS,6R,9S)-12-((1-((((R)-4-(difluoromethylene)-2-methylpiperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalen-2-ol

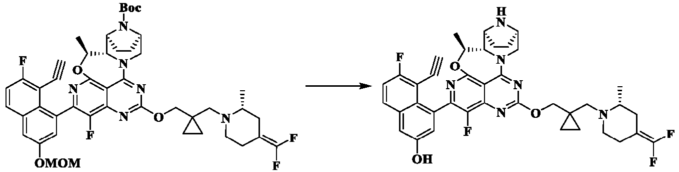

A solution of hydrochloric acid in dioxane (4 M, 8 mL) was added to tert-butyl (5S,5aS,6R,9S)-12-((1-((((R)-4-(difluoromethylene)-2-methylpiperidin-1-yl)methyl)cyclopropyl)methoxy)-2-(8-ethynyl-7-fluoro-3-(methoxymethoxynaphthalen-1-yl)-1-fluoro-5-methyl-5a,6,7,8,9,10-hexahydro-5H-4-oxa-3, 10a, 11, 13,14-pentaaza-6,9-methylnaphtho[1,8-ab]heptene-4-carboxylate (600 mg, 0.7 mmol) in dichloromethane (8 mL) and methanol (1.5 mL) at 0°C, and the obtained mixture was stirred at room temperature for 1 h after addition was completed. The reaction liquid was slowly added to a sodium bicarbonate aqueous solution (50 mL), and the obtained mixture was extracted with dichloromethane/methanol = 10/1, and then extracted with ethyl acetate/methanol = 10/1. The organic phase was washed with saturated sodium chloride aqueous solution, dried over anhydrous sodium sulfate, filtered, concentrated, and purified by silica gel column chromatography to obtain 4-((5S,5aS,6R,9S)-12-((1-((((R)-4-(difluoromethylene)-2-methylpiperidin-1-yl)methyl)cyclopropyl)methoxy)-1-fluoro-5-methyl-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalen-2-ol (380 mg, yield 68%).

¹H NMR (400 MHz, MeOD) δ 7.89 - 7.77 (m, 1H), 7.36 - 7.26 (m, 2H), 7.25 - 7.19 (m, 0.5H), 7.12 (m, 0.5H), 5.45 - 5.29 (m, 1H), 4.57 - 4.39 (m, 3H), 4.37 - 4.20 (m, 1H), 4.09 (d, *J =* 8 Hz, 1H), 3.77 - 3.58 (m, 2H), 3.25 - 2.92 (m, 4H), 2.80 - 2.62 (m, 1H), 2.50 - 2.33 (m, 1H), 2.34 - 2.13 (m, 3H), 2.13 - 1.99 (m, 2H), 1.98 - 1.70 (m, 3H), 1.59 (t, *J =* 8 Hz, 3H), 1.04 (d, *J =* 4 Hz, 3H), 0.79 - 0.63 (m, 2H), 0.61 - 0.42 (m, 2H).

MS m/z (ESI): 715 [M+H].

### Examples 96 to 120 refer to the synthesis of example 1-1:

| **Serial No.** | **Example structure** | **MS m/z (ESI)** | **Serial No.** | **Example structure** | **MS m/z (ESI)** |
|---|---|---|---|---|---|
| **97** | 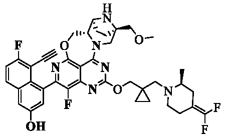 | 745 [M+H] | **98** | 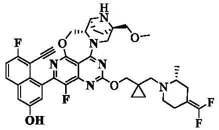 | 745 [M+H] |
| **99** | 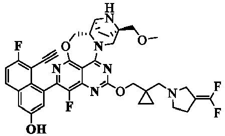 | 717 [M+H] | **100** | 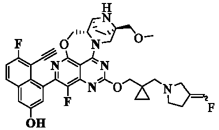 | 699 [M+H] |
| **101** | 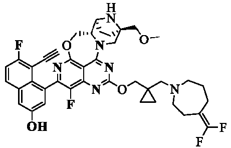 | 745 [M+H] | **102** | 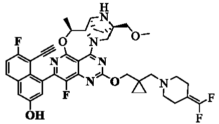 | 745 [M+H] |
| **103** | 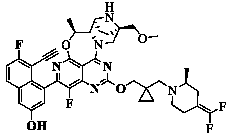 | 759 [M+H] | **104** | 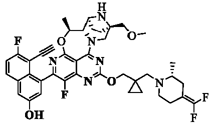 | 759 [M+H] |
| **105** | 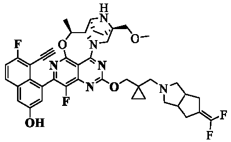 | 771 [M+H] | **106** | 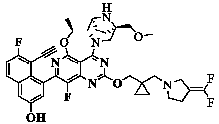 | 731 [M+H] |
| **107** | 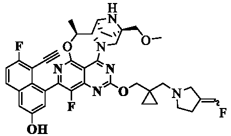 | 713 [M+H] | **108** | 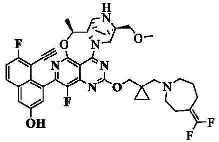 | 759 [M+H] |
| **109** | 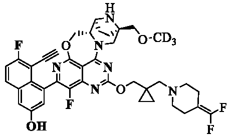 | 734 [M+H] | **110** | 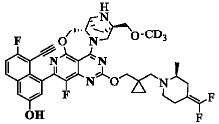 | 748 [M+H] |
| **111** | 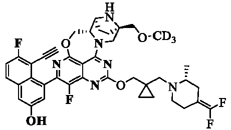 | 748 [M+H] | **112** | 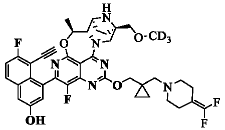 | 748 [M+H] |
| **113** | 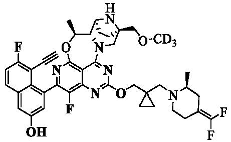 | 762 [M+H] | **114** | 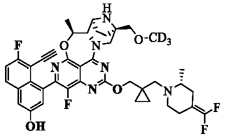 | 762 [M+H] |
| **115** | 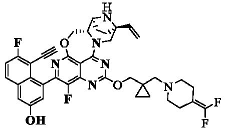 | 713 [M+H] | **116** | 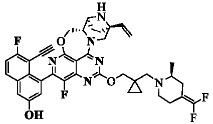 | 727 [M+H] |
| **117** | 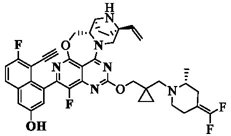 | 727 [M+H] | **118** | 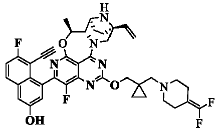 | 727 [M+H] |
| **119** | 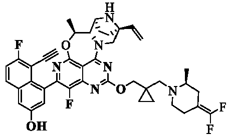 | 741 [M+H] | **120** | 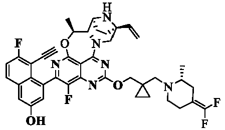 | 741 [M+H] |

**Examples 121 to 136 refer to the synthesis of example 1-1 or example 19:**

| **Serial No.** | **Example structure** | **MS m/z (ESI)** | **Serial No.** | **Example structure** | **MS m/z (ESI)** |
|---|---|---|---|---|---|
| **121** | 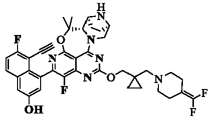 | 715 [M+H] | **122** | 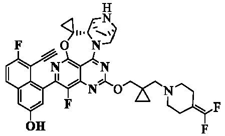 | 713 [M+H] |
| **Example 121:** ¹H NMR (500 MHz, Chloroform-d) δ 7.76 (ddd, *J* = 8.1, 3.8, 2.0 Hz, 1H), 7.68 (s, 1H), 7.43 (d*, J* = 2.2 Hz, 1H), 7.40 (t, *J* = 2.2 Hz, 1H), 7.26 (dd, *J* = 10.2, 8.1 Hz, 1H), 4.11 - 4.08 (m, 2H), 4.06 (s, 2H), 3.60 (d, *J =* 5.3 Hz, 1H), 3.42 (ddq, *J* = 6.9, 3.4, 1.7 Hz, 1H), 3.33 (s, 1H), 3.33 - 3.29 (m, 1H), 3.13 (t, *J =* 4.9 Hz, 1H), 2.75 (t, *J =* 3.3 Hz, 4H), 2.62 (s, 2H), 2.36 - 2.27 (m, 4H), 2.26 - 2.19 (m, 2H), 2.01 - 1.85 (m, 4H), 1.72 - 1.62 (m, 2H), 1.55 (s, 3H), 1.51 (s, 3H). | | | | | |
| **123** | 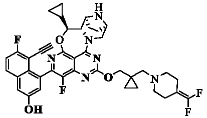 | 727 [M+H] | **124** | 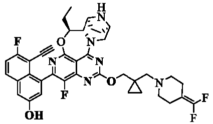 | 715 [M+H] |
| **Example 123:** ¹H NMR (500 MHz, Chloroform-d) δ 7.76 (ddd, *J* = 8.1, 3.8, 2.0 Hz, 1H), 7.68 (s, 1H), 7.43 (d, *J* = 2.2 Hz, 1H), 7.40 (t, *J* = 2.2 Hz, 1H), 7.26 (dd, *J* = 10.2, 8.1 Hz, 1H), 4.63 (dd, *J* = 8.9, 6.5 Hz, 1H), 4.06 (s, 2H), 4.03 - 3.92 (m, 2H), 3.60 (dd, *J =* 8.9, 4.9 Hz, 1H), 3.33 (s, 1H), 3.32 - 3.29 (m, 2H), 3.24 (dddd, *J* = 5.7, 4.8, 3.8, 1.8 Hz, 1H), 2.75 (t, *J =* 3.3 Hz, 4H), 2.62 (s, 2H), 2.36 - 2.26 (m, 4H), 2.25 - 2.20 (m, 2H), 2.03 - 1.91 (m, 4H), 1.75 - 1.67 (m, 2H), 1.56 (dtd, *J =* 12.6, 6.8, 5.9 Hz, 1H), 1.04 - 0.99 (m, 2H), 0.78 - 0.72 (m, 2H). | | | | | |
| **125** | 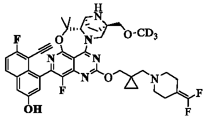 | 762 [M+H] | **126** | 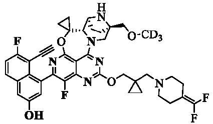 | 760 [M+H] |
| **127** | 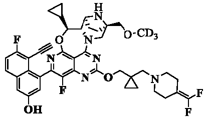 | 774 [M+H] | **128** | 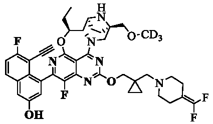 | 762 [M+H] |
| **129** | 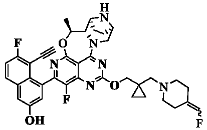 | 683 [M+H] | **130** | 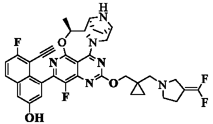 | 687 [M+H] |
| **Example 129:** ¹H NMR (400 MHz, MeOD) δ 7.92 - 7.76 (m, 1H), 7.37 - 7.26 (m, 2H), 7.23 (d*, J* = 4 Hz, 0.5H), 7.16 - 7.06 (m, 0.5H), 6.72 (s, 0.5H), 6.51 (s, 0.5H), 5.46 - 5.36 (m, 1H), 4.69 - 4.28 (m, 3H), 4.16 (d, *J =* 8 Hz, 1H), 3.92 - 3.59 (m, 5H), 3.11 - 2.79 (m, 5H), 2.60 - 2.43 (m, 2H), 2.37 - 2.24 (m, 2H), 2.20 - 2.07 (m, 1H), 2.02 - 1.75 (m, 3H), 1.59 (t, *J =* 8 Hz, 3H), 0.94 - 0.80 (m, 2H), 0.76 - 0.61 (m, 2H). | | | | | |
| **Example 130:** ¹H NMR (500 MHz, Chloroform-d) δ 7.76 (ddd, *J* = 8.1, 3.8, 2.0 Hz, 1H), 7.68 (s, 1H), 7.43 (d, *J* = 2.2 Hz, 1H), 7.40 (t, *J =* 2.2 Hz, 1H), 7.26 (dd, *J =* 10.2, 8.1 Hz, 1H), 4.97 (p, *J =* 7.2 Hz, 1H), 4.06 (s, 2H), 4.04 - 3.93 (m, 2H), 3.73 (dd, *J =* 7.7, 4.9 Hz, 1H), 3.33 (s, 1H), 3.32 - 3.25 (m, 2H), 2.99 - 2.96 (m, 2H), 2.93 (dd, *J* = 6.1, 5.2 Hz, 1H), 2.79 (t, *J* = 2.2 Hz, 2H), 2.64 (s, 2H), 2.44 (dp, *J* = 4.4, 2.3 Hz, 2H), 2.24 - 2.20 (m, 2H), 2.00 - 1.90 (m, 4H), 1.73 - 1.61 (m, 2H), 1.35 (d, *J =* 7.1 Hz, 3H). | | | | | |
| **131** | 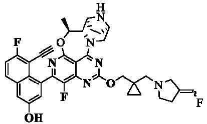 | 669 [M+H] | **132** | 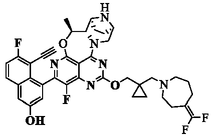 | 715 [M+H] |
| **Example 131:** ¹H NMR (400 MHz, DMSO) δ 10.12 (s, 1H), 7.96 (dt, *J =* 9.2, 5.7 Hz, 1H), 7.46 (td, *J =* 9.0, 7.5 Hz, 1H), 7.37 (d, *J =* 2.5 Hz, 1H), 7.13 (dd, *J =* 50.0, 2.6 Hz, 1H), 6.95 - 6.51 (m, 1H), 5.24 - 5.03 (m, 1H), 4.61 - 4.38 (m, 1H), 4.29 - 4.19 (m, 2H), 4.03 - 3.90 (m, 2H), 3.64 - 3.42 (m, 2H), 3.23 - 3.14 (m, 2H), 3.10 - 2.99 (m, 2H), 2.68 - 2.56 (m, 2H), 2.47 - 2.24 (m, 4H), 1.89 - 1.79 (m, 1H), 1.78 - 1.52 (m, 3H), 1.44 (dd, *J =* 8.1, 6.3 Hz, 3H), 0.75 - 0.53 (m, 2H), 0.50 - 0.41 (m, 2H). | | | | | |
| **Example 132:** ¹H NMR (400 MHz, MeOD) δ 7.84 (dt, *J=* 9.1, 6.1 Hz, 1H), 7.36 - 7.26 (m, 2H), 7.18 (dd, *J =* 42.4, 2.6 Hz, 1H), 5.43 - 5.32 (m, 1H), 4.60 - 4.32 (m, 4H), 4.10 (d, *J =* 8.6 Hz, 1H), 3.76 - 3.70 (m, 1H), 3.64 (d, *J =* 4.9 Hz, 1H), 3.24 - 3.15 (m, 1H), 2.98 (d, *J =* 22.2 Hz, 4H), 2.81 (d, *J =* 13.7 Hz, 2H), 2.42 (s, 2H), 2.34 - 2.25 (m, 2H), 2.06 (d, *J =* 9.1 Hz, 1H), 1.94 - 1.74 (m, 5H), 1.58 (t, *J =* 6.3 Hz, 3H), 0.79 (d, *J =* 4.7 Hz, 2H), 0.60 (s, 2H). | | | | | |
| **133** | 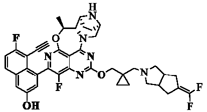 | 727 [M+H] | **134** | 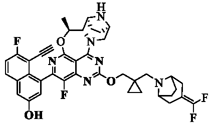 | 727 [M+H] |
| **Example 133:** ¹H NMR (500 MHz, Chloroform-d) δ 7.76 (ddd, J= 8.1, 3.8, 2.0 Hz, 1H), 7.68 (s, 1H), 7.43 (d, *J* = 2.2 Hz, 1H), 7.40 (t, *J =* 2.2 Hz, 1H), 7.26 (dd, *J* = 10.2, 8.1 Hz, 1H), 4.97 (p, *J =* 7.2 Hz, 1H), 4.06 (s, 2H), 4.04 - 3.94 (m, 2H), 3.73 (dd, *J* = 7.7, 4.9 Hz, 1H), 3.33 (s, 1H), 3.33 - 3.26 (m, 2H), 3.01 -2.91 (m, 5H), 2.57 - 2.52 (m, 2H), 2.49 (s, 2H), 2.43 - 2.36 (m, 2H), 2.25 - 2.19 (m, 2H), 2.17 - 2.08 (m, 2H), 2.02 - 1.91 (m, 4H), 1.74 - 1.63 (m, 2H), 1.35 (d, *J =* 7.1 Hz, 3H). | | | | | |
| **Example 134:** ¹H NMR (400 MHz, MeOD) δ 7.84 (dt, *J* = 9.1, 6.1 Hz, 1H), 7.36 - 7.26 (m, 2H), 7.27 - 7.12 (m, 1H), 5.43 - 5.32 (m, 1H), 4.64 - 4.36 (m, 3H), 4.33 - 4.15 (m, 3H), 3.85 - 3.70 (m, 4H), 3.23 - 3.06 (m, 2H), 2.84 (s, 2H), 2.54 (d, *J =* 15.5 Hz, 2H), 2.31 - 1.99 (m, 4H), 1.94 - 1.82 (m, 4H), 1.12 (d, *J* = 6.4 Hz, 3H), 0.99 - 0.81 (m, 4H). | | | | | |
| **135** | 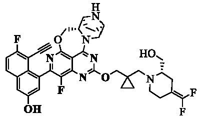 | 731 [M+H] | **136** | 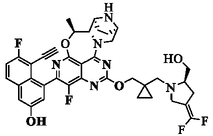 | 717 [M+H] |
| **Example 136:** ¹H NMR (400 MHz, MeOD) δ 7.89 - 7.79 (m, 1H), 7.37 - 7.26 (m, 2H), 7.22 (d*, J* = 4 Hz, 0.5H), 7.15 (d*, J =* 4 Hz, 0.5H), 5.13 - 4.92 (m, 1H), 4.50 - 4.37 (m, 1H), 4.19 - 4.08 (m, 1H), 4.04 - 3.86 (m, 2H), 3.83 - 3.69 (m, 3H), 3.65 - 3.52 (m, 4H), 3.27 - 3.17 (m, 1H), 2.95-2.80 (m, 1H), 2.76 - 2.49 (m, 2H), 2.35 - 2.14 (m, 1H), 2.00 - 1.77 (m, 4H), 1.75 - 1.64 (m, 1H), 1.59 (t, *J =* 8 Hz, 3H), 0.76 - 0.64 (m, 2H), 0.60-0.55 (m, 1H), 0.45-0.41 (m, 1H). | | | | | |

**The following synthesis methods can also be adopted for the following examples:**

### Example 124

### Step 1: Preparation of tert-butyl (1S,2S,SR)-3-benzyl-2-((S)-1-hydroxypropyl)-3,8-diazabicyclo[3.2.1]octane-8-carboxylate

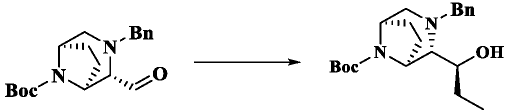

Tert-butyl (1R,2R,5S)-3-benzyl-2-formyl-3,8-diazabicyclo[3.2.1]octane-8-carboxylate (6.2 g, 18.76 mmol) was dissolved in tetrahydrofuran (120 mL), the obtained mixture was subjected to nitrogen replacement and cooled to -20°C, an ethylmagnesium bromide solution (38 mL, 38 mmol, 1 M in THF) was slowly added dropwise to the system. The reaction was kept warm for half an hour, then slowly warmed to room temperature and the stirring was continued for 3 h. After the reaction was completed, water was added to quench the reaction, the obtained mixture was extracted with ethyl acetate, washed with saturated sodium chloride aqueous solution. The organic phase was dried over anhydrous sodium sulfate, filtered, concentrated and separated by silica gel column chromatography to obtain tert-Butyl (1S,2S,5R)-3-benzyl-2-((S)-1-hydroxypropyl)-3,8-diazabicyclo[3.2.1] octane-8-carboxylate (3.4 g, yield 50.3%).

MS m/z (ESI):361 [M+H].

### Step 2: Preparation of tert-butyl (1S,2S,SR)-2-((S)-1-hydroxypropyl)-3,8-diazabicyclo[3.2.1]octane-8-carboxylate

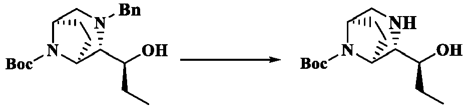

The raw material tert-butyl (1S,2S,SR)-3-benzyl-2-((S)-1-hydroxypropyl)-3,8-diazabicyclo[3.2.1]octane-8-carboxylate (3.4 g, 9.4 mmol) was dissolved in 60 mL of methanol, and palladium hydroxide/carbon (1.7 g, 10%wt) were weighed and added to the reaction liquid. The reaction flask was placed in a hydrogen gas tank with the pressure set to 65 psi, and hydrogen replacement was performed 3 times. After reacting at room temperature for 4 h, the reaction liquid was filtered through diatomaceous earth and methanol was used for washing. The filtrate was concentrated under reduced pressure and separated by silica gel column chromatography to obtain tert-butyl (1S,2S,5R)-2-((S)-1-hydroxypropyl)-3,8-diazabicyclo[3.2.1]octane-8-carboxylate (2.5 g, yield 98%).

MS m/z (ESI):271 [M+H].

### Step 3: Preparation of tert-butyl (1S,2S,5R)-2-((S)-1-((7-chloro-8-fluoro-4-hydroxy-2-(methylthio)pyrido[4,3-d]pyrimidin-5-yl)oxy)propyl)-3,8-diazabicyclo[3.2.1]octane-8-carboxylate

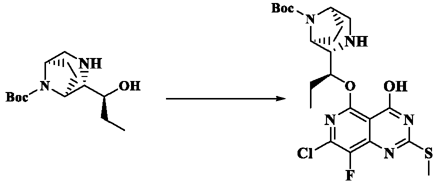

Sodium hydride (1.95 g, 81.37 mmol) was added in portions in small amount to a solution of tert-butyl (1S,2S,5R)-2-((S)-1-hydroxypropyl)-3,8-diazabicyclo[3.2.1]octane-8-carboxylate (4.4 g, 16.27 mmol) in tetrahydrofuran (80 mL) at 0°C. After 30 min of reaction, 5,7-dichloro-8-fluoro-2-methylsulfanyl-3H-pyrido[4,3-d]pyrimidin-4-one (9.12 g, 32.55 mmol) was added to the reaction system, and the obtained mixture was heated to 50°C and reacted overnight. After the reaction was completed, saturated ammonium chloride solution was added to quench the reaction, and the obtained mixture was extracted with ethyl acetate. The organic phases were combined, dried over anhydrous sodium sulfate, concentrated under reduced pressure, and separated by silica gel column chromatography to obtain tert-butyl (1S,2S,SR)-2-((S)-1-((7-chloro-8-fluoro-4-hydroxy-2-(methylthio)pyrido[4,3-d]pyrimidin-5-yl)oxy)propyl)-3,8-diazabicyclo[3.2.1] octane-8-carboxylate (6.6 g, yield 78.9%).

MS m/z (ESI):514 [M+H].

### Step 4: Preparation of tert-butyl (5S,5aS,6S,9R)-2-chloro-5-ethyl-1-fluoro-12-(methylthio)-5a,6,7,8,9,10-hexahydro-5H-4-oxa-3,10a,11,13,14-pentaaza-6,9-methylnaphtho[1,8-ab]heptene-4-carboxylate

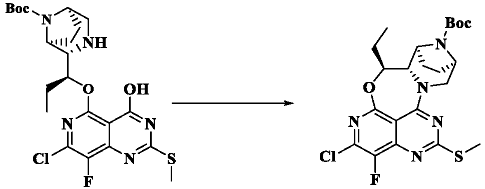

Tert-butyl (1S,2S,SR)-2-((S)-1-((7-chloro-8-fluoro-4-hydroxy-2-(methylthio) pyrido[4,3-d]pyrimidin-5-yl)oxy)propyl)-3,8-diazabicyclo[3.2.1]octane-8-carboxylate (6.6 g, 12.8 mmol), diisopropylethylamine (8.3 g, 64.2 mmol), bis(2-oxo-3-oxazolidinyl)phosphinic chloride (6.6 g, 25.7 mmol) were added to the chloroform (80 mL) solution, and the reaction was warmed to 70°C and refluxed for 2 h. After the reaction was completed, water was added to quench the reaction and the obtained mixture was extracted with dichloromethane. The organic phases were combined, dried over anhydrous sodium sulfate, filtered, concentrated, and separated by silica gel column chromatography to obtain tert-butyl (5S,5aS,6S,9R)-2-chloro-5-ethyl-1-fluoro-12-(methylthio)-5a,6,7,8,9,10-hexahydro-5H-4-oxa-3,10a,11,13,14-pentaaza-6,9-methylnaphtho[1,8-ab]heptene-4-carboxylate (5.8 g, yield 91.1%).

MS m/z (ESI):496 [M+H].

### Step 5: Preparation of tert-butyl (5S,5aS,6S,9R)-5-ethyl-1-fluoro-2-(7-fluoro-3-(methoxymethoxy)-8-((triisopropylsilyl)ethynyl)naphthalen-1-yl)-12-(methylthio)-5a,6,7,8,9,10-hexahydro-5H-4-oxa-3,10a,11,13,14-pentaaza-6,9-methylenenaphtho[1,8-ab]heptene-14-carboxylate

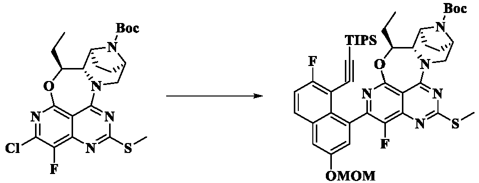

Tert-butyl (5S,5aS,6S,9R)-2-chloro-5-ethyl-1-fluoro-12-(methylthio)-5a,6,7,8,9, 10-hexahydro-5H-4-oxa-3, 10a, 11, 13,14-pentaaza-6,9-methylnaphtho[1,8-ab]heptene-4-carboxylate (5.8 g, 11.7 mmol), 2-[2-fluoro-6-(methoxymethoxy)-8-(4,4,5,5-tetramethyl-1,3,2-dioxyboran-2-yl)-1-naphthyl] ethynyltriisopropylsilane (8.99 g, 17.5 mmol), [(di(1-adamantyl) butylphosphino)-2-(2'-amino-1,1'-biphenyl)]palladium(II) methanesulfonate (2.55 g, 3.5 mmol), sodium hydroxide solution (28.3 mL, 2 M), tetrahydrofuran (100 mL) were added to the 250 mL reaction flask. The reaction mixture was subjected to nitrogen replacement, then heated to 100°C and stirred for 2 h. After the reaction was completed, water and ethyl acetate were added for layer separation and extraction. The organic phases were combined, dried over anhydrous sodium sulfate, concentrated under reduced pressure, and separated by silica gel column chromatography to obtain tert-butyl (5S,5aS,6S,9R)-5-ethyl-1-fluoro-2-(7-fluoro-3-(methoxymethoxy)-8-((triisopropylsilyl)ethynyl)naphthalen-1-yl)-12-(methylthio)-5a,6,7,8,9,10-hexahydro-5H-4-oxa-3,10a,11,13,14-pentaaza-6,9-methylenenaphtho[1,8-ab]heptene-14-carboxylate (8.4 g, yield 84.9%).

MS m/z (ESI):846 [M+H].

### Step 6: Preparation of tert-butyl (5S,5aS,6S,9R)-5-ethyl-1-fluoro-2-(7-fluoro-3-(methoxymethoxy)-8-((triisopropylsilyl)ethynyl)naphthalen-1-yl)-12-(methylsulfonyl)- 5a,6,7,8,9,10-hexahydro-5H-4-oxa-3,10a,11,13,14-pentaaza-6,9-methylnaphtho[1,8-ab]heptene-14-carboxylate

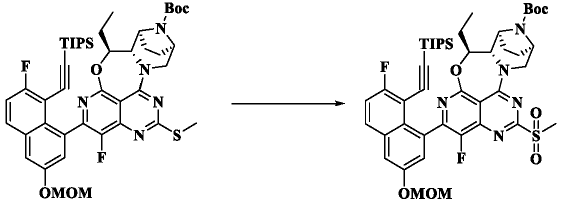

Tert-butyl (5S,5aS,6S,9R)-5-ethyl-1-fluoro-2-(7-fluoro-3-(methoxymethoxy)-8-((triisopropylsilyl)ethynyl)naphthalen-1-yl)-12-(methylthio)-5a,6,7,8,9,10-hexahydro-5H-4-oxa-3,10a,11,13,14-pentaaza-6,9-methylenenaphtho[1,8-ab]heptene-14-carboxylate (8.4 g, 9.93 mmol) was dissolved in dichloromethane (80 mL), and the reaction liquid was cooled to 0°C. M-chloroperoxybenzoic acid (5.14 g, 29.8 mmol) was added thereto, and the reaction mixture slowly returned to room temperature and stirred for 1.5 h. After the reaction was completed, a saturated sodium thiosulfate solution and a saturated sodium bicarbonate solution was added to quench the reaction, and the obtained mixture was extracted with dichloromethane, dried over anhydrous sodium sulfate, and separated by silica gel column chromatography to obtain tert-butyl (5S,5aS,6S,9R)-5-ethyl-1-fluoro-2-(7-fluoro-3-(methoxymethoxy)-8-((triisopropylsilyl)ethynyl)naphthalen-1-yl)-12-(methylsulfonyl)-5a,6,7,8,9,10-hexahydro-5H-4-oxa-3,10a,11,13,14-pentaaza-6,9-methanonaphtho[1,8-ab]heptene-14-carboxylate (5.7 g, yield 65.4%).

MS m/z (ESI):878 [M+H].

### Step 7: Preparation of tert-butyl (5S,5aS,6S,9R)-12-((1-((4-(difluoromethylene)piperidin-1-yl)methyl)cyclopropyl)methoxy)-5-ethyl-1-fluoro-2-(7-fluoro-3-(methoxymethoxy)-8-((triisopropylsilyl)ethynyl)naphthalen-1-yl)-5a,6,7,8,9,10-hexahydro-5H-4-oxa-3,10a,11,13,14-pentaaza-6,9-methylenenaphtho[1,8-ab]heptene-14-carboxylate

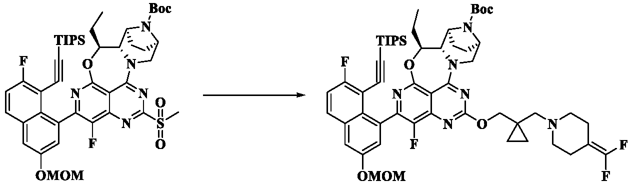

Sodium hydride (778.95 mg, 32.46 mmol) was added in portions in small amount to a solution of [1-[[4-(difluoromethylene)-1-piperidinyl]methyl] cyclopropyl]methanol (3.53 g, 16.23 mmol) in tetrahydrofuran (50 mL) at 0°C. After half an hour of reaction, a solution of tert-butyl (5S,5aS,6S,9R)-5-ethyl-1-fluoro-2-(7-fluoro-3-(methoxymethoxy)-8-((triisopropylsilyl)ethynyl)naphthalen-1-yl)-12-(methylsulfonyl)-5a,6,7,8,9,10-hexahydro-5H-4-oxa-3,10a,11,13,14-pentaaza-6,9-methanonaphtho[1,8-ab]heptene-14-carboxylate (5.7 g, 6.49 mmol) in tetrahydrofuran was added to the reaction liquid, and the obtained mixture was stirred for 1 h. After the reaction was completed, tert-butyl alcohol was added to quench the reaction, and the obtained mixture was stirred for a few min, and then directly concentrated under reduced pressure. The crude product was separated by silica gel column chromatography to obtain tert-butyl (5S,5aS,6S,9R)-12-((1-((4-(difluoromethylene)piperidin-1-yl)methyl)cyclopropyl)methoxy)-5-ethyl-1-fluoro-2-(7-fluoro-3-(methoxymethoxy)-8-((triisopropylsilyl)ethynyl)naphthalen-1-yl)-5a,6,7,8,9, 10-hexahydro-5H-4-oxa-3, 10a, 11, 13,14-pentaaza-6,9-methylenenaphtho[1,8-ab]heptene-14-carboxylate (4.5 g, yield 68.3%).

MS m/z (ESI):1015 [M+H].

### Step 8: Preparation of tert-butyl (5S,5aS,6S,9R)-12-((1-((4-(difluoromethylene)piperidin-1-yl)methyl)cyclopropyl)methoxy)-5-ethyl-2-(8-ethynyl-7-fluoro-3-(methoxymethoxynaphthalen-1-yl)-1-fluoro-5a,6,7,8,9,10-hexahydro-5H-4-oxa-3,10a,11,13,14-pentaaza-6,9-methylnaphtho[1,8-ab]heptene-4-carboxylate

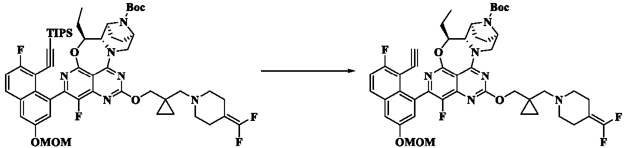

Tetrabutylammonium fluoride solution (18 mL, 1 M in THF) was added to a tetrahydrofuran (50 mL) solution containing tert-butyl (5S,5aS,6S,9R)-12-((1-((4-(difluoromethylene)piperidin-1-yl)methyl)cyclopropyl)methoxy)-5-ethyl-1-fluoro-2-(7-fluoro-3-(methoxymethoxy)-8-((triisopropylsilyl)ethynyl)naphthalen-1-yl)-5a,6,7,8,9,10-hexahydro-5H-4-oxa-3,10a,11,13,14-pentaaza-6,9-methylenenaphtho [1,8-ab]heptene-14-carboxylate (4.5 g, 4.43 mmol). The reaction was warmed to 60°C and reacted for 1 h. After the reaction was completed, water was added to quench the reaction, and the obtained mixture was extracted with ethyl acetate, dried over anhydrous sodium sulfate, concentrated under reduced pressure, and separated by silica gel column chromatography to obtain tert-butyl (5S,5aS,6S,9R)-12-((1-((4-(difluoromethylene)piperidin-1-yl)methyl)cyclopropyl)methoxy)-5-ethyl-2-(8-ethynyl-7-fluoro-3-(methoxymethoxynaphthalen-1-yl)-1-fluoro-5a,6,7,8,9,10-hexahydro-5H-4-oxa-3,10a,11,13,14-pentaaza-6,9-methylnaphtho[1,8-ab]heptene-4-carboxylate (3 g, yield 78.8%).

MS m/z (ESI):859 [M+H].

### Step 9: Preparation of 4-((5S,5aS,6S,9R)-12-((1-((4-(difluoromethylene) piperidin-1-yl)methyl)cyclopropyl)methoxy)-5-ethyl-1-fluoro-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalen-2-ol

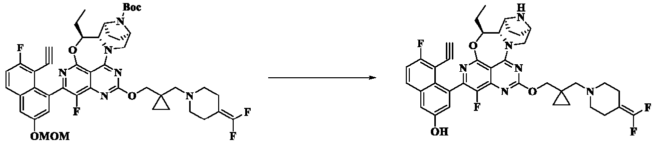

Tert-butyl (5S,5aS,6S,9R)-12-((1-((4-(difluoromethylene)piperidin-1-yl)methyl)cyclopropyl)methoxy)-5-ethyl-2-(8-ethynyl-7-fluoro-3-(methoxymethoxynaphthalen-1-yl)-1-fluoro-5a,6,7,8,9,10-hexahydro-5H-4-oxa-3,10a,11,13,14-pentaaza-6,9-methylnaphtho[1,8-ab]heptene-4-carboxylate (3 g, 3.49 mmol) was dissolved in a mixed solution of dichloromethane (10 mL) and methanol (1 mL), and a solution of hydrochloric acid in ethyl acetate (18 mL, 4M in EtOAc) was added. After 1 h of reaction, the reaction mixture was concentrated under reduced pressure and separated by silica gel column chromatography to obtain 4-((5S,5aS,6S,9R)-12-((1-((4-(difluoromethylene)piperidin-1-yl)methyl)cyclopropyl)methoxy)-5-ethyl-1-fluoro-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalen-2-ol (1.5 g, yield 57.2%).

MS m/z (ESI):715 [M+H].

¹H NMR (500 MHz, Chloroform-d) δ 7.76 (ddd, *J* = 8.1, 3.8, 2.0 Hz, 1H), 7.68 (s, 1H), 7.43 (d, *J =* 2.2 Hz, 1H), 7.40 (t, *J* = 2.2 Hz, 1H), 7.26 (dd, *J* = 10.2, 8.1 Hz, 1H), 4.48 (dtd, *J* = 8.4, 6.2, 1.5 Hz, 1H), 4.08 - 3.97 (m, 4H), 3.65 (dd, *J* = 8.4, 4.2 Hz, 1H), 3.33 (s, 1H), 3.31 (ddt, *J* = 6.3, 4.2, 2.1 Hz, 1H), 3.20 (t, *J* = 5.8 Hz, 1H), 3.17 (ddt, *J* = 5.6, 3.8, 1.9 Hz, 1H), 2.75 (t, *J =* 3.3 Hz, 4H), 2.62 (s, 2H), 2.36 - 2.26 (m, 4H), 2.26 - 2.19 (m, 2H), 2.03 - 1.90 (m, 5H), 1.77 - 1.64 (m, 3H), 0.96 (td, *J* = 7.6, 1.5 Hz, 3H).

### Example 129

### Step 1: Preparation of tert-butyl (5S,5aS,6S,9R)-2-chloro-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methylnaphtho[1,8-ab]heptene-14-carboxylate

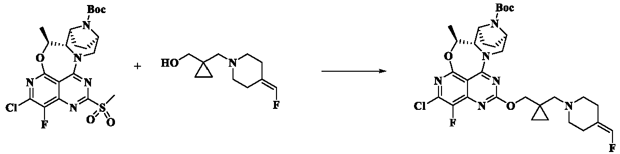

In an eggplant-shaped flask, tert-butyl (5S,5aS,6S,9R)-2-chloro-1-fluoro-5-methyl-12-(methylsulfonyl)-5a,6,7,8,9,10-hexahydro-5H-4-oxa-3,10a,11,13,14-pentaaza-6,9-methylnaphtho[1,8-ab]heptene-14-carboxylate (13.37 g, 26.00 mmol) and [1-[[4-(fluoromethylene)-1-piperidinyl]methyl]cyclopropyl]methanol (5.7 g, 28.61 mmol) were dissolved in tetrahydrofuran (150 mL). The obtained mixture was subjected to nitrogen replacement, and lithium bis(trimethylsilyl)amide (1 M, 78.01 mL) was added dropwise under ice bath. The obtained mixture was reacted under ice bath for 20 min. After the addition was completed, a saturated ammonium chloride solution was added to quench the reaction, and the obtained mixture was extracted with ethyl acetate. The organic phase was dried over anhydrous sodium sulfate, filtered, concentrated, and purified by silica gel column chromatography to obtain tert-butyl (5S,5aS,6S,9R)-2-chloro-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methylnaphtho[1,8-ab]heptene-14-carboxylate (12 g, yield 73%).

MS m/z(ESI):633 [M+H].

### Step 2: Preparation of tert-butyl (5S,5aS,6S,9R)-1-fluoro-2-(7-fluoro-3-(methoxymethoxy)-8-((triisopropylsilyl)ethynyl)naphthalen-1-yl)-12-(1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methylnaphtho[1,8-ab]heptene-14-carboxylate

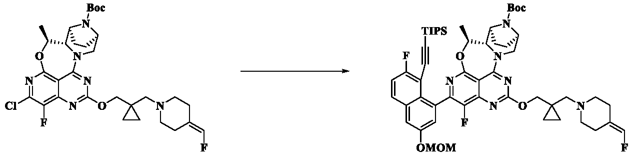

2-[2-fluoro-6-(methoxymethoxy)-8-(4,4,5,5-tetramethyl-1,3,2-dioxyboran-2-yl)-1-naphthyl]ethynyltriisopropylsilane (10.69 g, 20.85 mmol), tert-butyl (5S,5aS,6S,9R)-2-chloro-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methylnaphtho[1,8-ab]heptene-14-carboxylate (12 g, 18.95 mmol), methanesulfonic acid [n-butyldi(1-adamantyl)phosphine](2-amino-1,1'-biphenyl-2-yl)palladium(II) (690.16 mg, 947.68 µmol), cesium carbonate (12.35 g, 37.91 mmol), 120 mL of dioxane, and 24 mL of water were added to an eggplant-shaped flask. Nitrogen replacement was performed and the reaction was carried out in an oil bath at 85°C for 1 h. After the reaction was completed, the obtained mixture was concentrated and stirred, and purified by silica gel column chromatography to obtain tert-butyl (5S,5aS,6S,9R)-1-fluoro-2-(7-fluoro-3-(methoxymethoxy)-8-((triisopropylsilyl)ethynyl)naphthalen-1-yl)-12-(1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methylnaphtho[1,8-ab]heptene-14-carboxylate (16.5 g, yield 89%).

MS m/z(ESI): 983 [M+H].

### Step 3: Preparation of tert-butyl (5S,5aS,6S,9R)-2-(8-ethynyl-7-fluoro-3-(methoxy-methoxy)naphthalen-1-yl)-1-fluoro-12-((1-((4-(fluoromethylene)piperidin-1-yl)methyl)cyclopropyl)methoxy-5-methyl-5a,6,7,8,9,10-hexahydro-5H-4-oxo-3,10a,11,13,14-pentaaza-6,9-methylnaphtho[1,8-ab]heptene-14carboxylate

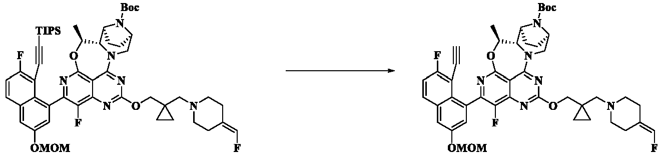

In an eggplant-shaped flask, tert-butyl (5S,5aS,6S,9R)-1-fluoro-2-(7-fluoro-3-(methoxymethoxy)-8-((triisopropylsilyl)ethynyl)naphthalen-1-yl)-12-(1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methylnaphtho[1,8-ab]heptene-14-carboxylate (16.5 g, 16.78 mmol) was dissolved in 70 mL of tetrahydrofuran, tetrabutylammonium fluoride (1 M, 33.56 mL) was added, and the obtained mixture was reacted at room temperature for 1 h. After the addition was completed, the reaction liquid was concentrated and purified by silica gel column chromatography to obtain tert-butyl (5S,5aS,6S,9R)-2-(8-ethynyl-7-fluoro-3-(methoxy-methoxy)naphthalen-1-yl)-1-fluoro-12-((1-((4-(fluoromethylene)piperidin-1-yl)methyl)cyclopropyl)methoxy-5-methyl-5a,6,7,8,9,10-hexahydro-5H-4-oxo-3,10a,11,13,14-pentaaza-6,9-methylnaphtho [1,8-ab]heptene-14carboxylate (12.5 g, yield 90%).

MS m/z(ESI):827 [M+H].

### Step 4: Preparation of 5-ethynyl-6-fluoro-4-((5S,5aS,6S,9R)-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methylnaphtho[1,8-ab]hept-2-yl)naphthalen-2-ol

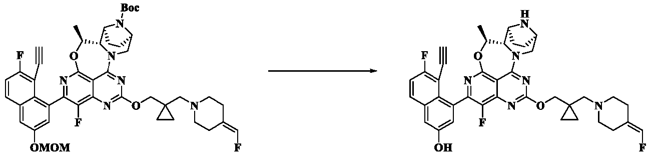

In an eggplant-shaped flask, tert-butyl (5S,5aS,6S,9R)-2-(8-ethynyl-7-fluoro-3-(methoxy-methoxy)naphthalen-1-yl)-1-fluoro-12-((1-((4-(fluoromethylene) piperidin-1-yl)methyl)cyclopropyl)methoxy-5-methyl-5a,6,7,8,9,10-hexahydro-5H-4-oxo-3,10a,11,13,14-pentaaza-6,9-methylnaphtho[1,8-ab]heptene-14carboxylate (10 g, 12.09 mmol) was dissolved in a mixed solvent of 160 mL of dichloromethane and 40 mL of methanol, and the obtained mixture was cooled under ice bath for 10 min, nitrogen replacement was performed, 200 mL of 4N hydrochloric acid in dioxane was slowly added, and the obtained mixture was reacted under ice bath for 1.5 h. After the addition was completed, sodium bicarbonate solution was added to the reaction liquid to quench the reaction, and the obtained mixture was extracted with dichloromethane/methanol at low temperature. The organic phase was dried over anhydrous sodium sulfate, filtered, concentrated, and purified by silica gel column chromatography to obtain the product 5-ethynyl-6-fluoro-4-((5S,5aS,6S,9R)-1-fluoro-12-((1-((4-(fluoromethylene)piperidin-1-yl)methyl)cyclopropyl)methoxy)-5-methyl-5a,6,7,8,9,10-hexahydro-5H-4-oxa-3,10a,11,13,14-pentaaza-6,9-methylnaphtho[1,8-ab]hept-2-yl)naphthalen-2-ol (5.3 g, yield 55%).

MS m/z(ESI):683 [M+H].

¹H NMR(400 MHz, MeOD) δ 7.83(dt, *J* = 9.2, 4.2 Hz, 1H), 7.34 - 7.26(m, 2H), 7.26 - 7.17(m, 1H), 6.31(s, 1H), 5.06(t, *J =* 12.1 Hz, 1H), 4.65 - 4.31(m, 5H), 4.14(s, 1H), 3.84 - 3.69(m, 1H), 3.05(s, 3H), 2.93 - 2.77(m, 4H), 2.38(s, 2H), 2.08 - 1.79(m, 6H), 1.40 - 1.32 (m, 3H), 0.86 - 0.65(m, 4H).

**Examples 137 to 144 refer to the synthesis of example 1-1 or example 124:**

| **Serial No.** | **Example structure** | **MS m/z (ESI)** | **Serial No.** | **Example structure** | **MS m/z (ESI)** |
|---|---|---|---|---|---|
| **137** | 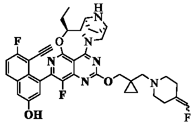 | 697 [M+H] | **138** | 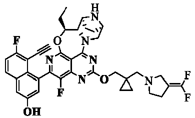 | 701 [M+H] |
| **Example 138:** ¹H NMR (500 MHz, Chloroform-d) δ 7.76 (ddd, *J* = 8.1, 3.8, 2.0 Hz, 1H), 7.68 (s, 1H), 7.43 (d, *J* = 2.2 Hz, 1H), 7.40 (t, *J* = 2.2 Hz, 1H), 7.26 (dd, *J* = 10.1, 8.1 Hz, 1H), 4.51 - 4.45 (m, 1H), 4.08 - 4.04 (m, 3H), 4.00 (dd, *J* = 13.0, 2.0 Hz, 1H), 3.65 (dd, *J* = 8.4, 4.2 Hz, 1H), 3.33 (s, 1H), 3.31 (ddt, *J* = 6.4, 4.2, 2.1 Hz, 1H), 3.20 (t, *J* = 5.8 Hz, 1H), 3.17 (dq, *J* = 6.0, 4.1, 2.1 Hz, 1H), 2.99 - 2.94 (m, 2H), 2.79 (t, *J =* 2.2 Hz, 2H), 2.64 (s, 2H), 2.44 (dp, *J =* 4.4, 2.3 Hz, 2H), 2.26 - 2.19 (m, 2H), 2.01 - 1.94 (m, 5H), 1.74 - 1.65 (m, 3H), 0.96 (td, *J* = 7.6, 1.5 Hz, 3H). | | | | | |
| **139** | 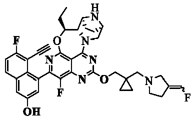 | 683 [M+H] | **140** | 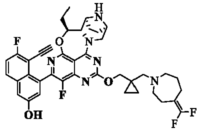 | 729 [M+H] |
| **Example 139:** ¹H NMR (500 MHz, Chloroform-d) δ 7.76 (ddd, *J* = 8.1, 3.8, 2.0 Hz, 1H), 7.68 (s, 1H), 7.41 (dd, *J =* 15.3, 2.2 Hz, 2H), 7.32 - 7.14 (m, 2H), 4.51 - 4.45 (m, 1H), 4.08 - 4.04 (m, 3H), 4.00 (dd, *J =* 13.0, 2.0 Hz, 1H), 3.65 (dd, *J* = 8.4, 4.2 Hz, 1H), 3.33 (s, 1H), 3.31 (ddq, *J* = 6.4, 4.2, 2.2 Hz, 1H), 3.20 (t, *J* = 5.8 Hz, 1H), 3.17 (ddq, *J* = 6.0, 4.1, 2.1 Hz, 1H), 2.97 (dd, *J* = 2.9, 0.8 Hz, 2H), 2.80 (t, *J* = 2.5 Hz, 2H), 2.67 (s, 2H), 2.43 (qd, *J =* 2.8, 0.8 Hz, 2H), 2.27 - 2.19 (m, 2H), 2.02 - 1.92 (m, 5H), 1.77 - 1.66 (m, 3H), 0.96 (td, *J =* 7.6, 1.5 Hz, 3H). | | | | | |
| **Example 140:** ¹H NMR (500 MHz, Chloroform-d) δ 7.76 (ddd, *J* = 8.1, 3.8, 2.0 Hz, 1H), 7.68 (s, 1H), 7.44 - 7.39 (m, 2H), 7.26 (dd, *J =* 10.2, 8.1 Hz, 1H), 4.50 - 4.45 (m, 1H), 4.08 - 4.04 (m, 3H), 4.00 (dd, *J* = 13.0, 2.0 Hz, 1H), 3.65 (dd, *J* = 8.4, 4.2 Hz, 1H), 3.33 (s, 1H), 3.31 (dq, *J* = 6.3, 2.1 Hz, 1H), 3.20 (t, *J* = 5.8 Hz, 1H), 3.17 (dq, *J* = 5.6, 2.0 Hz, 1H), 2.73 (t, *J* = 4.9 Hz, 2H), 2.63 (s, 2H), 2.59 (t, *J* = 6.2 Hz, 2H), 2.26 - 2.19 (m, 4H), 2.19 - 2.07 (m, 2H), 2.00 - 1.91 (m, 5H), 1.85 - 1.66 (m, 5H), 0.96 (td, *J =* 7.6, 1.5 Hz, 3H). | | | | | |
| **141** | 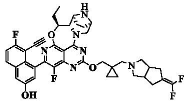 | 741 [M+H] | **142** | 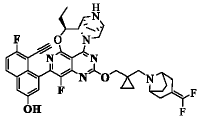 | 741 [M+H] |
| **Example 141:** ¹H NMR (400 MHz, MeOD) δ 7.86 - 7.79 (m, 1H), 7.35 - 7.27 (m, 2H), 7.17 (d, J = 2.6 Hz, 1H), 5.36 (d, J = 13.2 Hz, 1H), 4.54 - 4.31 (m, 3H), 4.11 - 4.04 (m, 1H), 3.69 - 3.67 (m, 1H), 3.62 - 3.57 (m, 1H), 3.22 - 3.13 (m, 1H), 2.91 - 2.66 (m, 4H), 2.60 - 2.39 (m, 4H), 2.34 - 2.23 (m, 2H), 2.18 - 2.03 (m, 3H), 1.95 - 1.47 (m, 6H), 1.36 - 1.29 (m, 2H), 1.12-0.98 (m, 3H), 0.71 - 0.49 (m, 4H). | | | | | |
| **143** | 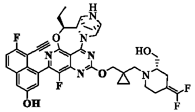 | 745 [M+H] | **144** | 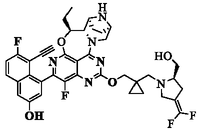 | 731 [M+H] |

**The following synthesis method can also be adopted for example 137:**

### Example 137

### Step 1: Preparation of tert-butyl (5S,5aS,6S,9R)-2-chloro-5-ethyl-1-fluoro-12-(methylsulfonyl)-5a,6,7,8,9,10-hexahydro-5H-4-oxa-3,10a,11,13,14-pentaaza-6,9-methylenenaphtho[1,8-ab]heptalene-14-carboxylate

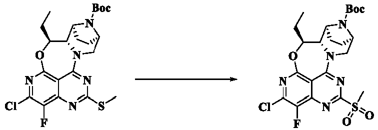

At 0°C, under nitrogen protection, mCPBA (10.23 g, 59.27 mmol) was added to a solution of tert-butyl (5S,5aS,6S,9R)-2-chloro-5-ethyl-1-fluoro-12-(methylthio)-5a,6,7,8,9,10-hexahydro-5H-4-oxa-3,10a,11,13,14-pentaaza-6,9-methylnaphtho[1,8-ab]heptene-4-carboxylate (7.00 g, 14.11 mmol) in dichloromethane (60 mL), and the obtained mixture was stirred at 0°C for 2 h. Saturated aqueous NaHCO₃ solution was added to the reaction liquid, and the obtained mixture was extracted with ethyl acetate, washed with saturated NaCl aqueous solution, dried over anhydrous sodium sulfate, filtered, concentrated, and then purified by silica gel column chromatography to obtain tert-butyl (5S,5aS,6S,9R)-2-chloro-5-ethyl-1-fluoro-12-(methylsulfonyl)-5a,6,7,8,9,10-hexahydro-5H-4-oxa-3,10a,11,13,14-pentaaza-6,9-methylenenaphtho[1,8-ab]heptalene-14-carboxylate (6.1 g, 81.9%).

MS m/z (ESI): 528 [M+H].

### Step 2: Preparation of tert-butyl (5S,5aS,6S,9R)-12-((1-(((tert-butyldiphenylsilyl)oxo)methyl)cyclopropyl)methoxy)-2-chloro-5-ethyl-1-fluoro-5a,6,7,8,9,10-hexahydro-5H-4-oxa-3,10a,11,13,14-pentaaza-6,9-methylenenaphtho[1,8-ab]heptalene-14-carboxylate

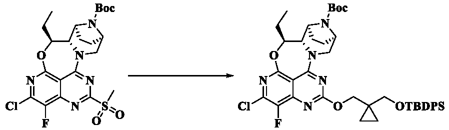

At 0°C, under nitrogen protection, LiHMDS (1 M, 34.66 mL) solution was added to a solution of tert-butyl (5S,5aS,6S,9R)-2-chloro-5-ethyl-1-fluoro-12-(methylsulfonyl)-5a,6,7,8,9,10-hexahydro-5H-4-oxa-3,10a,11,13,14-pentaaza-6,9-methylenenaphtho[1,8-ab]heptalene-14-carboxylate (6.1 g, 11.55 mmol), [1-[[tert-butyl (diphenyl)silyl]oxomethyl]cyclopropyl]methanol (5.11 g, 15.02 mmol) in THF (60 mL), and the obtained mixture was stirred at 0°C for 0.5 h. At 0°C, saturated aqueous NH₄Cl solution was added to the reaction liquid, and the obtained mixture was extracted with ethyl acetate, and the organic layer was washed with saturated aqueous sodium chloride solution. The organic layers were combined, dried over anhydrous sodium sulfate, filtered, concentrated, and then purified by silica gel column chromatography to obtain tert-butyl (5S,5aS,6S,9R)-12-((1-(((tert-butyldiphenylsilyl)oxo)methyl)cyclopropyl)methoxy)-2-chloro-5-ethyl-1-fluoro-5a,6,7,8,9,10-hexahydro-5H-4-oxa-3,10a,11,13,14-pentaaza-6,9-methylenenaphtho[1,8-ab]heptalene-14-carboxylate (8.8 g, yield 96.6%).

MS m/z (ESI): 788 [M+H].

### Step 3: Preparation of tert-butyl (5S,5aS,6S,9R)-12-((1-(((tert-butyldiphenylsilyl)oxo)methyl)cyclopropyl)methoxy)-5-ethyl-1-fluoro-2-(7-fluoro-3-(methoxymethoxy)-8-((triisopropylsilyl)ethynyl)naphthalen-1-yl)-5a,6,7,8,9,10-hexahydro-5H-4-oxa-3,10a,11,13,14-pentaaza-6,9-methylenenaphtho[1,8-ab]heptalene-14-carboxylate

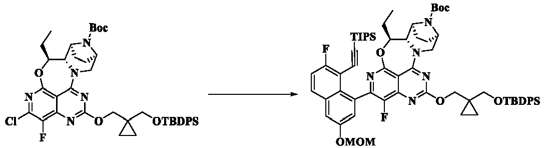

Under nitrogen protection, methylsulfonyloxy (diadamantyl-n-butylphosphino)-2'-amino-1,1'-biphenyl-2-yl)palladium(II) (812.56 mg, 1.12 mmol) and Cs₂CO₃ (10.88 g, 33.48 mmol) were added to a solution of tert-butyl (5S,5aS,6S,9R)-12-((1-(((tert-butyldiphenylsilyl)oxo)methyl)cyclopropyl) methoxy)-2-chloro-5-ethyl-1-fluoro-5a,6,7,8,9,10-hexahydro-5H-4-oxa-3,10a,11,13,14-pentaaza-6,9-methylenenaphtho[1,8-ab]heptalene-14-carboxylate (8.80 g, 11.16 mmol), 2-[2-fluoro-6-(methoxymethoxy)-8-(4,4,5,5-tetramethyl-1,3,2-dioxaborolan-2-yl)-1-naphthyl]ethynyl-triisopropyl-silane (9.73 g, 18.97 mmol) in dioxane (80 mL) and water (16 mL), and the obtained mixture was stirred at 105°C for 2 h. The reaction liquid was concentrated and then purified by silica gel column chromatography to obtain tert-butyl (5S,5aS,6S,9R)-12-((1-(((tert-butyldiphenylsilyl)oxo)methyl)cyclopropyl)methoxy)-5-ethyl-1-fluoro-2-(7-fluoro-3-(methoxymethoxy)-8-((triisopropylsilyl)ethynyl)naphthalen-1-yl)-5a,6,7,8,9,10-hexahydro-5H-4-oxa-3,10a,11,13,14-pentaaza-6,9-methylenenaphtho[1,8-ab]heptalene-14-carboxylate (10.8 g, yield 85.0%).

### Step 4: Preparation of tert-butyl (5S,5aS,6S,9R)-5-ethyl-1-fluoro-2-(7-fluoro-3-(methoxymethoxy)-8-((triisopropylsilyl)ethynyl)naphthalen-1-yl)-12-((1-(hydroxymethyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate

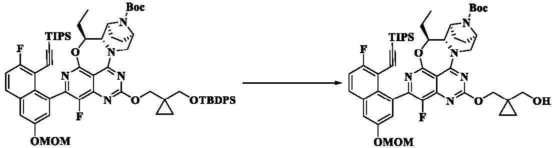

Under nitrogen protection, TBAF (1 M, 37.94 mL) solution was added to a solution of tert-butyl (5S,5aS,6S,9R)-12-((1-(((tert-butyldiphenylsilyl)oxo)methyl) cyclopropyl)methoxy)-5-ethyl-1-fluoro-2-(7-fluoro-3-(methoxymethoxy)-8-((triisopropylsilyl)ethynyl)naphthalen-1-yl)-5a,6,7,8,9,10-hexahydro-5H-4-oxa-3,10a,11,13,14-pentaaza-6,9-methylenenaphtho[1,8-ab]heptalene-14-carboxylate (10.80 g, 9.49 mmol) in THF (20 mL), and the obtained mixture was stirred at 20°C for 3 h. NH₄Cl aqueous solution was added to the reaction liquid, and the obtained mixture was extracted with ethyl acetate, washed with saturated NaCl aqueous solution, dried over anhydrous sodium sulfate, filtered, concentrated and purified by silica gel column chromatography to obtain tert-butyl (5S,5aS,6S,9R)-5-ethyl-1-fluoro-2-(7-fluoro-3-(methoxymethoxy)-8-((triisopropylsilyl)ethynyl)naphthalen-1-yl)-12-((1-(hydroxymethyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate (4.7 g, yield 55.0%).

MS m/z (ESI): 900 [M+H].

### Step 5: Preparation of tert-butyl (5S,5aS,6S,9R)-5-ethyl-1-fluoro-2-(7-fluoro-3-(methoxymethoxy)-8-((triisopropylsilyl)ethynyl)naphthalen-1-yl)-12-((1-(((methylsulfonyl)oxo)methyl)cyclopropyl)methoxy) -5a,6,7,8,9,10-hexahydro-5H-4-oxa-3,10a,11,13,14-pentaaza-6,9-methylenenaphtho[1,8-ab]heptalene-14-carboxylate

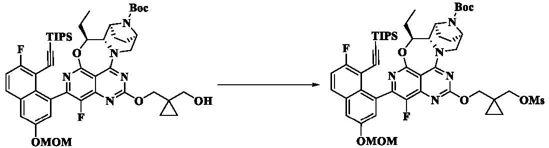

At 0°C, under nitrogen protection, a solution of TEA (1.35 g, 13.31 mmol, 1.86 mL) and methanesulfonyl chloride (1.02 g, 8.87 mmol, 686.80 µL) was added to a solution of tert-butyl (5S,5aS,6S,9R)-5-ethyl-1-fluoro-2-(7-fluoro-3-(methoxymethoxy)-8-((triisopropylsilyl)ethynyl)naphthalen-1-yl)-12-((1-(hydroxymethyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate (3.3 g, 3.67 mmol) in dichloromethane (30 mL), and the obtained mixture was stirred at 0°C for 0.5 h. Aqueous NaHCO₃ solution was added to the reaction liquid to quench the reaction, and the obtained mixture was extracted with dichloromethane, washed with saturated NaCl aqueous solution, dried over anhydrous sodium sulfate, filtered, and concentrated to obtain tert-butyl (5S,5aS,6S,9R)-5-ethyl-1-fluoro-2-(7-fluoro-3-(methoxymethoxy)-8-((triisopropylsilyl)ethynyl)naphthalen-1-yl)-12-((1-(((methylsulfonyl)oxo)methyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate (3.6 g).

### Step 6: Preparation of tert-butyl (SS,SaS,6S,9R)-12-((1-((4-(fluoromethylene)piperidin-1-yl)methyl)cyclopropyl)methoxy)-5-ethyl-2-(8-ethynyl-7-fluoro-3-(methoxymethoxynaphthalen-1-yl)-1-fluoro-5a,6,7,8,9,10-hexahydro-5H-4-oxa-3,10a,11,13,14-pentaaza-6,9-methylnaphtho[1,8-ab]heptene-4-carboxylate

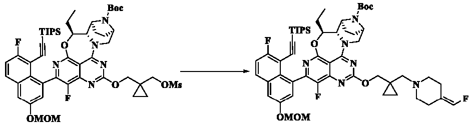

Under nitrogen protection, a solution of NaI (683.9 mg, 4.56 mmol) and DIEA (1.97 g, 15.21 mmol, 2.65 mL) was added to tert-butyl (5S,5aS,6S,9R)-5-ethyl-1-fluoro-2-(7-fluoro-3-(methoxymethoxy)-8-((triisopropylsilyl)ethynyl)naphthalen-1-yl)-12-((1-(((methylsulfonyl)oxo)methyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate (1.39 g, 6.08 mmol, TF) in acetonitrile (20 mL), and the obtained mixture was stirred at 55°C for 12 h. Saturated NaCl aqueous solution was added to the reaction liquid, and the obtained mixture was extracted with ethyl acetate, washed with saturated NaCl aqueous solution, dried over anhydrous sodium sulfate, filtered, concentrated, and purified by silica gel column chromatography to obtain tert-butyl (5S,5aS,6S,9R)-12-((1-((4-(fluoromethylene)piperidin-1-yl)methyl)cyclopropyl)methoxy)-5-ethyl-2-(8-ethynyl-7-fluoro-3-(methoxymethoxynaphthalen-1-yl)-1-fluoro-5a,6,7,8,9,10-hexahydro-5H-4-oxa-3,10a,11,13,14-pentaaza-6,9-methylnaphtho[1,8-ab]heptene-4-carboxylate (1 g, yield 78.2%).

MS m/z (ESI): 997 [M+H].

### Step 7: Preparation of 4-((SS,SaS,6S,9R)-12-((1-((4-(fluoromethylene)piperidin-1-yl)methyl)cyclopropyl)methoxy)-5-ethyl-1-fluoro-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalen-2-ol

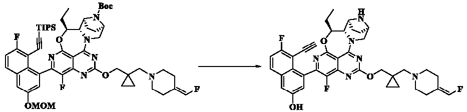

At 0°C, under nitrogen protection, tetrabutylammonium fluoride (1M, 2.6 mL) was added to a solution of tert-butyl (5S,5aS,6S,9R)-12-((1-((4-(fluoromethylene)piperidin-1-yl)methyl)cyclopropyl)methoxy)-5-ethyl-2-(8-ethynyl-7-fluoro-3-(methoxymethoxynaphthalen-1-yl)-1-fluoro-5a,6,7,8,9,10-hexahydro-5H-4-oxa-3,10a,11,13,14-pentaaza-6,9-methylnaphtho[1,8-ab]heptene-4-carboxylate (1 g, 1.19 mmol) in tetrahydrofuran (100 mL), and the obtained mixture was reacted at room temperature for 1 h. The reaction liquid was concentrated, and dichloromethane (50 mL) was added, and the obtained mixture was filtered and washed with dichloromethane (50 mL). MeOH (4 mL) was added, and HCl-dioxane (4 M, 12 mL) solution was added. The obtained mixture was stirred at 20°C for 2 h. At 0°C, the reaction liquid was poured into saturated aqueous NaHCO₃ solution and the obtained mixture was extracted with dichloromethane/methanol (10/1). The organic layer was washed with saturated aqueous sodium chloride solution. The organic layers were combined, dried over anhydrous sodium sulfate, filtered, concentrated, and then purified by silica gel column chromatography. Formic acid (1 eq) was added and the obtained mixture was lyophilized to obtain 4-((5S,5aS,6S,9R)-12-((1-((4-(fluoromethylene)piperidin-1-yl)methyl)cyclopropyl)methoxy)-5-ethyl-1-fluoro-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalen-2-ol (0.6 g, yield 80.5%).

MS m/z (ESI): 697 [M+H].

¹H NMR (400 MHz, MeOD) δ 7.95 - 7.74 (m, 1H), 7.37 - 7.28 (m, 2H), 7.21 -7.09 (m, 1H), 6.72 (s, 0.5H), 6.51 (s, 0.5H), 5.46 - 5.36 (m, 1H), 4.69 - 4.28 (m, 3H), 4.16 (d, *J =* 8 Hz, 1H), 3.92- 3.59 (m, 5H), 3.11 - 2.79 (m, 5H), 2.60 - 2.43 (m, 2H), 2.37 - 2.24 (m, 2H), 2.20 - 2.07 (m, 1H), 2.02 - 1.75 (m, 2H), 1.55 (t, *J* = 8 Hz, 3H), 1.19 - 1.12 (m, 3H), 0.94 - 0.80 (m, 2H), 0.76 - 0.64 (m, 2H).

**Examples 145 to 146 refer to the synthesis of example 1-1:**

| **Serial No.** | **Example structure** | **MS m/z (ESI)** | **Serial No.** | **Example structure** | **MS m/z (ESI)** |
|---|---|---|---|---|---|
| **145** | 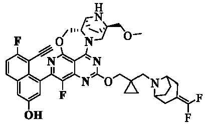 | 757 [M+H] | **146** | 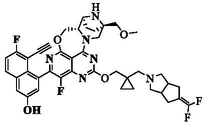 | 757 [M+H] |

**Examples 147 to 160 refer to the synthesis of example 1-1 or example 37:**

| **Serial No.** | **Example structure** | **MS m/z (ESI)** | **Serial No.** | **Example structure** | **MS m/z (ESI)** |
|---|---|---|---|---|---|
| **147** | 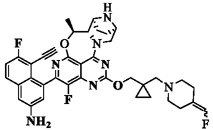 | 682 [M+H] | **148** | 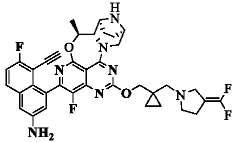 | 686 [M+H] |
| **149** | 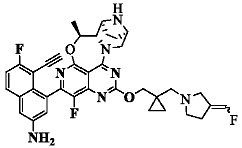 | 668 [M+H] | **150** | 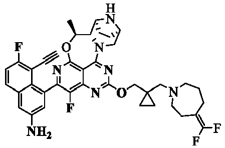 | 714 [M+H] |
| **151** | 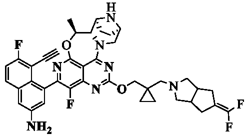 | 726 [M+H] | **152** | 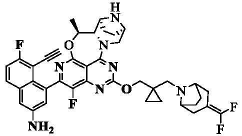 | 726 [M+H] |
| **153** | 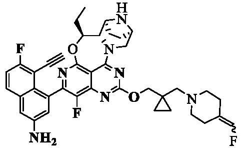 | 696 [M+H] | **154** | 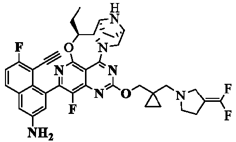 | 700 [M+H] |
| **155** | 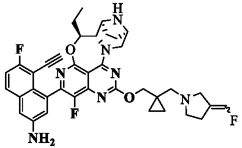 | 682 [M+H] | **156** | 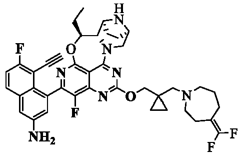 | 728 [M+H] |
| **157** | 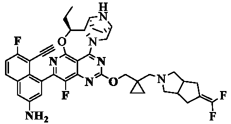 | 740 [M+H] | **158** | 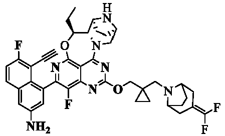 | 740 [M+H] |
| **159** | 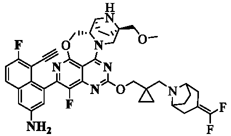 | 756 [M+H] | **160** | 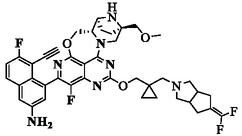 | 756 [M+H] |

**Example 161 and example 162 refer to the synthesis of example 1-1 or the following synthesis method:**

### Example 161

### Step 1: Preparation of tert-butyl (1S,2S,5R)-2-(hydroxymethyl)-3,8-diazabicyclo[3.2.1]octane-8-carboxylate

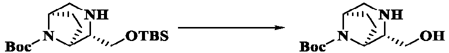

Tetrabutylammonium fluoride trihydrate (62 g, 238 mmol) was added to a solution of tert-butyl (1S,2S,5R)-2-[[tert-butyl (dimethyl)silyl]oxymethyl]-3,8-diazabicyclo[3.2.1]octane-8-carboxylate (50 g, 140 mmol) in tetrahydrofuran (600 mL) at room temperature, and the obtained mixture was stirred at room temperature for 13 h after the addition was completed. After the reaction liquid was concentrated, the crude product (109 g) was directly used in the next reaction.

MS m/z (ESI): 243 [M+H].

### Step 2: Preparation of 3-benzyl 8-(tert-butyl)(1S,2S,5R)-2-(hydroxymethyl)-3,8-diazabicyclo[3.2.1]octane-3,8-dicarboxylate

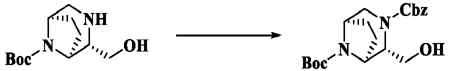

Sodium bicarbonate (23.4 g, 279 mmol) was added to a solution of tert-butyl (1S,2S,5R)-2-(hydroxymethyl)-3,8-diazabicyclo[3.2.1]octane-8-carboxylate (109 g, 139.5 mmol) in ethyl acetate (250 mL) and water (250 mL) at room temperature, then benzyl chloroformate (26.2 g, 153.4 mmol) was slowly added dropwise, and the obtained mixture was stirred at room temperature for 1 h after the addition was completed. Liquid separation was performed, and the aqueous phase was extracted with ethyl acetate. The organic phases were combined, washed with saturated sodium chloride aqueous solution, dried over anhydrous sodium sulfate, filtered, concentrated, and then purified by silica gel column chromatography to obtain 3-benzyl 8-(tert-butyl)(1S,2S,5R)-2-(hydroxymethyl)-3,8-diazabicyclo[3.2.1]octane-3,8-dicarboxylate (44 g, yield 84%).

MS m/z (ESI): 377 [M+H].

### Step 3: Preparation of 3-benzyl 8-(tert-butyl)(1S,2S,5R)-2-formyl-3,8-diazabicyclo[3.2.1]octane-3,8-dicarboxylate

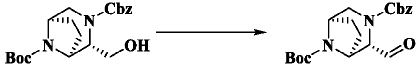

Under ice-water bath, sodium hypochlorite (39 g, 526 mmol) was added dropwise to a solution of 3-benzyl 8-(tert-butyl)(1S,2S,5R)-2-(hydroxymethyl)-3,8-diazabicyclo[3.2.1]octane-3,8-dicarboxylate (44 g, 117 mmol), 2,2,6,6-tetramethylpiperidine oxide (1.83 g, 11.7 mmol), potassium bromide (1.4 g, 11.7 mmol), and sodium bicarbonate (58.9 g, 701 mmol) in dichloromethane (800 mL) and water (700 mL). After the addition was completed, the obtained mixture was stirred for 30 min. TLC (petroleum ether/ethyl acetate = 3/1) showed that the raw material remained. Additional sodium hypochlorite (15.68 g, 210.6 mmol) was added and the obtained mixture was stirred for 30 min under ice-water bath. Layer separation was performed by letting the mixture stand, and the aqueous phase was extracted with dichloromethane. The organic phases were combined, washed with water, 10% sodium thiosulfate and saturated sodium chloride aqueous solution, dried over anhydrous sodium sulfate, filtered, concentrated, and purified by silica gel column chromatography to obtain 3-benzyl 8-(tert-butyl)(1S,2S,5R)-2-formyl-3,8-diazabicyclo[3.2.1]octane-3,8-dicarboxylate (35 g, yield 80%).

MS m/z (ESI): 375 [M+H].

### Step 4: Preparation of 3-benzyl 8-(tert-butyl)(1S,2S,5R)-2-((S)-1-hydroxyethyl-2,2,2-d3)-3,8-diazabicyclo[3.2.1]octane-3,8-dicarboxylate

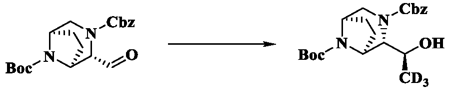

(Trideuteriomethyl)magnesium iodide (1 M, 243 mL) was added dropwise to a solution of 3-benzyl 8-(tert-butyl)(1S,2S,5R)-2-formyl-3,8-diazabicyclo[3.2.1]octane-3,8-dicarboxylate (70 g, 187 mmol) in tetrahydrofuran (680 mL) at -70°C (the dropwise addition was completed in 45 min), and the obtained mixture was stirred at -70°C for 45 min. The reaction liquid was slowly poured into ammonium chloride aqueous solution (1 L), the obtained mixture was extracted with ethyl acetate. The organic phase was dried over anhydrous sodium sulfate, filtered, and concentrated to obtain 3-benzyl 8-(tert-butyl)(1S,2S,5R)-2-((S)-1-hydroxyethyl-2,2,2-d3)-3,8-diazabicyclo[3.2.1]octane-3,8-dicarboxylate (73 g, yield 99%).

MS m/z (ESI): 394 [M+H].

### Step 5: Preparation of tert-butyl (1S,2S,5R)-2-(2,2,2-trideuterium-1-hydroxyethyl)-3,8-diazabicyclo[3.2.1]octane-8-carboxylate

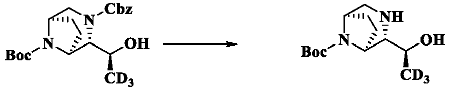

3-benzyl 8-(tert-butyl)(1S,2S,SR)-2-((S)-1-hydroxyethyl-2,2,2-d3)-3,8-diazabicyclo[3.2.1]octane-3,8-dicarboxylate (73 g, 185.5 mmol) and wet palladium on carbon (8 g, 7.52 mmol, purity 10%) were stirred at room temperature under a hydrogen balloon for 13 h. The reaction liquid was filtered, and the filtrate was concentrated and then purified by silica gel column chromatography to obtain tert-butyl (1S,2S,5R)-2-(2,2,2-trideuterium-1-hydroxyethyl)-3,8-diazabicyclo[3.2.1] octane-8-carboxylate (21 g, yield 44%).

MS m/z (ESI): 260 [M+H].

### Step 6: Preparation of tert-butyl (1S,2S,5R)-2-[(1S)-1-(7-chloro-8-fluoro-4-hydroxy-2-methylsulfanyl-pyrido[4,3-d]pyrimidin-5-yl)oxy-2,2,2-trideuterium-ethyl]-3,8-diazabicyclo[3.2.1]octane-8-carboxylate

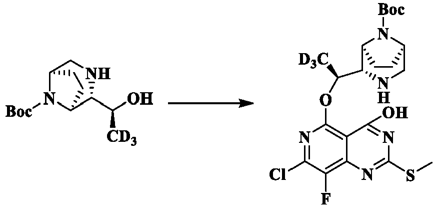

Sodium hydride (11.3 g, 283.4 mmol, purity 60%) was added to a solution of 5,7-dichloro-8-fluoro-2-methylsulfanyl-pyrido[4,3-d]pyrimidin-4-ol (25 g, 89 mmol) and tert-butyl (1S,25,5R)-2-(2,2,2-trideuterium-1-hydroxyethyl)-3,8-diazabicyclo[3.2.1]octane-8-carboxylate (21 g, 81 mmol) in tetrahydrofuran (500 mL) at 0°C, and the obtained mixture was stirred at room temperature for 1 h after the addition was completed. The reaction liquid was slowly poured into 10% ammonium chloride aqueous solution (800 mL) and ethyl acetate (500 mL). A large amount of solid precipitated. After filtration, the solid was dried to obtain tert-butyl (15,2S,5R)-2-[(1S)-1-(7-chloro-8-fluoro-4-hydroxy-2-methylsulfanyl-pyrido[4,3-d]pyrimidin-5-yl)oxy-2,2,2-trideuterium-ethyl]-3,8-diazabicyclo[3.2.1]octane-8-carboxylate (25 g, yield 61%).

MS m/z (ESI): 503 [M+H].

### Step 7: Preparation of tert-butyl (5S,5aS,6R,9S)-2-chloro-1-fluoro-5-(methyl-d3)-12-(methylthio)-5a,6,7,8,9,10-hexahydro-5H-4-oxa-3,10a,11,13,14-pentaaza-6,9-methylnaphtho[1,8-ab]heptene-4-carboxylate

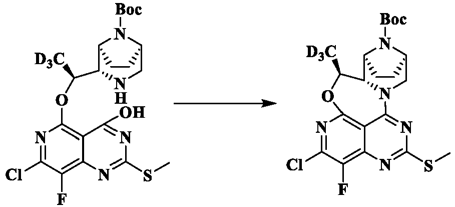

N,N-diisopropylethylamine (38.5 g, 298 mmol, 52 mL) was added to a solution of tert-butyl (1S,2S,5R)-2-[(1S)-1-(7-chloro-8-fluoro-4-hydroxy-2-methylsulfanyl-pyrido[4,3-d]pyrimidin-5-yl)oxy-2,2,2-trideuterium-ethyl]-3,8-diazabicyclo[3.2.1] octane-8-carboxylate (25 g, 49.7 mmol) in dichloromethane (250 mL) at 0°C, then phosphorus oxychloride (18.3 g, 119 mmol, 10.9 mL) was added dropwise, and the obtained mixture was stirred at room temperature for 1 h after the addition was completed. The reaction liquid was poured into sodium bicarbonate aqueous solution (500 mL), the obtained mixture was extracted with dichloromethane. The organic phase was washed with ammonium chloride aqueous solution, washed with saturated sodium chloride aqueous solution, dried over anhydrous sodium sulfate, filtered, concentrated and slurried with ethyl acetate/petroleum ether = 1/1 (150 mL), and the obtained slurry was filtered and the filter cake was dried to obtain tert-butyl (5S,5aS,6R,9S)-2-chloro-1-fluoro-5-(methyl-d3)-12-(methylthio)-5a,6,7,8,9,10-hexahydro-5H-4-oxa-3,10a,11,13,14-pentaaza-6,9-methylnaphtho[1,8-ab]heptene-4-carboxylate (20.4 g, yield 85%).

MS m/z (ESI): 485 [M+H].

### Step 8: Preparation of tert-butyl (5S,5aS,6R,9S)-2-chloro-1-fluoro-5-(methyl-d3)-12-(methylsulfonyl)-5a,6,7,8,9,10-hexahydro-5H-4-oxa-3,10a,11,13,14-pentaaza-6,9-methylnaphtho[1,8-ab]heptene-4-carboxylate

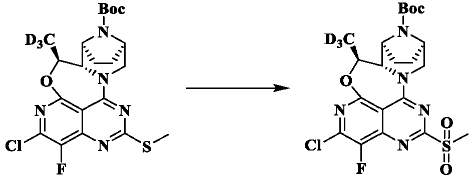

M-chloroperoxybenzoic acid (30.5 g, 150.2 mmol, purity 85%) was added to a solution of tert-butyl (5S,5aS,6R,9S)-2-chloro-1-fluoro-5-(methyl-d3)-12-(methylthio)-5a,6,7,8,9,10-hexahydro-5H-4-oxa-3,10a,11,13,14-pentaaza-6,9-methylnaphtho[1,8-ab]heptene-4-carboxylate (20.4 g, 42.1 mmol) in dichloromethane (200 mL) at 5°C, and the obtained mixture was stirred for 1.5 h after the addition was completed. The reaction liquid was filtered, and the filtrate was stirred with aqueous sodium thiosulfate solution (500 mL) and ethyl acetate (500 mL) for 30 min. Liquid separation was performed, and the aqueous phase was extracted with ethyl acetate. The organic phases were combined, washed with sodium bicarbonate aqueous solution and washed with saturated sodium chloride aqueous solution, dried over anhydrous sodium sulfate, filtered, concentrated, and purified by silica gel column chromatography to obtain tert-butyl (5S,5aS,6R,9S)-2-chloro-1-fluoro-5-(methyl-d3)-12-(methylsulfonyl)-5a,6,7,8,9,10-hexahydro-5H-4-oxa-3,10a,11,13,14-pentaaza-6,9-methylnaphtho[1,8-ab]heptene-4-carboxylate (18 g, yield 83%).

MS m/z (ESI): 517 [M+H].

### Step 9: Preparation of tert-butyl (5S,5aS,6S,9R)-2-chloro-12-((1-((4-(difluoromethylene)piperidin-1-yl)methyl)cyclopropyl)methoxy)-1-fluoro-5-(methyl-d3)-5a,6,7,8,9,10-hexahydro-5H-4-oxa-3,10a,11,13,14-pentaaza-6,9-methylnaphtho[1,8-ab]heptene-4-carboxylate

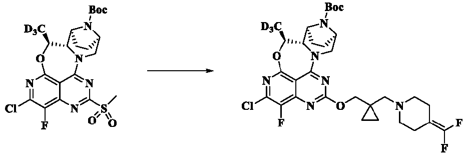

In an eggplant-shaped flask, tert-butyl (5S,5aS,6S,9R)-2-chloro-1-fluoro-5-(methyl-d3)-12-(methylsulfonyl)-5a,6,7,8,9,10-hexahydro-5H-4-oxa-3,10a,11,13,14-pentaaza-6,9-methylnaphtho[1,8-ab]heptene-4-carboxylate (14.00 g, 27.08 mmol) and [1-[[4-(difluoromethylene)-1-piperidinyl]methyl]cyclopropyl] methanol (7.65 g, 35.20 mmol) were dissolved in tetrahydrofuran (140 mL). The obtained mixture was subjected to nitrogen replacement, and lithium bis(trimethylsilyl)amide (1 M, 81.24 mL) was added dropwise under ice bath. The obtained mixture was reacted under ice bath for 20 min. A saturated ammonium chloride solution was added to quench the reaction, and the obtained mixture was extracted with ethyl acetate, dried over anhydrous sodium sulfate, filtered, concentrated, and purified by silica gel column chromatography to obtain tert-butyl (5S,5aS,6S,9R)-2-chloro-12-((1-((4-(difluoromethylene)piperidin-1-yl)methyl)cyclopropyl)methoxy)-1-fluoro-5-(methyl-d3)-5a,6,7,8,9,10-hexahydro-5H-4-oxa-3,10a,11,13,14-pentaaza-6,9-methylnaphtho[1,8-ab]heptene-4-carboxylate (13 g, yield 73.4%).

MS m/z (ESI): 654[M+H].

### Step 10: Preparation of tert-butyl (5S,5aS,6S,9R)-12-((1-((4-(difluoromethylene)piperidin-1-yl)methyl)cyclopropyl)methoxy)-1-fluoro-2-(7-fluoro-3-(methoxymethoxy)-8-((triisopropylsilyl)ethynyl)naphthalen-1-yl)-5-(methyl-d3)-5a,6,7,8,9,10-hexahydro-5H-4-oxa-3,10a,11,13,14-pentaaza-6,9-methylnaphtho[1,8-ab]heptene-14carboxylate

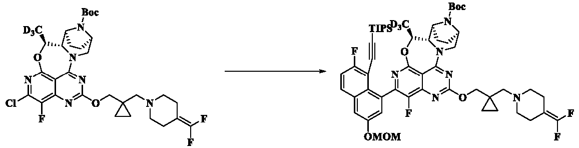

In an eggplant-shaped bottle, 2-[2-fluoro-6-(methoxymethoxy)-8-(4,4,5,5-tetramethyl-1,3,2-dioxyboran-2-yl)-1-naphthyl]ethynyltriisopropylsilane (10.19 g, 19.87 mmol), tert-butyl (5S,5aS,6S,9R)-2-chloro-12-((1-((4-(difluoromethylene)piperidin-1-yl)methyl)cyclopropyl)methoxy)-1-fluoro-5-(methyl-d3)-5a,6,7,8,9,10-hexahydro-5H-4-oxa-3,10a,11,13,14-pentaaza-6,9-methylnaphtho[1,8-ab]heptene-4-carboxylate (13 g, 19.87 mmol), methanesulfonic acid [n-butyldi(1-adamantyl)phosphine](2-amino-1,1'-biphenyl-2-yl)palladium(II) (723.66 mg, 993.68 µmol), and cesium carbonate (12.95 g, 39.75 mmol) were dissolved in a mixed solvent of dioxane (30 mL) and water (6 mL). Nitrogen replacement was performed and the reaction was carried out in an oil bath at 85°C for 1 h. After the addition was completed, the mixture was concentrated and purified by silica gel chromatography to obtain tert-butyl (5S,5aS,6S,9R)-12-((1-((4-(difluoromethylene)piperidin-1-yl)methyl)cyclopropyl)methoxy)-1-fluoro-2-(7-fluoro-3-(methoxymethoxy)-8-((triisopropylsilyl)ethynyl)naphthalen-1-yl)-5-(methyl-d3)-5a,6,7,8,9,10-hexahydro-5H-4-oxa-3,10,11,13,14-pentaaza-6,9-methylnaphtho[1,8-ab]heptene-14carboxylate (13 g, yield 65.1%).

MS m/z (ESI): 1004[M+H].

### Step 11: Preparation of tert-butyl (5S,5aS,6S,9R)-12-((1-((4-(difluoromethylene)piperidin-1-yl)methyl)cyclopropyl)methoxy)-2-(8-ethynyl-7-fluoro-3-(methoxymethoxynaphthalen-1-yl)-1-fluoro-5-(methyl-d3)-5a,6,7,8,9,10-hexahydro-5H-4-oxo-3,10a,11,13,14-pentaaza-6,9-methylnaphtho[1,8-ab]heptene-4-carboxylate

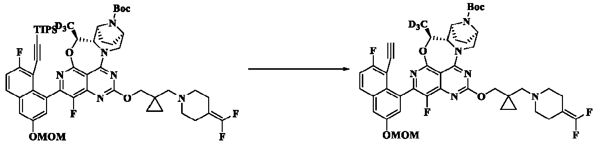

In an eggplant-shaped flask, tert-butyl (5S,5aS,6S,9R)-12-((1-((4-(difluoromethylene)piperidin-1-yl)methyl)cyclopropyl)methoxy)-1-fluoro-2-(7-fluoro-3-(methoxymethoxy)-8-((triisopropylsilyl)ethynyl)naphthalen-1-yl)-5-(methyl-d3)-5,6,7,8,9,10-hexahydro-5-4-oxa-3,10,11,13,14-pentaaza-6,9-methylnaphtho[1,8-ab]heptene-14carboxylate (13 g, 12.94 mmol) was dissolved in tetrahydrofuran (120 mL), tetrabutylammonium fluoride (1 M, 25.89 mL) was added, and the obtained mixture was reacted at room temperature for 1 h. The reaction liquid was concentrated and purified by silica gel column chromatography to obtain tert-butyl (5S,5aS,6S,9R)-12-((1-((4-(difluoromethylene)piperidin-1-yl)methyl) cyclopropyl)methoxy)-2-(8-ethynyl-7-fluoro-3-(methoxymethoxynaphthalen-1-yl)-1-fluoro-5-(methyl-d3)-5a,6,7,8,9,10-hexahydro-5H-4-oxo-3,10a,11,13,14-pentaaza-6,9-methylnaphtho[1,8-ab]heptene-4-carboxylate (9.7 g, yield 88.4%).

MS m/z (ESI): 848[M+H].

### Step 12: Preparation of 4-((5S,5aS,6S,9R)-12-((1-((4-(difluoromethylene)piperidin-1-yl)methyl)cyclopropyl)methoxy)-1-fluoro-5-(methyl-d3)-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalen-2-ol

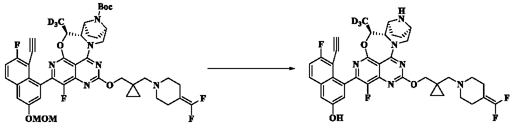

tert-butyl (5S,5aS,6S,9R)-12-((1-((4-(difluoromethylene)piperidin-1-yl)methyl)cyclopropyl)methoxy)-2-(8-ethynyl-7-fluoro-3-(methoxymethoxynaphthalen-1-yl)-1-fluoro-5-(methyl-d3)-5a,6,7,8,9,10-hexahydro-5H-4-oxo-3,10a, 11, 13,14-pentaaza-6,9-methylnaphtho[1,8-ab]heptene-4-carboxylate (9.7 g, 11.44 mmol) was dissolved in a solution of dichloromethane (160 mL) and methanol (40 mL), and the obtained mixture was cooled under ice bath for 10 min. Nitrogen replacement was performed, and a solution of 4N hydrochloric acid in dioxane (194 mL) was slowly added, and the obtained mixture was reacted under ice bath for 1.5 h. Sodium bicarbonate solution was added to the reaction liquid to quench the reaction, and the obtained mixture was extracted with dichloromethane/methanol at low temperature. The organic phase was dried over anhydrous sodium sulfate, filtered, concentrated, and purified by silica gel column chromatography to obtain the product 4-((5S,5aS,6S,9R)-12-((1-((4-(difluoromethylene)piperidin-1-yl)methyl)cyclopropyl)methoxy)-1-fluoro-5-(methyl-d3)-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalen-2-ol (6.2 g, yield 75.3%).

MS m/z(ESI):704 [M+H].

¹H NMR(400 MHz, MeOD) δ 7.84(dt, *J* = 9.2, 5.9 Hz, 1H), 7.36 - 7.27(m, 2H), 7.27 - 7.14(m, 1H), 5.45(dt, *J* = 14.0, 3.3 Hz, 1H), 4.65 - 4.18(m, 4H), 3.98(d, *J* = 5.7 Hz, 1H), 3.91 - 3.86(m, 1H), 3.81 - 3.65(m, 3H), 2.99 - 2.82(m, 5H), 2.37(q, *J* = 6.4 Hz, 4H), 2.25 - 2.13(m, 1H), 2.04 - 1.86(m, 3H), 0.87 - 0.63(m, 4H).

### Example 162

### Step 1: Preparation of tert-butyl (5S,5aS,6R,9S)-2-chloro-12-((1-((((R)-4-(difluoromethylene)-2-methylpiperidin-1-yl)methyl)cyclopropyl)methoxy)-1-fluoro-5-(methyl-d3)-5a,6,7,8,9,10-hexahydro-5H-4-oxa-3,10a,11,13,14-pentaaza-6,9-methylnaphtho[1,8-ab]heptene-4-carboxylate

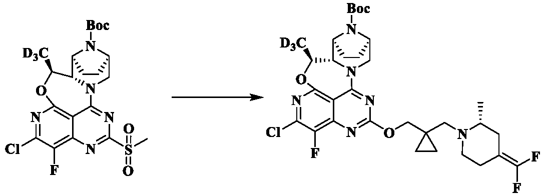

Lithium bis(trimethylsilyl)amide (1 M, 81.2 mL) was added dropwise to a solution of tert-butyl (5S,5aS,6R,9S)-2-chloro-1-fluoro-5-(methyl-d3)-12-(methylsulfonyl)-5a,6,7,8,9,10-hexahydro-5H-4-oxa-3,10a,11,13,14-pentaaza-6,9-methylnaphtho[1,8-ab]heptene-4-carboxylate (14 g, 27.1 mmol) and [1-[[(2R)-4-(difluoromethylene)-2-methyl-1-piperidinyl]methyl]cyclopropyl]methanol (8.14 g, 35.2 mmol) in tetrahydrofuran (140 mL) at 0°C (15 min) and the obtained mixture was stirred at 0°C for 15 min after the addition was completed. The reaction liquid was poured into ammonium chloride aqueous solution (300 mL), and the obtained mixture was extracted with ethyl acetate. The organic phase was washed with saturated sodium chloride aqueous solution, dried over anhydrous sodium sulfate and filtered; after concentration, the obtained product was dissolved in petroleum ether/ethyl acetate = 10/1 (140 mL), and the obtained mixture was stirred for 30 min and filtered, and the filtrate was concentrated to obtain tert-butyl (5S,5aS,6R,9S)-2-chloro-12-((1-((((R)-4-(difluoromethylene)-2-methylpiperidin-1-yl)methyl) cyclopropyl)methoxy)-1-fluoro-5-(methyl-d3)-5a,6,7,8,9,10-hexahydro-5H-4-oxa-3,10a,11,13,14-pentaaza-6,9-methylnaphtho[1,8-ab]heptene-4-carboxylate (17.8 g, yield 98%).

MS m/z (ESI): 668 [M+H].

### Step 2: Preparation of tert-butyl (5S,5aS,6R,9S)-12-((1-((((R)-4-(difluoromethylene)-2-methylpiperidin-1-yl)methyl)cyclopropyl)methoxy)-1-fluoro-2-(7-fluoro-3-(methoxymethoxy)-8-((triisopropylsilyl)ethynyl)naphthalen-1-yl)-5-(methyl-d3)-5a,6,7,8,9,10-hexahydro-5H-4-oxa-3,10a,11,13,14-pentaaza-6,9-methylnaphtho[1,8-ab]heptene-4-carboxylate

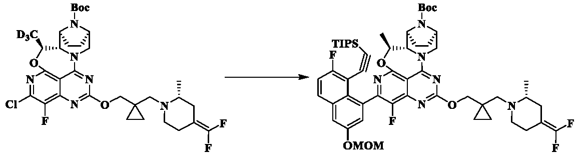

tert-butyl (5S,5aS,6R,9S)-2-chloro-12-((1-((((R)-4-(difluoromethylene)-2-methylpiperidin-1-yl)methyl)cyclopropyl)methoxy)-1-fluoro-5-(methyl-d3)-5a,6,7,8,9, 10-hexahydro-5H-4-oxa-3, 10a, 11, 13,14-pentaaza-6,9-methylnaphtho [1,8-ab]heptene-4-carboxylate (17.8 g, 26.64 mmol), 2-[2-fluoro-6-(methoxymethoxy)-8-(4,4,5,5-tetramethyl-1,3,2-dioxyboran-2-yl)-1-naphthyl] ethynyltriisopropylsilane (13.65 g, 26.6 mmol), methanesulfonate (dimethyl-n-butylphosphino)-2'-amino-1,1'-biphenyl-2-yl) palladium(II) dichloromethane adduct (970 mg, 1.33 mmol), and cesium carbonate (17.4 g, 53.3 mmol) were stirred in dioxane (180 mL) and water (36 mL) at 85°C for 1 h. Water (300 mL) was added to the reaction liquid, and the obtained mixture was extracted with ethyl acetate, dried over anhydrous sodium sulfate, filtered, concentrated, and purified by silica gel chromatography to obtain tert-butyl (5S,5aS,6R,9S)-12-((1-((((R)-4-(difluoromethylene)-2-methylpiperidin-1-yl)methyl)cyclopropyl)methoxy)-1-fluoro-2-(7-fluoro-3-(methoxymethoxy)-8-((triisopropylsilyl)ethynyl)naphthalen-1-yl)-5-(methyl-d3)-5a,6,7,8,9,10-hexahydro-5H-4-oxa-3,10a,11,13,14-pentaaza-6,9-methylnaphtho[1,8-ab]heptene-4-carboxylate (20 g, yield 74%).

MS m/z (ESI): 1015 [M+H].

### Step 3: Preparation of tert-butyl (SS,SaS,6R,9S)-12-((1-((((R)-4-(difluoromethylene)-2-methylpiperidin-1-yl)methyl)cyclopropyl)methoxy)-2-(8-ethynyl-7-fluoro-3-(methoxymethoxynaphthalen-1-yl)-1-fluoro-5-(methyl-d3)-5a,6,7,8,9,10-hexahydro-5H-4-oxa-3,10a,11,13,14-pentaaza-6,9-methylnaphtho[1,8-ab]heptene-4-carboxylate

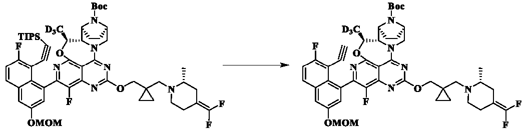

A solution of tetrabutylammonium fluoride in tetrahydrofuran (1 M, 40 mL) was added to a solution of tert-butyl (5S,5aS,6R,9S)-12-((1-((((R)-4-(difluoromethylene)-2-methylpiperidin-1-yl)methyl)cyclopropyl)methoxy)-1-fluoro-2-(7-fluoro-3-(methoxymethoxy)-8-((triisopropylsilyl)ethynyl)naphthalen-1-yl)-5-(methyl-d3)-5a,6,7,8,9,10-hexahydro-5H-4-oxa-3,10a,11,13,14-pentaaza-6,9-methylnaphtho[1,8-ab]heptene-4-carboxylate (20 g, 19.64 mmol) in tetrahydrofuran (200 mL) at room temperature, and the obtained mixture was stirred at room temperature for 1 h after addition was completed. Water (300 mL) was added, and the obtained mixture was extracted with ethyl acetate. The organic phase was washed with water, dried over anhydrous sodium sulfate, filtered, concentrated, and purified by silica gel chromatography to obtain tert-butyl (5S,5aS,6R,9S)-12-((1-((((R)-4-(difluoromethylene)-2-methylpiperidin-1-yl)methyl)cyclopropyl) methoxy)-2-(8-ethynyl-7-fluoro-3-(methoxymethoxynaphthalen-1-yl)-1-fluoro-5-(methyl-d3)-5a,6,7,8,9,10-hexahydro-5H-4-oxa-3,10a,11,13,14-pentaaza-6,9-methylnaphtho[1,8-ab]heptene-4-carboxylate (16 g, yield 95%).

MS m/z (ESI): 862 [M+H].

### Step 4: Preparation of 4-((5S,5aS,6R,9S)-12-((1-((((R)-4-(difluoromethylene)-2-methylpiperidin-1-yl)methyl)cyclopropyl)methoxy)-1-fluoro-5-(methyl-d3)-5a,6,7,8,9,10-hexahydro-5H-4-oxa-3,10a,11,13,14-pentaaza-6,9-methylnaphtho[1,8-ab]hept-2-yl)-5-ethynyl-6-fluoronaphthalen-2-ol

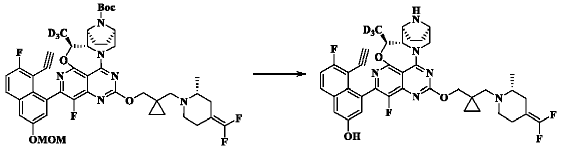

Hydrochloric acid (4 M, 203 mL) was added dropwise to a solution of tert-butyl (5S,5aS,6R,9S)-12-((1-((((R)-4-(difluoromethylene)-2-methylpiperidin-1-yl)methyl)cyclopropyl)methoxy)-2-(8-ethynyl-7-fluoro-3-(methoxymethoxynaphthalen-1-yl)-1-fluoro-5-(methyl-d3)-5a,6,7,8,9,10-hexahydro-5H-4-oxa-3,10a,11,13,14-pentaaza-6,9-methylnaphtho[1,8-ab]heptene-4-carboxylate (10 g, 11.6 mmol) in a solution of dichloromethane (200 mL) and methanol (40 mL) at 5°C (< 10°C). After addition was completed, the obtained mixture was stirred at 0°C for 2 h. The reaction liquid was slowly added to a sodium bicarbonate aqueous solution (800 mL), and the obtained mixture was extracted with ethyl acetate/methanol = 10/1. The organic phase was washed with saturated sodium chloride aqueous solution, dried over anhydrous sodium sulfate, concentrated, and purified by silica gel column chromatography to obtain 4-((5S,5aS,6R,9S)-12-((1-((((R)-4-(difluoromethylene)-2-methylpiperidin-1-yl)methyl)cyclopropyl) methoxy)-1-fluoro-5-(methyl-d3)-5a,6,7,8,9,10-hexahydro-5H-4-oxa-3, 10a, 11, 13,14-pentaaza-6,9-methylnaphtho[1,8-ab]hept-2-yl)-5-ethynyl-6-fluoronaphthalen-2-ol (4.27 g, yield 49%).

¹H NMR (400 MHz, DMSO) δ 10.13 (s, 1H), 8.02 - 7.89 (m, 1H), 7.46 (q, *J* = 8 Hz, 1H), 7.37 (s, 1H), 7.19 (d, *J =* 4 Hz, 0.5H), 7.05 (d, *J* = 4 Hz, 0.5H), 5.27 - 5.04 (m, 1H), 4.55 - 4.30 (m, 2H), 4.27 - 4.05 (m, 2H), 4.01 - 3.85 (m, 1H), 3.63 - 3.49 (m, 1H), 3.45 (t, *J* = 4 Hz, 1H), 3.09 - 2.94 (m, 1H), 2.92 - 2.71 (m, 3H), 2.70 - 2.54 (m, 1H), 2.37 - 2.22 (m, 1H), 2.22 - 1.98 (m, 4H), 1.95 - 1.42 (m, 5H), 0.93 (d, *J* = 4 Hz, 3H), 0.74 - 0.52 (m, 2H), 0.51 - 0.29 (m, 2H).

MS m/z (ESI): 718 [M+H].

**Examples 163 to 171 refer to the synthesis of example 1-1 or example 161:**

| **Serial No.** | **Example structure** | **MS m/z (ESI)** | **Serial No.** | **Example structure** | **MS m/z (ESI)** |
|---|---|---|---|---|---|
| 163 | 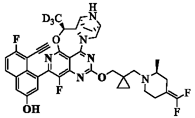 | 718 [M+H] | 164 | 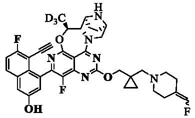 | 686 [M+H] |
| **Example 163:** ¹H NMR (400 MHz, MeOD) δ 7.90 - 7.76 (m, 1H), 7.37 - 7.25 (m, 2H), 7.24 - 7.12 (m, 1H), 5.45 - 5.30 (m, 1H), 4.58 - 4.39 (m, 3H), 4.37 - 4.21 (m, 1H), 4.10 (d, *J* = 8 Hz, 1H), 3.76 - 3.59 (m, 2H), 3.25 - 2.91 (m, 4H), 2.81 - 2.63 (m, 1H), 2.49 - 2.33 (m, 1H), 2.33 - 2.13 (m, 3H), 2.13 - 1.97 (m, 2H), 1.98 - 1.69 (m, 5H), 1.59 (t, *J* = 8 Hz, 3H), 0.79 - 0.63 (m, 2H), 0.61 - 0.42 (m, 2H). | | | | | |
| **Example 164:** ¹H NMR (400 MHz, MeOD) δ 7.90 - 7.79 (m, 1H), 7.37 - 7.28 (m, 2H), 7.27 - 7.22 (m, 0.5H), 7.16 - 7.10 (m, 0.5H), 6.76 (s, 0.5H), 6.55 (s, 0.5H), 5.46 (d, *J* = 16 Hz, 1H), 4.69 - 4.33 (m, 3H), 4.26 (d, *J =* 12 Hz, 1H), 4.07 - 3.86 (m, 2H), 3.81 - 3.63 (m, 3H), 3.27 - 2.98 (m, 5H), 2.73 - 2.49 (m, 2H), 2.45 - 2.31 (m, 2H), 2.25 - 2.12 (m, 1H), 2.11 - 1.79 (m, 3H), 0.99 - 0.85 (m, 2H), 0.81 - 0.64 (m, 2H). | | | | | |
| **165** | 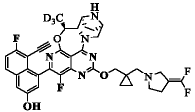 | 690 [M+H] | **166** | 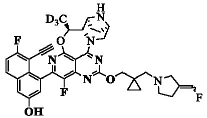 | 672 [M+H] |
| **Example 165:** ¹H NMR (400 MHz, MeOD) δ 7.83 - 7.76 (m, 1H), 7.36 - 7.28 (m, 2H), 7.25 - 7.09 (m, 1H), 5.12 - 5.00 (m, 1H), 4.55 - 4.42 (m, 4H), 4.42 - 4.24 (m, 1H), 4.20 - 4.12 (m, 1H), 3.87 - 3.68 (m, 1H), 3.27 - 3.05 (m, 4H), 3.04 - 2.47 (m, 4H), 2.46 - 2.23 (m, 2H), 2.10 - 1.72 (m, 3H), 0.84 - 0.70 (m, 2H), 0.69 - 0.45 (m, 2H). | | | | | |
| **Example 166:** ¹H NMR (400 MHz, DMSO) δ 10.12 (s, 1H), 7.96 (dt, *J* = 9.2, 5.7 Hz, 1H), 7.46 (td, *J* = 9.0, 7.5 Hz, 1H), 7.37 (d, *J* = 2.5 Hz, 1H), 7.13 (dd, *J* = 50.0, 2.6 Hz, 1H), 6.95 - 6.51 (m, 1H), 5.24 - 5.03 (m, 1H), 4.61 - 4.38 (m, 1H), 4.29 - 4.19 (m, 2H), 4.03 - 3.90 (m, 2H), 3.64 - 3.42 (m, 2H), 3.23 - 3.14 (m, 2H), 3.10 - 2.99 (m, 2H), 2.68 - 2.56 (m, 2H), 2.47 - 2.24 (m, 4H), 1.89 - 1.79 (m, 1H), 1.78 - 1.52 (m, 3H), 0.75 - 0.53 (m, 2H), 0.50 - 0.41 (m, 2H). | | | | | |
| **167** | 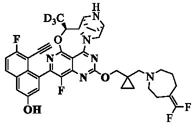 | 718 [M+H] | **168** | 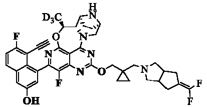 | 730 [M+H] |
| **Example 167:** ¹H NMR (400 MHz, MeOD) δ 7.83 (dt, *J =* 9.1, 6.1 Hz, 1H), 7.35 - 7.27 (m, 2H), 7.19 (dd, *J* = 43.1, 2.5 Hz, 1H), 5.42 - 5.32 (m, 1H), 4.54 - 4.32 (m, 3H), 4.09 (d, *J* = 8.7 Hz, 1H), 3.74 - 3.70 (m, 1H), 3.63 (d, *J* = 4.9 Hz, 1H), 3.38 (s, 1H), 3.19 (t, *J* = 11.6 Hz, 1H), 3.07 - 2.70 (m, 6H), 2.35 (d, *J* = 45.5 Hz, 4H), 2.06 (d, *J* = 8.6 Hz, 1H), 1.92 - 1.71 (m, 5H), 0.78 (d, *J* = 4.8 Hz, 2H), 0.59 (s, 2H). | | | | | |
| **Example 168:** ¹H NMR (400 MHz, MeOD) δ 7.86 - 7.79 (m, 1H), 7.35 - 7.27 (m, 2H), 7.17 (d, *J* = 2.6 Hz, 1H), 5.36 (d, *J* = 13.2 Hz, 1H), 4.54 - 4.31 (m, 3H), 4.11 - 4.04 (m, 1H), 3.69 - 3.67 (m, 1H), 3.62 - 3.57 (m, 1H), 3.22 - 3.13 (m, 1H), 2.91 - 2.66 (m, 4H), 2.60 - 2.39 (m, 4H), 2.34 - 2.23 (m, 2H), 2.18 - 2.03 (m, 3H), 1.85 - 1.77 (m, 2H), 1.36 - 1.29 (m, 2H), 0.71 - 0.49 (m, 4H). | | | | | |
| **169** | 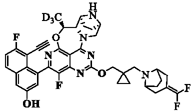 | 730 [M+H] | **170** | 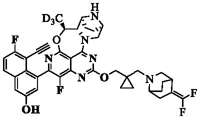 | 730 [M+H] |
| **Example 169:** ¹H NMR (400 MHz, MeOD) δ 7.87 - 7.78 (m, 1H), 7.34 - 7.26 (m, 2H), 7.14 (d, *J* = 2.7 Hz, 1H), 5.35 (d, *J* = 13.3 Hz, 1H), 4.54 - 4.30 (m, 3H), 4.11 - 4.03 (m, 1H), 3.70 - 3.67 (m, 1H), 3.63 - 3.57 (m, 1H), 3.21 - 3.13 (m, 1H), 2.90 - 2.66 (m, 4H), 2.60 - 2.39 (m, 4H), 2.34 - 2.23 (m, 2H), 2.18 - 2.03 (m, 3H), 1.85 - 1.77 (m, 2H), 1.36 - 1.29 (m, 2H), 0.70 - 0.48 (m, 4H). | | | | | |
| **171** | 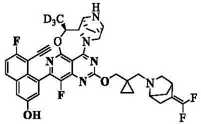 | 716 [M+H] | | | |

**Examples 172 to 175 refer to the synthesis of example 1-1 or example 124:**

| **Serial No.** | **Example structure** | **MS m/z (ESI)** | **Serial No.** | **Example structure** | **MS m/z (ESI)** |
|---|---|---|---|---|---|
| **172** | 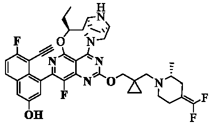 | 729 [M+H] | **173** | 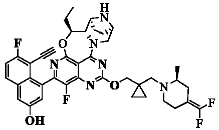 | 729 [M+H] |
| **Example 172:** ¹H NMR (400 MHz, DMSO) δ 10.15 (s, 1H), 7.96 (ddd, *J* = 8.7, 5.9, 2.4 Hz, 1H), 7.46 (td, *J* = 9.0, 3.7 Hz, 1H), 7.37 (t, *J* = 2.4 Hz, 1H), 7.11 (dd, *J* = 47.0, 2.5 Hz, 1H), 5.17 (dd, *J* = 12.9, 2.7 Hz, 1H), 4.45 - 4.12 (m, 4H), 4.05 (d, *J* = 9.2 Hz, 1H), 3.60 - 3.51 (m, 1H), 3.47 (t, *J* = 7.0 Hz, 1H), 3.04 (t, *J* = 11.4 Hz, 1H), 2.92 - 2.75 (m, 3H), 2.68 - 2.55 (m, 1H), 2.36 - 2.24 (m, 1H), 2.22 - 2.01 (m, 4H), 1.95 - 1.47 (m, 7H), 1.12 - 0.98 (m, 3H), 0.93 (d, *J* = 6.2 Hz, 3H), 0.74 - 0.53 (m, 2H), 0.50 - 0.33 (m, 2H). | | | | | |
| **174** | 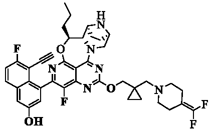 | 729 [M+H] | **175** | 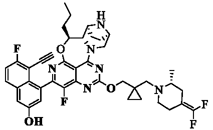 | 743 [M+H] |
| **Example 174:** ¹H NMR (400 MHz, DMSO) δ 10.15 (s, 1H), 7.96 (m, 1H), 7.46 (td, *J* = 9.0, 3.7 Hz, 1H), 7.37 (t, *J =* 2.4 Hz, 1H), 7.11 (dd, *J* = 47.0, 2.5 Hz, 1H), 5.17 (dd, *J* = 12.9, 2.7 Hz, 1H), 4.45 - 4.12 (m, 4H), 4.05 (d, *J* = 9.2 Hz, 1H), 3.60 - 3.51 (m, 1H), 3.47 (t, *J* = 7.0 Hz, 1H), 3.04 (t, *J* = 11.4 Hz, 1H), 2.92 - 2.75 (m, 3H), 2.68 - 2.55 (m, 1H), 2.36 - 2.24 (m, 3H), 2.22 - 2.01 (m, 4H), 1.95 - 1.47 (m, 8H), 0.93 (d, *J* = 6.2 Hz, 3H), 0.74 - 0.53 (m, 2H), 0.50 - 0.33 (m, 2H). | | | | | |

**Example 176 refers to the synthesis of example 1-1, and Example 177 refers to the synthesis of example 1-1 or example 19:**

| **Serial No.** | **Example structure** | **MS m/z (ESI)** | **Serial No.** | **Example structure** | **MS m/z (ESI)** |
|---|---|---|---|---|---|
| **176** | 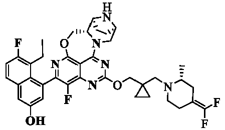 | 705 [M+H] | **177** | 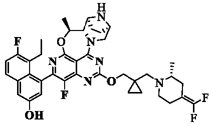 | 719 [M+H] |

**Examples 178 to 179 refer to the synthesis of example 1-1 or example 161:**

| **Serial No.** | **Example structure** | **MS m/z (ESI)** | **Serial No.** | **Example structure** | **MS m/z (ESI)** |
|---|---|---|---|---|---|
| **178** | 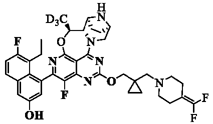 | 708 | **179** | 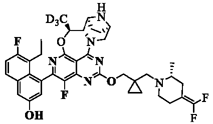 | 722 |
| | | [M+H] | | | [M+H] |
| **Example 178:** ¹H NMR (400 MHz, MeOD) δ 7.65 - 7.60 (m, 1H), 7.34 - 7.17 (m, 2H), 7.14 - 7.01 (m, 1H), 5.22 - 5.02 (m, 1H), 4.68 - 4.54 (m, 3H), 4.54 - 4.35 (m, 2H), 4.26 - 4.11 (m, 1H), 3.92 - 3.72 (m, 2H), 2.86 - 2.64 (m, 4H), 2.62 - 2.36 (m, 2H), 2.30 (s, 3H), 2.24 - 2.13 (m, 1H), 2.08 - 1.75 (m, 5H), 0.89 (t, *J* = 8 Hz, 2H), 0.86 - 0.72 (m, 3H), 0.66 - 0.47 (m, 2H). | | | | | |
| **Example 179:** ¹H NMR (400 MHz, MeOD) δ 7.71 - 7.60 (m, 1H), 7.34 - 7.16 (m, 2H), 7.14 - 7.01 (m, 1H), 5.21 - 5.02 (m, 1H), 4.70 - 4.55 (m, 3H), 4.53 - 4.34 (m, 2H), 4.27 - 4.10 (m, 1H), 3.92 - 3.71 (m, 2H), 2.86 - 2.65 (m, 4H), 2.62 - 2.36 (m, 2H), 2.30 (s, 3H), 2.24 - 2.13 (m, 1H), 2.08 - 1.75 (m,7H), 0.89 (t, *J* = 8 Hz, 2H), 0.86 - 0.71 (m, 3H), 0.66 - 0.48 (m, 2H). | | | | | |

**Examples 180 to 196 refer to the synthesis of example 1-1 or example 124:**

| **Serial No.** | **Example structure** | **MS m/z (ESI)** | **Serial No.** | **Example structure** | **MS m/z (ESI)** |
|---|---|---|---|---|---|
| **180** | 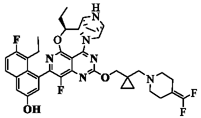 | 719 | **181** | 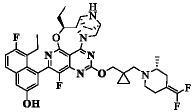 | 733 |
| | | [M+H] | | | [M+H] |
| **182** | 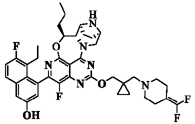 | 733 | **183** | 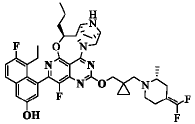 | 747 |
| | | [M+H] | | | [M+H] |
| **184** | 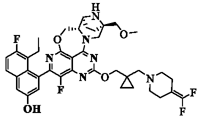 | 735 | **185** | 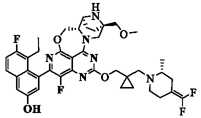 | 749 |
| | | [M+H] | | | [M+H] |
| **186** | 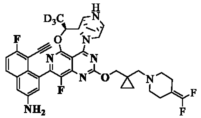 | 703 | **187** | 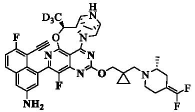 | 717 |
| | | [M+H] | | | [M+H] |
| **188** | 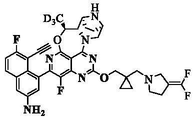 | 689 | **189** | 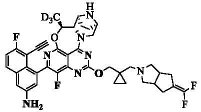 | 729 |
| | | [M+H] | | | [M+H] |
| **190** | 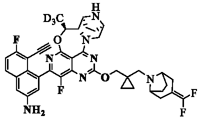 | 729 | **191** | 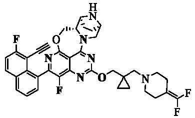 | 671 |
| | | [M+H] | | | [M+H] |
| **192** | 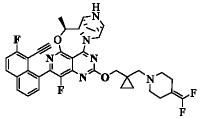 | 685 | **193** | 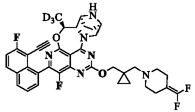 | 688 |
| | | [M+H] | | | [M+H] |
| **194** | 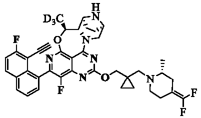 | 702 | **195** | 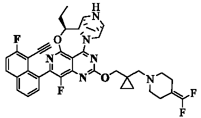 | 699 |
| | | [M+H] | | | [M+H] |
| **196** | 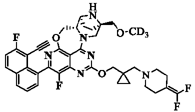 | 718 | | | |
| | | [M+H] | | | |

**Embodiment 197 refers to the synthesis of example 1-1 or the following synthesis method:**

### Example 197

### Step 1: Preparation of tert-butyl (1S,2S,5R)-2-((S)-1-((7-bromo-2,6-dichloro-8-fluoro-4-hydroxyquinazolin-5-yl)oxy)propyl)-3,8-diazabicyclo[3.2.1]octane-8-carboxylate

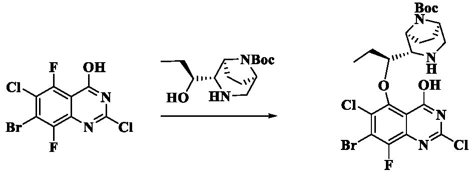

In a round-bottom flask, 7-bromo-2,6-dichloro-5,8-difluoroquinazolin-4-ol (3.2 g, 9.9 mmol) and tert-butyl (1S,2S,5R)-2-((S)-1-hydroxypropyl)-3,8-diazabicyclo[3.2.1]octane-8-carboxylate (2.7 g, 10.7 mmol) were dissolved in tetrahydrofuran (50 mL). The obtained mixture was subjected to nitrogen replacement, then cooled to 0°C, and sodium hydride (356 mg, 14.9 mmol) was slowly added in portions. The reaction liquid was stirred at 25°C for 1 h. Saturated ammonium chloride solution was slowly added to the reaction liquid to quench the reaction, and then the obtained mixture was extracted twice with ethyl acetate. The organic phases were combined, washed with water and saturated sodium chloride aqueous solution, dried over anhydrous sodium sulfate, filtered, concentrated, and purified by silica gel column chromatography to obtain tert-butyl (1S,2S,5R)-2-((S)-1-((7-bromo-2,6-dichloro-8-fluoro-4-hydroxylquinazolin-5-yl)oxy)propyl)-3,8-diazabicyclo[3.2.1]octane-8-carboxylate (3.5 g, yield 61%).

MS m/z (ESI): 579 [M+H].

### Step 2: Preparation of tert-butyl (5S,5as,6S,9R)-2-bromo-3,13-dichloro-5-ethyl-1-fluoro-5a,6,7,8,9,10-hexahydro-5H-6,9-aminoazabicyclo[2',1': 3,4][1,4]oxazapyrido[5,6,7]quinazoline-15-carboxylate

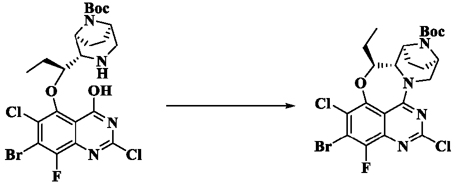

In a round-bottom flask, tert-butyl (1S,2S,5R)-2-((S)-1-((7-bromo-2,6-dichloro-8-fluoro-4-hydroxylquinazolin-5-yl)oxy)propyl)-3,8-diazabicyclo[3.2.1] octane-8-carboxylate (3.5 g, 5.9 mmol) was dissolved in dichloromethane (30 mL). Phosphorus oxychloride (1.1 mL, 11.9 mmol) and N,N-diisopropylethylamine (3.1 mL, 17.8 mmol) were added in portions under ice bath. The reaction liquid was stirred at 25°C for 1 h. After the addition was completed, the reaction liquid was cooled to 0°C, and saturated ammonium chloride solution was added dropwise to quench the reaction, and the obtained mixture was extracted with ethyl acetate, dried over anhydrous sodium sulfate, filtered, concentrated, and purified by silica gel column chromatography to obtain tert-butyl (5S,5as,6S,9R)-2-bromo-3,13-dichloro-5-ethyl-1-fluoro-5a,6,7,8,9,10-hexahydro-5H-6,9-aminoazabicyclo[2',1': 3,4][1,4]oxazapyrido[5,6,7]quinazoline-15-carboxylate (2.7 g, yield 81%).

MS m/z (ESI): 561 [M+H].

### Step 3: Preparation of tert-butyl (5S,5as,6S,9R)-2-bromo-3-chloro-13-((1-((4-(difluoromethylene)piperidin-1-yl)methyl)cyclopropyl)methoxy)-5-ethyl-1-fluoro-5a,6,7,8,9,10-hexahydro-5H-6,9-aminoazapyrido[2',1':3,4][1,4]oxazapyrido [5,6,7]quinazoline-15-carboxylate

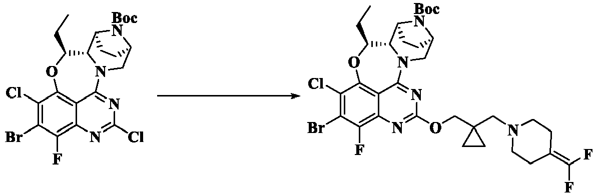

In a round-bottom flask, tert-butyl (5S,5as,6S,9R)-2-bromo-3, 13-dichloro-5-ethyl-1-fluoro-5a,6,7,8,9,10-hexahydro-5H-6,9-aminoazabicyclo[2',1': 3,4][1,4]oxazabicyclopyrido[5,6,7]quinazoline-15-carboxylate (2.7 g, 4.8 mmol) and (1-((4-(difluoromethylene)piperidin-1-yl)methyl)cyclopropyl)methanol (1.3 g, 5.7 mmol) were dissolved in tetrahydrofuran (25 mL). The obtained mixture was subjected to nitrogen replacement, then cooled to 0°C, and sodium hydride (230 mg, 9.6 mmol) was slowly added in portions. The reaction liquid was stirred at 25°C for 1 h. Saturated ammonium chloride solution was slowly added to the reaction liquid to quench the reaction, and then the obtained mixture was extracted twice with ethyl acetate. The organic phases were combined, washed with water and saturated sodium chloride aqueous solution, dried over anhydrous sodium sulfate, filtered, concentrated, and purified by silica gel column chromatography to obtain tert-butyl (5S,5as,6S,9R)-2-bromo-3-chloro-13-((1-((4-(difluoromethylene)piperidin-1-yl)methyl)cyclopropyl)methoxy)-5-ethyl-1-fluoro-5a,6,7,8,9,10-hexahydro-5H-6,9-aminoazapyrido[2',1':3,4][1,4]oxazapyrido[5,6,7]quinazoline-15-carboxylate (3.1 g, yield 86%).

MS m/z (ESI): 741[M+H].

### Step 4: Preparation of tert-butyl (5S,5as,6S,9R)-3-chloro-13-((1-((4-(difluoromethylene)piperidin-1-yl)methyl)cyclopropyl)methoxy)-5-ethyl-1-fluoro-2- (4,4,5,5-tetramethyl-1,3,2-dioxaboran-2-yl)-5a,6,7,8,9,10-hexahydro-5H-6,9-oxazabicyclopyrido[2',1': 3,4][1,4]oxazapyrido[5,6,7]quinazoline-15-carboxylate

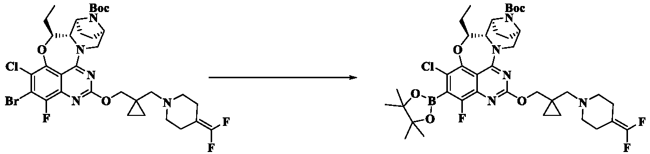

In a round-bottom flask, tert-butyl (5S,5as,6S,9R)-2-bromo-3-chloro-13-((1-((4-(difluoromethylene)piperidin-1-yl)methyl)cyclopropyl)methoxy)-5-ethyl-1-fluoro-5a,6,7,8,9,10-hexahydro-5H-6,9-aminoazapyrido[2',1':3,4][1,4] oxazabicyclopyrido[5,6,7]quinazoline-15-carboxylate (3.1 g, 4.1 mmol) was dissolved in dioxane (30 mL), and bis(pinacolato)diboron (1.5 g, 6.2 mmol), potassium acetate (1.2 g, 12.3 mmol) and 1,1-bis(diphenylphosphine)ferrocene palladium dichloride (0.3 g, 0.4 mmol) were added. The reaction liquid was stirred at 105°C for 16 h. After the addition was completed, the reaction liquid was cooled to room temperature, filtered through diatomaceous earth, concentrated, and purified by silica gel column chromatography to obtain tert-butyl (5S,5as,6S,9R)-3-chloro-13-((1-((4-(difluoromethylene)piperidin-1-yl)methyl)cyclopropyl)methoxy)-5-ethyl-1-fluoro-2-(4,4,5,5-tetramethyl-1,3,2-dioxaboran-2-yl)-5a,6,7,8,9,10-hexahydro-5H-6,9-oxazabicyclopyrido[2',1': 3,4][1,4]oxapyrido[5,6,7]quinazoline-15-carboxylate (2.9 g, yield 92%).

MS m/z (ESI): 790 [M+H].

### Step 5: Preparation of tert-butyl (5S,5as,6S,9R)-2-(6-amino-4-chloro-3-(trifluoromethyl)pyridin-2-yl)-3-chloro-13-(1-((4-(difluoromethylene)piperidin-1-yl)methyl)cyclopropyl)methoxy)-5-ethyl-1-fluoro-5a,6,7,8,9,10-hexahydro-5H-6,9-oxazabicyclicpyrido[2',1':3,4][1,4]oxapyrido[5,6,7]quinazoline-15-carboxylate

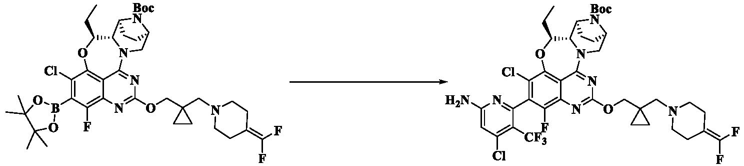

In a round-bottom flask, tert-butyl (5S,5as,6S,9R)-3-chloro-13-((1-((4-(difluoromethylene)piperidin-1-yl)methyl)cyclopropyl)methoxy)-5-ethyl-1-fluoro-2-(4,4,5,5-tetramethyl-1,3,2-dioxaboran-2-yl)-5a,6,7,8,9,10-hexahydro-5H-6,9-oxazabicyclopyrido[2',1':3,4][1,4]oxazapyrido[5,6,7]quinazoline-15-carboxylate (2.9 g, 3.7 mmol) and 6-bromo-4-chloro-5-(trifluoromethyl)pyridin-2-amine (1.2 g, 4.5 mmol) were dissolved in dioxane (30 mL) and water (6 mL), and cesium carbonate (4.2 g, 11.1 mmol) and methanesulfonic acid (dimethyl-n-butylphosphino)-2'-amino-1,1'-biphenyl-2-yl)palladium(II) dichloromethane adduct (0.3 g, 0.4 mmol) were added. The reaction liquid was stirred at 100°C for 2 h. After the reaction was completed, the reaction liquid was cooled to room temperature, saturated ammonium chloride solution was added dropwise, and the obtained mixture was extracted twice with ethyl acetate. The combined organic phases were washed with water and saturated sodium chloride aqueous solution, dried over anhydrous sodium sulfate, filtered, concentrated, and purified by silica gel column chromatography to obtain tert-butyl (5S,5as,6S,9R)-2-(6-amino-4-chloro-3-(trifluoromethyl)pyridin-2-yl)-3-chloro-13-(1-((4-(difluoromethylene)piperidin-1-yl)methyl)cyclopropyl)methoxy)-5-ethyl-1-fluoro-5a,6,7,8,9,10-hexahydro-5H-6,9-oxazabicyclopyrido[2',1':3,4][1,4]oxazapyrido[5,6,7]quinazoline-15-carboxylate (1.4 g, yield 45%).

MS m/z (ESI): 858 [M+H].

### Step 6: Preparation of 4-chloro-6-((5S,5as,6S,9R)-3-chloro-13-((1-((4-(difluoromethylene)piperidin-1-yl)methyl)cyclopropyl)methoxy)-5-ethyl-1-fluoro-5a,6,7,8,9,10-hexahydro-5H-6,9-oxazabicyclopyrido[2',1':3,4][1,4]oxazapyrido [5,6,7]quinazolin-2-yl)-5-(trifluoromethyl)pyridin-2-amine

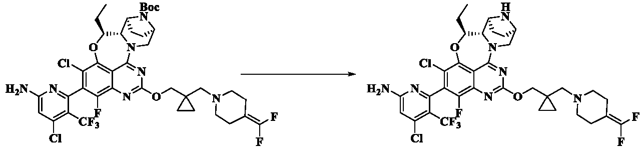

In a round-bottom flask, tert-butyl (5S,5as,6S,9R)-2-(6-amino-4-chloro-3-(trifluoromethyl)pyridin-2-yl)-3-chloro-13-(1-((4-(difluoromethylene)piperidin-1-yl)methyl)cyclopropyl)methoxy)-5-ethyl-1-fluoro-5a,6,7,8,9,10-hexahydro-5H-6,9-oxazabicyclopyrido[2',1':3,4][1,4]oxazapyrido[5,6,7]quinazoline-15-carboxylate (1.4 g, 1.6 mmol) was dissolved in dichloromethane (15 mL), and trifluoroacetic acid (5 mL) was added under ice bath. The reaction liquid was stirred at 25°C for 1 h. After the addition was completed, the reaction liquid was concentrated to dryness, purified by high performance liquid column chromatography, and lyophilized to obtain 4-chloro-6-((5S,5as,6S,9R)-3-chloro-13-((1-((4-(difluoromethylene)piperidin-1-yl)methyl)cyclopropyl)methoxy)-5-ethyl-1-fluoro-5a,6,7,8,9,10-hexahydro-5H-6,9-oxazabicyclopyrido[2',1':3,4][1,4] oxazapyrido[5,6,7]quinazoline-2-yl)-5-(trifluoromethyl)pyridin-2-amine (0.6 g, yield 48%).

MS m/z (ESI): 758 [M+H].

¹H NMR (400 MHz, DMSO) δ 7.11 (dd, *J* = 47.0, 2.5 Hz, 1H), 6.96 - 6.91 (m, 2H), 5.17 (dd, *J =* 12.9, 2.7 Hz, 1H), 4.45 - 4.12 (m, 4H), 4.05 (d, *J* = 9.2 Hz, 1H), 3.60 - 3.51 (m, 1H), 3.47 (t, *J* = 7.0 Hz, 1H), 3.04 (t, *J* = 11.4 Hz, 1H), 2.92 - 2.75 (m, 3H), 2.68 - 2.55 (m, 1H), 2.36 - 2.24 (m, 1H), 2.22 - 2.01 (m, 4H), 1.95 - 1.47 (m, 7H), 0.93 (d, *J =* 6.2 Hz, 3H), 0.74 - 0.53 (m, 2H), 0.50 - 0.33 (m, 2H).

**Examples 198 to 204 refer to the synthesis of example 1-1 or example 197:**

| **Serial No.** | **Example structure** | **MS m/z (ESI)** | **Serial No.** | **Example structure** | **MS m/z (ESI)** |
|---|---|---|---|---|---|
| **198** | 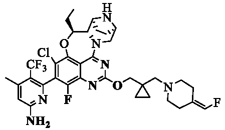 | 720 | **199** | 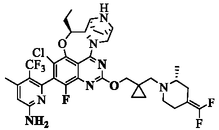 | 752 |
| | | [M+H] | | | [M+H] |
| **200** | 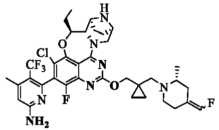 | 734 | **201** | 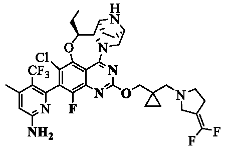 | 724 |
| | | [M+H] | | | [M+H] |
| **202** | 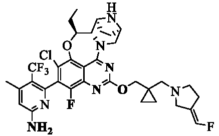 | 706 | **203** | 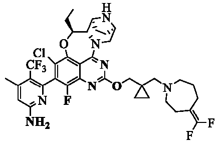 | 752 |
| | | [M+H] | | | [M+H] |
| **204** | 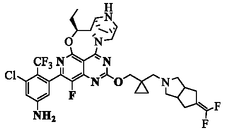 | 750 | | | |
| | | [M+H] | | | |

**Embodiment 205 refers to the synthesis of example 1-1 or the following synthesis method:**

### Example 205

### Step 1: Synthesis of 4-((SS,SaS,6S,9R)-12-((1-((4-(difluoromethylene) piperidin-1-yl)methyl)cyclopropyl)methoxy)-2-(8-ethynyl-7-fluoro-3-hydroxylnaphthalen-1-yl)-1-fluoro-5-methyl-5a,6,7,8,9,10-hexahydro-5H-4-oxa-3,10a,11,13,14-pentaaza-6,9-methylnaphtho[1,8-ab]heptene-4-carbonyl)oxetan-2-one

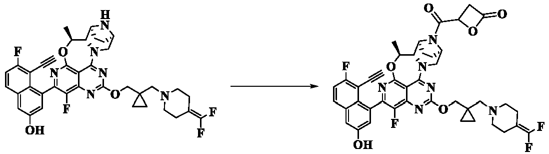

(Ethyl 2-oximino-cyanoacetate)-N,N-dimethyl-morpholinourea hexafluorophosphate (42.4 mg, 0.1 mmol), 0.5 mL of anhydrous DMF and 2 drops of lutidine were added to a microwave tube, and the obtained mixture was reacted at room temperature for 15 min. 4-((SS,SaS,6S,9R)-12-((1-((4-(difluoromethylene)piperidin-1-yl)methyl)cyclopropyl)methoxy)-1-fluoro-5-methyl-5a,6,7,8,9,10-hexahydro-5H-4-oxa-3,10a,11,13,14-pentyl-6,9-methylnaphtho[1,8-ab]hept-2-yl)-5-ethynyl-6-fluoronaphthalen-2-ol (46.7 mg, 0.07 mmol) was continuously added to the reaction liquid, and the reaction was continued at room temperature for 15 min. The obtained mixture was washed by adding saturated sodium bicarbonate solution, extracted with ethyl acetate. The organic phase was dried over anhydrous sodium sulfate, filtered, concentrated, and purified by preparative column chromatography to obtain 4-((SS,SaS,6S,9R)-12-((1-((4-(difluoromethylene)piperidin-1-yl)methyl)cyclopropyl)methoxy)-2-(8-ethynyl-7-fluoro-3-hydroxylnaphthalen-1-yl)-1-fluoro-5-methyl-5a,6,7,8,9,10-hexahydro-5H-4-oxa-3,10a,11,13,14-pentaaza-6,9-methylnaphtho[1,8-ab]heptene-4-carbonyl)oxetan-2-one (32.4 mg, yield 61%).

MS m/z(ESI): 799 [M+H].

¹H NMR (400 MHz, MeOD) δ 7.83 (dt, *J =* 9.2, 5.9 Hz, 1H), 7.36 - 7.27 (m, 2H), 7.27 - 7.14 (m, 1H), 5.45 - 5.23 (m, 2H), 4.65 - 4.18 (m, 4H), 4.02 - 3.65 (m, 6H), 2.99 - 2.82 (m, 5H), 2.37 (q, *J* = 6.4 Hz, 4H), 2.25 - 2.13 (m, 1H), 2.04 - 1.86 (m, 3H), 1.12 (d, *J* = 6.4 Hz, 3H), 0.87 - 0.63 (m, 4H).

**Examples 206 to 268 refer to the synthesis of example 1-1 or example 205:**

| **Serial No.** | **Example structure** | **MS m/z (ESI)** | **Serial No.** | **Example structure** | **MS m/z (ESI)** |
|---|---|---|---|---|---|
| **206** | 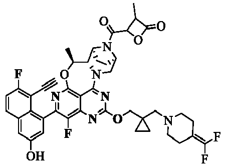 | 813 | **207** | 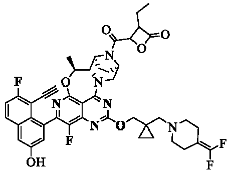 | 827 |
| | | [M+H] | | | [M+H] |
| **208** | 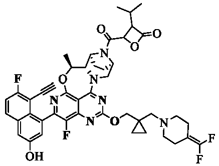 | 841 | **209** | 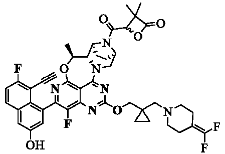 | 827 |
| | | [M+H] | | | [M+H] |
| **210** | 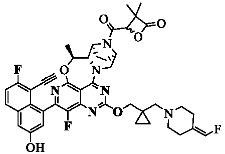 | 809 | **211** | 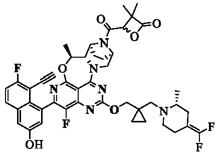 | 841 |
| | | [M+H] | | | [M+H] |
| **212** | 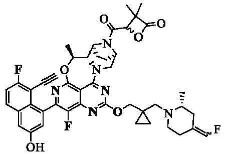 | 823 | **213** | 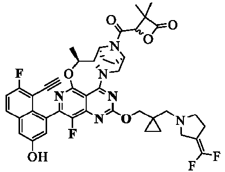 | 813 |
| | | [M+H] | | | [M+H] |
| **214** | 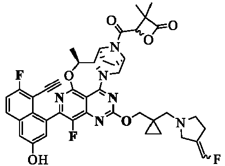 | 795 | **215** | 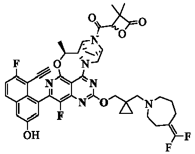 | 841 |
| | | [M+H] | | | [M+H] |
| **216** | 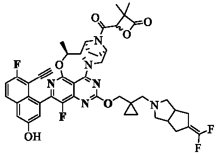 | 853 | **217** | 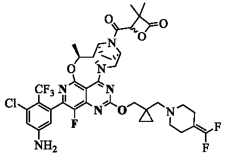 | 836 |
| | | [M+H] | | | [M+H] |
| **218** | 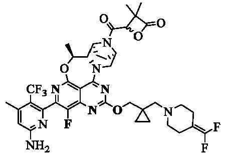 | 817 | **219** | 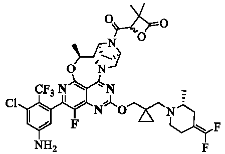 | 850 |
| | | [M+H] | | | [M+H] |
| **220** | 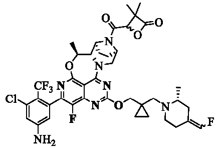 | 832 | **221** | 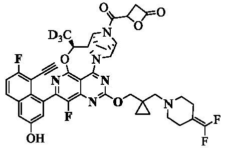 | 802 |
| | | [M+H] | | | [M+H] |
| **222** | 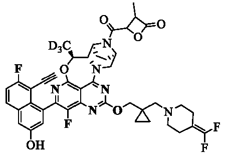 | 816 | **223** | 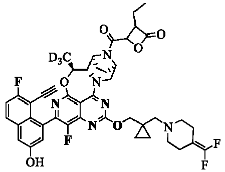 | 830 |
| | | [M+H] | | | [M+H] |
| **224** | 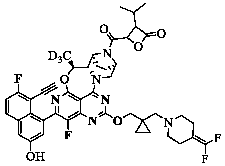 | 844 | **225** | 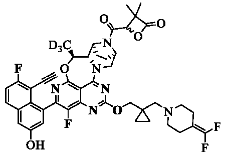 | 830 |
| | | [M+H] | | | [M+H] |
| **226** | 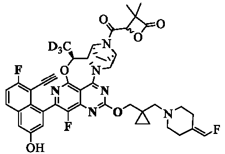 | 812 | **227** | 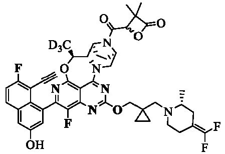 | 844 |
| | | [M+H] | | | [M+H] |
| **228** | 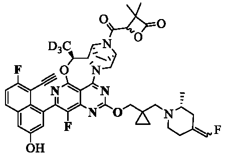 | 826 | **229** | 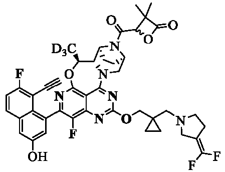 | 816 |
| | | [M+H] | | | [M+H] |
| **230** | 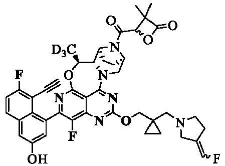 | 798 | **231** | 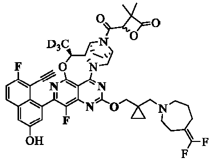 | 844 |
| | | [M+H] | | | [M+H] |
| **232** | 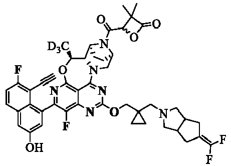 | 856 | **233** | 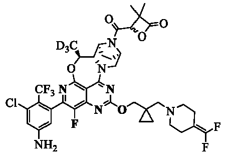 | 839 |
| | | [M+H] | | | [M+H] |
| **234** | 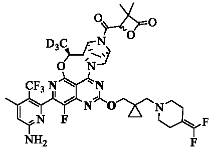 | 820 | **235** | 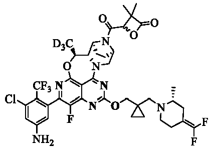 | 853 |
| | | [M+H] | | | [M+H] |
| **236** | 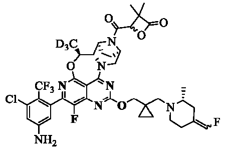 | 835 | **237** | 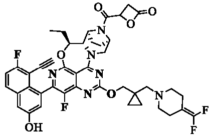 | 813 |
| | | [M+H] | | | [M+H] |
| **238** | 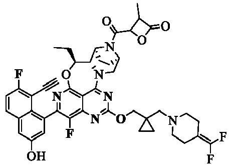 | 827 | **239** | 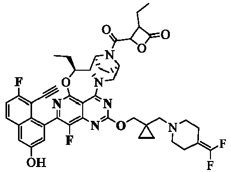 | 841 |
| | | [M+H] | | | [M+H] |
| **240** | 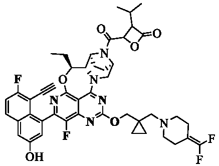 | 855 | **241** | 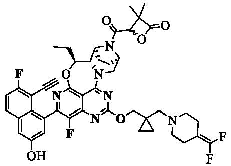 | 841 |
| | | [M+H] | | | [M+H] |
| **242** | 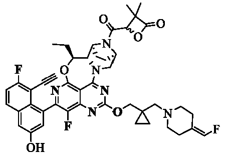 | 823 | **243** | 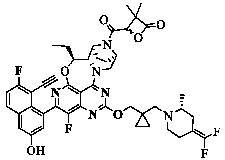 | 855 |
| | | [M+H] | | | [M+H] |
| **244** | 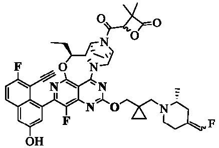 | 837 | **245** | 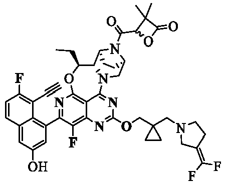 | 827 |
| | | [M+H] | | | [M+H] |
| **246** | 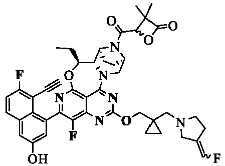 | 809 | **247** | 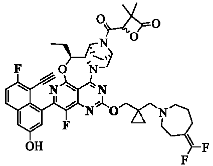 | 855 |
| | | [M+H] | | | [M+H] |
| **248** | 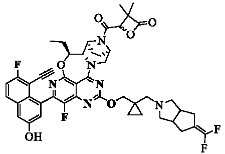 | 867 | **249** | 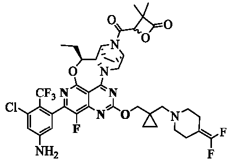 | 850 |
| | | [M+H] | | | [M+H] |
| **250** | 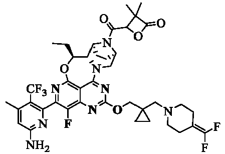 | 831 | **251** | 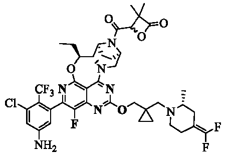 | 864 |
| | | [M+H] | | | [M+H] |
| **252** | 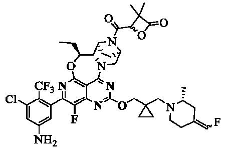 | 846 | **253** | 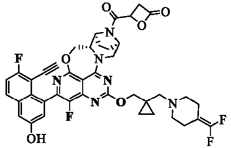 | 785 |
| | | [M+H] | | | [M+H] |
| **254** | 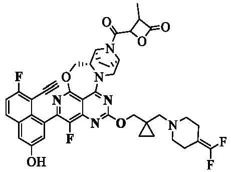 | 799 | **255** | 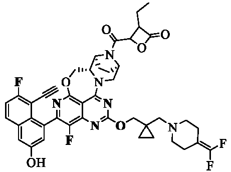 | 813 |
| | | [M+H] | | | [M+H] |
| **256** | 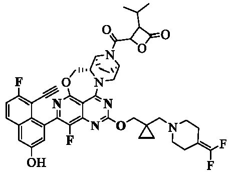 | 827 | **257** | 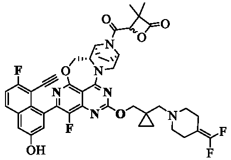 | 813 |
| | | [M+H] | | | [M+H] |
| **258** | 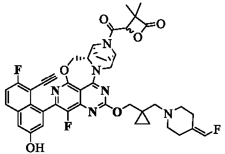 | 795 | **259** | 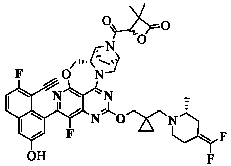 | 827 |
| | | [M+H] | | | [M+H] |
| **260** | 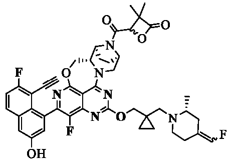 | 809 | **261** | 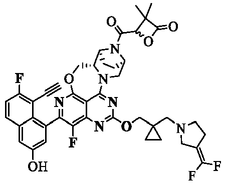 | 799 |
| | | [M+H] | | | [M+H] |
| **262** | 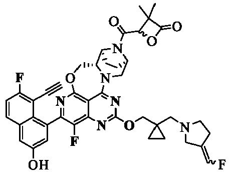 | 781 | **263** | 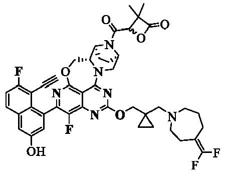 | 827 |
| | | [M+H] | | | [M+H] |
| **264** | 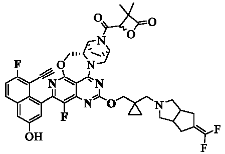 | 839 | **265** | 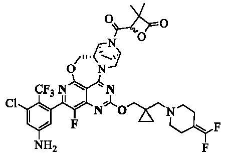 | 822 |
| | | [M+H] | | | [M+H] |
| **266** | 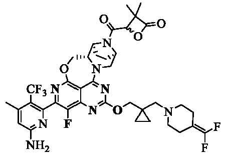 | 803 | **267** | 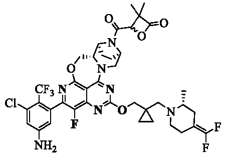 | 836 |
| | | [M+H] | | | [M+H] |
| **268** | 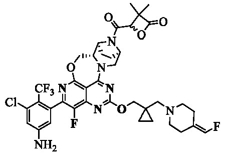 | 804 | | | |
| | | [M+H] | | | |

**Examples 269 to 304 refer to the synthesis of example 19:**

| **Serial No.** | **Example structure** | **MS m/z (ESI)** | **Serial No.** | **Example structure** | **MS m/z (ESI)** |
|---|---|---|---|---|---|
| **269** | 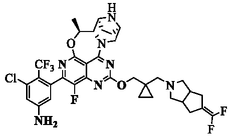 | 736 [M+H] | **270** | 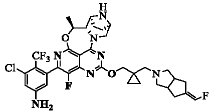 | 718 [M+H] |
| **Example 270:** ¹H NMR (400 MHz, MeOD) δ 7.07 (s, 1H), 6.95 (s, 1H), 6.08 - 5.86 (m, 1H), 4.45 - 4.37 (m, 2H), 4.22 - 4.09 (m, 4H), 4.02 - 3.93 (m, 2H), 3.76 - 3.53 (m, 4H), 3.45 - 3.35 (m, 2H), 2.82- 2.53 (m, 5H), 2.46 - 2.35 (m, 4H), 2.14 - 1.79 (m, 4H), 0.84 - 0.76 (m, 2H), 0.66 - 0.58 (m, 2H). | | | | | |
| **271** | 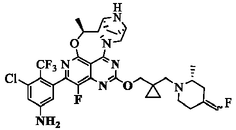 | 706 [M+H] | **272** | 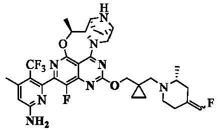 | 687 [M+H] |
| **Example 271:** ¹H NMR (400 MHz, MeOD) δ 7.07 (s, 1H), 6.96 (s, 1H), 6.10 - 5.84 (m, 1H), 4.47 - 4.37 (m, 2H), 3.75 - 3.66 (m, 2H), 3.32 - 3.06 (m, 4H), 2.86 - 2.72 (m, 2H), 2.56 - 2.39 (m, 2H), 2.23 - 2.12 (m, 2H), 2.03 - 1.89 (m, 4H), 1.76 - 1.61 (m, 4H), 1.43 - 1.26 (m, 5H), 0.83 - 0.72 (m, 2H), 0.66 - 0.53 (m, 2H). | | | | | |
| **Example 272:** ¹H NMR (400 MHz, MeOD) δ 7.07 (s, 1H), 6.12 - 5.88 (m, 1H), 4.42 - 4.36 (m, 2H), 3.77 - 3.65 (m, 2H), 3.27 - 2.93 (m, 4H), 2.79 - 2.63 (m, 2H), 2.46 - 2.35 (m, 5H), 2.16 - 2.09 (m, 2H), 2.07 - 1.93 (m, 4H), 1.86 - 1.63 (m, 4H), 1.37 - 1.13 (m, 5H), 0.86 - 0.69 (m, 2H), 0.63 - 0.49 (m, 2H). | | | | | |
| **273** | 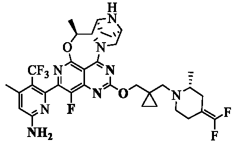 | 705 [M+H] | **274** | 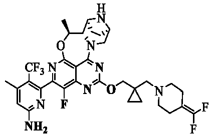 | 691 [M+H] |
| **Example** 273: ¹H NMR (400 MHz, MeOD) δ 7.01 (s, 1H), 4.36 - 4.31 (m, 2H), 3.79 - 3.69 (m, 2H), 3.25 - 2.91 (m, 4H), 2.81 - 2.63 (m, 2H), 2.54 - 2.33 (m, 5H), 2.21 - 2.13 (m, 2H), 2.10 - 1.97 (m, 4H), 1.89 - 1.59 (m, 4H), 1.33 - 1.13 (m, 5H), 0.79 - 0.63 (m, 2H), 0.58 - 0.44 (m, 2H). | | | | | |
| **Example 274:** NMR (400 MHz, MeOD) δ 7.04 (s, 1H), 4.36 - 4.31 (m, 2H), 4.21 - 4.14 (m, 4H), 4.05 - 3.96 (m, 2H), 3.79 - 3.54 (m, 4H), 3.41 - 3.22 (m, 2H), 3.08 (s, 3H), 2.81 - 2.54 (m, 5H), 2.49 - 2.41 (m, 2H), 2.10 - 1.76 (m, 4H), 0.87 - 0.77 (m, 2H), 0.67 - 0.55 (m, 2H). | | | | | |
| **275** | 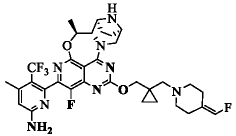 | 673 [M+H] | **276** | 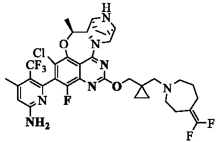 | 738 [M+H] |
| **Example 275:** ¹H NMR (400 MHz, MeOD) δ 7.08 (s, 1H), 6.06 - 5.84 (m, 1H), 4.36 - 4.34 (m, 2H), 4.25 - 4.16 (m, 4H), 4.06 - 3.93 (m, 2H), 3.75 - 3.57 (m, 4H), 3.46 - 3.26 (m, 2H), 3.10 (s, 3H), 2.79 - 2.51 (m, 5H), 2.43 - 2.36 (m, 2H), 2.12 - 1.69 (m, 4H), 0.79 - 0.69 (m, 2H), 0.59 - 0.48 (m, 2H). | | | | | |
| **Example 276:** ¹H NMR (400 MHz, MeOD) δ 7.08 (s, 1H), 4.45 - 4.35 (m, 2H), 4.30 - 4.21 (m, 4H), 4.09 - 3.87 (m, 2H), 3.84 - 3.56 (m, 4H), 3.53 - 3.29 (m, 4H), 3.16 (s, 3H), 3.05 - 2.89 (m, 2H), 2.81 - 2.54 (m, 5H), 2.03 - 1.77 (m, 2H), 1.51 - 1.20 (m, 2H), 0.84 - 0.72 (m, 2H), 0.69 - 0.58 (m, 2H). | | | | | |
| **277** | 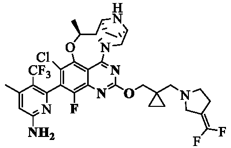 | 710 [M+H] | **278** | 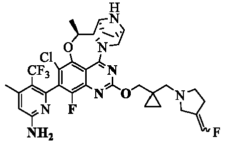 | 692 [M+H] |
| **Example 277:** ¹H NMR (400 MHz, MeOD) δ 7.03 (s, 1H), 4.36 - 4.28 (m, 2H), 4.26 - 4.10 (m, 4H), 4.09 - 3.93 (m, 2H), 3.87 - 3.66 (m, 4H), 3.53 - 3.29 (m, 2H), 3.14 (s, 3H), 2.81 - 2.54 (m, 5H), 2.07 - 1.65 (m, 4H), 0.84 - 0.75 (m, 2H), 0.69 - 0.54 (m, 2H). | | | | | |
| **Example 278:** ¹H NMR (400 MHz, MeOD) δ 7.07 (s, 1H), 6.03 - 5.84 (m, 1H), 4.39 - 4.28 (m, 2H), 4.21 - 4.12 (m, 4H), 4.06 - 3.91 (m, 2H), 3.85 - 3.68 (m, 4H), 3.56 - 3.25 (m, 2H), 3.18 (s, 3H), 2.81 - 2.58 (m, 5H), 2.17 - 1.68 (m, 4H), 0.81 - 0.70 (m, 2H), 0.58 - 0.46 (m, 2H). | | | | | |
| **279** | 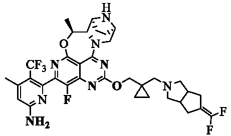 | 717 [M+H] | **280** | 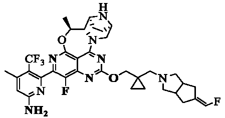 | 699 [M+H] |
| **Example 279:** ¹H NMR (400 MHz, MeOD) δ 7.05 (s, 1H), 6.08 - 5.86 (m, 1H), 4.39 - 4.30 (m, 2H), 4.24 - 4.12(m, 4H), 4.06 - 3.93 (m, 2H), 3.82 - 3.56 (m, 4H), 3.41 - 3.26 (m, 2H), 3.06 (s, 3H), 2.83- 2.56 (m, 5H), 2.51 - 2.39 (m, 4H), 2.14 - 1.75 (m, 4H), 0.87 - 0.72 (m, 2H), 0.63 - 0.59 (m, 2H). | | | | | |
| **Example 280:** ¹H NMR (400 MHz, MeOD) δ 7.09 (s, 1H), 6.08 - 5.86 (m, 1H), 4.42 - 4.33 (m, 2H), 4.22 - 4.09 (m, 4H), 4.02 - 3.93 (m, 2H), 3.78 - 3.54 (m, 4H), 3.41 - 3.24 (m, 2H), 3.08 (s, 3H), 2.82- 2.50 (m, 5H), 2.46 - 2.31 (m, 4H), 2.14 - 1.79 (m, 4H), 0.84 - 0.73 (m, 2H), 0.66 - 0.55 (m, 2H). | | | | | |
| **281** | 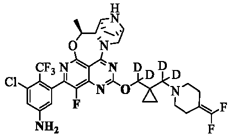 | 714 [M+H] | **282** | 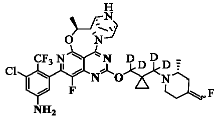 | 710 [M+H] |
| **Example 281:** ¹H NMR (400 MHz, MeOD) δ 7.04 (s, 1H), 6.93 (s, 1H), 4.35 - 3.95 (m, 2H), 3.79 - 3.67 (m, 2H), 3.62 - 3.51 (m, 2H), 3.34 - 3.08 (m, 3H), 2.48 - 2.36 (m, 2H), 2.15 - 2.03 (m, 4H), 1.95 - 1.57 (m, 4H), 1.46 - 1.41 (m, 2H), 0.72 - 0.59 (m, 2H), 0.50 - 0.36 (m, 2H). | | | | | |
| **Example 282:** ¹H NMR (400 MHz, MeOD) δ 7.05 (s, 1H), 6.94 (s, 1H), 6.10 - 5.88 (m, 1H), 4.36 - 4.31 (m, 2H), 3.79 - 3.69 (m, 2H), 3.25 - 2.91 (m, 3H), 2.81 - 2.63 (m, 4H), 2.54 - 2.33 (m, 3H), 2.21 - 2.13 (m, 2H), 2.10 - 1.97 (m, 2H), 1.89 - 1.59 (m, 2H), 1.33 - 1.13 (m, 3H), 0.79 - 0.63 (m, 2H), 0.58 - 0.44 (m, 2H). | | | | | |
| **283** | 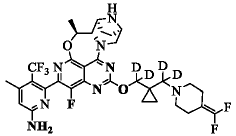 | 695 [M+H] | **284** | 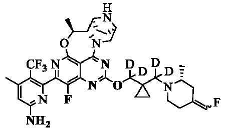 | 691 [M+H] |
| **Example 283:** ¹H NMR (400 MHz, MeOD) δ 7.14 (s, 1H), 4.46 - 4.41 (m, 1H), 4.11 - 4.04 (m, 3H), 3.74 - 3.50 (m, 1H), 3.51 - 3.32 (m, 1H), 3.18 (s, 3H),2.53 (q, *J* = 5.0 Hz, 4H), 2.02 (t, *J* = 5.4 Hz, 4H), 1.92 (d, *J* = 8.6 Hz, 1H), 1.79 - 1.55 (m, 3H), 1.63 - 1.48 (m, 3H), 0.68 - 0.5 (m, 2H), 0.45 (d, *J* = 1.8 Hz, 2H). | | | | | |
| **Example 284:** ¹H NMR (400 MHz, MeOD) δ 7.24 (s, 1H), 6.92 (s, 0.5H), 6.61 (s, 0.5H), 4.56 - 4.51 (m, 1H), 4.21 - 4.14 (m, 3H), 3.89 - 3.64 (m, 1H), 3.41 - 3.22 (m, 1H), 3.18 (s, 3H),2.53 (q, *J* = 5.0 Hz, 3H), 2.01 (t, *J* = 5.4 Hz, 4H), 1.87 (d, *J* = 8.6 Hz, 1H), 1.75 - 1.45 (m, 3H), 1.43 - 1.38 (m, 3H), 1.14 (d, *J* = 4 Hz, 3H), 0.71 - 0.59 (m, 2H), 0.39 (d, *J* = 1.8 Hz, 2H). | | | | | |
| **285** | 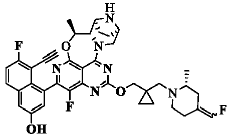 | 697 [M+H] | **286** | 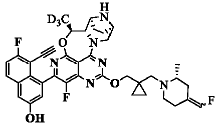 | 700 [M+H] |
| **Example 285:** ¹H NMR (400 MHz, MeOD) δ 7.79 - 7.75 (m, 1H), 7.30 - 7.20 (m, 2H), 7.15 - 7.10 (m, 0.5H), 7.01 (m, 0.5H), 6.62 (s, 0.5H), 6.41 (s, 0.5H), 5.55 - 5.39 (m, 1H), 4.47 - 4.29 (m, 3H), 4.27 - 4.10 (m, 1H), 4.09 (d, *J* = 8 Hz, 1H), 3.77 - 3.58 (m, 2H), 3.25 - 2.82 (m, 4H), 2.70 - 2.52 (m, 1H), 2.40 - 2.31 (m, 1H), 2.24 - 2.03 (m, 3H), 2.00 - 1.89 (m, 2H), 1.78 - 1.60 (m, 3H), 1.49 (t, *J* = 8 Hz, 3H), 1.14 (d, J = 4 Hz, 3H), 0.89 - 0.73 (m, 2H), 0.63 - 0.47 (m, 2H). | | | | | |
| **Example 286:** ¹H NMR (400 MHz, DMSO) δ 10.53 (s, 1H), 8.42 - 7.89 (m, 1H), 7.86 (q, *J* = 8 Hz, 1H), 7.57 (s, 1H), 7.39 (d, *J* = 4 Hz, 0.5H), 7.15 (d, *J* = 4 Hz, 0.5H), 6.96 (s, 0.5H), 6.95 (s, 0.5H), 5.37 - 5.14 (m, 1H), 4.58 - 4.38 (m, 2H), 4.17 - 4.06 (m, 2H), 4.01 - 3.81 (m, 1H), 3.61 - 3.46 (m, 1H), 3.41 (t, *J* = 4 Hz, 1H), 3.19 - 2.98 (m, 1H), 2.94 - 2.72 (m, 3H), 2.71 - 2.64 (m, 1H), 2.33 - 2.23 (m, 1H), 2.22 - 1.98 (m, 4H), 1.97 - 1.42 (m, 5H), 0.99 (d, *J* = 4 Hz, 3H), 0.84 - 0.62 (m, 2H), 0.53 - 0.31 (m, 2H). | | | | | |
| **287** | 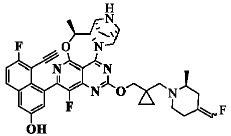 | 697 [M+H] | **288** | 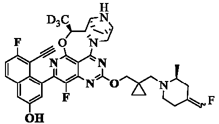 | 700 [M+H] |
| **Example 287:** ¹H NMR (400 MHz, MeOD) δ 7.81 - 7.72 (m, 1H), 7.31 - 7.20 (m, 2H), 7.21 - 7.11 (m, 0.5H), 7.12 (m, 0.5H), 6.73 (s, 0.5H), 6.52 (s, 0.5H), 5.45 - 5.26 (m, 1H), 4.55 - 4.36 (m, 3H), 4.32 - 4.21 (m, 1H), 4.09 (d, *J* = 8 Hz, 1H), 3.72 - 3.52 (m, 2H), 3.23 - 2.92 (m, 4H), 2.81 - 2.62 (m, 1H), 2.54 - 2.32 (m, 1H), 2.31 - 2.13 (m, 3H), 2.11 - 1.90 (m, 2H), 1.90 - 1.70 (m, 3H), 1.69 (t, *J* = 8 Hz, 3H), 1.14 (d, J = 4 Hz, 3H), 0.89 - 0.63 (m, 2H), 0.71 - 0.52 (m, 2H). | | | | | |
| **Example 288:** ¹H NMR (400 MHz, DMSO) δ 10.12 (s, 1H), 8.02 - 7.81 (m, 1H), 7.41 (q, *J* = 8 Hz, 1H), 7.37 (s, 1H), 7.10 (d, *J* = 4 Hz, 0.5H), 7.05 (d, *J* = 4 Hz, 0.5H), 6.76 (s, 0.5H), 6.65 (s, 0.5H), 5.37 - 5.14 (m, 1H), 4.45 - 4.30 (m, 2H), 4.17 - 4.05 (m, 2H), 4.01 - 3.80 (m, 1H), 3.61 - 3.41 (m, 1H), 3.42 (t, *J* = 4 Hz, 1H), 3.19 - 2.94 (m, 1H), 2.92 - 2.61 (m, 3H), 2.70 - 2.64 (m, 1H), 2.38 - 2.22 (m, 1H), 2.22 - 1.98 (m, 4H), 1.85 - 1.42 (m, 5H), 0.91 (d, *J* = 4 Hz, 3H), 0.66 - 0.52 (m, 2H), 0.52 - 0.39 (m, 2H). | | | | | |
| **289** | 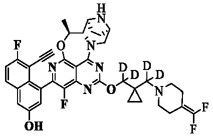 | 705 [M+H] | **290** | 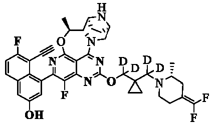 | 719 [M+H] |
| **Example 289:** ¹H NMR (400 MHz, DMSO) δ 8.18 (s, 1H), 7.96 (dt, *J* = 9.2, 5.8 Hz, 1H), 7.46 (td, *J* = 9.0, 7.8 Hz, 1H), 7.37 (d, *J* = 2.5 Hz, 1H), 7.19 (d, *J =* 2.5 Hz, 0.5H), 7.06 (d, *J* = 2.5 Hz, 0.5H), 5.25 - 5.11 (m, 1H), 4.51 (ddd, *J* = 24.2, 8.7, 6.4 Hz, 1H), 4.22 (m, 0.5H), 4.03 (d, *J* = 8.8 Hz, 1H), 3.96 (m, 0.5H), 3.73 - 3.64 (m, 1H), 3.56 (h, *J* = 6.1 Hz, 1H), 3.26 (dd, *J* = 10.6, 5.9 Hz, 1H), 3.20 - 3.03 (m, 1H), 2.43 (q, *J* = 5.0 Hz, 4H), 2.12 (t, *J* = 5.4 Hz, 4H), 1.89 (d, *J* = 8.6 Hz, 1H), 1.85 - 1.55 (m, 3H), 1.53 - 1.38 (m, 3H), 0.71 - 0.59 (m, 2H), 0.42 (d, J = 1.8 Hz, 2H). | | | | | |
| **Example 290:** ¹H NMR (400 MHz, DMSO) δ 8.08 (s, 1H), 7.76 (dt, *J* = 9.2, 5.8 Hz, 1H), 7.56 (td, *J* = 9.0, 7.8 Hz, 1H), 7.31 (d, *J* = 2.5 Hz, 1H), 7.10 (d, *J* = 2.5 Hz, 0.5H), 7.00 (d, *J* = 2.5 Hz, 0.5H), 5.35 - 5.21 (m, 1H), 4.41 (ddd, *J* = 24.2, 8.7, 6.4 Hz, 1H), 4.12 (m, 0.5H), 4.00 (d, *J* = 8.8 Hz, 1H), 3.81 (m, 0.5H), 3.53 - 3.41 (m, 1H), 3.26 (h, *J* = 6.1 Hz, 1H), 3.06 (dd, *J* = 10.6, 5.9 Hz, 1H), 3.00 - 2.83 (m, 1H), 2.43 (q, *J* = 5.0 Hz, 3H), 2.11 (t, *J* = 5.4 Hz, 4H), 1.80 (d, *J* = 8.6 Hz, 1H), 1.80 - 1.55 (m, 3H), 1.53 - 1.33 (m, 3H), 0.83 (d, *J =* 4 Hz, 3H), 0.61 - 0.57 (m, 2H), 0.39 (d, *J* = 1.8 Hz, 2H). | | | | | |
| **292** | 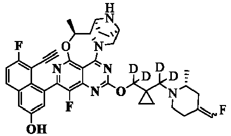 | 701 [M+H] | **293** | 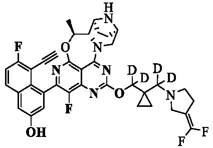 | 691 [M+H] |
| **Example 292:** ¹H NMR (400 MHz, DMSO) δ 8.38 (s, 1H), 7.90 (dt, *J* = 9.2, 5.8 Hz, 1H), 7.36 (td, *J* = 9.0, 7.8 Hz, 1H), 7.22 (d, *J* = 2.5 Hz, 1H), 7.15 (d, J = 2.5 Hz, 0.5H), 7.06 (d, *J* = 2.5 Hz, 0.5H), 6.71 (s, 0.5H), 6.41 (s, 0.5H), 5.28 - 5.11 (m, 1H), 4.53 (ddd, *J* = 24.2, 8.7, 6.4 Hz, 1H), 4.32 (m, 1 H), 4.03 (d, *J* = 8.8 Hz, 1H), 3.83 - 3.64 (m, 1H), 3.46 (h, *J* = 6.1 Hz, 1H), 3.21 (dd, *J* = 10.6, 5.9 Hz, 1H), 3.10 - 3.03 (m, 1H), 2.48 (q, *J* = 5.0 Hz, 3H), 2.16 (t, *J* = 5.4 Hz, 4H), 1.85 (d, *J* = 8.6 Hz, 1H), 1.75 - 1.49 (m, 3H), 1.44 - 1.31 (m, 3H), 0.95 (d, *J* = 4 Hz, 3H), 0.71 - 0.59 (m, 2H), 0.40 (d, *J* = 1.8 Hz, 2H). | | | | | |
| **Example 293:** ¹H NMR (400 MHz, DMSO) δ 10.12 (s, 1H), 7.96 (dt, *J* = 9.2, 5.7 Hz, 1H), 7.46 (td, *J* = 9.0, 7.5 Hz, 1H), 7.37 (d, *J* = 2.5 Hz, 1H), 7.13 (dd, J = 50.0, 2.6 Hz, 1H), 5.24 - 5.03 (m, 1H), 4.61 - 4.38 (m, 1H), 4.03 - 3.90 (m, 2H), 3.64 - 3.42 (m, 2H), 3.23 - 3.14 (m, 2H), 3.10 - 2.99 (m, 2H), 2.68 - 2.56 (m, 2H), 2.47 - 2.24 (m, 2H), 1.89 - 1.79 (m, 1H), 1.78 - 1.52 (m, 3H), 1.44 (dd, *J* = 8.1, 6.3 Hz, 3H), 0.75 - 0.53 (m, 2H), 0.50 - 0.41 (m, 2H). | | | | | |
| **294** | 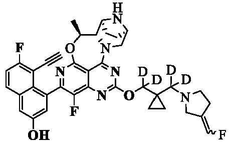 | 673 [M+H] | **295** | 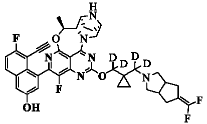 | 731 [M+H] |
| **Example 294:** ¹H NMR (400 MHz, DMSO) δ 10.02 (s, 1H), 7.86 (dt, *J* = 9.2, 5.7 Hz, 1H), 7.56 (td, *J* = 9.0, 7.5 Hz, 1H), 7.37 (d, *J* = 2.5 Hz, 1H), 7.18 (dd, *J* = 50.0, 2.6 Hz, 1H), 6.85 - 6.53 (m, 1H), 5.14 - 5.03 (m, 1H), 4.62 - 4.38 (m, 1H), 4.06 - 3.80 (m, 2H), 3.54 - 3.32 (m, 2H), 3.21 - 3.14 (m, 2H), 3.10 - 2.91 (m, 2H), 2.61 - 2.50 (m, 2H), 2.47 - 2.24 (m, 2H), 1.89 - 1.79 (m, 1H), 1.68 - 1.51 (m, 3H), 1.34 (dd, *J* = 8.1, 6.3 Hz, 3H), 0.71 - 0.50 (m, 2H), 0.40 - 0.31 (m, 2H). | | | | | |
| **Example 295:** ¹H NMR (400 MHz, MeOD) δ 7.86 - 7.79 (m, 1H), 7.35 - 7.27 (m, 2H), 7.17 (d, *J* = 2.6 Hz, 1H), 5.36 (d, *J* = 13.2 Hz, 1H), 4.54 - 4.31 (m, 1H), 4.11 - 4.04 (m, 1H), 3.69 - 3.67 (m, 1H), 3.62 - 3.57 (m, 1H), 3.22 - 3.13 (m, 1H), 2.91 - 2.66 (m, 4H), 2.60 - 2.39 (m, 4H), 2.18 - 2.03 (m, 3H), 1.85 - 1.77 (m, 2H), 1.57 (t, *J* = 6.2 Hz, 3H), 1.36 - 1.29 (m, 2H), 0.71 - 0.49 (m, 4H). | | | | | |
| **296** | 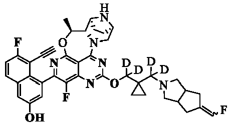 | 713 [M+H] | **297** | 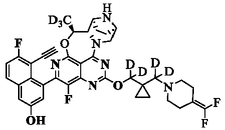 | 708 [M+H] |
| **Example 296:** ¹H NMR (400 MHz, MeOD) δ 7.65 - 7.59 (m, 1H), 7.15 - 7.01 (m, 2H), 6.99 (d, *J* = 2.6 Hz, 1H), 6.85 - 6.50 (m, 1H), 5.46 (d, *J* = 13.2 Hz, 1H), 4.64 - 4.32 (m, 1H), 4.18 - 4.04 (m, 1H), 3.51 - 3.47 (m, 1H), 3.32 - 3.27 (m, 1H), 3.20 - 3.13 (m, 1H), 2.91 - 2.69 (m, 4H), 2.62 - 2.31 (m, 4H), 2.12 - 2.05 (m, 3H), 1.88 - 1.75 (m, 2H), 1.52 (t, *J* = 6.2 Hz, 3H), 1.21 - 1.12 (m, 2H), 0.61 - 0.39 (m, 4H). | | | | | |
| **298** | 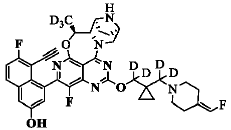 | 690 [M+H] | **299** | 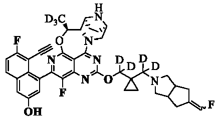 | 716 [M+H] |
| **300** | 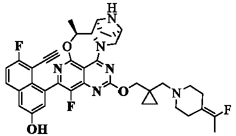 | 697 [M+H] | **301** | 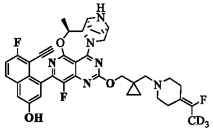 | 700 [M+H] |
| **302** | 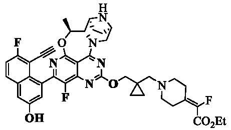 | 755 [M+H] | **303** | 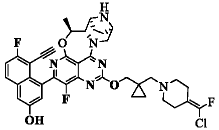 | 717 [M+H] |
| **304** | 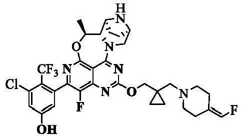 | 693 [M+H] | | | |

### Example 291

### Step 1: Preparation of (1-((4-(fluoromethylene)piperidin-1-yl)methyl-d2)cyclopropyl)methane-d2-ol

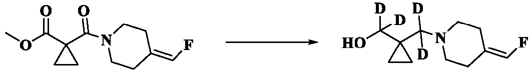

In a round-bottom flask, methyl 1-(4-(fluoromethylene)piperidin-1-carbonyl)cyclopropane-1-carboxylate (7.5 g, 31.11 mmol) was dissolved in THF (80 mL). The obtained mixture was subjected to nitrogen replacement, then cooled to 0°C. Deuterated lithium aluminum hydride (75 mL, 74.65 mmol, 1 M) was slowly added dropwise. The reaction liquid was stirred at 0°C for 1 h. Sodium sulfate decahydrate was slowly added to the reaction liquid in portions to quench the reaction. Then, the obtained mixture was further stirred for 2 h, and filtered, and the filter cake was washed with THF. The organic phase was concentrated, and purified by silica gel column chromatography to obtain (1-((4-(fluoromethylene)piperidin-1-yl)methyl-d2)cyclopropyl)methane-d2-ol (5.5 g, yield 87.1%).

MS m/z (ESI): 204 [M+H].

### Step 2: Preparation of tert-butyl (5S,5aS,6S,9R)-2-(8-ethynyl-7-fluoro-3-(methoxymethoxy)naphthalen-1-yl)-1-fluoro-12-((1-((4-(fluoromethylene)piperidin-1-yl)methyl-d2)cyclopropyl)methoxy-d2)-5-methyl-5a,6,7,8,9,10-hexahydro-5H-4-oxa-3,10a,11,13,14-pentaaza-6,9-methylnaphtho[1,8-ab]heptene-4-carboxylate

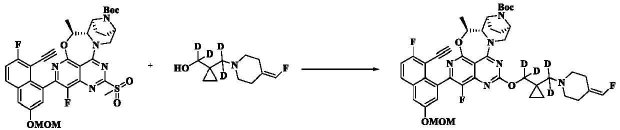

In a round-bottom flask, tert-butyl (5S,5aS,6S,9R)-2-(8-ethynyl-7-fluoro-3-(methoxymethoxy)naphthalen-1-yl)-1-fluoro-5-methyl-12-(methylsulfonyl)-5a,6,7,8,9,10-hexahydro-5H-4-oxa-3,10a,11,13,14-pentaaza-6,9-methylnaphtho[1,8-ab]heptene-4-carboxylate (2 g, 2.83 mmol) and (1-((4-(fluoromethylene)piperidin-1-yl)methyl-d2)cyclopropyl)methane-d2-ol (0.9 g, 4.25 mmol) were dissolved in THF (20 mL), and NaH (203 mg, 8.49 mmol) was added in portions under ice bath. The reaction liquid was stirred at 25°C for 1 h. The reaction liquid was cooled to 0°C, the reaction was quenched by adding dropwise saturated ammonium chloride solution, and the obtained mixture was extracted with ethyl acetate, dried over anhydrous sodium sulfate, filtered, concentrated, and purified by silica gel column chromatography to obtain tert-butyl (5S,5aS,6S,9R)-2-(8-ethynyl-7-fluoro-3-(methoxymethoxy)naphthalen-1-yl)-1-fluoro-12-((1-((4-(fluoromethylene)piperidin-1-yl)methyl-d2)cyclopropyl)methoxy-d2)-5-methyl-5a,6,7,8,9,10-hexahydro-5H-4-oxa-3,10a,11,13,14-pentaaza-6,9-methylnaphtho[1,8-ab]heptene-4-carboxylate (1.6 g, yield 68.5%).

MS m/z (ESI): 831 [M+H].

### Step 3: Preparation of 5-ethynyl-6-fluoro-4-((5S,5aS,6S,9R)-1-fluoro-12-((1-((4-(fluoromethylene)piperidin-1-yl)methyl-d2)cyclopropyl)methoxy-d2)-5-methyl-5a,6,7,8,9,10-hexahydro-5H-4-oxa-3,10a,11,13,14-pentaaza-6,9-methylnaphtho[1,8-ab]hept-2-yl)naphthalen-2-ol

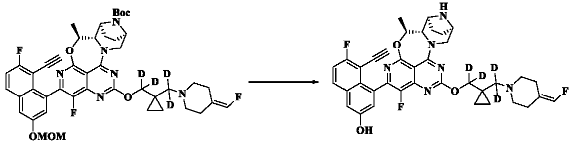

In a round-bottom flask, tert-butyl (5S,5aS,6S,9R)-2-(8-ethynyl-7-fluoro-3-(methoxymethoxy)naphthalen-1-yl)-1-fluoro-12-((1-((4-(fluoromethylene) piperidin-1-yl)methyl-d2)cyclopropyl)methoxy-d2)-5-methyl-5a,6,7,8,9,10-hexahydro-5H-4-oxa-3,10a,11,13,14-pentaaza-6,9-methylnaphtho[1,8-ab]heptene-4-carboxylate (1.6 g, 1.93 mmol) was dissolved in MeOH (5 mL) and dichloromethane (25 mL), and HCl (4 M, 15 mL) was added dropwise under ice bath. The reaction liquid was stirred at 25°C for 1 h. The reaction liquid was cooled to 0°C, and saturated sodium bicarbonate solution was added dropwise to adjust pH>7, the obtained mixture was extracted with CH₂Cl₂/MeOH (10/1), dried over anhydrous sodium sulfate, filtered, concentrated, separated by high performance liquid chromatography, and lyophilized to obtain 5-ethynyl-6-fluoro-4-((55,5As,6S,9R)-1-fluoro-12-((1-((4-(fluoromethylene)piperidin-1-yl)methyl-d2)cyclopropyl)methoxy-d2)-5-methyl-5a,6,7,8,9,10-hexahydro-5H-4-oxa-3,10a,11,13,14-pentaaza-6,9-methylnaphtho[1,8-ab]hept-2-yl)naphthalen-2-ol (480 mg, yield 36.2%).

MS m/z (ESI): 687 [M+H].

¹H NMR (400 MHz, DMSO) δ 8.58 (s, 1H), 7.96 (dt, *J =* 9.2, 5.8 Hz, 1H), 7.56 (td, J = 9.0, 7.8 Hz, 1H), 7.27 (d, J = 2.5 Hz, 1H), 7.10 (d, *J* = 2.5 Hz, 0.5H), 7.00 (d, *J* = 2.5 Hz, 0.5H), 6.82 (s, 0.5H), 6.61 (s, 0.5H), 5.29 - 5.18 (m, 1H), 4.59 (ddd, J = 24.2, 8.7, 6.4 Hz, 1H), 4.12 (m, 1 H), 4.00 (d, *J* = 8.8 Hz, 1H), 3.63 - 3.44 (m, 1H), 3.36 (h, *J* = 6.1 Hz, 1H), 3.18 (dd, *J* = 10.6, 5.9 Hz, 1H), 3.10 - 3.03 (m, 1H), 2.49 (q, *J* = 5.0 Hz, 4H), 2.16 (t, *J* = 5.4 Hz, 4H), 1.79 (d, *J* = 8.6 Hz, 1H), 1.65 - 1.45 (m, 3H), 1.43 - 1.38 (m, 3H), 0.78 - 0.61 (m, 2H), 0.49 (d, *J* = 1.8 Hz, 2H).

**Examples 305 to 308 refer to the synthesis of example 161:**

| **Serial No.** | **Example structure** | **MS m/z (ESI)** | **Serial No.** | **Example structure** | **MS m/z (ESI)** |
|---|---|---|---|---|---|
| **305** | 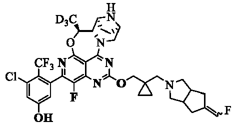 | 722 [M+H] | **306** | 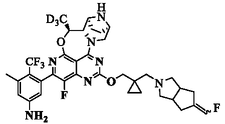 | 701 [M+H] |
| **307** | 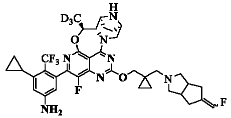 | 727 [M+H] | **308** | 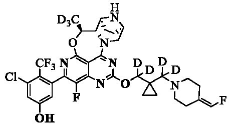 | 700 [M+H] |

**Examples 309 to 314 refer to the synthesis of example 197.**

| **Serial No.** | **Example structure** | **MS m/z (ESI)** | **Serial No.** | **Example structure** | **MS m/z (ESI)** |
|---|---|---|---|---|---|
| **309** | 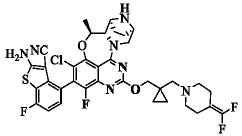 | 740 [M+H] | **310** | 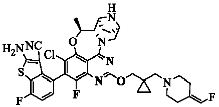 | 722 [M+H] |
| **311** | 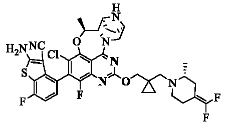 | 754 [M+H] | **312** | 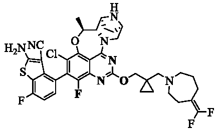 | 754 [M+H] |
| **313** | 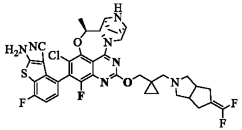 | 766 [M+H] | **314** | 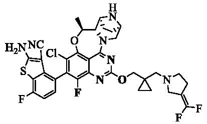 | 726 [M+H] |

**Examples 315 to 320 refer to the synthesis of example 37:**

| **Serial No.** | **Example structure** | **MS m/z (ESI)** | **Serial No.** | **Example structure** | **MS m/z (ESI)** |
|---|---|---|---|---|---|
| **315** | 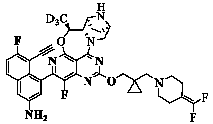 | 703 [M+H] | **316** | 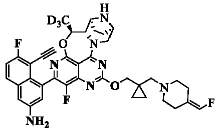 | 685 [M+H] |
| **317** | 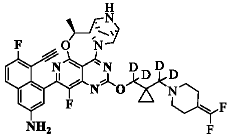 | 704 [M+H] | **318** | 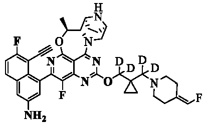 | 686 [M+H] |
| **319** | 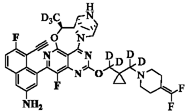 | 707 [M+H] | **320** | 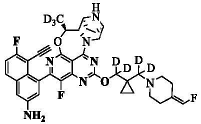 | 689 [M+H] |

### Example 322

### Step 1: Preparation of tert-butyl (5S,5aS,6R,9S)-2-chloro-5-ethyl-1-fluoro-12-(1-((4-(fluoromethylene)piperidin-1-yl)methyl)cyclopropyl)methoxy)-5a,6,7,8,9,10-hexahydro-5H-4-oxa-3,10a,11,13,14-pentaaza-6,9-methylnaphtho[1,8-ab]heptene-4-carboxylate

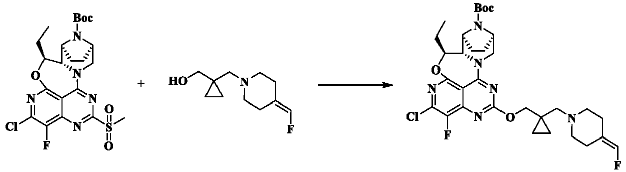

Lithium bis(trimethylsilyl)amide (1 M, 11.4 mL) was added dropwise to a solution of tert-butyl (5S,5aS,6R ,95)-2-chloro-5-ethyl-1-fluoro-12-(methylsulfonyl)-5a,6,7,8,9,10-hexahydro-5H-4-oxa-3,10a,11,13,14-pentaaza-6,9-methylnaphtho[1,8-ab]heptene-4-carboxylate (2 g, 3.8 mmol) and (1-((4-(fluoromethylene)piperidin-1-yl)methyl)cyclopropyl)methanol (981 mg, 4.9 mmol) in tetrahydrofuran (10 mL) at 0°C, and the obtained mixture was stirred at 0°C for 5 min after the addition was completed. The reaction liquid was poured into ammonium chloride aqueous solution (100 mL), and the obtained mixture was extracted with ethyl acetate, dried over anhydrous sodium sulfate, filtered, concentrated, and purified by silica gel column chromatography to obtain tert-butyl (5S,5aS,6R,9S)-2-chloro-5-ethyl-1-fluoro-12-(1-((4-(fluoromethylene)piperidin-1-yl)methyl)cyclopropyl)methoxy)-5a,6,7,8,9,10-hexahydro-5H-4-oxa-3,10a,11,13,14-pentaaza-6,9-methylnaphtho[1,8-ab]heptene-4-carboxylate (2.45 g, yield 100%).

MS m/z (ESI): 647 [M+H].

### Step 2: Preparation of tert-butyl (5S,5aS,6R,9S)-2-(5-amino-3-chloro-2-(trifluoromethyl)phenyl)-5-ethyl-1-fluoro-12-((1-(4-(fluoromethylene)piperidin-1-yl)methyl)cyclopropyl)methoxy)-5a,6,7,8,9,10-hexahydro-5H-4-oxa-3,10a,11,13,14-pentaaza-6,9-methylnaphtho[1,8-ab]heptene-4-carboxylate

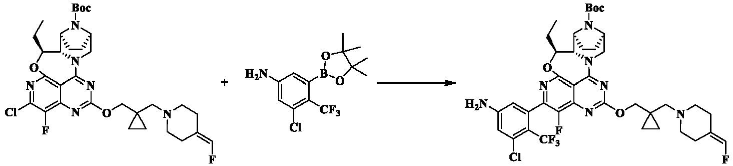

Tert-butyl (5S,5aS,6R,9S)-2-chloro-5-ethyl-1-fluoro-12-(1-((4-(fluoromethylene)piperidin-1-yl)methyl)cyclopropyl)methoxy)-5a,6,7,8,9,10-hexahydro-5H-4-oxa-3,10a, 11, 13,14-pentaaza-6,9-methylnaphtho[1,8-ab]heptene-4-carboxylate (2.45 g, 3.8 mmol), 3-chloro-5-(4,4,5,5-tetramethyl-1,3,2-dioxaboran-2-yl)-4-(trifluoromethyl)aniline (1.8 g, 5.7 mmol), tetrakis(triphenylphosphine)palladium (878 mg, 0.76 mmol), and cesium carbonate (3.7 g, 11.4 mmol) were stirred in dioxane (30 mL) and water (6 mL) at 100°C for 1 h. Water was added to the reaction liquid, and the obtained mixture was extracted with ethyl acetate, dried over anhydrous sodium sulfate, filtered, concentrated, and purified by silica gel column chromatography to obtain yellow tert-butyl (5S,5aS,6R,9S)-2-(5-amino-3-chloro-2-(trifluoromethyl)phenyl)-5-ethyl-1-fluoro-12-((1-(4-(fluoromethylene)piperidin-1-yl)methyl)cyclopropyl)methoxy)-5a,6,7,8,9,10-hexahydro-5H-4-oxa-3,10a,11,13,14-pentaaza-6,9-methylnaphtho[1,8-ab]heptene-4-carboxylate (1 g, yield 33%).

MS m/z (ESI): 806 [M+H].

### Step 3: Preparation of 3-chloro-5-((5S,5aS,6R,9S)-5-ethyl-1-fluoro-12-((1-((4-(fluoromethylene)piperidin-1-yl)methyl)cyclopropyl)methoxy)-5a,6,7,8,9,10-hexahydro-5H-4-oxa-3,10a,11,13,14-pentaaza-6,9-methylnaphtho[1,8-ab]hept-2-yl)-4-(trifluoromethyl)aniline

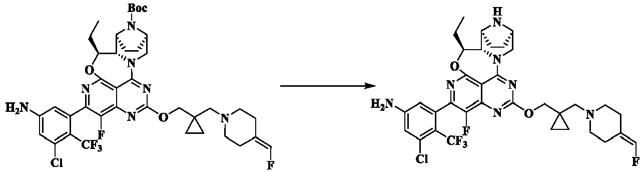

Trifluoroacetic acid (7 mL) was added to a solution of tert-butyl (5S,5aS,6R,9S)-2-(5-amino-3-chloro-2-(trifluoromethyl)phenyl)-5-ethyl-1-fluoro-12-((1-(4-(fluoromethylene)piperidin-1-yl)methyl)cyclopropyl)methoxy)-5a,6,7,8,9,10-hexahydro-5H-4-oxa-3,10a,11,13,14-pentaaza-6,9-methylnaphtho [1,8-ab]heptene-4-carboxylate (1 g, 1.24 mmol) in dichloromethane (21 mL) at room temperature, and the obtained mixture was stirred at room temperature for 0.5 h after the addition was completed. Concentration and then high performance liquid column chromatography purification gave 3-chloro-5-((5S,5aS,6R,9S)-5-ethyl-1-fluoro-12-((1-((4-(fluoromethylene)piperidin-1-yl)methyl)cyclopropyl)methoxy)-5a,6,7,8,9,10-hexahydro-5H-4-oxa-3,10a,11,13,14-pentaaza-6,9-methylnaphtho[1,8-ab]hept-2-yl)-4-(trifluoromethyl)aniline (500 mg, yield 57%).

¹H NMR (400 MHz, MeOD) δ 7.76 - 7.55 (m, 0.5 H), 6.88 (s, 1H), 6.69 (s, 0.5 H), 6.48 (t, *J =* 20 Hz, 1H), 5.37 (d, *J* = 12 Hz, 1H), 4.44 (s, 2H), 4.38 - 4.24 (m, 1H), 4.23 - 4.12 (m, 1H), 3.83 - 3.62 (m, 5H), 3.22 (d, *J* = 12 Hz, 1H), 2.97 - 2.71 (m, 5H), 2.46 (s, 2H), 2.23 (s, 2H), 2.15 - 1.65 (m, 4H), 1.20 - 1.04 (m, 3H), 0.83 (s, 2H), 0.64 (s, 2H).

MS m/z (ESI): 706 [M+H].

### Example 330

### Step 1: Preparation of tert-butyl (5S,5aS,6R,9S)-2-chloro-5-ethyl-1-fluoro-12-(1-((4-(fluoromethylene)piperidin-1-yl)methyl-d2)cyclopropyl)methoxy-d2)-5a,6,7,8,9,10-hexahydro-5H-4-oxa-3,10a,11,13,14-pentaaza-6,9-methylnaphtho[1,8-ab]heptene-4-carboxylate

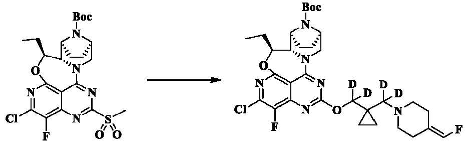

Lithium bis(trimethylsilyl)amide (1 M, 30.7 mL) was added dropwise to a solution of tert-butyl (5S,5aS,6R,95)-2-chloro-5-ethyl-1-fluoro-12-(methylsulfonyl)-5a,6,7,8,9,10-hexahydro-5H-4-oxa-3,10a,11,13,14-pentaaza-6,9-methylnaphtho[1,8-ab]heptene-4-carboxylate (5 g, 9.47 mmol) and (1-((4-(fluoromethylene)piperidin-1-yl)methyl-d2)cyclopropyl)methane-d2-ol (2.5 g, 12.3 mmol) in tetrahydrofuran (10 mL) at 0°C, and the obtained mixture was stirred at 0°C for 15 min after the addition was completed. The reaction liquid was poured into ammonium chloride aqueous solution (100 mL), and the obtained mixture was extracted with ethyl acetate, dried over anhydrous sodium sulfate, filtered, concentrated, and purified by silica gel column chromatography to obtain tert-butyl (5S,5aS,6R,9S)-2-chloro-5-ethyl-1-fluoro-12-(1-((4-(fluoromethylene)piperidin-1-yl)methyl-d2)cyclopropyl)methoxy-d2)-5a,6,7,8,9,10-hexahydro-5H-4-oxa-3,10a,11,13,14-pentaaza-6,9-methylnaphtho[1,8-ab]heptene-4-carboxylate (5.5 g, yield 89.1%).

MS m/z (ESI): 651 [M+H].

### Step 2: Preparation of tert-butyl (5S,5aS,6R,9S)-2-(5-amino-3-chloro-2-(trifluoromethyl)phenyl)-5-ethyl-1-fluoro-12-((1-(4-(fluoromethylene)piperidin-1-yl)methyl-d2)cyclopropyl)methoxy-d2)-5a,6,7,8,9,10-hexahydro-5H-4-oxa-3,10a,11,13,14-pentaaza-6,9-methylnaphtho[1,8-ab]heptene-4-carboxylate

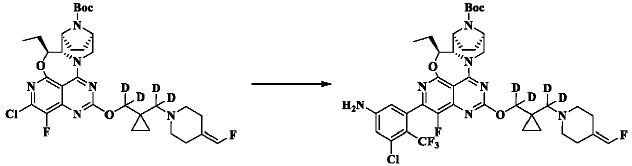

tert-butyl (5S,5aS,6R,9S)-2-chloro-5-ethyl-1-fluoro-12-(1-((4-(fluoromethylene) piperidin-1-yl)methyl-d2)cyclopropyl)methoxy-d2)-5a,6,7,8,9,10-hexahydro-5H-4-oxa-3,10a,11,13,14-pentaaza-6,9-methylnaphtho[1,8-ab]heptene-4-carboxylate (5.3 g, 8.14 mmol), 3-chloro-5-(4,4,5,5-tetramethyl-1,3,2-dioxaboran-2-yl)-4-(trifluoromethyl)aniline (3.93 g, 12.2 mmol), tetrakis(triphenylphosphine)palladium (1.88 g, 1.63 mmol), and cesium carbonate (8 g, 24.55 mmol) were dissolved in dioxane/water (100 mL/20 mL), and the obtained mixture was subjected to nitrogen replacement, heated to 100°C and stirred for 3 h. The obtained mixture was cooled to room temperature, water was added, and the obtained mixture was extracted with ethyl acetate. The organic phase was dried over anhydrous sodium sulfate, filtered, concentrated, and purified by silica gel column chromatography to obtain tert-butyl (5S,5aS,6R,9S)-2-(5-amino-3-chloro-2-(trifluoromethyl)phenyl)-5-ethyl-1-fluoro-12-((1-(4-(fluoromethylene)piperidin-1-yl)methyl-d2)cyclopropyl)methoxy-d2)-5a,6,7,8,9,10-hexahydro-5H-4-oxa-3,10a,11,13,14-pentaaza-6,9-methylnaphtho[1,8-ab]heptene-4-carboxylate (3.4 g, yield 51.6%).

MS m/z (ESI): 810 [M+H].

### Step 3: Preparation of 3-chloro-5-((5S,5aS,6R,9S)-5-ethyl-1-fluoro-12-((1-((4-(fluoromethylene)piperidin-1-yl)methyl-d2)cyclopropyl)methoxy-d2)-5a,6,7,8,9,10-hexahydro-5H-4-oxa-3,10a,11,13,14-pentaaza-6,9-methylnaphtho[1,8-ab]hept-2-yl)-4-(trifluoromethyl)aniline

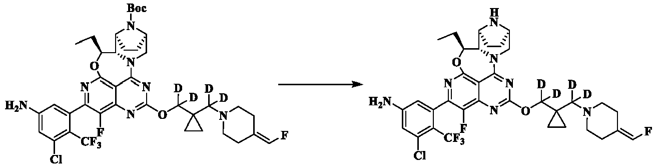

Trifluoroacetic acid (15 mL) was added to a solution of tert-butyl (5S,5aS,6R,9S)-2-(5-amino-3-chloro-2-(trifluoromethyl)phenyl)-5-ethyl-1-fluoro-12-((1-(4-(fluoromethylene)piperidin-1-yl)methyl-d2)cyclopropyl)methoxy-d2)-5a,6,7,8,9,10-hexahydro-5H-4-oxa-3,10a,11,13,14-pentaaza-6,9-methylnaphtho [1,8-ab]heptene-4-carboxylate (3.3 g, 4.07 mmol) in dichloromethane (100 mL) at room temperature, and the obtained mixture was stirred at room temperature for 1 h after the addition was completed. Concentration and then high performance liquid column chromatography purification gave 3-chloro-5-((5S,5aS,6R,9S)-5-ethyl-1-fluoro-12-((1-((4-(fluoromethylene)piperidin-1-yl)methyl-d2)cyclopropyl) methoxy-d2)-5a,6,7,8,9,10-hexahydro-5H-4-oxa-3,10a,11,13,14-pentaaza-6,9-methylnaphtho[1,8-ab]hept-2-yl)-4-(trifluoromethyl)aniline (1.9 g, yield 65.7%).

MS m/z (ESI): 710 [M+H].

¹H NMR(400 MHz, MeOD) δ 7.82(dt, *J* = 9.0, 4.1 Hz, 1H), 7.33 - 7.27(m, 2H), 7.26 - 7.18(m, 1H), 6.29(s, 1H), 5.08 - 5.01 (m, 1H), 4.65 - 4.30(m, 5H), 3.05 - 2.98 (m, 3H), 2.93 - 2.77(m, 4H), 2.08 - 1.79(m, 6H), 1.37 - 1.30 (m, 2H), 0.86 - 0.65(m, 7H).

**Examples 321 to 335 refer to the synthesis of example 124:**

| **Serial No.** | **Example structure** | **MS m/z (ESI)** | **Serial No.** | **Example structure** | **MS m/z (ESI)** |
|---|---|---|---|---|---|
| **321** | 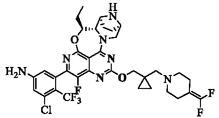 | 734 [M+H] | **322** | 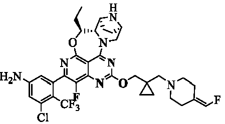 | 706 [M+H] |
| **323** | 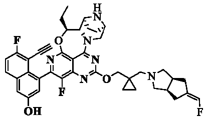 | 723 [M+H] | **324** | 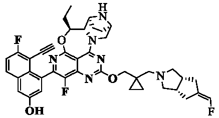 | 723 [M+H] |
| **325** | 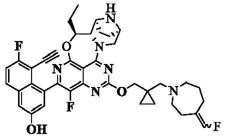 | 711 [M+H] | **326** | 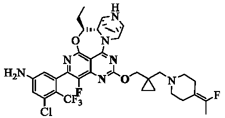 | 720 [M+H] |
| **327** | 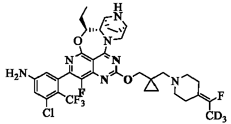 | 723 [M+H] | **328** | 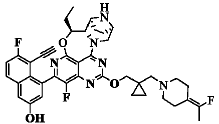 | 711 [M+H] |
| **329** | 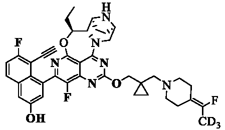 | 714 [M+H] | **330** | 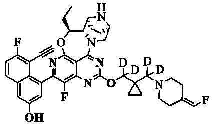 | 701 [M+H] |
| **331** | 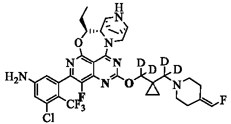 | 710 [M+H] | **332** | 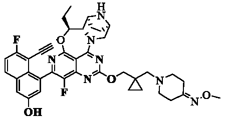 | 710 [M+H] |
| **333** | 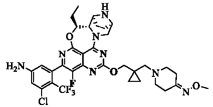 | 719 [M+H] | **334** | 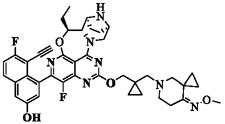 | 736 [M+H] |
| **335** | 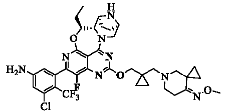 | 745 [M+H] | | | |

**Examples 336 to 351 refer to the synthesis of example 21.**

| **Serial No.** | **Example structure** | **MS m/z (ESI)** | **Serial No.** | **Example structure** | **MS m/z (ESI)** |
|---|---|---|---|---|---|
| **336** | 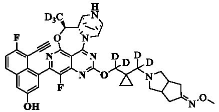 | 729 [M+H] | **337** | 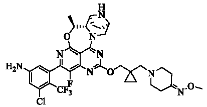 | 705 [M+H] |
| **338** | 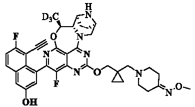 | 699 [M+H] | **339** | 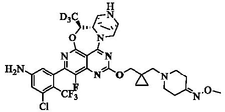 | 708 [M+H] |
| **340** | 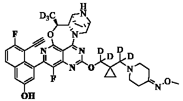 | 703 [M+H] | **341** | 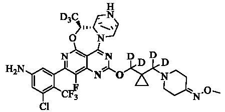 | 712 [M+H] |
| **342** | 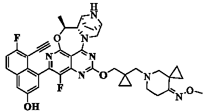 | 722 [M+H] | **343** | 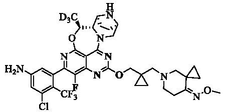 | 734 [M+H] |
| **344** | 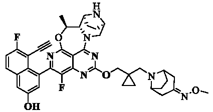 | 722 [M+H] | **345** | 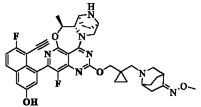 | 722 [M+H] |
| **346** | 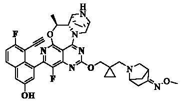 | 708 [M+H] | **347** | 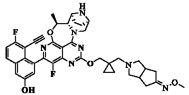 | 722 [M+H] |
| **348** | 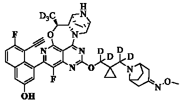 | 729 [M+H] | **349** | 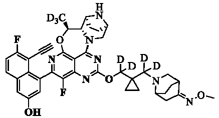 | 729 [M+H] |
| **350** | 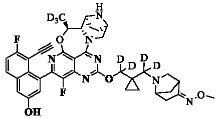 | 715 [M+H] | | | |

**The following synthesis methods can also be adopted for the following examples:**

### Example 337

### Step 1: Preparation of tert-butyl (SS,SaS,6S,9R)-12-((1-((tertbutyldiphenylsilyl)oxy)methyl)cyclopropyl)methoxy)-2-chloro-1-fluoro-5-methyl-5a,6,7,8,9, 10-hexahydro-5H-4-oxa-3, 10a, 11, 13,14-pentyl-6,9-methylnaphtho[1,8-ab]heptene-4-carboxylate

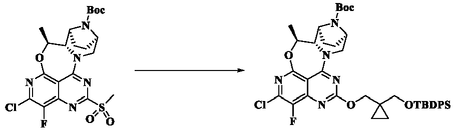

In an eggplant-shaped flask, tert-butyl (5S,5aS,6S,9R)-2-chloro-1-fluoro-5-methyl-12-(methylsulfonyl)-5a,6,7,8,9,10-hexahydro-5H-4-oxa-3,10a,11,13,14-pentaaza-6,9-methylnaphtho[1,8-ab]heptene-14-carboxylate (8.00 g, 15.57 mmol) and [1-[[tert-butyl(diphenyl)silyl]oxymethyl]cyclopropyl]methanol (7.95 g, 23.35 mmol) were dissolved in tetrahydrofuran (100 mL). Nitrogen replacement was performed, and lithium bis(trimethylsilyl)amide (1 M, 46.70 mL) was added dropwise under ice bath. The obtained mixture was reacted under ice bath for 20 min. A saturated ammonium chloride solution was added to quench the reaction, and the obtained mixture was extracted with ethyl acetate. The organic phase was dried over anhydrous sodium sulfate, filtered, concentrated, and purified by silica gel column chromatography to obtain tert-butyl (SS,SaS,6S,9R)-12-((1-((tert-butyldiphenylsilyl)oxy)methyl)cyclopropyl)methoxy)-2-chloro-1-fluoro-5-methyl-5a,6,7,8,9,10-hexahydro-5H-4-oxa-3,10a,11,13,14-pentyl-6,9-methylnaphtho[1,8-ab]heptene-4-carboxylate (8.5 g, yield 71%).

### Step 2: Preparation of tert-butyl (5S,5aS,6S,9R)-2-chloro-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methylnaphtho[1,8-ab]heptene-14carboxylate

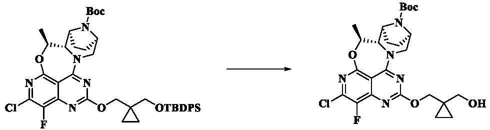

In an eggplant-shaped flask, tert-butyl (5S,5aS ,6S,9R)-12-((1-((tert-butyldiphenylsilyl)oxy)methyl)cyclopropyl)methoxy)-2-chloro-1-fluoro-5-methyl-5a,6,7,8,9,10-hexahydro-5H-4-oxa-3,10a,11,13,14-pentyl-6,9-methylnaphtho[1,8-ab]heptene-4-carboxylate (8.5 g, 10.98 mmol) was dissolved in tetrahydrofuran (60 mL), a solution of tetrabutylammonium fluoride in tetrahydrofuran (1 M, 21.95 mL) was added, and then the obtained mixture was reacted at room temperature for 2 h. Water was added to the reaction liquid and the obtained mixture was extracted with ethyl acetate. The organic phase was dried over anhydrous sodium sulfate, filtered, concentrated, and purified by silica gel column chromatography to obtain tert-butyl (5S,5a8,6S,9R)-2-chloro-1-fluoro-12-((1-(hydroxymethyl)cyclopropyl)methoxy)-5-methyl-5a,6,7,8,9, 10-hexahydro-5H-4-oxa-3, 10a, 11, 13,14-pentaaza-6,9-methylnaphtho[1,8-ab]heptene-14carboxylate (5.2 g, yield 88%).

### Step 3: Preparation of tert-butyl (5S,5aS,6S,9R)-2-chloro-1-fluoro-5-methyl-12-((1-(methylsulfonyl)oxy)methyl)cyclopropyl)methoxy)-5a,6,7,8,9,10-hexahydro-5H-4-oxa-3,10a,11,13,14-pentaaza-6,9-methylnaphtho[1,8-ab]heptene-14carboxylate

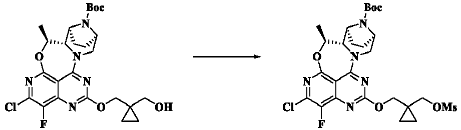

In an eggplant-shaped flask, tert-butyl (5S,5aS,6S,9R)-2-chloro-1-fluoro-12-((1-(hydroxymethyl)cyclopropyl)methoxy)-5-methyl-5a,6,7,8,9,10-hexahydro-5H-4-oxa-3,10a,11,13,14-pentaaza-6,9-methylnaphtho[1,8-ab]heptene-14carboxylate (5.2 g, 9.70 mmol) was dissolved in 20 mL of dichloromethane. N,N-diisopropylethylamine (6.27 g, 48.51 mmol, 8.45 mL) and methylsulfonyl chloride (2.78 g, 24.25 mmol) were added under ice bath, and the obtained mixture was reacted at room temperature for 1 h. Saturated sodium bicarbonate solution was used to quench the reaction, and dichloromethane was used for extraction; the organic phase was dried over anhydrous sodium sulfate, filtered, and concentrated to obtain crude tert-butyl (5S,5aS,6S,9R)-2-chloro-1-fluoro-5-methyl-12-((1-(methylsulfonyl)oxy)methyl)cyclopropyl)methoxy)-5a,6,7,8,9,10-hexahydro-5H-4-oxa-3,10a,11,13,14-pentaaza-6,9-methylnaphtho[1,8-ab]heptene-14carboxylate (5.96 g, yield 100%).

### Step 4: Preparation of tert-butyl (5S,5aS,6S,9R)-2-chloro-1-fluoro-12-((1-((4-(methoxyimino)piperidin-1-yl)methyl)cyclopropyl)methoxy)-5-methyl-5a,6,7,8,9,10-hexahydro-5H-4-oxa-3,10a,11,13,14-pentaaza-6,9-methylnaphtho[1,8-ab]heptene-14carboxylate

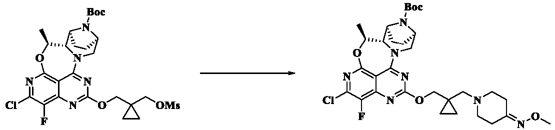

N-methoxypiperidin-4-imine trifluoroformate (9.05 g, 37.37 mmol), tert-butyl (5S,5aS,6S,9R)-2-chloro-1-fluoro-5-methyl-12-((1-(methylsulfonyl)oxy)methyl) cyclopropyl)methoxy)-5a,6,7,8,9,10-hexahydro-5H-4-oxa-3,10a,11,13,14-pentaaza-6,9-methylnaphtho[1,8-ab]heptene-14carboxylate (5.96 g, 9.71 mmol), N,N-diisopropylethylamine (6.27 g, 48.53 mmol, 8.45 mL), sodium iodide (4.36 g, 29.12 mmol) and 50 mL of acetonitrile were added to an eggplant-shaped flask. Nitrogen replacement was performed and the reaction was carried out in an oil bath at 55°C for 16 h. Water was added to the reaction liquid and the obtained mixture was extracted with ethyl acetate. The organic phase was dried over anhydrous sodium sulfate, filtered, concentrated, and purified by silica gel column chromatography to obtain tert-butyl (5S,5aS,6S,9R)-2-chloro-1-fluoro-12-((1-((4-(methoxyimino)piperidin-1-yl)methyl)cyclopropyl)methoxy)-5-methyl-5a,6,7,8,9,10-hexahydro-5H-4-oxa-3,10a,11,13,14-pentaaza-6,9-methylnaphtho [1,8-ab]heptene-14carboxylate (5.4 g, yield 86%).

MS m/z(ESI):646 [M+H].

### Step 5: Preparation of tert-butyl (5S,5aS,6S,9R)-2-(5-amino-3-chloro-2-(trifluoromethyl)phenyl)-1-fluoro-12-((1-((4-(methoxyimino)piperidin-1-yl)methyl)cyclopropyl)methoxy)-5-methyl-5a,6,7,8,9,10-hexahydro-5H-4-oxo-3,10a,11,13,14-pentaaza-6,9-methylnaphtho[1,8-ab]heptene-14carboxylate

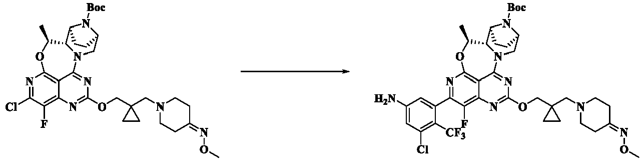

3-chloro-5-(4,4,5,5-tetramethyl-1,3,2-dioxaboran-2-yl)-4-(trifluoromethyl) aniline (4.03 g, 12.54 mmol), tert-butyl (5S,5aS,6S,9R)-2-chloro-1-fluoro-12-((1-((4-(methoxyimino)piperidin-1-yl)methyl)cyclopropyl)methoxy)-5-methyl-5a,6,7,8,9,10-hexahydro-5H-4-oxa-3,10a,11,13,14-pentaaza-6,9-methylnaphtho [1,8-ab]heptene-14carboxylate (5.4 g, 8.36 mmol), tetrakis(triphenylphosphine) palladium (1.93 g, 1.67 mmol), cesium carbonate (8.17 g, 25.07 mmol), 80 mL of dioxane and 20 mL of water were added to an eggplant-shaped flask. Nitrogen replacement was performed and the reaction was carried out in an oil bath at 100°C for 1 h. The reaction liquid was cooled, extracted with ethyl acetate. The organic phase was dried over anhydrous sodium sulfate, filtered, concentrated, and purified by silica gel chromatography to obtain tert-butyl (5S,5aS,6S,9R)-2-(5-amino-3-chloro-2-(trifluoromethyl)phenyl)-1-fluoro-12-((1-((4-(methoxyimino)piperidin-1-yl)methyl)cyclopropyl)methoxy)-5-methyl-5a,6,7,8,9,10-hexahydro-5H-4-oxo-3,10a,11,13,14-pentaaza-6,9-methylnaphtho[1,8-ab]heptene-14carboxylate (3.6 g, yield 53%).

MS m/z(ESI):805 [M+H].

### Step 6: Preparation of 1-((1-((((5S,5aS,6S,9R)-2-(5-amino-3-chloro-2-(trifluoromethyl)phenyl)-1-fluoro-5-methyl-5a,6,7,8,9,10-hexahydro-5H-4-oxa-3,10a,11,13,14-pentaaza-6,9-methylnaphtho[1,8-ab]heptene-12-yl)oxy)methyl)cyclopropyl)methyl)piperidin-4-one O-methyloxime

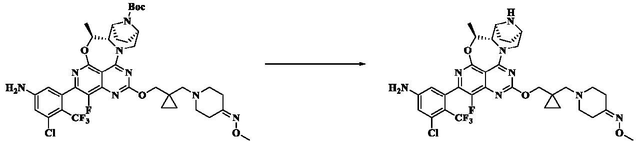

In an eggplant-shaped flask, tert-butyl (5S,5aS,6S,9R)-2-(5-amino-3-chloro-2-(trifluoromethyl)phenyl)-1-fluoro-12-((1-((4-(methoxyimino)piperidin-1-yl)methyl)cyclopropyl)methoxy)-5-methyl-5a,6,7,8,9,10-hexahydro-5H-4-oxo-3,10a,11,13,14-pentaaza-6,9-methylnaphtho[1,8-ab]heptene-14carboxylate (3.6 g, 4.47 mmol) was dissolved in dichloromethane (35 mL), trifluoroacetic acid (18.42 g, 161.55 mmol) was added under ice bath, and the obtained mixture was reacted at room temperature for 1 h. The reaction liquid was concentrated and purified by silica gel column chromatography to obtain 1-((1-((((5S,5aS,6S,9R)-2-(5-amino-3-chloro-2-(trifluoromethyl)phenyl)-1-fluoro-5-methyl-5a,6,7,8,9,10-hexahydro-5H-4-oxa-3,10a,11,13,14-pentaaza-6,9-methylnaphtho[1,8-ab]heptene-12-yl)oxy)methyl) cyclopropyl)methyl)piperidine-4-one O-methyloxime (2.8 g, yield 88.9%).

MS m/z(ESI):705 [M+H].

¹H NMR (400 MHz, MeOD) δ 6.90 (d, *J* = 2.4 Hz, 1H), 6.47 (d, *J* = 57.6 Hz, 1H), 5.51 (dd, *J* = 14.6, 2.8 Hz, 1H), 4.67 (t, *J* = 7.4 Hz, 1H), 4.48 (s, 2H), 4.35 (d, *J* = 8.5 Hz, 1H), 4.30 (s, 1H), 4.24 - 4.19 (m, 1H), 3.84 (s, 3H), 3.45 (d, *J* = 14.8 Hz, 1H), 3.36 (s, 6H), 2.72 (s, 2H), 2.39 - 2.26 (m, 1H), 2.21 - 1.99 (m, 3H), 1.60 (d, *J* = 6.3 Hz, 3H), 1.31 (d, *J* = 18.0 Hz, 2H), 0.99 (d, *J* = 4.4 Hz, 2H), 0.92 - 0.84 (m, 2H).

### Example 338

### Step 1: Preparation of (5S,5aS,6S,9R)-2-(8-ethynyl-7-fluoro-3-(methoxymethoxy)naphthalen-1-yl)-1-fluoro-5-deuterated methyl-12-((1-(((methylsulfonyl)oxo)methyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate

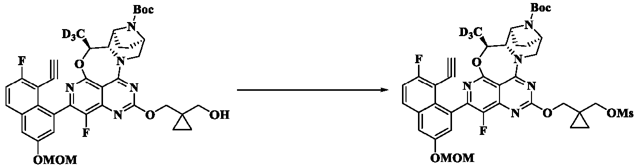

tert-butyl (5S,5aS,6S,9R)-2-(8-ethynyl-7-fluoro-3-(methoxymethoxy)naphthalen-1-yl)-1-fluoro-12-((1-(hydroxymethyl)cyclopropyl)methoxy)-5-deuterated methyl-5a,6,7,8,9,10-hexahydro-5H-4-oxa-3,10a,11,13,14-pentaaza-6,9-methylenenaphtho [1,8-ab]heptalene-14-carboxylate (1 g, 1.37 mmol) and DIPEA (0.5 g, 4.11 mmol) were added to dichloromethane (10 mL). After mixing evenly, MsCl (235 mg, 2.05 mmol) was added dropwise at 0°C. After the addition was completed, the obtained mixture was reacted for 0.5 h. The reaction liquid was quenched by adding NaHCO3 aqueous solution, extracted with dichloromethane, washed with saturated NaCl aqueous solution, dried over anhydrous sodium sulfate, filtered and concentrated to obtain crude (5S,5aS,6S,9R)-2-(8-ethynyl-7-fluoro-3-(methoxymethoxy) naphthalen-1-yl)-1-fluoro-5-deuterated methyl-12-((1-(((methylsulfonyl)oxo) methyl)cyclopropyl)methoxy)-5a,6,7,8,9,10-hexahydro-5H-4-oxa-3,10a,11,13,14-pentaaza-6,9-methylenenaphtho[1,8-ab]heptalene-14-carboxylate (1 g).

### Step 2: Preparation of tert-butyl (5S,5aS,6S,9R)-2-(8-ethynyl-7-fluoro-3-(methoxymethoxy)naphthalen-1-yl)-1-fluoro-12-((1-((4-(methoxyimino)piperidin-1-yl)methyl)cyclopropyl)methoxy)-5-deuterated methyl-5a,6,7,8,9,10-hexahydro-5H-4-oxa-3,10a,11,13,14-pentaaza-6,9-methylenenaphtho[1,8-ab]heptalene-14-carboxylate

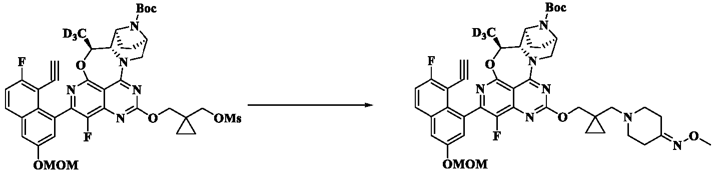

(5 S, 5 aS, 6 S, 9R)-2-(8-ethynyl-7-fluoro-3 -(methoxymethoxy)naphthalen-1-yl)-1-fluoro-5-deuterated methyl-12-((1-(((methylsulfonyl)oxo)methyl)cyclopropyl) methoxy)-5a,6,7,8,9,10-hexahydro-5H-4-oxa-3,10a,11,13,14-pentaaza-6,9-methylenenaphtho[1,8-ab]heptalene-14-carboxylate (1 g, 1.23 mmol), piperidin-4-one O-methyloxime (475 mg, 3.7 mmol), DIPEA (0.8 g, 6.17 mmol), NaI (0.55 g, 3.7 mmol) were added to acetonitrile (10 mL). After mixing evenly, the obtained mixture was reacted at 50°C overnight, and water was added, and the obtained mixture was extracted with ethyl acetate, dried over anhydrous sodium sulfate, filtered, concentrated, and purified by silica gel column chromatography to obtain the target compound tert-butyl (5S,5aS,6S,9R)-2-(8-ethynyl-7-fluoro-3-(methoxymethoxy)naphthalen-1-yl)-1-fluoro-12-((1-((4-(methoxyimino)piperidin-1-yl)methyl)cyclopropyl)methoxy)-5-deuterated methyl-5a,6,7,8,9,10-hexahydro-5H-4-oxa-3,10a,11,13,14-pentaaza-6,9-methylenenaphtho[1,8-ab]heptalene-14-carboxylate (1 g, yield 96.2%).

MS m/z (ESI):843 [M+H].

### Step 3: Preparation of 1-((1-((((5S,5aS,6S,9R)-2-(8-ethynyl-7-fluoro-3-hydroxynaphthalen-1-yl)-1-fluoro-5-deuterated methyl-5a,6,7,8,9,10-hexahydro-5H-4-oxa-3,10a,11,13, 14-pentaaza-6,9-methylenenaphtho[1,8-ab]heptalen-12-yl)oxo)methyl)cyclopropyl)methyl)piperidin-4-one O-methyloxime formate

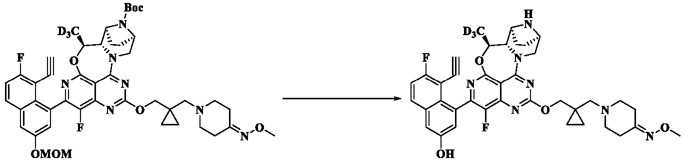

Tert-butyl (5S,5aS,6S,9R)-2-(8-ethynyl-7-fluoro-3-(methoxymethoxy) naphthalen-1-yl)-1-fluoro-12-((1-((4-(methoxyimino)piperidin-1-yl)methyl) cyclopropyl)methoxy)-5-deuterated methyl-5a,6,7,8,9,10-hexahydro-5H-4-oxa-3,10a,11,13,14-pentaaza-6,9-methylenenaphtho[1,8-ab]heptalene-14-carboxylate (1 g, 1.19 mmol) was added to the dichloromethane/methanol solution (10 mL, volume ratio of 4:1), and after mixing evenly, a solution of hydrochloric acid in dioxane (10 mL, 4 M) was added at 0°C. After the addition was completed, the temperature was warmed to room temperature, and the reaction was continued for 40 min. The mixture was neutralized with the aqueous NaHCO₃ solution, extracted with ethyl acetate, filtered, concentrated, and purified by preparative column chromatography to obtain 1-((1-((((5S,5aS,6S,9R)-2-(8-ethynyl-7-fluoro-3-hydroxylnaphthalen-1-yl)-1-fluoro-5-deuterated methyl-5a,6,7,8,9,10-hexahydro-5H-4-oxa-3,10a,11,13,14-pentaaza-6,9-methylenenaphtho[1,8-ab]heptalene-12-yl)oxo)methyl)cyclopropyl)methyl)piperidine-4-one O-methyloxime formate (100 mg, yield 11%).

MS m/z (ESI):699 [M+H].

¹H NMR (400 MHz, DMSO) δ 10.09 (s, 1H), 8.12 (s, 1H), 7.96 (dt, *J* = 9.6, 5.7 Hz, 1H), 7.51 - 7.40 (m, 1H), 7.36 (d, *J* = 2.5 Hz, 1H), 7.11 (dd, *J* = 50.6, 2.5 Hz, 1H), 5.21 (d, *J* = 13.1 Hz, 1H), 4.62 - 4.43 (m, 1H), 4.37 - 4.22 (m, 2H), 4.22 - 3.90 (m, 2H), 3.83 - 3.59 (m, 4H), 3.21 - 3.05 (m, 2H), 2.55 (s, 2H), 2.48 - 2.30 (m, 6H), 2.20 (d, *J* = 5.6 Hz, 2H), 1.98 - 1.59 (m, 4H), 0.73 - 0.60 (m, 2H), 0.50 - 0.24 (m, 2H).

### Example 339

### Step 1: Preparation of tert-butyl (SS,SaS,6R,9S)-12-((1-((((tert-butyldiphenylsilyl)oxy)methyl)cyclopropyl)methoxy)-2-chloro-1-fluoro-5-(methyl-d3)-5a,6,7,8,9,10-hexahydro-5H-4-oxa-3,10a,11,13,14-pentaaza-6,9-methylnaphtho[1,8-ab]heptene-4-carboxylate

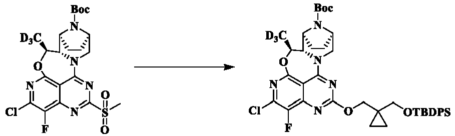

Lithium bis(trimethylsilyl)amide (1 M, 11.6 mL) was added dropwise to a solution of tert-butyl (5S,5aS,6R,9S)-2-[(1S)-1-(7-chloro-8-fluoro-4-hydroxy-2-methylsulfanyl-pyrido[4,3-d]pyrimidin-5-yl)oxy-2,2,2-trideuterium-ethyl]-3,8-diazabicyclo[3.2.1]octane-8-carboxylate (2 g, 3.87 mmol) and [1-[[tert-butyl (diphenyl)silyl]oxymethyl]cyclopropyl]methanol (1.71 g, 5.03 mmol) in tetrahydrofuran (20 mL) at 0°C (15 min), and the obtained mixture was reacted at 0°C for 5 min after the addition was completed. The reaction liquid was poured into ammonium chloride aqueous solution (50 mL), and the obtained mixture was extracted with ethyl acetate. The organic phase was washed with saturated sodium chloride aqueous solution, dried over anhydrous sodium sulfate, filtered, and concentrated to obtain tert-butyl (SS,SaS,6R,9S)-12-((1-((((tert-butyldiphenylsilyl)oxy)methyl)cyclopropyl)methoxy)-2-chloro-1-fluoro-5-(methyl-d3)-5a,6,7,8,9,10-hexahydro-5H-4-oxa-3,10a,11,13,14-pentaaza-6,9-methylnaphtho[1,8-ab]heptene-4-carboxylate (3 g, yield 100%).

MS m/z (ESI): 777 [M+H].

### Step 2: Preparation of tert-butyl (5S,5aS,6R,9S)-2-chloro-1-fluoro-12-((1-(hydroxymethyl)cyclopropyl)methoxy)-5-(methyl-d3)-5a,6,7,8,9,10-hexahydro-5H-4-oxa-3,10a,11,13,14-pentaaza-6,9-methylnaphtho[1,8-ab]heptene-14carboxylate

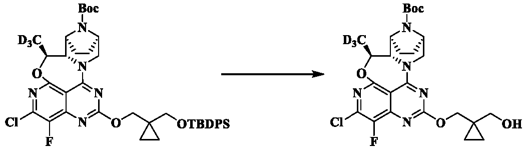

Tetrabutylammonium fluoride (1 M, 20 mL) was added to a solution of tert-butyl (5S,5aS,6R,9S)-12-((1-((((tert-butyldiphenylsilyl)oxy)methyl)cyclopropyl) methoxy)-2-chloro-1-fluoro-5-(methyl-d3)-5a,6,7,8,9,10-hexahydro-5H-4-oxa-3,10a,11,13,14-pentaaza-6,9-methylnaphtho[1,8-ab]heptene-4-carboxylate (3 g, 3.86 mmol) in tetrahydrofuran (30 mL) at room temperature, and the obtained mixture was stirred at room temperature for 1 h after the addition was completed. Water (100 mL) was added to the reaction liquid, and the obtained mixture was extracted with ethyl acetate. The organic phase was washed with water and saturated sodium chloride aqueous solution, dried over anhydrous sodium sulfate, filtered, concentrated, and purified by silica gel column chromatography to obtain tert-butyl (5S,5aS,6R,9S)-2-chloro-1-fluoro-12-((1-(hydroxymethyl)cyclopropyl)methoxy)-5-(methyl-d3)-5a,6,7,8,9,10-hexahydro-5H-4-oxa-3,10a,11,13,14-pentaaza-6,9-methylnaphtho[1,8-ab]heptene-14carboxylate (2 g, yield 96%).

MS m/z (ESI): 539 [M+H].

### Step 3: Preparation of tert-butyl (5S,5aS,6R,9S)-2-chloro-1-fluoro-5-(methyl-d3)-12-(1-(((methylsulfonyl)oxy)methyl)cyclopropyl)methoxy)-5a,6,7,8,9,10-hexahydro-5H-4-oxa-3,10a,11,13,14-pentaaza-6,9-methylnaphtho[1,8-ab]heptene-4-carboxylate

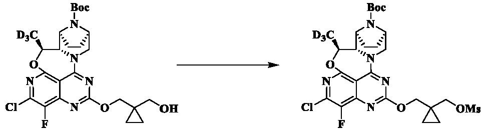

Triethylamine (1.13 g, 11.13 mmol, 1.55 mL) and methanesulfonyl chloride (850 mg, 7.42 mmol) were added to a solution of tert-butyl (SS,SaS,6R,9S)-2-chloro-1-fluoro-12-((1-(hydroxymethyl)cyclopropyl)methoxy)-5-(methyl-d3)-5a,6,7,8,9, 10-hexahydro-5H-4-oxa-3, 10a, 11, 13,14-pentaaza-6,9-methylnaphtho [1,8-ab]heptene-14carboxylate (2 g, 3.71 mmol) in dichloromethane (30 mL) at 0°C, and the obtained mixture was stirred at 0°C for 30 min after the addition was completed. The reaction liquid was added to an sodium bicarbonate aqueous solution (50 mL), and the obtained mixture was extracted with dichloromethane, and the organic phase was washed with an ammonium chloride aqueous solution, dried over anhydrous sodium sulfate, filtered, and concentrated to obtain a crude product tert-butyl (5S,5aS,6R,9S)-2-chloro-1-fluoro-5-(methyl-d3)-12-(1-(((methylsulfonyl) oxy)methyl)cyclopropyl)methoxy)-5a,6,7,8,9,10-hexahydro-5H-4-oxa-3,10a,11,13,14-pentaaza-6,9-methylnaphtho[1,8-ab]heptene-4-carboxylate (2.29 g).

MS m/z (ESI): 617 [M+H].

### Step 4: Preparation of tert-butyl (5S,5aS,6R,9S)-2-chloro-1-fluoro-12-((1-((4-(methoxyimino)piperidin-1-yl)methyl)cyclopropyl)methoxy)-5-(methyl-d3)-5a,6,7,8,9,10-hexahydro-5H-4-oxa-3,10a,11,13,14-pentaaza-6,9-methylnaphtho[1,8-ab]heptene-14carboxylate

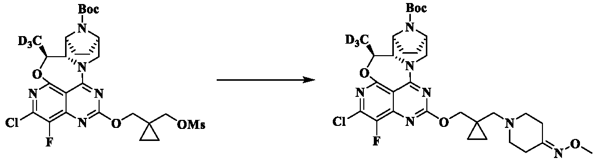

Tert-butyl (5S,5aS,6R,9S)-2-chloro-1-fluoro-5-(methyl-d3)-12-(1-(((methylsulfonyl)oxy)methyl)cyclopropyl)methoxy)-5a,6,7,8,9,10-hexahydro-5H-4-oxa-3,10a,11,13,14-pentaaza-6,9-methylnaphtho[1,8-ab]heptene-4-carboxylate (2.29 g, 3.71 mmol), N-methoxypiperidin-4-imine trifluoroacetate (2.07 g, 8.54 mmol), sodium iodide (1.67 g, 11.13 mmol) and N,N-diisopropylethylamine (4.8 g, 37.1 mmol, 6.46 mL) were dissolved in acetonitrile (40 mL), and the obtained mixture was heated to 50°C and stirred for 13 h. After cooling, water (60 mL) was added to the reaction liquid, and the obtained mixture was extracted with ethyl acetate. The organic phase was dried over anhydrous sodium sulfate, filtered, concentrated, and purified by silica gel chromatography to obtain tert-butyl (5S,5aS,6R,9S)-2-chloro-1-fluoro-12-((1-((4-(methoxyimino)piperidin-1-yl)methyl)cyclopropyl)methoxy)-5-(methyl-d3)-5a,6,7,8,9,10-hexahydro-5H-4-oxa-3,10a,11,13,14-pentaaza-6,9-methylnaphtho[1,8-ab]heptene-14carboxylate (1.4 g, yield 58%).

MS m/z (ESI): 649 [M+H].

### Step 5: Preparation of tert-butyl (5S,5aS,6R,9S)-2-(5-amino-3-chloro-2-(trifluoromethyl)phenyl)-1-fluoro-12-(1-((4-(methoxyimino)piperidin-1-yl)methyl)cyclopropyl)methoxy)-5-(methyl-d3)-5a,6,7,8,9,10-hexahydro-5H-4-oxa-3,10a,11,13,14-pentaaza-6,9-methylnaphtho[1,8-ab]heptene-14carboxylate

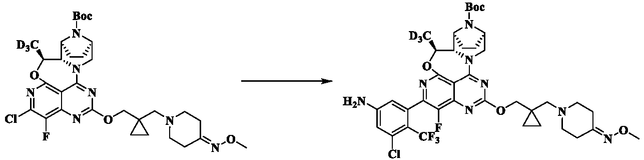

Tert-butyl (5S,5aS,6R,9S)-2-chloro-1-fluoro-12-((1-((4-(methoxyimino) piperidin-1-yl)methyl)cyclopropyl)methoxy)-5-(methyl-d3)-5a,6,7,8,9,10-hexahydro-5H-4-oxa-3,10a, 11, 13,14-pentaaza-6,9-methylnaphtho[1,8-ab]heptene-14carboxylate (1.4 g, 2.16 mmol) and methanesulfonic acid (dimethyl-n-butylphosphino)-2'-amino-1,1'-biphenyl-2-yl)palladium(II) dichloromethane adduct (157 mg, 0.22 mmol) were dissolved in dioxane (20 mL), and the obtained mixture was heated to 80°C and stirred for 0.5 h. The obtained mixture was cooled to room temperature, and 3-chloro-5-(4,4,5,5-tetramethyl-1,3,2-dioxaboran-2-yl)-4-(trifluoromethyl)aniline (2.08 g, 6.47 mmol) and sodium hydroxide aqueous solution (2 M, 4.3 mL) were added, then the obtained mixture was stirred at 70°C for 1.5 h. Ammonium chloride aqueous solution (50 mL) was added, and the obtained mixture was extracted with ethyl acetate. The organic phase was dried over anhydrous sodium sulfate, filtered, concentrated, and purified by silica gel column chromatography to obtain tert-butyl (5S,5aS,6R,9S)-2-(5-amino-3-chloro-2-(trifluoromethyl)phenyl)-1-fluoro-12-(1-((4-(methoxyimino)piperidin-1-yl)methyl)cyclopropyl)methoxy)-5-(methyl-d3)-5a,6,7,8,9,10-hexahydro-5H-4-oxa-3,10a,11,13,14-pentaaza-6,9-methylnaphtho[1,8-ab]heptene-14carboxylate (1.2 g, yield 69%).

MS m/z (ESI): 808[M+H].

### Step 6: Preparation of 1-((1-((((5S,5aS,6S,9R)-2-(5-amino-3-chloro-2-(trifluoromethyl)phenyl)-1-fluoro-5-methyl-d3)-5a,6,7,8,9,10-hexahydro-5H-4-oxa-3,10a,11,13,14-pentaaza-6,9-methylnaphtho[1,8-ab]heptene-12-yl)oxy)methyl)cyclopropyl)methyl)piperidin-4-one O-methyloxime

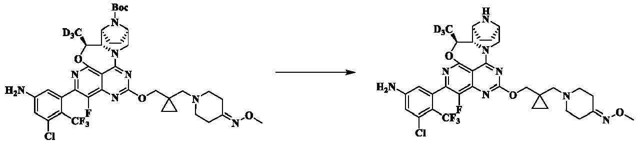

Trifluoroacetic acid (6 mL) was added to a solution of tert-butyl (5S,5aS,6R,9S)-2-(5-amino-3-chloro-2-(trifluoromethyl)phenyl)-1-fluoro-12-(1-((4-(methoxyimino)piperidin-1-yl)methyl)cyclopropyl)methoxy)-5-(methyl-d3)-5a,6,7,8,9,10-hexahydro-5H-4-oxa-3,10a,11,13,14-pentaaza-6,9-methylnaphtho [1,8-ab]heptene-14carboxylate (1.2 g, 1.48 mmol) in dichloromethane (18 mL) at room temperature. After the addition was completed, the obtained mixture was stirred at room temperature for 30 min. After concentration, high performance liquid phase column purification and lyophilization gave 1-((1-((((5S,5aS,6R,9S)-2-(5-amino-3-chloro-2-(trifluoromethyl)phenyl)-1-fluoro-5-(methyl-d3)-5a,6,7,8,9,10-hexahydro-5H-4-oxa-3,10a,11,13,14-pentaaza-6,9-methylnaphtho[1,8-ab]heptene-12-yl)oxy)methyl)cyclopropyl)methyl)piperidine-4-one O-methyloxime (870 mg, yield 74%).

MS m/z (ESI): 708 [M+H].

¹H NMR (400 MHz, MeOD) δ 6.89 (s, 1H), 6.46 (d, *J* = 60 Hz, 1H), 5.56 - 5.45 (m, 1H), 4.66 (d, *J =* 8 Hz, 1H), 4.58 - 4.39 (m, 2H), 4.38 - 4.25 (m, 2H), 4.21 (d, *J =* 4 Hz, 1H), 4.10 - 3.88 (m, 1H), 3.84 (s, 3H), 3.44 (d, *J* = 16 Hz, 2H), 3.35 (s, 2H), 3.24 - 2.87 (m, 6H), 2.72 (s, 2H), 2.62 - 2.39 (m, 1H), 2.37 - 2.23 (m, 1H), 2.21 - 1.96 (m, 3H), 1.00 (s, 2H), 0.87 (s, 2H).

### Example 341

### Step 1: Preparation of cyclopropyl-1,1-di(methyl-d2-ol)

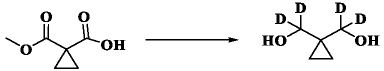

1-(methylformate)cyclopropyl-1-carboxylic acid (1 g, 6.94 mmol) was dissolved in THF (50 mL). The obtained mixture was subjected to nitrogen replacement, then cooled to 0°C. Deuterated lithium aluminum hydride (20 mL, 20 mmol, 1 M) was slowly added dropwise. The reaction liquid was stirred at 0°C for 5 h. Sodium sulfate decahydrate was slowly added to the reaction liquid in portions to quench the reaction. Then, the obtained mixture was further stirred for 2 h, and filtered, and the filter cake was washed with THF. The organic phase was concentrated to obtain cyclopropyl-1,1-di(methyl-d2-ol) (620 mg).

### Step 2: Preparation of (1-(((tert-butyldiphenylsilyl)oxo)methyl-d2)cyclopropyl)methyl-d2-ol

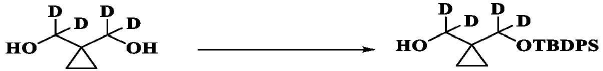

Cyclopropyl-1,1-di(methyl-d2-ol) (600 mg, 5.65 mmol) and TEA (580 mg, 5.73 mmol) were added to dichloromethane (50 mL). After mixing evenly, tert-butyl diphenylchlorosilane (780 mg, 2.84 mmol) was added at room temperature, and the reaction was continued for 16 h. Saturated ammonium chloride solution was added to the reaction liquid, and the obtained mixture was extracted with ethyl acetate. The organic phase was dried over anhydrous sodium sulfate, filtered, concentrated, and purified by flash silica gel column chromatography to obtain (1-(((tert-butyldiphenylsilyl)oxo)methyl-d2)cyclopropyl)methyl-d2 ol (1.6 g, yield 82.1%).

### Step 3: Preparation of tert-butyl (SS,SaS,6S,9R)-12-((1-((tert-butyldiphenylsilyl)oxy)methyl-d2)cyclopropyl)methoxy-d2)-2-chloro-1-fluoro-5-(methyl-d3)-5a,6,7,8,9,10-hexahydro-5H-4-oxa-3,10a,11,13,14-pentyl-6,9-methylnaphtho[1,8-ab]heptene-4-carboxylate

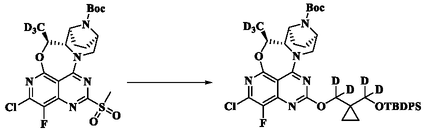

Tert-butyl (5S,5aS,6S,9R)-2-chloro-1-fluoro-5-(methyl-d3)-12-(methylsulfonyl)-5a,6,7,8,9,10-hexahydro-5H-4-oxa-3,10a,11,13,14-pentaaza-6,9-methylnaphtho[1,8-ab]heptene-14-carboxylate (1.55 g, 3.0 mmol) and (1-(((tert-butyldiphenylsilyl)oxo)methyl-d2)cyclopropyl)methyl-d2 ol (1.5 g, 4.35 mmol) were dissolved in tetrahydrofuran (80 mL). Nitrogen replacement was performed, and lithium bis(trimethylsilyl)amide (1 M, 8.8 mL) was added dropwise under ice bath. The obtained mixture was reacted under ice bath for 30 min. A saturated ammonium chloride solution was added to quench the reaction, and the obtained mixture was extracted with ethyl acetate. The organic phase was dried over anhydrous sodium sulfate, filtered, concentrated, and purified by silica gel column chromatography to obtain tert-butyl (SS,SaS,6S,9R)-12-((1-((tert-butyldiphenylsilyl)oxy)methyl-d2)cyclopropyl)methoxy-d2)-2-chloro-1-fluoro-5-(methyl-d3)-5a,6,7,8,9,10-hexahydro-5H-4-oxa-3,10a,11,13,14-pentyl-6,9-methylnaphtho[1,8-ab]heptene-4-carboxylate (1.8 g, yield 76.9%).

### Step 4: Preparation of tert-butyl (5S,5aS,6S,9R)-2-chloro-1-fluoro-12-((1-(hydroxymethyl-d2)cyclopropyl)methoxy-d2)-5-(methyl-d3)-5a,6,7,8,9,10-hexahydro-5H-4-oxa-3,10a,11,13,14-pentaaza-6,9-methylnaphtho[1,8-ab]heptene-14carboxylate

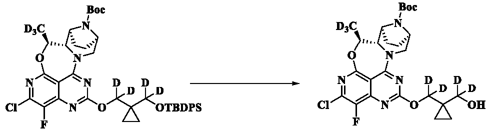

In an eggplant-shaped flask, tert-butyl (SS,SaS,6S,9R)-12-((1-((tert-butyldiphenylsilyl)oxy)methyl-d2)cyclopropyl)methoxy-d2)-2-chloro-1-fluoro-5-(methyl-d3)-5a,6,7,8,9,10-hexahydro-5H-4-oxa-3,10a,11,13,14-pentyl-6,9-methylnaphtho[1,8-ab]heptene-4-carboxylate (1.8 g, 2.30 mmol) was dissolved in tetrahydrofuran (50 mL), a solution of tetrabutylammonium fluoride in tetrahydrofuran (1 M, 5 mL) was added, and then the obtained mixture was reacted at room temperature for 3 h. Water was added to the reaction liquid and the obtained mixture was extracted with ethyl acetate. The organic phase was dried over anhydrous sodium sulfate, filtered, concentrated, and purified by silica gel column chromatography to obtain tert-butyl (5S,5aS,6S,9R)-2-chloro-1-fluoro-12-((1-(hydroxymethyl-d2)cyclopropyl)methoxy-d2)-5-(methyl-d3)-5a,6,7,8,9,10-hexahydro-5H-4-oxa-3,10a,11,13,14-pentaaza-6,9-methylnaphtho[1,8-ab]heptene-14carboxylate (1.05 g, yield 84.0%).

MS m/z(ESI):543 [M+H].

### Step 5: Preparation of tert-butyl (5S,5aS,6S,9R)-2-chloro-1-fluoro-5-(methyl-d3)-12-((1-(methylsulfonyl)oxy)methyl-d2)cyclopropyl)methoxy-d2)-5a,6,7,8,9,10-hexahydro-5H-4-oxa-3,10a,11,13,14-pentaaza-6,9-methylnaphtho[1,8-ab]heptene-14carboxylate

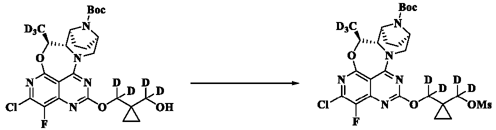

In an eggplant-shaped flask, tert-butyl (5S,5aS,6S,9R)-2-chloro-1-fluoro-12-((1-(hydroxymethyl-d2)cyclopropyl)methoxy-d2)-5-(methyl-d3)-5a,6,7,8,9,10-hexahydro-5H-4-oxa-3,10a,11,13,14-pentaaza-6,9-methylnaphtho[1,8-ab]heptene-14carboxylate (1.0 g, 1.84 mmol) was dissolved in dichloromethane (20 mL), N,N-diisopropylethylamine (950 mg, 7.36 mmol) and methylsulfonyl chloride (420 mg, 3.67 mmol) were added under ice bath, and the obtained mixture was reacted at room temperature for 1 h. Saturated sodium bicarbonate solution was used to quench the reaction, and dichloromethane was used for extraction; the organic phase was dried over anhydrous sodium sulfate, filtered, and concentrated to obtain crude tert-butyl (5S,5aS,6S,9R)-2-chloro-1-fluoro-5-(methyl-d3)-12-((1-(methylsulfonyl)oxy) methyl-d2)cyclopropyl)methoxy-d2)-5a,6,7,8,9,10-hexahydro-5H-4-oxa-3,10a,11,13,14-pentaaza-6,9-methylnaphtho[1,8-ab]heptene-14carboxylate (1.2 g).

### Step 6: Preparation of tert-butyl (5S,5aS,6S,9R)-2-chloro-1-fluoro-12-((1-((4-(methoxyimino)piperidin-1-yl)methyl-d2)cyclopropyl)methoxy-d2)-5-(methyl-d3)-5a,6,7,8,9,10-hexahydro-5H-4-oxa-3,10a,11,13,14-pentaaza-6,9-methylnaphtho[1,8-ab]heptene-14carboxylate

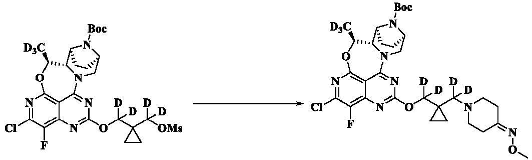

N-methoxypiperidin-4-imine trifluoroformate (1 g, 5.74 mmol), tert-butyl (5S,5aS,6S,9R)-2-chloro-1-fluoro-5-(methyl-d3)-12-((1-(methylsulfonyl)oxy) methyl-d2)cyclopropyl)methoxy-d2)-5a,6,7,8,9,10-hexahydro-5H-4-oxa-3,10a,11,13,14-pentaaza-6,9-methylnaphtho[1,8-ab]heptene-14carboxylate (1.2 g, 1.84 mmol), N,N-diisopropylethylamine (1.2 g, 9.3 mmol) and sodium iodide (830 mg, 5.54 mmol) were dissolved in acetonitrile (50 mL). The obtained mixture was subjected to nitrogen replacement, heated to 55°C and reacted for 16 h. The reaction liquid was cooled to room temperature, water was added, and the obtained mixture was extracted with ethyl acetate. The organic phase was dried with anhydrous sodium sulfate, filtered, concentrated, and purified by silica gel column chromatography to obtain tert-butyl (5S,5aS,6S,9R)-2-chloro-1-fluoro-12-((1-((4-(methoxyimino)piperidin-1-yl)methyl-d2)cyclopropyl)methoxy-d2)-5-(methyl-d3)-5a,6,7,8,9,10-hexahydro-5H-4-oxa-3,10a,11,13,14-pentaaza-6,9-methylnaphtho [1,8-ab]heptene-14carboxylate (980 g, yield 81.7%).

MS m/z(ESI):653 [M+H].

### Step 7: Preparation of tert-butyl (5S,5aS,6S,9R)-2-(5-amino-3-chloro-2-(trifluoromethyl)phenyl)-1-fluoro-12-((1-((4-(methoxyimino)piperidin-1-yl)methyl-d2)cyclopropyl)methoxy-d2)-5-(methyl-d3)-5a,6,7,8,9,10-hexahydro-5H-4-oxo-3,10a,11,13,14-pentaaza-6,9-methylnaphtho[1,8-ab]heptene-14carboxylate

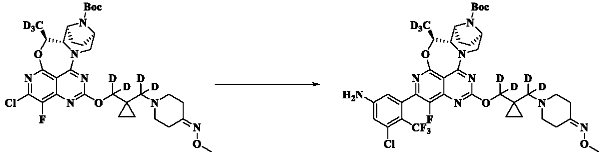

3-chloro-5-(4,4,5,5-tetramethyl-1,3,2-dioxaboran-2-yl)-4-(trifluoromethyl)aniline (370 mg, 1.15 mmol), tert-butyl (SS,SaS,6S,9R)-2-chloro-1-fluoro-12-((1-((4-(methoxyimino)piperidin-1-yl)methyl-d2)cyclopropyl)methoxy -d2)-5-(methyl-d3)-5a,6,7,8,9,10-hexahydro-5H-4-oxa-3,10a,11,13,14-pentaaza-6,9-methylnaphtho[1,8-ab]heptene-14carboxylate (500 mg, 0.77 mmol), tetrakis(triphenylphosphine)palladium (179 mg, 0.155 mmol), and cesium carbonate (760 mg, 2.33 mmol) were added to dioxane/water (5 mL/1 mL). Nitrogen replacement was performed and the reaction was carried out under microwave at 120°C for 1 h. The reaction liquid was cooled, water was added, and the obtained mixture was extracted with ethyl acetate. The organic phase was dried over anhydrous sodium sulfate, filtered, concentrated, and purified by silica gel chromatography to obtain tert-butyl (5S,5aS,6S,9R)-2-(5-amino-3-chloro-2-(trifluoromethyl)phenyl)-1-fluoro-12-((1-((4-(methoxyimino)piperidin-1-yl)methyl-d2)cyclopropyl)methoxy-d2)-5-(methyl-d3)-5a,6,7,8,9,10-hexahydro-5H-4-oxo-3,10a,11,13,14-pentaaza-6,9-methylnaphtho[1,8-ab]heptene-14carboxylate (350 g, yield 56.0%).

MS m/z(ESI):812 [M+H].

### Step 8: Preparation of 1-((1-((((5S,5aS,6S,9R)-2-(5-amino-3-chloro-2-(trifluoromethyl)phenyl)-1-fluoro-5-(methyl-d3)-5a,6,7,8,9,10-hexahydro-5H-4-oxa-3,10a,11,13,14-pentaaza-6,9-methylnaphtho[1,8-ab]heptene-12-yl)oxy)methyl-d2)cyclopropyl)methyl-d2)piperidin-4-one O-methyloxime

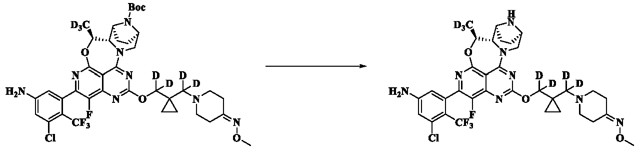

In an eggplant-shaped flask, tert-butyl (5S,5aS,6S,9R)-2-(5-amino-3-chloro-2-(trifluoromethyl)phenyl)-1-fluoro-12-((1-((4-(methoxyimino)piperidin-1-yl)methyl-d2)cyclopropyl)methoxy-d2)-5-(methyl-d3)-5a,6,7,8,9,10-hexahydro-5H-4-oxo-3,10a,11,13,14-pentaaza-6,9-methylnaphtho[1,8-ab]heptene-14carboxylate (350 mg, 0.43 mmol) was dissolved in dichloromethane (20 mL). Trifluoroacetic acid (3 mL) was added under ice bath and the obtained mixture was gradually warmed to room temperature and reacted for half an hour. The reaction liquid was concentrated and purified by high performance liquid column chromatography to obtain 1-((1-((((5S,5aS,6S,9R)-2-(5-amino-3-chloro-2-(trifluoromethyl)phenyl)-1-fluoro-5-(methyl-d3)-5a,6,7,8,9,10-hexahydro-5H-4-oxa-3,10a,11,13,14-pentaaza-6,9-methylnaphtho[1,8-ab]heptene-12-yl)oxy)methyl-d2)cyclopropyl)methyl-d2)piperidine-4-one O-methyloxime (130 mg, yield 42.2%).

MS m/z(ESI):712[M+H].

¹H NMR (400 MHz, MeOD) δ 6.92 (d, *J* = 2.4 Hz, 1H), 6.47 (d, *J* = 57.2 Hz, 1H), 5.52 - 5.47 (m, 1H), 4.66 (t, *J =* 7.4 Hz, 1H), 4.45 (s, 2H), 4.34 - 4.31 (m, 1H), 4.29 (s, 1H), 4.25 - 4.20 (m, 1H), 3.83 - 3.79 (m, 1H), 3.45 - 3.40 (m, 1H), 3.36 - 3.20 (m, 6H), 2.70 (s, 2H), 2.36 - 2.21 (m, 1H), 2.20 - 2.05 (m, 1H), 1.30 - 1.25 (m, 2H), 0.99 - 0.94 (m, 2H), 0.92 - 0.84 (m, 2H).

### Example 342

### Step 1: Preparation of tert-butyl 8-(methoxyimino)-5-azaspiro[2.5]octane-5-carboxylate

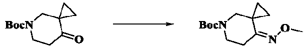

In a round-bottom flask, tert-butyl 8-oxo-5-azaspiro[2.5]octane-5-carboxylate (2 g, 8.88 mmol) and methoxyamine hydrochloride (1.11 g, 13.3 mmol) were dissolved in methanol (30 mL), and potassium carbonate (2.82 g, 26.6 mmol) was added. After nitrogen replacement, the reaction liquid was stirred at 70°C for 1 h. After the addition was completed, the obtained mixture was concentrated directly and purified by silica gel column chromatography to obtain tert-butyl 8-(methoxyimino)-5-azaspiro[2.5]octane-5-carboxylate (1.9 g, yield 84%).

MS m/z (ESI): 255 [M+H].

### Step 2: Preparation of 5-azaspiro[2.5]octan-8-one O-methyloxime

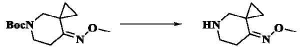

In a round-bottom flask, tert-butyl 8-(methoxyimino)-5-azaspiro[2.5]octane-5-carboxylate (300 mg, 1.18 mmol) was dissolved in dichloromethane (6 mL) and trifluoroacetic acid (2 mL) was added dropwise. The reaction liquid was stirred at 25°C for 1 h. After the addition was completed, the reaction liquid was concentrated to obtain 5-azaspiro[2.5]octan-8-one O-methyloxime (308 mg, yield 99%) which was directly used in the next step.

MS m/z (ESI): 155 [M+H].

### Step 3: Preparation of tert-butyl (5S,5aS,6S,9R)-2-(8-ethynyl-7-fluoro-3-(methoxymethoxy)naphthalen-1-yl)-1-fluoro-12-((1-((8-(methoxyimino)-5-azaspiro[2.5]oct-5-yl)methyl)cyclopropyl)methoxy-5-methyl-5a,6,7,8,9,10-hexahydro-5H-4-oxa-3,10a,11,13,14-pentaaza-6,9-methylnaphtho[1,8-ab]heptene-4-carboxylate

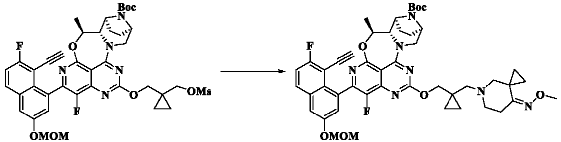

In a round-bottom flask, tert-butyl (5S,5aS,6S,9R)-2-(8-ethynyl-7-fluoro-3-(methoxymethoxy)naphthalen-1-yl)-1-fluoro-5-methyl-12-((1-((methylsulfonyl) oxy)methyl)-cyclopropyl)methoxy]-5a,6,7,8,9,10-hexahydro-5H-4-oxa-3,10a,11,13,14-pentaaza-6,9-methylnaphtho[1,8-ab]heptene-4-carboxylate (332 mg, 0.41 mmol) and (Z)-5-azaspiro[2.5]oct-8-one O-methyloxime (308 mg, 1.15 mmol) were dissolved in acetonitrile (30 mL). N,N-diisopropylethylamine (531 mg, 4.1 mmol) and sodium iodide (185 mg, 1.23 mmol) were added. After nitrogen replacement, the reaction liquid was stirred at 55°C for 13 h. After the addition was completed, the reaction liquid was cooled to room temperature, water was added to the reaction liquid, and the obtained mixture was extracted with ethyl acetate. The organic phase was washed with an ammonium chloride aqueous solution and then with saturated saline, dried over anhydrous sodium sulfate, filtered, and purified by silica gel column chromatography to obtain tert-butyl (SS,SaS,6S,9R)-2-(8-ethynyl-7-fluoro-3-(methoxymethoxy)naphthalen-1-yl)-1-fluoro-12-((1-((8-(methoxyimino)-5-azaspiro[2.5]oct-5-yl)methyl)cyclopropyl)methoxy-5-methyl-5a,6,7,8,9,10-hexahydro-5H-4-oxa-3,10a,11,13,14-pentaaza-6,9-methylnaphtho [1,8-ab]heptene-4-carboxylate (300 mg, yield 84%).

MS m/z (ESI): 866 [M+H].

### Step 4: Preparation of 5-((1-((((5S,5aS,6S,9R)-2-(8-ethynyl-7-fluoro-3-hydroxynaphthalen-1-yl)-1-fluoro-5-methyl-5a,6,7,8,9,10-hexahydro-5H-4-oxa-3,10a,11,13,14-pentaaza-6,9-methylnaphtho[1,8-ab]heptene-12-yl)oxy)methyl)cyclopropyl)methyl)-5-azaspiro[2.5]octan-8-one O-methyloxime

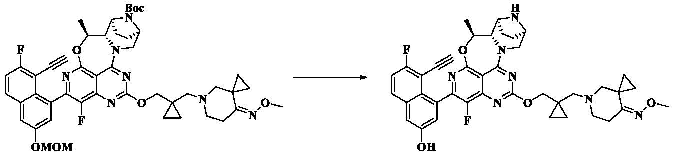

In a round-bottom flask, tert-butyl (5S,5aS,6S,9R)-2-(8-ethynyl-7-fluoro-3-(methoxymethoxy)naphthalen-1-yl)-1-fluoro-12-((1-((8-(methoxyimino)-5-azaspiro[2.5]oct-5-yl)methyl)cyclopropyl)methoxy-5-methyl-5a,6,7,8,9,10-hexahydro-5H-4-oxa-3,10a,11,13,14-pentaaza-6,9-methylnaphtho[1,8-ab]heptene-4-carboxylate (300 mg, 0.35 mmol) was dissolved in methanol (0.8 mL) and dichloromethane (4 mL), and a solution of hydrochloric acid in dioxane (4 M, 4 mL) was added dropwise under ice bath. The reaction liquid was stirred at 25°C for 30 min. The reaction liquid was slowly added to a sodium bicarbonate aqueous solution (100 mL), and the obtained mixture was extracted with dichloromethane/methanol = 10/1, and the organic phase was washed with saturated sodium chloride aqueous solution, dried over anhydrous sodium sulfate, filtered, concentrated, purified by high performance liquid column chromatography and lyophilized to obtain 5-((1-((((5S,5aS,6S,9R)-2-(8-ethynyl-7-fluoro-3-hydroxylnaphthalen-1-yl)-1-fluoro-5-methyl-5a,6,7,8,9,10-hexahydro-5H-4-oxa-3,10a,11,13,14-pentaaza-6,9-methylnaphtho[1,8-ab]heptene-12-yl)oxy)methyl)cyclopropyl)methyl)-5-azaspiro[2.5]octan-8-one O-methyloxime (143 mg, yield 51%).

MS m/z (ESI): 722 [M+H].

¹H NMR (400 MHz, MeOD) δ 7.89 - 7.76 (m, 1H), 7.35 - 7.25 (m, 2H), 7.17 (d, *J =* 44 Hz, 1H), 5.48 - 5.37 (m, 1H), 4.62 - 4.35 (m, 3H), 4.15 (d, *J =* 8 Hz, 1H), 3.90 - 3.75 (m, 3H), 3.73 (s, 3H), 3.27 - 3.18 (m, 1H), 3.03 - 2.83 (m, 2H), 2.83 - 2.55 (m, 6H), 2.25 - 2.05 (m, 1H), 2.02 - 1.74 (m, 3H), 1.59 (t, *J =* 4 Hz, 3H), 1.09 - 0.95 (m, 2H), 0.85 - 0.67 (m, 4H), 0.65 - 0.50 (m, 2H).

### Example 345

### Step 1: Preparation of tert-butyl 5-(methoxyimino)-2-azabicyclo[2.2.2]octane-2-carboxylate

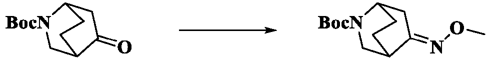

In a round-bottom flask, tert-butyl 5-oxo-2-azabicyclo[2.2.2]octane-2-carboxylate (2 g, 8.88 mmol) and methoxyamine hydrochloride (1.48 g, 17.76 mmol) was dissolved in methanol (20 mL), and potassium carbonate (2.82 g, 26.63 mmol) was added. After nitrogen replacement, the reaction liquid was stirred at 70°C for 1 h. After the addition was completed, the obtained mixture was filtered through diatomaceous earth, and the filter cake was washed with dichloromethane. The filtrate was collected, concentrated, and purified by silica gel column chromatography to obtain tert-butyl 5-(methoxyimino)-2-azabicyclo[2.2.2]octane-2-carboxylate (1.9 g, yield 84%).

MS m/z (ESI): 255 [M+H].

### Step 2: Preparation of 2-azabicyclo[2.2.2]oct-5-one-O-methyloxime

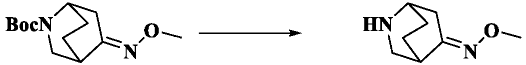

In a round-bottom flask, tert-butyl 5-(methoxyimino)-2-azabicyclo[2.2.2]octane-2-carboxylate (0.3 g, 1.18 mmol) was dissolved in dichloromethane (3 mL), and 1 mL of trifluoroacetic acid was added dropwise. The reaction liquid was stirred at 25°C for 1 h. After the reaction was completed, the reaction liquid was concentrated to obtain 2-azabicyclo[2.2.2]oct-5-one-O-methyloxime (0.3 g) which can be used directly in the next reaction.

MS m/z (ESI): 155 [M+H].

### Step 3: Preparation of tert-butyl (5S,5as,6S,9R)-2-(8-ethynyl-7-fluoro-3-(methoxymethoxy)naphthalen-1-yl)-1-fluoro-12-((1-((5-(methoxyimino)-2-azabicyclo[2.2.2]oct-2-yl)methyl)cyclopropyl)methoxy-5-methyl-5a,6,7,8,9,10-hexahydro-5H-4-oxa-3,10a,11,13,14-pentaaza-6,9-methylnaphtho[1,8-ab]heptene-4-carboxylate

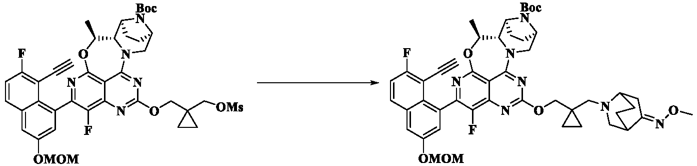

In a round-bottom flask, tert-butyl (5S,5as,6S,9R)-2-(8-ethynyl-7-fluoro-3-(methoxymethoxy)naphthalen-1-yl)-1-fluoro-S-methyl-12-((1-((methylsulfonyl) oxy)methyl)-cyclopropyl)methoxy]-5a,6,7,8,9,10-hexahydro-5H-4-oxa-3,10a,11,13,14-pentaaza-6,9-methylnaphtho[1,8-ab]heptene-4-carboxylate (330 mg, 0.41 mmol) and 2-azabicyclo[2.2.2]octan-5-one-O-methyloxime (0.18 g, 1.17 mmol) were dissolved in acetonitrile (7 mL). Triethylamine (0.4 mL, 2.04 mmol) and sodium iodide (0.15 g, 1.23 mmol) were added. After nitrogen replacement, the reaction liquid was stirred at 55°C for 16 h. After the addition was completed, the reaction liquid was cooled to room temperature, quenched with saturated ammonium chloride solution, extracted with ethyl acetate, and the combined organic phases were washed with water and sodium chloride aqueous solution, dried over anhydrous sodium sulfate, filtered, concentrated, and purified by silica gel column chromatography to obtain tert-butyl (5S,5as,6S,9R)-2-(8-ethynyl-7-fluoro-3-(methoxymethoxy)naphthalen-1-yl)-1-fluoro-12-((1-((5-(methoxyimino)-2-azabicyclo[2.2.2]oct-2-yl)methyl)cyclopropyl)methoxy-5-methyl-5a,6,7,8,9,10-hexahydro-5H-4-oxa-3,10a,11,13,14-pentaaza-6,9-methylnaphtho[1,8-ab]heptene-4-carboxylate (300 mg, yield 84%).

MS m/z (ESI): 866 [M+H].

### Step 4: Preparation of 2-((1-((((5S,5aS,6S,9R)-2-(8-ethyl-7-fluoro-3-hydroxynaphthalen-1-yl)-1-fluoro-5-methyl-5a,6,7,8,9,10-hexahydro- SH-4-oxo-3,10a,11,13,14-pentaaza-6,9-methylnaphtho[1,8-ab]heptene-12-yloxy)methyl) cyclopropyl)methyl)-2-azacyclo[2.2.2]octane-5-1-O-methyloxime

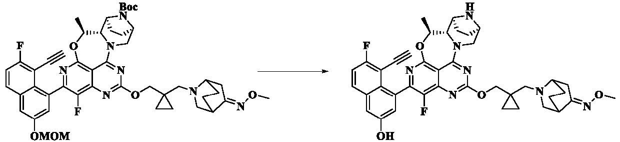

In a round-bottom flask, tert-butyl (5S,5as,6S,9R)-12-((1-((5-(difluoromethylene)hexahydrocyclopenta[c]pyrrole-2(1H)-yl)methyl)cyclopropyl) methoxy)-2-(8-ethynyl-7-fluoro-3-(methoxymethoxynaphthalen-1-yl)-1-fluoro-5-methyl-5a,6,7,8,9,10-hexahydro-5H-4-oxa-3,10a, 11, 13,14-pentaaza-6,9-methylnaphtho[1,8-ab]heptene-4-carboxylate (300 mg, 0.35 mmol) was dissolved in 1 mL of methanol and 4 mL of dichloromethane, and a solution of hydrochloric acid in dioxane (4 M, 4 mL) was added dropwise under ice bath. The reaction liquid was stirred at 25°C for 1 h. After the addition was completed, the reaction liquid was cooled to 0°C, saturated sodium bicarbonate solution was added dropwise to adjust the pH > 7, and the obtained mixture was extracted with a mixed solution of dichloromethane and methanol (10/1), dried over anhydrous sodium sulfate, filtered, concentrated, purified by high performance liquid column chromatography, and lyophilized to obtain 2-((1-((((5S,5aS,6S,9R)-2-(8-ethyl-7-fluoro-3-hydroxylnaphthalen-1-yl)-1-fluoro-5-methyl-5a,6,7,8,9,10-hexahydro- 5H -4-oxo-3,10a,11,13,14-pentaza-6,9-methylnaphtho[1,8-ab]heptene-12-yloxy)methyl) cyclopropyl)methyl)-2-azacyclo[2.2.2]octane-5- 1- o-methyloxime (50 mg, yield 20%).

MS m/z (ESI): 722 [M+H].

¹H NMR (400 MHz, MeOD) δ 7.87 - 7.77 (m, 1H), 7.37 - 7.28 (m, 2H), 7.19 (d, *J =* 44 Hz, 1H), 5.46 - 5.39 (m, 1H), 4.65 - 4.39 (m, 3H), 4.18 (m, 1H), 3.87 - 3.76 (m, 3H), 3.68 (s, 3H), 3.29 - 3.14 (m, 1H), 3.05 - 2.86 (m, 2H), 2.76 - 2.46 (m, 6H), 2.13 - 1.85 (m, 4H), 1.76 - 1.59 (m, 3H), 1.29 - 1.02 (m, 2H), 0.89 - 0.73 (m, 4H), 0.66 - 0.55 (m, 2H).

**Example 351 refers to the synthesis of example 1-1:**

| **Serial No.** | **Example structure** | **MS m/z (ESI)** |
|---|---|---|
| **351** | 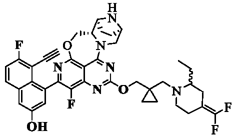 | 714 [M+H] |

**Examples 352 to 379 refer to the synthesis of example 337:**

| **Serial No.** | **Example structure** | **MS m/z (ESI)** | **Serial No.** | **Example structure** | **MS m/z (ESI)** |
|---|---|---|---|---|---|
| **352** | 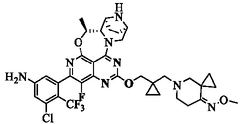 | 731 [M+H] | **353** | 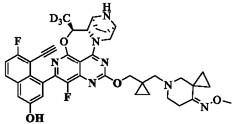 | 725 [M+H] |
| **354** | 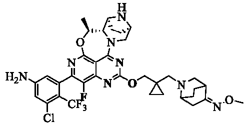 | 731 [M+H] | **355** | 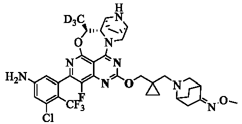 | 734 [M+H] |
| **356** | 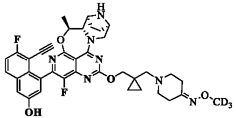 | 699 [M+H] | **357** | 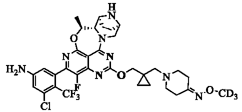 | 708 [M+H] |
| **358** | 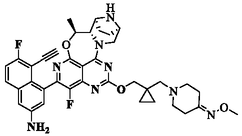 | 695 [M+H] | **359** | 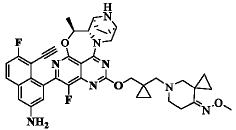 | 721 [M+H] |
| **360** | 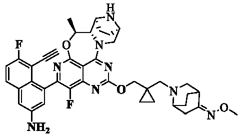 | 721 [M+H] | **361** | 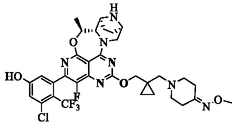 | 706 [M+H] |
| **362** | 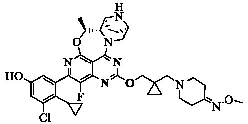 | 678 [M+H] | **363** | 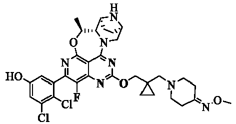 | 672 [M+H] |
| **364** | 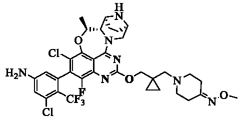 | 738 [M+H] | **365** | 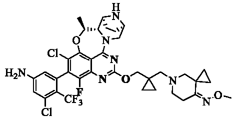 | 764 [M+H] |
| **366** | 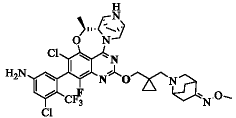 | 764 [M+H] | **367** | 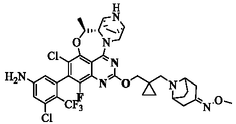 | 764 [M+H] |
| **368** | 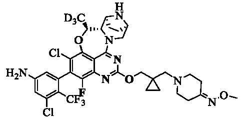 | 741 [M+H] | **369** | 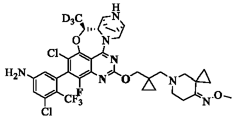 | 767 [M+H] |
| **370** | 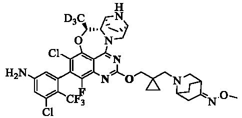 | 767 [M+H] | **371** | 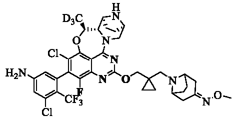 | 767 [M+H] |
| **372** | 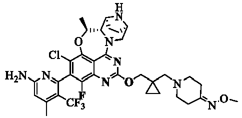 | 719 [M+H] | **373** | 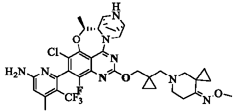 | 745 [M+H] |
| **374** | 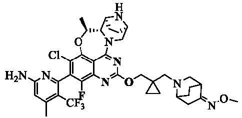 | 745 [M+H] | **375** | 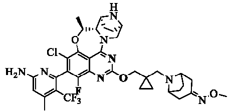 | 745 [M+H] |
| 376 | 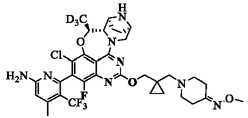 | 722 [M+H] | **377** | 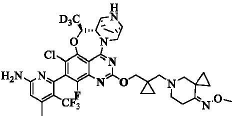 | 748 [M+H] |
| 378 | 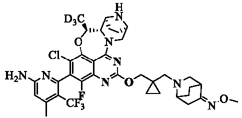 | 748 [M+H] | **379** | 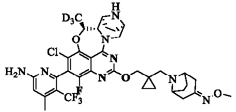 | 748 [M+H] |

**Examples 380 to 391 refer to the synthesis of example 341:**

| **Serial No.** | **Example structure** | **MS m/z (ESI)** | **Serial No.** | **Example structure** | **MS m/z (ESI)** |
|---|---|---|---|---|---|
| **380** | 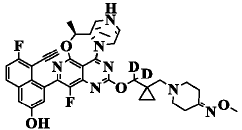 | 698 [M+H] | **381** | 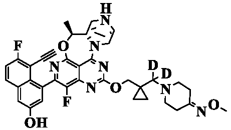 | 698 [M+H] |
| **382** | 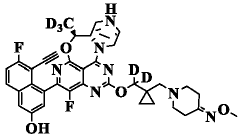 | 701 [M+H] | **383** | 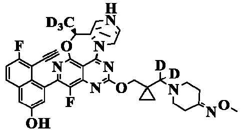 | 701 [M+H] |
| **384** | 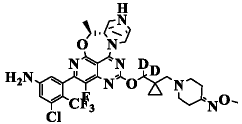 | 707 [M+H] | **385** | 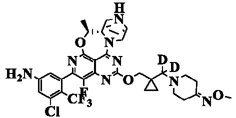 | 707 [M+H] |
| **386** | 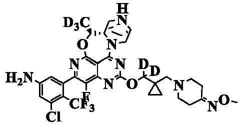 | 710 [M+H] | **387** | 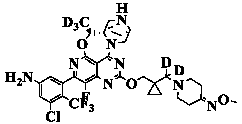 | 710 [M+H] |
| **388** | 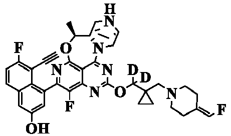 | 685 [M+H] | **389** | 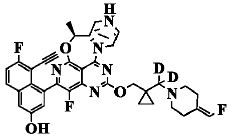 | 685 [M+H] |
| **390** | 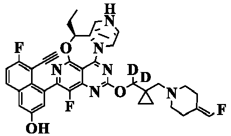 | 699 [M+H] | **391** | 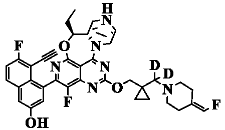 | 699 [M+H] |

### Biological test and evaluation

The present invention will be further described and explained below in conjunction with test examples, but these examples are not meant to limit the scope of the present invention.

### I. Determination of the activity of the compounds of the present invention in blocking KRAS G12D-SOS1 protein binding

1. Experimental objective: The purpose of this test example is to test the ability of the compounds to block the binding of KRAS G12D and SOS1 proteins.
2. Experimental reagents and instruments

### 2.1 Experimental instruments:

The centrifuge (5810R) was purchased from Eppendorf; the pipette was purchased from Eppendorf or Rainin; and the microplate reader was purchased from BioTek (United States) with a model of SynergyH1 multifunctional microplate reader.

### 2.2 Experimental reagents:

KRAS-G12D/SOS1 Binding Assay Kit was purchased from Cisbio, with a catalog number of 63ADK000CB17PEG; GTP was purchased from Sigma, with a catalog number of G8877; 384-well plate was purchased from Perkin Elmer, with a catalog number of 6007299.

### 3. Experimental method:

The Diluent buffer in the kit was used to dilute the compound into 10× compound solution of different concentrations, and 2 uL was added to each well of the 384-well plate. Tag2-KRAS G12D protein and GTP were formulated into a mixed solution of 5×KRAS G12D-GTP protein using Diluent buffer, and Tag1-SOS1 protein was formulated into a 5×SOS1 protein solution using Diluent buffer. Each solution was added to the 384-well plate separately at 4 uL per well, and incubated at room temperature for 15 min. A mixed solution of 2× anti-Tag1-Tb³⁺ and anti-Tag2-XL665 formulated using detection buffer was added to the 384-well plate at 10 µL per well, and incubated at 4°C in the dark for 3 h. The plate was read using the time-resolved fluorescence program of the Biotek Synergy H1 instrument to detect fluorescence values at emission wavelengths of 665 nm and 620 nm.

### 4. Experimental processing method:

The signal ratio (665nm/620nm*10,000) was calculated, and the nonlinear fitting of the signal ratio and sample concentration was performed using a fourparameter equation in GraphPad Prism 6 to obtain the IC₅₀ value.

### 5. Experimental results:

**Table 1: Activity of compounds in blocking KRAS G12D-SOS1 protein binding**

| **Compound No.** | **G12D SOS1 biochemical IC₅₀ (nM)** | **KRAS WT Biochemical IC₅₀ (nM)** |
|---|---|---|
| Example 1-1 | 1.8 | 79 |
| Example 3-1 | 2.2 | 62 |
| Example 3-2 | 3.9 | 40 |
| Example 14-1 | 4.3 | 153 |
| Example 15-1 | 3.0 | 26 |
| Example 17-1 | 2.6 | 58 |
| Example 21-1 | 1.9 | 60 |

### 6. Experimental conclusion: The compounds of the present invention can effectively block the binding of KRAS G12D and SOS1 protein.

### II. Determination of the inhibition activity of the compounds of the present invention on the proliferation of KRAS G12D mutant tumor cell lines

1. Experimental objective: The inhibition activity of the example compounds on the proliferation of 5 KRAS G12D mutant cell lines SNU-1, HPAF-II, Panc0403, AsPC-1 and GP2D was determined.
2. Experimental instruments:
2.1 Instruments: Microplate reader (BioTek Synergy H1); Pipette (Eppendorf & Rainin).
2.2 Reagents: HPAF-II, PANC0403, AsPC-1 and GP2D were purchased from COBIOER BIOSCIENCES CO., LTD; SNU-1 was purchased from the cell bank of Chinese Academy of Sciences, Cell Titer-Glo was purchased from Promega, with a catalog number of G7573; RPMI 1640 was purchased from Gibco, with a catalog number of 22400089; DMEM was purchased from Gibco, with a catalog number of 11995065; FBS was purchased from Gibco, with a catalog number of 10091148; PBS was purchased from Gibco, with a catalog number of 10010023; trypsin was purchased from Gibco, with a catalog number of 25200056; cell culture plates were purchased from Corning Company, with a catalog number of 3610.

3. Experimental method: When SNU-1, HPAF-II, Panc0403, AsPC-1 or GP2D cells were cultured to the appropriate confluence, and the SNU-1, HPAF-II, Panc0403, AsPC-1 or GP2D cells were collected. The cells were adjusted to the appropriate cell density using complete culture medium, the cell suspension was plated onto a 96-well plate at 90 µL per well, and the plate was placed in the incubator at 37°C and 5% CO₂ for adherent culture overnight. Solutions of the compound with different concentrations were formulated using DMSO and culture medium. A vehicle control was set up. The solutions of the compound were added to the 96-well plate at 10 µL per well, and the plate was placed in the incubator at 37°C and 5% CO₂ for further culture for about 72 h. Then, CellTiter-Glo solution was added, and the obtained mixture was shaken and mixed evenly, incubated in the dark for 10 min, and read with a BioTek Synergy H1 microplate reader.
4. Experimental data processing method: The luminescence signal value was used to calculate the inhibition rate. Graphpad Prism software was used to fit the concentration and inhibition rate to a nonlinear regression curve, so as to obtain the IC₅₀ value.
5. Experimental results:

**Table 2: Determination of the inhibition activity of the compounds on the proliferation of KRAS G12D mutant tumor cell line GP2D**

| **Compound No.** | **GP2D IC₅₀ (nM)** | **Compound No.** | **GP2D IC₅₀ (nM)** |
|---|---|---|---|
| Example 1-1 | 1.20 | Example 162 | 0.20 |
| Example 3-1 | 1.00 | Example 164 | 0.32 |
| Example 3-2 | 1.20 | Example 167 | 0.39 |
| Example 14-1 | 1.50 | Example 168 | 0.5 |
| Example 15-1 | 0.76 | Example 172 | 0.59 |
| Example 17-1 | 1.20 | Example 285 | 0.16 |
| Example 19-1 | 0.16 | Example 286 | 0.32 |
| Example 21-1 | 0.75 | Example 289 | 0.28 |
| Example 35-1 | 0.21 | Example 290 | 0.30 |
| Example 51-1 | 1.30 | Example 291 | 0.15 |
| Example 89 | 0.9 | Example 292 | 0.21 |
| Example 90 | 0.51 | Example 298 | 0.28 |
| Example 91 | 0.28 | Example 321 | 1.50 |
| Example 124 | 0.98 | Example 322 | 0.61 |
| Example 129 | 0.17 | Example 323 | 0.86 |
| Example 130 | 0.33 | Example 324 | 0.73 |
| Example 131 | 0.17 | Example 325 | 0.84 |
| Example 132 | 0.24 | Example 330 | 0.17 |
| Example 133 | 0.53 | Example 337 | 0.11 |
| Example 134 | 1.00 | Example 338 | 0.34 |
| Example 137 | 0.41 | Example 339 | 0.10 |
| Example 140 | 0.79 | Example 341 | 0.10 |
| Example 141 | 0.94 | Example 342 | 0.41 |
| Example 161 | 0.24 | Example 345 | 0.18 |

**Table 3: Determination of the inhibition activity of the compounds on the proliferation of KRAS G12D mutant tumor cell line AsPC-1**

| **Compound No.** | **AsPC-1 IC₅₀ (nM)** | **Compound No.** | **AsPC-1 IC₅₀ (nM)** |
|---|---|---|---|
| Example 19-1 | 3.1 | Example 164 | 6.6 |
| Example 21-1 | 3.1 | Example 167 | 6.9 |
| Example 35-1 | 3.8 | Example 172 | 8.8 |
| Example 89 | 8.9 | Example 285 | 4.8 |
| Example 90 | 8.8 | Example 286 | 7.8 |
| Example 91 | 5.8 | Example 289 | 5.7 |
| Example 129 | 3.6 | Example 290 | 6.2 |
| Example 130 | 4.2 | Example 291 | 4.6 |
| Example 131 | 5.0 | Example 292 | 6.1 |
| Example 132 | 4.9 | Example 337 | 1.2 |
| Example 161 | 4.4 | Example 339 | 3.5 |
| Example 162 | 4.0 | Example 341 | 2.6 |

6. Experimental conclusion: The example compounds of the present invention have significant inhibition effects on the proliferation of SNU-1, HPAF-II, Panc0403, AsPC-1 and GP2D cells.

### III. Inhibition activity of the compounds of the present invention on p-ERK in AsPC-1 cells

1. Experimental objective: The inhibition activity of the example compounds on the phosphorylated ERK level in KRAS G12D mutant cells AsPC-1 was determined.
2. Experimental instruments:
2.1 Instruments: Microplate reader (BioTek Synergy H1); Pipette (Eppendorf & Rainin)
2.2 Reagents: Phosphorylated ERK1/2 (T202-Y204) LANCE Ultra Cellular Detection Kit was purchased from PerkinElmer, with a catalog number of TRF4000M; DMEM was purchased from Gibco, with a catalog number of 11995065; FBS was purchased from Gibco, with a catalog number of 10091148; PBS was purchased from Gibco, with a catalog number of 10010023; trypsin was purchased from Gibco, with a catalog number of 25200056; cell culture plates were purchased from Corning Company, with a catalog number of 3610; White opaque OptiPlate^{™}-384 plates were purchased from PerkinElmer, with a catalog number of 6007290.

3. Experimental method: When AsPC-1 cells were cultured to appropriate confluence, the AsPC-1 cells were collected, and the cell density was adjusted to 1 × 10⁶/mL using complete culture medium. The cell suspension was plated onto a 96-well plate at 50 µL per well, and the plate was placed in the incubator at 37°C and 5% CO₂ for adherent culture overnight. Solutions of the compound with different concentrations were formulated using DMSO and complete culture medium, and a vehicle control was set up. The solutions of the compound were added to the 96-well plate at 25 µL per well, and the plate was placed in the incubator at 37°C and 5% CO₂ for further culture for 2 h. Then, the supernatant of the cell culture plate was discarded, 50 µL of lysis buffer was added to each well, the cells were lysed at room temperature under shaking for 30 min, then placed in a centrifuge and centrifuged at 1000 rpm for 1 min. 15 µL of supernatant was transferred to a 384-well plate, 5 µL of detection mixture was added to each well (Eu-labeled anti-ERK1/2 (T202-Y204) Antibody at a final detection concentration of 0.5 nM and ULight labeled anti-ERK1/2 Antibody at a final detection concentration of 5 nM), centrifuged at 1000 rpm for 1 min, mixed evenly, and reacted at room temperature overnight. The plate was read using BioTek Synergy H1 and the signal values at the emission wavelengths of 620 nm and 665 nm were detected using a time-resolved fluorescence program.
4. Experimental data processing method: The ratio of the signal values at the emission wavelengths of 665 nm and 620 nm was calculated, and the inhibition rate was calculated using the ratio. Graphpad Prism software was used to fit the concentration and inhibition rate to a nonlinear regression curve, so as to obtain the IC₅₀ value.
5. Experimental results:

**Table 4: Inhibition activity of the compounds on p-ERK in AsPC-1 cells**

| Compound No. | AsPC-1_pERK IC50(nM) |
|---|---|
| Example 1-1 | 1.0 |
| Example 3-1 | 2.0 |
| Example 3-2 | 1.6 |
| Example 14 | 3.0 |
| Example 15 | 1.4 |
| Example 17 | 1.4 |
| Example 21 | 0.6 |

6. Experimental conclusion: The example compounds of the present invention have good inhibition effect on pERK in AsPC-1 cells.

### IV. Inhibition activity of the compounds of the present invention on p-ERK in AGS cells

1. Experimental objective: The inhibition activity of the example compounds on the phosphorylated ERK level in KRAS G12D mutant cells AGS was determined.
2. Experimental instruments:
2.1 Instruments: Envison; Pipette (Eppendorf & Rainin)
2.2 Reagents: AGS cells were purchased from COBIOER BIOSCIENCES CO., LTD, with a catalog number of CBP60476; Phosphorylated ERK1/2 (T202-Y204) LANCE Ultra Cellular Detection Kit was purchased from PerkinElmer, with a catalog number of TRF4000M; RPMI 1640 was purchased from Gibco, with a catalog number of 22400071; FBS was purchased from Gibco, with a catalog number of 10091148; PBS was purchased from Gibco, with a catalog number of 10010023; trypsin was purchased from Gibco, with a catalog number of 25200056; cell culture plates were purchased from Corning Company, with a catalog number of 3610; White opaque OptiPlate^{™}-384 plates were purchased from PerkinElmer, with a catalog number of 6007290.

3. Experimental method:
When AGS cells were cultured to appropriate confluence, the AGS cells were collected, and the cell density was adjusted to 4 × 105/mL using complete culture medium. The cell suspension was plated onto a 96-well plate at 100 µL per well, and the plate was placed in the incubator at 37°C and 5% CO₂ for adherent culture overnight. Solutions of the compound with different concentrations were formulated using DMSO and complete culture medium, and a vehicle control was set up. The solutions of the compound were added to the 96-well plate at 25 µL per well, and the plate was placed in the incubator at 37°C and 5% CO2 for further culture for 2 h. Then, the supernatant of the cell culture plate was discarded, 50 µL of lysis buffer was added to each well, the cells were lysed at room temperature under shaking for 30 min, then placed in a centrifuge and centrifuged at 1000 rpm for 1 min. 15 µL of supernatant was transferred to a 384-well plate, 5 µL of detection mixture was added to each well (Eu-labeled anti-ERK1/2 (T202-Y204) Antibody at a final detection concentration of 0.5 nM and ULight labeled anti-ERK1/2 Antibody at a final detection concentration of 5 nM), centrifuged at 1000 rpm for 1 min, mixed evenly, and reacted at room temperature overnight. The plate was read using Envison and the signal values at the emission wavelengths of 620 nm and 665 nm were detected using a time-resolved fluorescence program.
4. Experimental data processing method:
The ratio of the signal values at the emission wavelengths of 665 nm and 620 nm was calculated, and the inhibition rate was calculated using the ratio. Graphpad Prism software was used to fit the concentration and inhibition rate to a nonlinear regression curve, so as to obtain the IC50 value.

**Table 5: Inhibition activity of the compounds on p-ERK in AGS cells**

| Compound No. | AGS_pERK IC50(nM) |
|---|---|
| Example 1-1 | 7.0 |
| Example 19-1 | 5.6 |
| Example 35-1 | 2.7 |
| Example 129 | 2.4 |
| Example 137 | 5.8 |
| Example 161 | 4.9 |
| Example 164 | 3.6 |
| Example 291 | 2.3 |
| Example 330 | 3.8 |
| Example 337 | 4.6 |
| Example 338 | 2.8 |
| Example 339 | 4.4 |
| Example 341 | 4.2 |

### 6. Experimental conclusion:

The example compounds of the present invention have good inhibition effect on pERK in AGS cells.

### V. Pharmacokinetic determination in mice

1. Study objective: Balb/c mice were used as test animals to study the pharmacokinetic behavior of the compounds in mice (plasma) after oral administration.
2. Test scheme
   2.1 Test drugs: The example compounds of the present invention, made in house;
   2.2 Test animals: Balb/c mice, male, purchased from Shanghai Jiesijie Experimental Animal Co., Ltd., animal production license number (SCXK (Shanghai) 2013-0006 N0.311620400001794).
   2.3 Drug formulation:
      Formulation of a drug for oral administration: 10% Captisol in 50 mM citrate buffer pH 5.0.

Formulation of 50 mM citric acid: 4.8 g of citric acid was weighed into a 1000 ml glass bottle, ultrapure water was added to 300 ml, and the mixture was magnetically stirred until completely dissolved, additional ultrapure water was then added to 500 ml to formulate 50 mM citric acid.

Formulation of a solution containing 50 mM citric acid (10% Captisol, PH = 5): 50 g of Captisol powder was weighed and added to a 1000 ml glass bottle, 300 ml of 50 mM citric acid was added, and the mixture was magnetically stirred until completely dissolved, then additional citric acid was added to 500 ml, and the PH was adjusted to 5 using 10 M NaOH.

The example compounds were weighed and added to a 4 mL glass bottle separately, 2.4 mL of the solution was added and the mixture was subjected to ultrasound treatment for 10 min to obtain a colorless clear solution with a concentration of 3 mg/mL.

Formulation of a drug for intravenous administration: 5%DMSO+10%Solutol HS15+85%PBS.

The example compounds were weighed, and 5% DMSO was added according to the total administration volume; the mixture was vortexed and sonicated for 2 min to ensure complete dissolution; then, 10% Solutol HS15 was added, and the mixture was vortexed and sonicated for 2 min to achieve complete dissolution; finally, 85% PBS was added, and the mixture was vortexed and sonicated for 5 min; the obtained mixture was filtered through a 0.22 um membrane to obtain a colorless, transparent, and clear solution with a concentration of 0.2 mg/mL.

### 2.4 Administration:

3 Balb/c mice, male; After overnight fasting, the animals were administered orally (PO) at a dose of 30 mg/kg with an administration volume of 10 mL/kg.

3 Balb/C mice, male; After overnight fasting, the animals were administered intravenously (IV) at a dose of 1 mg/kg with an administration volume of 5 mL/kg.

### 2.5 Sample collection:

0.04 mL of blood was collected from the orbit before administration and at 0.25 h, 0.5 h, 1 h, 2 h, 4 h, 6 h, 8 h, and 24 h after administration and placed in an EDTA-2K test tube, and the test tube was centrifuged at 6000 rpm for 6 min at 4°C to separate the plasma, which was stored at -20°C; the animals were fed 4 h after administration.

### 3. Determination results:

The final determination results obtained by applying the LCMS/MS method are shown in the table below:

**Table 6: Pharmacokinetic parameters of the compounds administered intravenously in mice**

| **Example number (PO)** | **C0 (ng/mL)** | **AUC_{o-∞} (ng/mL*hr)** | **T_{1/2} (hr)** | **CL (mL/min/kg)** |
|---|---|---|---|---|
| Example 1-1 | 886 | 583 | 8.6 | 28.6 |
| Example 19-1 | 904 | 476 | 11.2 | 35.0 |
| Example 21-1 | 939 | 253 | 9.1 | 14.1 |
| Example 35-1 | 693 | 144 | 3.2 | 116.1 |
| Example 91 | 662 | 366 | 9.3 | 45.6 |
| Example 124 | 412 | 374 | 11.5 | 44.6 |
| Example 339 | 188 | 133 | 4.8 | 125.0 |
| MRTX-1133 | 854 | 305 | 11.1 | 55 |

**Table 7: Pharmacokinetic parameters of the compounds administered orally in mice**

| **Example number** (**PO)** | **Tmax (hr)** | **Cmax (ng/mL)** | **AUC_{0-∞} (ng/mL*hr)** | **T_{1/2} (hr)** | **F (%)** |
|---|---|---|---|---|---|
| Example 1-1 | 0.5 | 227 | 2114 | 9.1 | 12.1 |
| Example 19-1 | 1.0 | 316 | 1606 | 7.2 | 11.2 |
| Example 89 | 1.0 | 259 | 1513 | 7.7 | / |
| Example 91 | 2.0 | 295 | 1818 | 5.7 | 16.6 |
| Example 124 | 1.0 | 391 | 2612 | 6.6 | 23 |
| Example 131 | 1.0 | 151 | 559 | 18.3 | / |
| Example 161 | 2.0 | 221 | 1548 | / | / |
| Example 337 | 1.0 | 204 | 632 | 1.7 | / |
| Example 339 | 1.0 | 143 | 476 | 1.8 | 13.8 |
| MRTX-1133 | 2.0 | 11 | NA | NA | NA |

| | | | | | |
|---|---|---|---|---|---|
| "NA" means not detected; "/" indicates no test. | | | | | |

### 4. Experimental conclusion

The results show that the example compounds have a high oral exposure. Compared with the compound MRTX-1133, which needs to be administered intravenously in clinical practice, the present invention provides a class of compounds that can be used for oral administration.

### VI. Pharmacokinetic determination in rats

### 1. Study objective:

SD rats were used as test animals to study the pharmacokinetic behavior of the example compounds in rats (plasma) after oral administration.

### 2. Test scheme

### 2.1 Test drugs:

The example compounds of the present invention, made in house.

### 2.2 Test animals:

3 SD rats per group, male. Shanghai Jiesijie Experimental Animal Co., Ltd., animal production license number (SCXK (Shanghai) 2013-0006 N0.311620400001794).

### 2.3 Drug formulation:

Formulation of a drug for oral administration: 10% Captisol in 50 mM citrate buffer pH 5.0.

Formulation of 50 mM citric acid: 4.8 g of citric acid was weighed into a 1000 ml glass bottle, ultrapure water was added to 300 ml, and the mixture was magnetically stirred until completely dissolved, additional ultrapure water was then added to 500 ml to formulate 50 mM citric acid.

Formulation of a solution containing 50 mM citric acid (10% Captisol, PH = 5): 50 g of Captisol powder was weighed and added to a 1000 ml glass bottle, 300 ml of 50 mM citric acid was added, and the mixture was magnetically stirred until completely dissolved, then additional citric acid was added to 500 ml, and the PH was adjusted to 5 using 10 M NaOH.

The example compounds were weighed and dissolved in the solution and the mixture was shaken well and subjected to ultrasound treatment for 15 min to obtain a colorless clear solution with a concentration of 3 mg/mL.

Formulation of a drug for intravenous administration: 5%DMSO+10%Solutol HS15+85%PBS.

The example compounds were weighed, and 5% DMSO was added according to the total administration volume; the mixture was vortexed and sonicated for 2 min to ensure complete dissolution; then, 10% Solutol HS15 was added, and the mixture was vortexed and sonicated for 2 min to achieve complete dissolution; finally, 85% PBS was added, and the mixture was vortexed and sonicated for 5 min; the obtained mixture was filtered through a 0.22 um membrane to obtain a colorless, transparent, and clear solution with a concentration of 0.2 mg/mL.

### 2.4 Administration:

3 SD rats per group, male; After overnight fasting, the animals were administered orally (PO) at a dose of 30 mg/kg with an administration volume of 10 mL/kg.

3 SD rats per group, male; After overnight fasting, the animals were administered intravenously (IV) at a dose of 1 mg/kg with an administration volume of 5 mL/kg.

### 2.5 Sample collection:

0.2 mL of blood was collected from the jugular vein before administration and at 0.25 h, 0.5 h, 1.0 h, 2.0 h, 4.0 h, 6.0 h, 8.0 h, and 24.0 h after administration and placed in an EDTA-2K test tube, and the test tube was centrifuged at 6000 rpm for 6 min at 4°C to separate the plasma, which was stored at -20°C. the animals were fed 4 h after administration.

### 3. Experimental results:

The final determination results obtained by applying the LCMS/MS method are shown in the table below:

**Table 8: Pharmacokinetic parameters of the compounds administered intravenously in rats**

| **Example number (PO)** | **C0 (ng/mL)** | **AUC_{o-∞} (ng/mL*hr)** | **T_{1/2} (hr)** | **CL (mL/min/kg)** |
|---|---|---|---|---|
| Example 1-1 | 1766 | 2964 | 16.3 | 5.6 |
| Example 19-1 | 1717 | 1903 | 10.1 | 8.8 |
| Example 35-1 | 2812 | 571 | 5.7 | 29.2 |
| Example 91 | 995 | 660 | 11.9 | 25.3 |
| Example 124 | 524 | 1113 | 9.0 | 15.0 |
| Example 129 | 2121 | 578 | 11.7 | 28.8 |
| Example 137 | 1634 | 793 | 11.3 | 21.0 |
| Example 161 | 704 | 1420 | 8.4 | 11.7 |
| Example 162 | 438 | 514 | 10.8 | 32.4 |
| Example 164 | 838 | 774 | 17.6 | 21.5 |
| Example 322 | 326 | 219 | 11.6 | 76.2 |
| Example 339 | 174 | 98 | 2.6 | 169.5 |
| MRTX-1133 | 2486 | 367 | 2.7 | 45.0 |

**Table 9: Pharmacokinetic parameters of the compounds administered orally in rats**

| **Example number (PO)** | **Tmax (hr)** | **Cmax (ng/mL)** | **AUC_{o-∞} (ng/mL*hr)** | **T_{1/2} (hr)** | **F (%)** |
|---|---|---|---|---|---|
| Example 1-1 | 6.0 | 254 | 1893 | 5.1 | 2.1 |
| Example 3-1 | 4.0 | 140 | 601 | 5.4 | / |
| Example 3-2 | 4.0 | 145 | 839 | 7.1 | / |
| Example 19-1 | 2.0 | 329 | 3076 | 8.2 | 5.4 |
| Example 35-1 | 2.0 | 108 | 601 | 3.4 | 3.5 |
| Example 91 | 6.0 | 251 | 1925 | 4.8 | 9.7 |
| Example 124 | 6.0 | 471 | 6171 | 6.8 | 18 |
| Example 129 | 1.0 | 103 | 938 | 9.6 | 5.4 |
| Example 132 | 2.0 | 246 | 2349 | / | / |
| Example 133 | 4.0 | 588 | 6512 | 7.0 | / |
| Example 137 | 4.0 | 271 | 2111 | 6.2 | 9.7 |
| Example 140 | 2.0 | 118 | 2277 | 11.0 | / |
| Example 141 | 2.0 | 869 | 17342 | 18.9 | / |
| Example 161 | 6.0 | 254 | 2887 | 6.9 | 7 |
| Example 162 | 4.0 | 89 | 1591 | 10.5 | 10.3 |
| Example 164 | 2.0 | 135 | 1584 | 14.0 | / |
| Example 167 | 2 | 127 | 1808 | 11.9 | / |
| Example 168 | 4.0 | 459 | 6795 | 12.6 | / |
| Example 172 | 2.0 | 94 | 2599 | 19.3 | / |
| Example 285 | 2.0 | 73 | 598 | / | / |
| Example 286 | 2.0 | 50 | 600 | 10.8 | / |
| Example 289 | 4.0 | 280 | 2106 | 6.3 | / |
| Example 322 | 6.0 | 40 | 694 | 10.4 | 10 |
| Example 337 | 4.0 | 41 | 374 | 4.2 | / |
| Example 339 | 2.0 | 43 | 220 | 3.0 | 6.6 |
| MRTX-1133 | 2.0 | 11 | NA | NA | NA |

| | | | | | |
|---|---|---|---|---|---|
| "NA" means not detected; "/" indicates no test. | | | | | |

### 4. Experimental conclusion

The results show that the example compounds have a high oral exposure. Compared with the compound MRTX-1133, which needs to be administered intravenously in clinical practice, the present invention provides a class of compounds that can be used for oral administration.

### VII. Anti-tumor efficacy experiment in mouse transplanted tumor model of human colorectal cancer cell line (GP2D)

### 1. Experimental objective

8-10 week old female conventional immunodeficient mice (BALB/c nude) with a body weight of 18 g-22 g were used as experimental animals, and a transplanted tumor model of human colorectal cancer cell line (GP2D) was used to conduct in vivo efficacy experiments to test the anti-tumor results of the example compounds after gavage administration.

### 2. Experimental instruments and reagents:

### 2.1 Instruments:

Ultra-clean workbench (BSC-1300II A2, Shanghai Boxun Industrial Co., Ltd. Medical Equipment Factory); CO₂ incubator (Thermo-311, Thermo); Centrifuge (Centrifuge 5720R, Eppendorf); Full-automatic cell counter (Countess II, Life Technologies); Pipette (10-20 µL, Eppendorf); Microscope (Ts 2, Nikon); Vernier caliper (CD-6"AX, Mitutoyo, Japan); Cell culture flask (T25/T75/T225, Corning); Thermostat water tank (HWS12, Shanghai Yiheng Science)

### 2.2 Reagents:

DMEM(11995-065, Gibco); fetal bovine serum (FBS) (10091-148, Gibco); 0.25% trypsin (25200-056, Gibco); penicillin-streptomycin dual antibiotics (P/S) (SV30010, GE); phosphate buffer solution (PBS) (10010-023, Gibco); Matrigel (356234, Corning); Gln (25030-081, Gibco)

### 3. Experimental method

### 3.1 Experimental compounds

The example compounds of the present invention, made in house.

### 3.2 Formulation of compounds

A certain amount of the example compound was weighed, and an appropriate amount of 10% vehicle (Solutol HS15) was added. The mixture was stirred on a magnetic stirrer for 0.5 h to formulate a homogeneous suspension at the desired concentration.

### 3.3 Cell culture and passaging

The human colorectal cancer cell line (GP2D) was derived from COBIOER BIOSCIENCES CO., LTD, cultured on DMEM medium (Gibco, 11995-065) supplemented with 10% fetal calf serum (Gibco, 10091-148) and 1% penicillin/streptomycin (Gibco, 15140-122) and observed under a microscope (Thermo Fisher, 311). The cells were routinely passaged when the cells reached 80%-90% confluence in the culture flask. The passaged cells were cultured in a 37°C, 5% CO₂ incubator (Thermo Fisher, 311).

### 3.4 Cell seeding and group administration

Under aseptic conditions, human colorectal cancer cells (GP2D) in the logarithmic growth phase were harvested using 0.25% trypsin-EDTA (Gibco, 25200-056), washed and resuspended with phosphate buffer. The cell suspension was mixed with Matrigel (Corning, 356234) at a 1 : 1 ratio (v/v) and implanted subcutaneously into the right anterior dorsal region of immunodeficient mice (BALB/c nude). Each mouse was inoculated with 5*10⁶ cells in a volume of 100 microliters (µL). After inoculation, when the tumor volume reached 100-200 mm³, the mice would be evenly randomized into 5 groups based on the tumor size, with 5 mice per group, for in vivo efficacy experiments. The positive control group was the compound MRTX-1133, and the negative control group was the vehicle group.

Mode of administration: The positive control group was intraperitoneally injected, and the example compounds were administered orally. The dosage and administration period are as shown in the table.

### 3.5 Tumor measurement and tumor inhibition rate calculation

Twice a week, the long and short diameters of the tumor were measured using a vernier caliper, and the tumor volume (mm³) was calculated using the formula: V = 0.5*D*d*d, where D and d are the long and short diameters of the tumor, respectively. The anti-tumor efficacy was determined by dividing the average increase in tumor volume of compound-treated animals by the average increase in tumor volume of untreated animals. The calculation formula for tumor inhibition rate was: When there is no tumor regression, TGI(%) = 100-[(Vₜ-V₀) administration group/(Vₜ-V₀) vehicle control group]*100%. When tumor regression occurs, TGI(%) = [1-(V₁-V₀) administration group/V₀ vehicle control group]* 100%. All animals were euthanized after the experiment.

### 4. Experimental results and conclusions

**Table 10: Efficacy parameters of the compounds in mice with transplanted tumors**

| **Grouping** | **Tumor volume (mm³, Mean ± SD)** | | **ΔT/ΔC(%)** | **TGI(%)** |
|---|---|---|---|---|
| | Day 0 | Day 14 | Day 14 | Day 14 |
| Vehicle, PO*BID | 167 | 790 | / | / |
| MRTX-1133, 10mpk, IV*BID | 167 | 94 | 11.89 | 143.79 |
| Example 131, 100mpk, PO*BID | 167 | 122 | 15.42 | 127.11 |
| Example 161, 30mpk, PO*BID | 167 | 110 | 13.93 | 134.18 |
| Example 161, 100mpk, PO*BID | 167 | 77 | 9.79 | 153.75 |
| Example 162, 30mpk, PO*BID | 167 | 130 | 16.47 | 122.15 |
| Example 162, 100mpk, PO*BID | 167 | 111 | 14.04 | 133.67 |
| Example 129, 30mpk, PO*BID | 167 | 112 | 14.15 | 133.12 |
| Example 129, 100mpk, PO*BID | 167 | 79 | 9.94 | 153.01 |
| Example 285, 100mpk, PO*BID | 167 | 87 | 10.99 | 148.04 |

As shown in the table above, the example compound at an oral administration dose of 10-100 mg/kg and the MRTX-1133 compound at an intravenous administration dose of 10 mg/kg can both achieve the inhibition effect of tumor regression, and the body weight of mice at each dose did not significantly decrease, exhibiting that the oral administration of the example compound has a good tumor inhibition effect and safety, and may provide a new medication regimen for future clinical applications.

### VIII. Antitumor efficacy experiment in mouse transplanted tumor model of human pancreatic cancer cell line (HPAC)

### 1. Experimental objective

8-10 week old female conventional immunodeficient mice (BALB/c nude) with a body weight of 18 g-22 g were used as experimental animals, and a transplanted tumor model of human pancreatic cancer cell line (HPAC) was used to conduct in vivo efficacy experiments to test the anti-tumor results of the example compounds after gavage administration.

### 2. Experimental instruments and reagents:

### 2.1 Instruments:

Ultra-clean workbench (BSC-1300II A2, Shanghai Boxun Industrial Co., Ltd. Medical Equipment Factory); CO₂ incubator (Thermo-311, Thermo); Centrifuge (Centrifuge 5720R, Eppendorf); Full-automatic cell counter (Countess II, Life Technologies); Pipette (10-20 µL, Eppendorf); Microscope (Ts 2, Nikon); Vernier caliper (CD-6" AX, Mitutoyo, Japan); Cell culture flask (T25/T75/T225, Corning); Thermostat water tank (HWS12, Shanghai Yiheng Science)

### 2.2 Reagents:

DMEM/F12(11330-032, Gibco); fetal bovine serum (FBS) (10091-148, Gibco); Insulin(51500-056, Gibco); EGF(PHG0311, Gibco); 0.25% trypsin (25200-056, Gibco); phosphate buffer solution (PBS) (10010-023, Gibco); Matrigel (356234, Corning); Gln (25030-081, Gibco)

### 3. Experimental method

### 3.1 Experimental compounds

The example compounds of the present invention, made in house.

### 3.2 Formulation of compounds

A certain amount of the example compound was weighed, and an appropriate amount of 10% vehicle (Solutol HS15) was added. The mixture was stirred on a magnetic stirrer for 0.5 h to formulate a homogeneous suspension at the desired concentration.

### 3.3 Cell culture and passaging

Human pancreatic cancer cell line (HPAC) was derived from COBIOER BIOSCIENCES CO., LTD, cultured on DMEM/F12 medium (Gibco, 11330-032) supplemented with 10% fetal calf serum (Gibco, 10091-148), 1×Insulin (51500-056, Gibco) and 10 ng/ml of EGF (PHG0311, Gibco) and observed under a microscope (Thermo Fisher, 311). The cells were routinely passaged when the cells reached 80%-90% confluence in the culture flask. The passaged cells were cultured in a 37°C, 5% CO₂ incubator (Thermo Fisher, 311).

### 3.4 Cell seeding and group administration

Under aseptic conditions, human pancreatic cancer cells (HPAC) in the logarithmic growth phase were harvested using 0.25% trypsin-EDTA (Gibco, 25200-056), washed and resuspended with phosphate buffer. The cell suspension was mixed with Matrigel (Corning, 356234) at a 1 : 1 ratio (v/v) and implanted subcutaneously into the right anterior dorsal region of immunodeficient mice (BALB/c nude). Each mouse was inoculated with 3*10⁶ cells in a volume of 100 microliters (µL). After inoculation, when the tumor volume reached 100-200 mm³, the mice would be evenly randomized into 5 groups based on the tumor size, with 5 mice per group, for in vivo efficacy experiments. The negative control group was the vehicle group.

Mode of administration: The example compounds were administered orally. The dosage and administration period are as shown in the table.

### 3.5 Tumor measurement and tumor inhibition rate calculation

Twice a week, the long and short diameters of the tumor were measured using a vernier caliper, and the tumor volume (mm³) was calculated using the formula: V = 0.5*D*d*d, where D and d are the long and short diameters of the tumor, respectively. The anti-tumor efficacy was determined by dividing the average increase in tumor volume of compound-treated animals by the average increase in tumor volume of untreated animals. The calculation formula for tumor inhibition rate was: When there is no tumor regression, TGI(%) = 100-[(Vₜ-V₀) administration group/(Vₜ-V₀) vehicle control group]*100%. When tumor regression occurs, TGI(%) = [1-(Vₜ-V₀) administration group/V₀ vehicle control group]*100%. All animals were euthanized after the experiment.

### 4. Experimental results and conclusions

**Table 11: Efficacy parameters of the compounds in mice with transplanted tumors**

| **Grouping** | **Tumor volume (mm³, Mean ± SD)** | | **ΔT/ΔC(%)** | **TGI(%)** |
|---|---|---|---|---|
| | Day 0 | Day 21 | Day 21 | Day 21 |
| Vehicle, PO*BID | 209 | 1141 | / | / |
| Example 129, 100mpk, PO*BID | 209 | 231 | 20.27 | 97.63 |
| Example 161, 100mpk, PO*BID | 209 | 131 | 11.47 | 137.45 |
| Example 162, 100mpk, PO*BID | 210 | 155 | 13.54 | 126.15 |
| Example 291, 100mpk, PO*BID | 210 | 153 | 13.29 | 127.51 |

**Table 12: Efficacy parameters of the compounds in mice with transplanted tumors**

| **Grouping** | **Tumor volume (mm³, Mean ± SD)** | | **ΔT/ΔC(%)** | **TGI(%)** |
|---|---|---|---|---|
| | Day 0 | Day 21 | Day 14 | Day 14 |
| Vehicle, PO*BID | 468 | 956 | / | / |
| Example 35-1, 100mpk, PO*BID | 469 | 585 | 61.00 | 76.39 |
| Example 330, 100mpk, PO*BID | 468 | 418 | 43.78 | 110.18 |
| Example 339, 100mpk, PO*BID | 469 | 185 | 19.36 | 160.47 |
| Example 341, 100mpk, PO*BID | 469 | 180 | 18.73 | 161.42 |

As shown in the table above, the example compound at an oral administration dose of 100 mg/kg can achieve the effect of inhibiting tumor proliferation or causing tumor regression, and the body weight of mice at each dose did not significantly decrease, exhibiting that the oral administration of the example compound has a good tumor inhibition effect and safety, and may provide a new medication regimen for future clinical applications.

### IX. Plasma stability test

### 1. Experimental objective

The purpose of this experiment was to detect the stability of the example compounds in plasma of mouse, rat, dog and human.

### 2. Experimental instruments and materials

Liquid chromatography-mass spectrometer, centrifuge, vortexer, pipette, continuous dispenser, 96-well plate, 50% methanol aqueous solution, acetonitrile solution containing internal standard, blank matrix (plasma of rat, dog, or human), Propantheline (propantheline bromide), Mevinolin (lovastatin), and test compound.

### 3. Experimental steps

### 3.1 Solution formulation

### 1), plasma preparation

After whole blood was collected from animals or humans, it was placed in a test tube containing an anticoagulant and centrifuged at 3500 rpm for 10 min to collect the upper pale yellow plasma.

### 2), 10 µM test compound (m/M/V=C)

The compound was weighed and the stock solution was formulated with DMSO and the working solution was formulated with 100 mM phosphate buffer.

### 3), 10 µM positive control

### (1) Propantheline (propantheline bromide Mr = 449.4 Da)

2.36 mg of propantheline bromide was weighed and diluted with 1 mL of DMSO into a 10 mM stock solution; 10 µL of 10 mM stock solution was pipetted into 1 mL of 100 mM phosphate buffer to achieve a final concentration of 100 µM.

### (2) Mevinolin (lovastatin Mr = 404.5 Da)

4.05 mg of lovastatin was weighed and diluted with 1 mL of DMSO into a 10 mM stock solution; 10 µL of 10 mM stock solution was pipetted into 1 mL of 100 mM phosphate buffer to achieve a final concentration of 100 µM.

### 3.2 Experimental process:

1) In a 96-well plate, 285 µL of plasma and 15 µL of 10 µM compound (test compound) were added in sequence and incubated at 37°C.
2) 40 µL was taken at 0, 15, 30, 60, 90, and 120 min (the sampling point can be fine-tuned) and 160 µL of acetonitrile stop solution containing internal standard was add.
3) After centrifugation (3500 rpm, 10 min), 50 µL of supernatant was taken, and 50 µL of DDH2O added for dilution and then the obtained mixture was injected into LC-MS/MS.

### 4. Chromatography conditions

Instruments: Shimadzu LC-20 AD
Chromatographic column: Phenomenex Gemiu ^{®} C18 (50*4.6 mm, 5 µm particle size);
mobile phase: A: Acetonitrile, B: 0.1% formic acid solution 0-8 min: 5% A→95% A, 2.0-2.1 min: 90%A→5% A; Flow rate: 0.8 mL/min; Run time: 5.0 min; Injection volume: 5 µL.

### 5. Mass spectrum conditions

Instrument: API4000 liquid chromatography-mass spectrometer, AB Company, USA;
the ion source was electrospray ionization source (ESI);
drying gas (N₂) temperature was 500°C;
electrospray voltage was 5500V;
the detection method was positive ion detection;
the scanning method was selected reaction monitoring (MRM);
the scanning time was 0.1s.

### 6. Experimental results

**Table 13: Plasma stability results of the example compounds**

| Species | Number | Remaining rate (%) | | | | | *t*_{1/2} (h) |
|---|---|---|---|---|---|---|---|
| | | 0 min | 15 min | 30 min | 60 min | 120 min | |
| Human | Propantheline bromide | 100.00 | 26.78 | 5.05 | 0.25 | 0.05 | 0.27 |
| | Example 1-1 | 100.00 | 100.68 | 101.72 | 97.62 | 88.35 | 31.41 |
| | Example 19-1 | 100.00 | 95.61 | 93.15 | 85.08 | 83.37 | 15.53 |
| | Example 129 | 100.00 | 109.00 | 97.59 | 101.54 | 105.91 | ∞ |
| | Example 161 | 100.00 | 93.48 | 92.44 | 95.19 | 97.25 | 141.80 |
| | Example 164 | 100.00 | 97.48 | 91.02 | 93.68 | 89.66 | 30.32 |
| | Example 291 | 100.00 | 102.43 | 95.46 | 105.44 | 96.68 | 115.42 |
| Rat | Lovastatin | 100.00 | 21.15 | 1.77 | 0.36 | 0.30 | 6.69 |
| | Example 1-1 | 100.00 | 95.76 | 92.93 | 88.38 | 80.60 | 13.46 |
| | Example 19-1 | 100.00 | 96.27 | 91.39 | 95.69 | 94.09 | 86.34 |
| | Example 129 | 100.00 | 110.22 | 98.09 | 106.43 | 95.62 | 41.24 |
| | Example 161 | 100.00 | 94.50 | 98.84 | 98.63 | 95.03 | 29.39 |
| | Example 164 | 100.00 | 97.07 | 93.36 | 97.21 | 95.03 | 91.57 |
| | Example 291 | 100.00 | 99.67 | 102.83 | 103.48 | 98.20 | 38.34 |
| Mouse | Propantheline bromide | 100.00 | 79.79 | 48.66 | 23.39 | 6.66 | 27.74 |
| | Example 1-1 | 100.00 | 94.74 | 96.27 | 96.21 | 98.50 | 145.96 |
| | Example 19-1 | 100.00 | 101.30 | 98.20 | 91.51 | 92.09 | 27.39 |
| | Example 129 | 100.00 | 97.30 | 103.47 | 104.94 | 91.62 | 37.65 |
| | Example 161 | 100.00 | 101.36 | 101.38 | 98.35 | 101.35 | 228.58 |
| | Example 164 | 100.00 | 99.34 | 95.56 | 101.65 | 100.99 | 148.72 |
| | Example 291 | 100.00 | 97.50 | 82.58 | 81.82 | 63.23 | 184.89 |
| Dog | Propantheline bromide | 100.00 | 95.25 | 95.45 | 75.66 | 36.25 | 80.22 |
| | Example 1-1 | 100.00 | 100.25 | 105.57 | 104.40 | 93.99 | 48.92 |
| | Example 19-1 | 100.00 | 95.89 | 101.96 | 101.84 | 94.59 | 75.55 |
| | Example 129 | 100.00 | 100.48 | 100.22 | 97.42 | 110.22 | ∞ |
| | Example 161 | 100.00 | 91.74 | 92.09 | 90.97 | 88.28 | 31.44 |
| | Example 164 | 100.00 | 92.40 | 94.94 | 96.81 | 91.42 | 51.90 |
| | Example 291 | 100.00 | 104.39 | 94.89 | 103.00 | 99.65 | 94.56 |

### 7. Experimental conclusion

The above data show that the compounds of the present invention have high plasma stability and small species differences.

## Claims

1. A compound represented by general formula (I), or a stereoisomer or pharmaceutically acceptable salt thereof, **characterized in that** 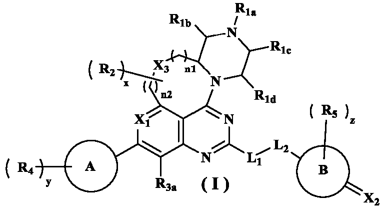
X₁ is selected from N or CR_{3b};
X₂ is selected from N-OR₆ₐ or CR_{6b}R_{6c};
X₃ is selected from O, S or NRₙ₁; preferably O or S;
L₁ is selected from a bond, O, S or NRₙ₂; preferably O or S; more preferably O;
L₂ is selected from C₁-C₄ alkylene, wherein the C₁-C₄ alkylene is optionally substituted with 1 to 4 Rₐ; preferably C₁-C₄ alkylene or C₁-C₄ deuterated alkylene, wherein the C₁-C₄ alkylene and C₁-C₄ deuterated alkylene are optionally substituted with 1 to 4 Rₐ;
ring A is selected from C₃₋₁₂ cycloalkyl, 3- to 12-membered heterocyclyl, C₆₋₁₄ aryl, or 5- to 14-membered heteroaryl; preferably C₃₋₁₀ cycloalkyl, 3- to 10-membered heterocyclyl, C₆₋₁₂ aryl, or 5- to 12-membered heteroaryl;
ring B is selected from C₃₋₁₂ cycloalkyl, 3- to 12-membered heterocyclyl, C₆₋₁₄ aryl, or 5- to 14-membered heteroaryl; preferably C₃₋₁₀ cycloalkyl, 3- to 10-membered heterocyclyl, C₆₋₁₂ aryl or 5- to 12-membered heteroaryl; more preferably 5- to 8-membered saturated or unsaturated heterocyclyl, 7- to 10-membered fused heterocyclyl or 6- to 10-membered bridged heterocyclyl;
R₁ₐ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -C(O)(CH₂)ₙ₃R_{b}, -C(O)O(CH₂)ₙ₃R_{b} or - C(O)[(CH₂NR_{c}C(O))]ₙ₄(CH₂)ₙ₃R_{b}, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl and any CH₂ of (CH₂)ₙ₃ are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl, -C(O)(CH₂)ₙ₃Rₙ, -C(O)O(CH₂)ₙ₃Rₙ or - C(O)[(CH₂NR_{c}C(O))]ₙ₄(CH₂)ₙ₃R_{b}, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl and any CH₂ of (CH₂)ₙ₃ are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl;
R₁ₐ is preferably hydrogen or Pg, wherein the Pg is selected from trityl, benzyl, p-toluenesulfonyl, p-methoxybenzyl, formate, acetyl, benzyloxycarbonyl, tert-butoxycarbonyl, p-methoxyphenyl, -C(O)O(CRₐₐR_{bb})ₙ₉OC(O)CR_{dd}Rₑₑ)ₙ₁₀R_{b}, - C(O)(CRₐₐR_{bb})ₙ₉R_{d}, -C(O)O(CRₐₐR_{bb})ₙ₉R_{b} or -C(O)[(CRₐₐR_{bb}NR_{cc}C(O))] ₙ₉(CR_{dd}Rₑₑ)ₙ₁₀R_{b};
each of R_{1b}, R_{1c} or R_{1d} is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
or R_{1b} and R_{1c} together with adjacent atoms to which they are attached form 3- to 12-membered heterocyclyl or 5- to 14-membered heteroaryl optionally substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, -(CH₂)ₙ₇-C₁₋₆ alkoxy, -(CH₂)ₙ₇-C₁₋₆ deuterated alkoxy, -(CH₂)ₙ₇-C₁₋₆ haloalkoxy, -(CH₂)ₙ₇-C₂₋₆ alkenyl, -(CH₂)ₙ₇-C₂₋₆ alkynyl, -(CH₂)ₙ₇-C₃₋₁₂ cycloalkyl, -(CH₂)ₙ₇-3- to 12-membered heterocyclyl, -(CH₂)ₙ₇-C₆₋₁₄ aryl or - (CH₂)ₙ₇-5- to 14-membered heteroaryl; preferably form 3- to 12-membered heterocyclyl or 5- to 14-membered heteroaryl optionally substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; more preferably form C₃₋₈ cycloalkyl, 3- to 8-membered heterocyclyl, C₆₋₁₀ aryl or 5- to 10-membered heteroaryl optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₂ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl; more preferably hydrogen, deuterium, methyl, ethyl, deuterated methyl, deuterated ethyl, halomethyl or haloethyl;
or any two R₂ substituents together with adjacent atoms to which they are attached form C₃₋₁₂ cycloalkyl or 3- to 12-membered heterocyclyl optionally substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; more preferably form C₃₋₈ cycloalkyl or 3- to 8-membered heterocyclyl optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
each of R₃ₐ or R_{3b} is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₄ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ alkyl-C(O)O-, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -OC(O)(CH₂)ₙ₅R_{d}, -O(CH₂)ₙ₅C(O)R_{d}, -O(CH₂)ₙ₅C(O)OR_{d}, - O(CH₂)ₙ₅OC(O)OR_{d}, -OC(O)O(CH₂)ₙ₅R_{d}, -O(CH₂)ₙ₅C(O)NR_{d}Rₑ, - O(CH₂)ₙ₅OC(O)NR_{d}Rₑ or -O(CH₂)ₙ₅P(=O)R_{d}Rₑ, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl and any CH₂ of (CH₂)ₙ₅ are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl; preferably hydrogen, deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₁₋₃ alkyl-C(O)O-, C₂₋₄ alkenyl, C₂₋₄ alkynyl, C₃₋₈ cycloalkyl, 3- to 8-membered heterocyclyl, C₆₋₁₀ aryl, 5-to 10-membered heteroaryl, -OC(O)(CH₂)ₙ₅R₄, -O(CH₂)ₙ₅C(O)R_{d}, - O(CH₂)ₙ₅C(O)OR_{d}, -O(CH₂)ₙ₅OC(O)OR_{d}, -OC(O)O(CH₂)ₙ₅R_{d}, - O(CH₂)ₙ₅C(O)NR_{d}Rₑ, -O(CH₂)ₙ₅OC(O)NR_{d}Rₑ or -O(CH₂)ₙ₅P(=O)R_{d}Rₑ, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₁₋₃ alkyl-C(O)O-, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl and any CH₂ of (CH₂)ₙ₅ are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₅ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ alkyl-C(O)O-, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -OC(O)(CH₂)ₙ₆R_{f}, -O(CH₂)ₙ₆C(O)R_{f}, -O(CH₂)ₙ₁C(O)OR_{f}, - OC(O)O(CH₂)ₙ₆R_{f}, -O(CH₂)ₙ₆C(O)NR_{f}R_{g}, -O(CH₂)ₙ₆OC(O)NR_{f}R_{g} or - O(CH₂)ₙ₆P(=O) R_{f}R_{g}, wherein the amino, hydroxyl, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl and any CH₂ of (CH₂)ₙ₆ are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl; preferably hydrogen, deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl, -OC(O)(CH₂)ₙ₆R_{f}, -O(CH₂)ₙ₆C(O)R_{f}, -O(CH₂)ₙ₆C(O)OR_{f}, - OC(O)O(CH₂)ₙ₆R_{f}, -O(CH₂)ₙ₆C(O)NR_{f}R_{g}, -O(CH₂)ₙ₆OC(O)NR_{f}R_{g} or - O(CH₂)ₙ₆P(=O)R_{f}R_{g}, wherein the amino, hydroxyl, C₁₋₃ alkyl, C₁₋₃ deuterated alkyl, C₁₋₃ haloalkyl, C₁₋₃ hydroxyalkyl, C₁₋₃ alkoxy, C₁₋₃ alkylthio, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl, 5-to 10-membered heteroaryl and any CH₂ of (CH₂)ₙ₆ are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
or any two R₅ together with adjacent atoms to which they are attached form C₃₋₁₂ cycloalkyl or 3- to 12-membered heterocyclyl optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl; preferably form C₃₋₈ cycloalkyl or 3- to 8-membered heterocyclyl optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
each of R₆ₐ, R_{6b} or R_{6c} is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
or R_{6b} and R_{6c} together with adjacent atoms to which they are attached form C₃₋₁₂ cycloalkyl or 3- to 12-membered heterocyclyl optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl; preferably form C₃₋₈ cycloalkyl or 3- to 8-membered heterocyclyl optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
each of Rₙ₁ or Rₙ₂ is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
each of Rₐ, R_{b}, R_{c}, R_{d}, Rₑ, R_{f} or R_{g} is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
or two Rₐ attached to the same carbon atom are connected to form C₃₋₁₂ cycloalkyl or 3- to 12-membered heterocyclyl optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl; preferably form C₃₋₈ cycloalkyl or 3- to 8-membered heterocyclyl optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
each of Rₐₐ, R_{bb}, R_{cc}, R_{dd} or Rₑₑ is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
x is selected from 0, 1, 2, 3, 4, 5 or 6;
y is selected from 0, 1, 2, 3, 4, 5 or 6;
z is selected from 0, 1, 2, 3, 4, 5 or 6;
n1 is selected from 0, 1 or 2;
n2 is selected from 0, 1 or 2;
n3 is selected from 0, 1, 2, 3, 4, 5 or 6;
n4 is selected from 0, 1, 2, 3, 4, 5 or 6;
n5 is selected from 0, 1, 2, 3, 4, 5 or 6;
n6 is selected from 0, 1, 2, 3, 4, 5 or 6;
n7 is selected from 0, 1 or 2;
n9 is selected from 0, 1 or 2; and
n10 is selected from 0, 1 or 2.

2. The compound or the stereoisomer or pharmaceutically acceptable salt thereof of claim 1, **characterized in that** R_{1b} and R_{1c} together with adjacent atoms to which they are attached form the following structure 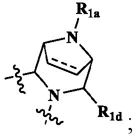 preferably form the following structure 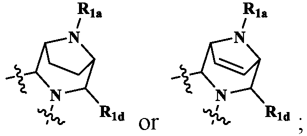 more preferably form the following structure

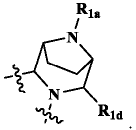

3. The compound, or the stereoisomer or pharmaceutically acceptable salt thereof of claim 1 or 2, **characterized in that** L₂ is selected from 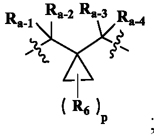 preferably 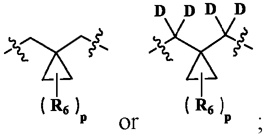
each of Rₐ₋₁, Rₐ₋₂, Rₐ₋₃ and Rₐ₋₄ is independently selected from hydrogen, deuterium, halogen, amino, hydroxyl, cyano, C₁₋₆ alkyl, C₁₋₆ deuterated alkyl, C₁₋₆ haloalkyl or C₁₋₆ hydroxyalkyl; preferably hydrogen, deuterium, halogen, amino, hydroxyl, cyano, C₁₋₃ alkyl, C₁₋₃ deuterated alkyl, C₁₋₃ haloalkyl or C₁₋₃ hydroxyalkyl; more preferably hydrogen or deuterium;
R₆ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl;
and p is selected from 0, 1, 2, 3 or 4.

4. The compound or the stereoisomer or pharmaceutically acceptable salt thereof of any one of claims 1-3, **characterized in that** ring A is selected from phenyl, pyridyl, naphthyl, benzothienyl, benzopyrazolyl, quinolyl or isoquinolyl.

5. The compound or the stereoisomer or pharmaceutically acceptable salt thereof of any one of claims 1-4, **characterized in that** the compound is further represented by general formula (II) or (II-A): 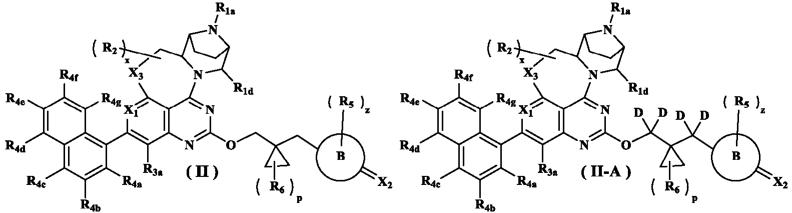
R_{4b} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ alkyl-C(O)O-, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl, -OC(O)(CH₂)ₙ₅R_{d}, -O(CH₂)ₙ₅C(O)R_{d}, -O(CH₂)ₙ₅C(O)OR_{d}, - O(CH₂)ₙ₅OC(O)OR_{d}, -OC(O)O(CH₂)ₙ₅R_{d}, -O(CH₂)ₙ₅C(O)NR_{d}Rₑ, - O(CH₂)ₙ₅OC(O)NR_{d}Rₑ or -O(CH₂)ₙ₅P(=O)R_{d}Rₑ, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl, 5- to 14-membered heteroaryl and any CH₂ of (CH₂)ₙ₅ are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl; preferably selected from hydrogen, deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₁₋₃ alkyl-C(O)O-, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl, -OC(O)(CH₂)ₙ₅R₄, -O(CH₂)ₙ₅C(O)R_{d}, - O(CH₂)ₙ₅C(O)OR_{d}, -O(CH₂)ₙ₅OC(O)OR_{d}, -OC(O)O(CH₂)ₙ₅R_{d}, - O(CH₂)ₙ₅C(O)NR_{d}Rₑ, -O(CH₂)ₙ₅OC(O)NR_{d}Rₑ or -O(CH₂)ₙ₅P(=O)R_{d}Rₑ, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₁₋₃ alkyl-C(O)O-, C₂₋₄ alkenyl, C₂₋₄ alkynyl, C₃₋₈ cycloalkyl, 3- to 8-membered heterocyclyl, C₆₋₁₀ aryl, 5- to 10-membered heteroaryl and any CH₂ of (CH₂)ₙ₅ are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
each of R₄ₐ, R_{4c}, R_{4d} or R₄ₑ is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{4f} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{4g} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl; more preferably C₂₋₄ alkynyl;
R₆ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
p is selected from 0, 1, 2, 3 or 4;
and R₁ₐ, R_{1d}, R₂, R₃ₐ, R_{3b}, R₅, X₁, X₂, X₃, x and z are as defined in claim 1.

6. The compound or the stereoisomer or pharmaceutically acceptable salt thereof of any one of claims 1-4, **characterized in that** the compound is further represented by general formula (III): 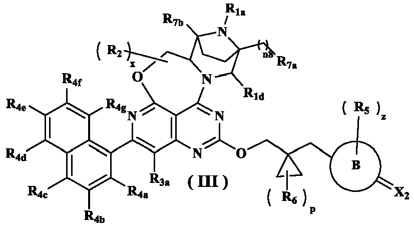
R_{4b} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₁₋₆ alkyl-C(O)O-, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl; preferably hydrogen, deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₁₋₃ alkyl-C(O)O-, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₁₋₃ alkyl-C(O)O-, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
each of R₄ₐ, R_{4c}, R_{4d} or R₄ₑ is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{4f} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{4g} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₆ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₇ₐ is selected from deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ deuterated alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to **14-**membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ deuterated alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{7b} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
p is selected from 0, 1, 2, 3 or 4;
n8 is selected from 0, 1 or 2;
and R₁ₐ, R_{1d}, R₂, R₃ₐ, R₅, X₂, X₃, x and z are as defined in claim 1.

7. The compound or the stereoisomer or pharmaceutically acceptable salt thereof of any one of claims 1-4, **characterized in that** the compound is further represented by general formula (II-B) or (II-C): 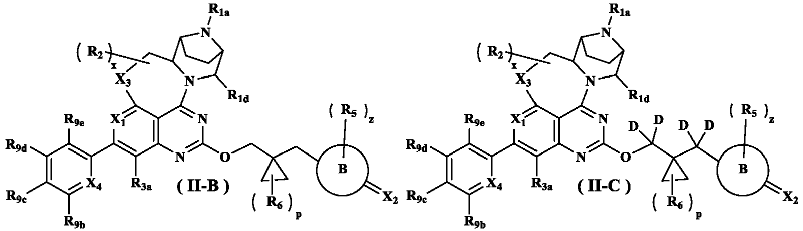
preferably, the compound is further represented by general formula (II-B-1) or (II-C-1): 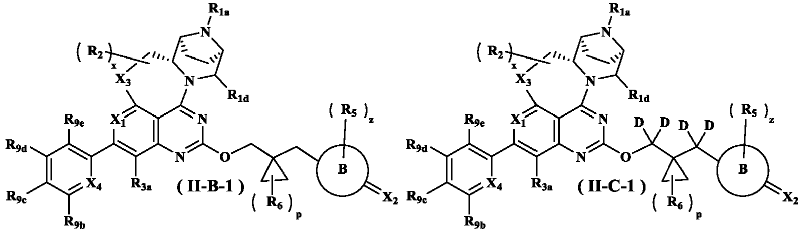
X₂ is selected from N-OR₆ₐ or CR_{6b}R_{6c}; preferably N-OR₆ₐ;
X₄ is selected from N or CR₉ₐ;
R₉ₐ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{9b} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{9c} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R_{9d} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₉ₑ is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
R₆ is selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
p is selected from 0, 1, 2, 3 or 4;
and R₁ₐ, R_{1d}, R₂, R₃ₐ, R₅, X₂, X₃, x and z are as defined in claim 1.

8. The compound or the stereoisomer or pharmaceutically acceptable salt thereof of claim 1, **characterized in that** the compound is further represented by general formula (V): 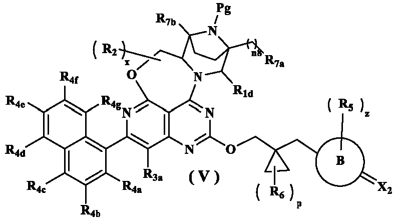
Pg is selected from trityl, benzyl, p-toluenesulfonyl, p-methoxybenzyl, formate, acetyl, benzyloxycarbonyl, tert-butoxycarbonyl, p-methoxyphenyl, - C(O)O(CRₐₐR_{bb})ₙ₉OC(O)(CR_{dd}Rₑₑ)ₙ₁₀R_{b}, -C(O)(CRₐₐR_{bb})ₙ₉R_{b}, - C(O)O(CRₐₐR_{bb})ₙ₉R_{b} or -C(O)[(CRₐₐR_{bb}NR_{cc}C(O))]ₙ₉(CR_{dd}Rₑₑ)ₙ₁₀R_{b};
R_{b} is selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably selected from hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl;
each of Rₐₐ, R_{bb}, R_{cc}, R_{dd} or Rₑₑ is independently selected from hydrogen, deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl, wherein the amino, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl and 5- to 14-membered heteroaryl are optionally further substituted with one or more substituents of deuterium, halogen, amino, nitro, hydroxyl, cyano, mercapto, oxo, thio, C₁₋₆ alkyl, C₁₋₆ deuterated alkyl, C₁₋₆ haloalkyl, C₁₋₆ hydroxyalkyl, C₁₋₆ alkoxy, C₁₋₆ haloalkoxy, C₂₋₆ alkenyl, C₂₋₆ alkynyl, C₃₋₁₂ cycloalkyl, 3- to 12-membered heterocyclyl, C₆₋₁₄ aryl or 5- to 14-membered heteroaryl; preferably hydrogen, deuterium, fluorine, chlorine, bromine, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl or 5- to 10-membered heteroaryl, wherein the amino, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl are optionally further substituted with one or more substituents of deuterium, halogen, amino, nitro, hydroxyl, cyano, mercapto, oxo, thio, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, C₃₋₈ cycloalkyl, 3- to 8-membered heterocyclyl, C₆₋₁₀ aryl and 5- to 10-membered heteroaryl; more preferably hydrogen, deuterium, fluorine, chlorine, bromine, amino, nitro, hydroxyl, cyano, mercapto, methyl, ethyl, propyl or isopropyl;
n9 is selected from 0, 1 or 2;
n10 is selected from 0, 1 or 2;
X₂, Ring B, R_{1d}, R₂, R₃ₐ, R₅, R₆ₐ, R_{6b}, R_{6c}, x and z are as defined in claim 1;
and R₄ₐ, R_{4b}, R_{4c}, R_{4d}, R₄ₑ, R_{4f}, R_{4g}, R₆, R₇ₐ, R_{7b}, p and n8 are as defined in claim 6.

9. The compound or the stereoisomer or pharmaceutically acceptable salt thereof of any one of claims 1-6, **characterized in that** the compound is further represented by general formula (IV): 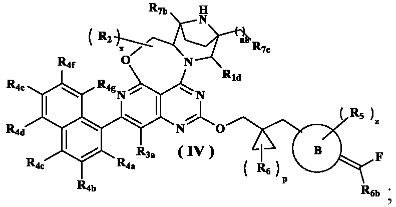
preferably, the compound is further represented by general formula (IV-1): 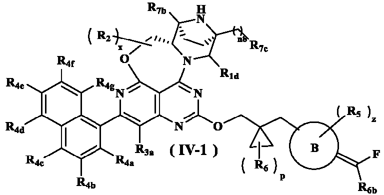
R_{1d} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen;
R₂ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen, deuterium, fluorine, chlorine, bromine, methyl, ethyl or cyclopropyl;
or two R₂ substituents together with adjacent atoms to which they are attached form C₃₋₈ cycloalkyl; preferably form cyclopropyl;
R₃ₐ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl or isopropyl; more preferably hydrogen or fluorine;
R₄ₐ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R_{4b} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, -OC(O) C₁₋₆ alkyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl, ethynyl, acetoxy, propionyloxy, butyryloxy, p-pivaloyloxy or cyclopropyl; more preferably hydrogen, deuterium, fluorine, chlorine, hydroxyl, amino, methyl, acetoxy, propionyloxy, butyryloxy or p-pivaloyloxy;
R_{4c} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R_{4d} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R₄ₑ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R_{4f} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R_{4g} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine, hydroxyl, cyano, methyl, ethyl or ethynyl;
R₅ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen, deuterium, fluorine, chlorine, amino, hydroxyl, cyano, methyl or ethyl;
R_{6b} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen, fluorine, difluoromethyl or trifluoromethyl;
R_{7c} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ deuterated alkoxy, C₁₋₃ haloalkoxy, C₂₋₄ alkenyl, C₂₋₄ haloalkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, deuterated methoxy, deuterated ethoxy, hydroxymethyl, hydroxyethyl, vinyl, halovinyl, or ethynyl; more preferably hydrogen, deuterium, fluorine, chlorine, methyl, ethyl, methoxy, ethoxy, deuterated methoxy, deuterated ethoxy, vinyl, monofluorovinyl or difluorovinyl;
R_{7b} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen or deuterium;
x is selected from 0, 1 or 2;
z is selected from 0, 1, 2, 3, 4, 5 or 6;
p is selected from 0, 1 or 2;
and n8 is selected from 0, 1 or 2.

10. The compound or the stereoisomer or pharmaceutically acceptable salt thereof of any one of claims 1-9, **characterized in that** ring B is selected from 5- to 8-membered saturated or unsaturated monocyclic heterocyclyl, 5- or 6-membered heterocyclyl fused C₃₋₆ cycloalkyl, 5- or 6-membered heterocyclyl fused 5- or 6-membered heterocyclyl, 5- or 6-membered heterocyclyl fused phenyl, 5- or 6-membered heterocyclyl fused 5- or 6-membered heteroaryl or 6- to 10-membered bridged heterocyclyl;
preferably selected from

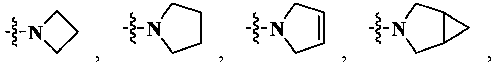

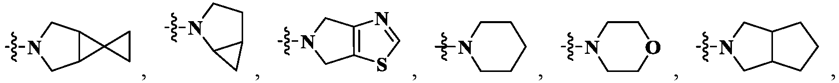

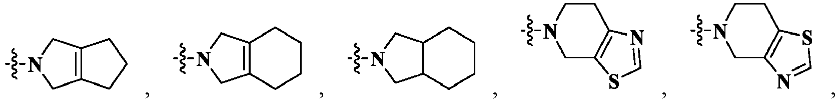

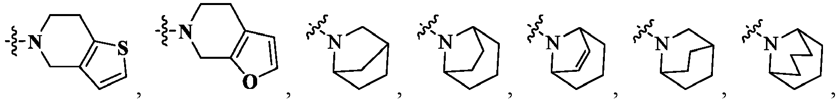

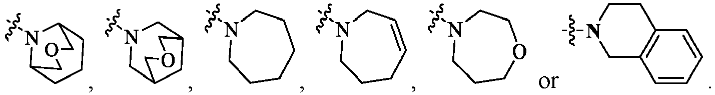

11. The compound or the stereoisomer or pharmaceutically acceptable salt thereof of any one of claims 1-6, **characterized in that** the compound is further represented by general formula (III-A) or (III-B): 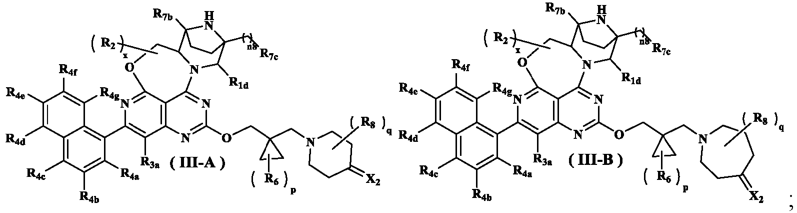
preferably the compound is further represented by general formula (III-A-1) or (III-B-1): 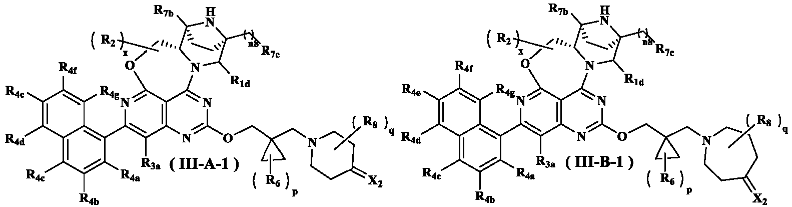
X₂ is selected from N-OR₆ₐ or CR_{6b}R_{6c}; preferably CR_{6b}R_{6c};
R_{1d} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen;
R₂ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen, deuterium, fluorine, chlorine, bromine, methyl, ethyl or cyclopropyl;
or two R₂ substituents together with adjacent atoms to which they are attached form C₃₋₈ cycloalkyl; preferably form cyclopropyl;
R₃ₐ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen or fluorine;
R₄ₐ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R_{4b} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl, -OC(O) C₁₋₆ alkyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl, ethynyl, acetoxy, propionyloxy, butyryloxy, p-pivaloyloxy or cyclopropyl; more preferably hydrogen, deuterium, fluorine, chlorine, hydroxyl, amino, methyl, acetoxy, propionyloxy, butyryloxy or p-pivaloyloxy;
R_{4c} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R_{4d} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R₄ₑ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R_{4f} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine or methyl;
R_{4g} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl, hydroxyethyl, vinyl or ethynyl; more preferably hydrogen, deuterium, fluorine, chlorine, hydroxyl, cyano, methyl, ethyl or ethynyl;
R₆ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen;
R₆ₐ is selected from hydrogen, deuterium, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy or C₃₋₈ cycloalkyl; preferably hydrogen, deuterium, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably methyl, ethyl, -CH₂CH₂F, - CH₂CHF₂, -CH₂CF₃ or cyclopropyl;
R_{6b} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen, fluorine, difluoromethyl or trifluoromethyl;
R_{6c} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen or fluorine;
R_{7c} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ deuterated alkoxy, C₁₋₃ haloalkoxy, C₂₋₄ alkenyl, C₂₋₄ haloalkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, deuterated methoxy, deuterated ethoxy, hydroxymethyl, hydroxyethyl, vinyl, halovinyl, or ethynyl; more preferably hydrogen, deuterium, fluorine, chlorine, methyl, ethyl, methoxy, ethoxy, deuterated methoxy, deuterated ethoxy, vinyl, monofluorovinyl or difluorovinyl;
R_{7b} is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl or C₂₋₄ alkynyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen or deuterium;
R₈ is selected from hydrogen, deuterium, halogen, amino, nitro, hydroxyl, cyano, mercapto, C₁₋₃ alkyl, C₁₋₃ deuterated alkyl, C₁₋₃ haloalkyl, C₁₋₃ hydroxyalkyl, C₁₋₃ alkoxy, C₁₋₃ haloalkoxy, C₂₋₄ alkenyl, C₂₋₄ alkynyl or C₃₋₈ cycloalkyl; preferably hydrogen, deuterium, fluorine, chlorine, bromine, amino, nitro, hydroxyl, cyano, mercapto, methyl, ethyl, propyl, isopropyl, deuterated methyl, deuterated ethyl, deuterated propyl, deuterated isopropyl, halomethyl, haloethyl, halopropyl, haloisopropyl, methoxy, ethoxy, halomethoxy, haloethoxy, hydroxymethyl or hydroxyethyl; more preferably hydrogen, deuterium, fluorine, chlorine, amino, hydroxyl, cyano, methyl or ethyl;
or any two R₈ substituents together with adjacent atoms to which they are attached form C₃₋₈ cycloalkyl or 3- to 8-membered heterocyclyl; preferably form cyclopropyl, 5-membered nitrogen-containing heterocyclyl or 6-membered nitrogen-containing heterocyclyl;
x is selected from 0, 1 or 2;
p is selected from 0, 1 or 2;
q is selected from 0, 1, 2, 3, 4, 5 or 6;
and n8 is selected from 0, 1 or 2.

12. The compound or the stereoisomer or pharmaceutically acceptable salt thereof of any one of claims 1-11, **characterized in that** the compound has the following structure:

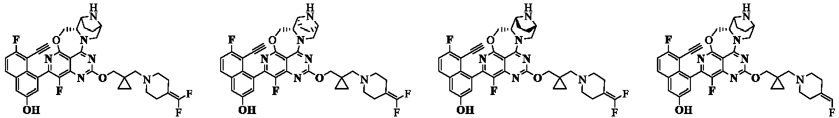

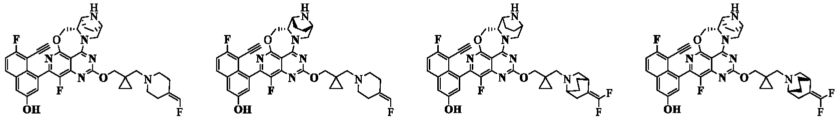

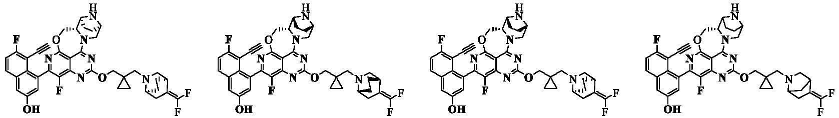

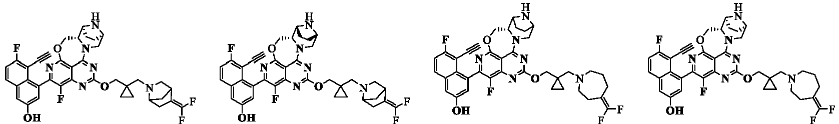

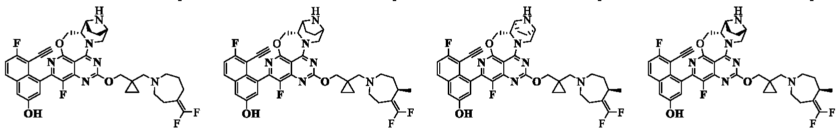

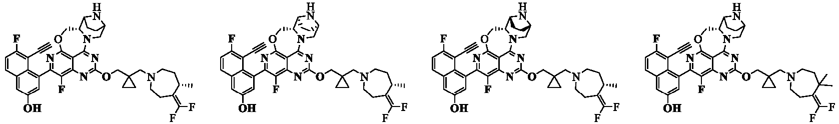

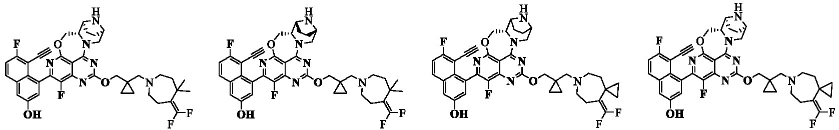

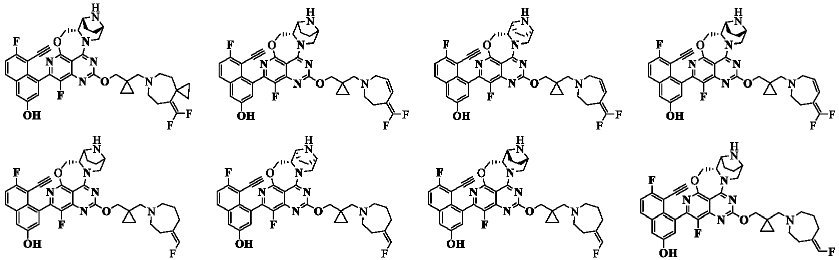

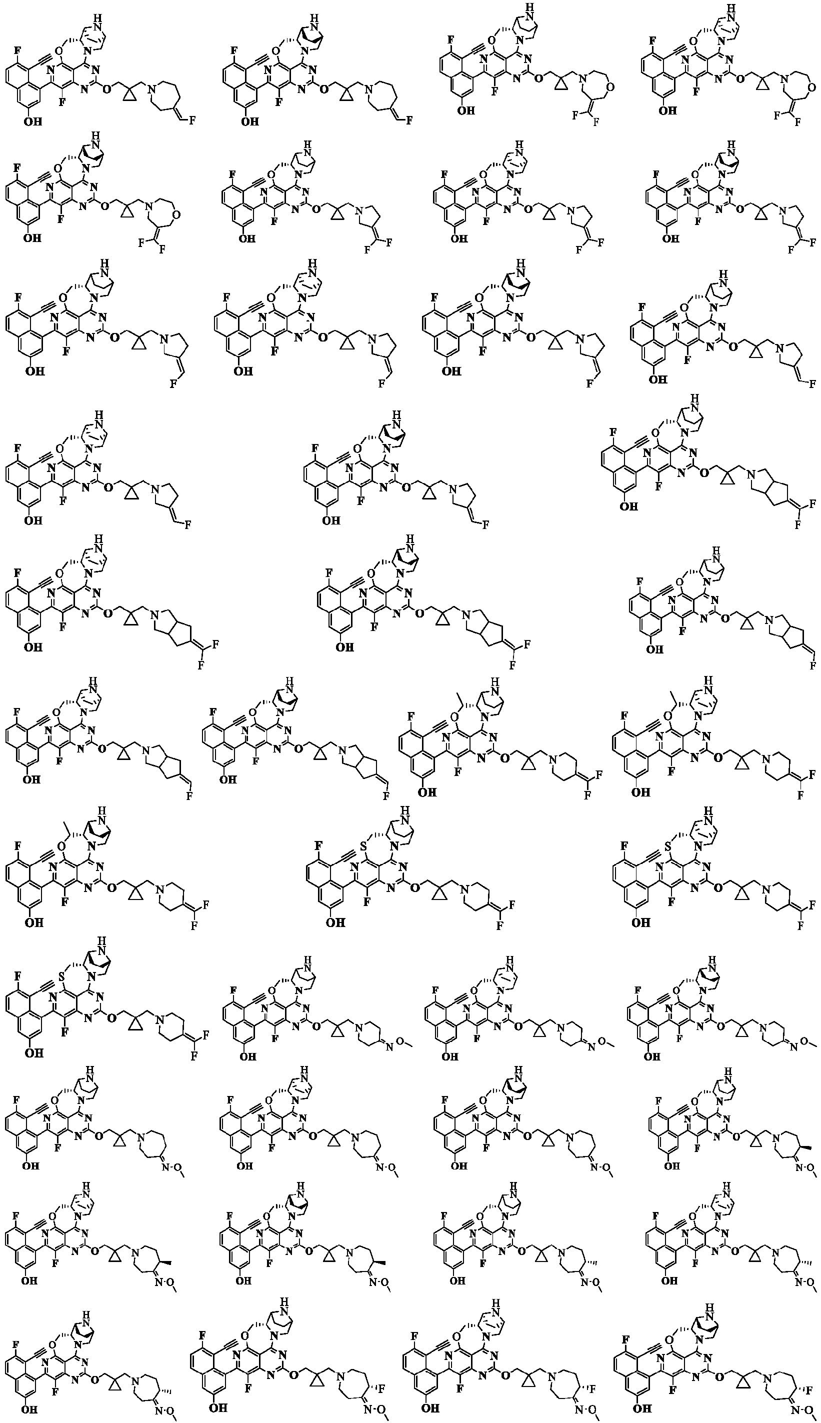

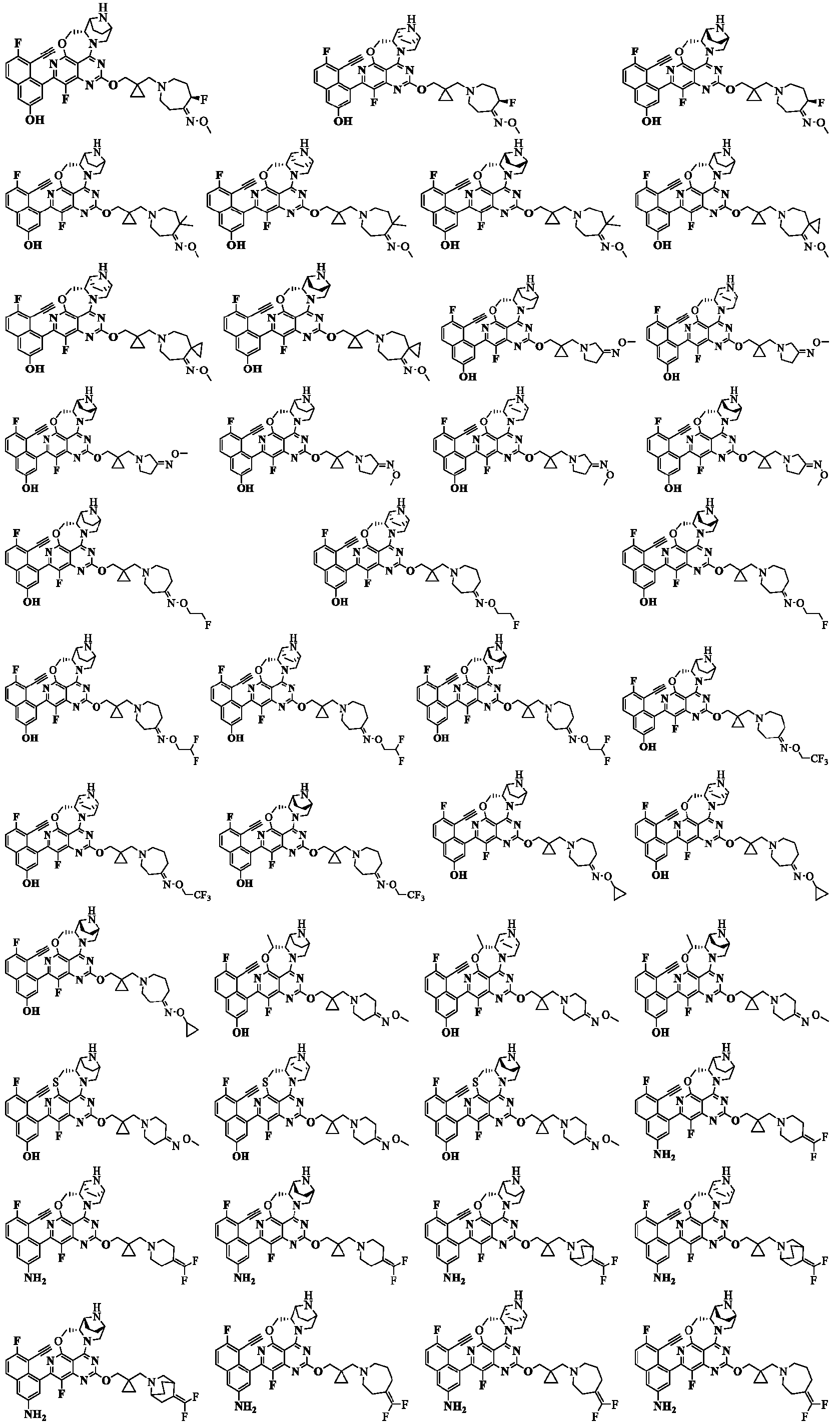

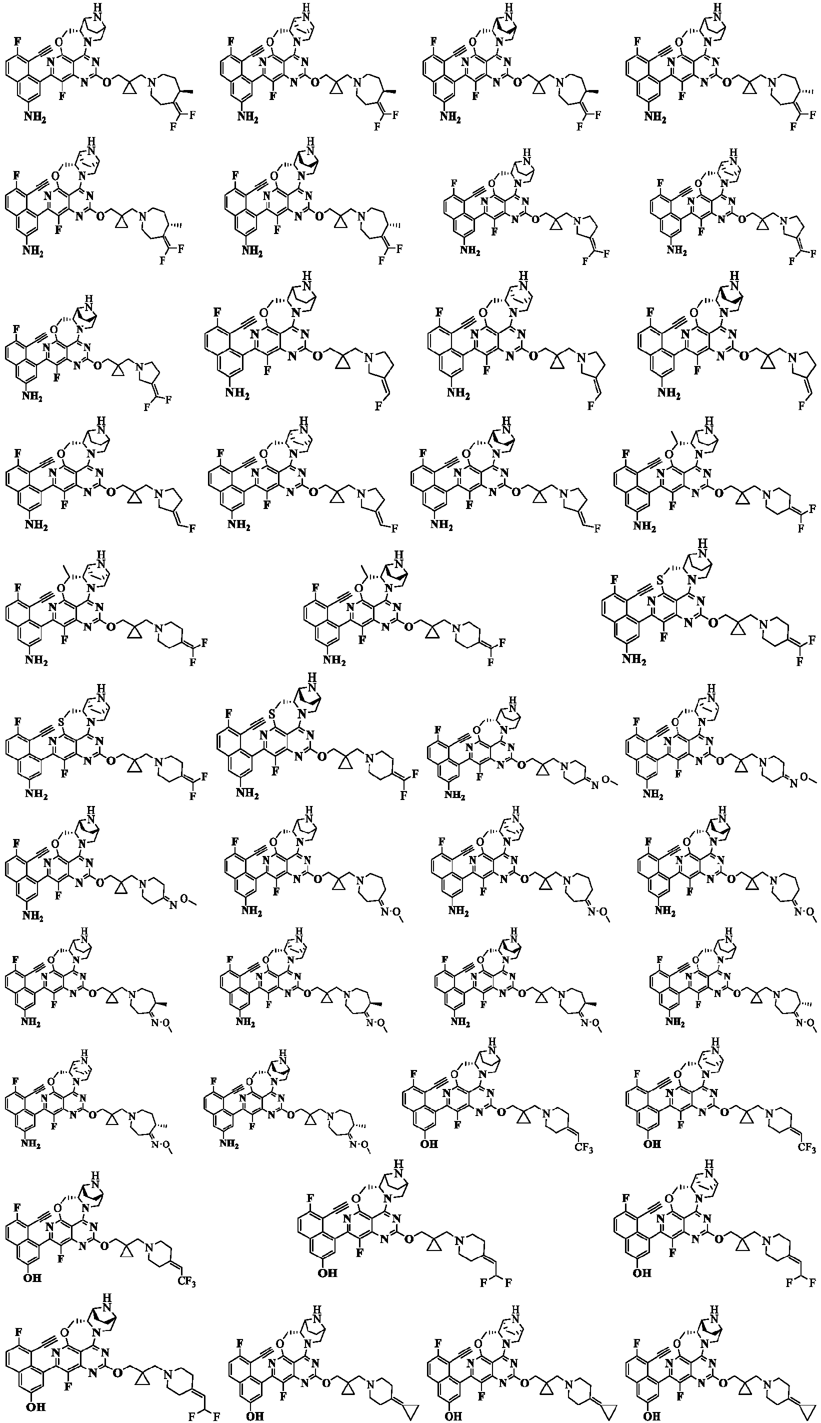

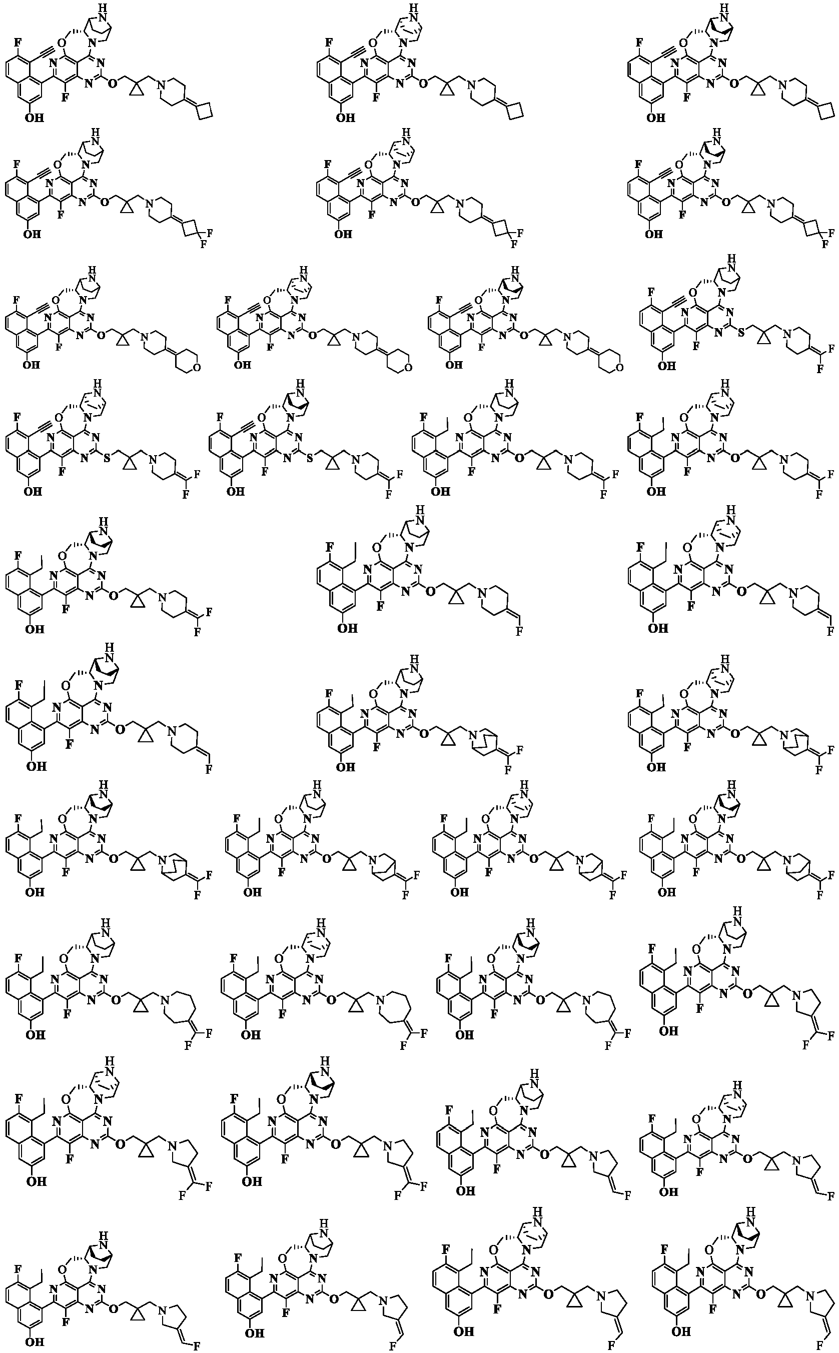

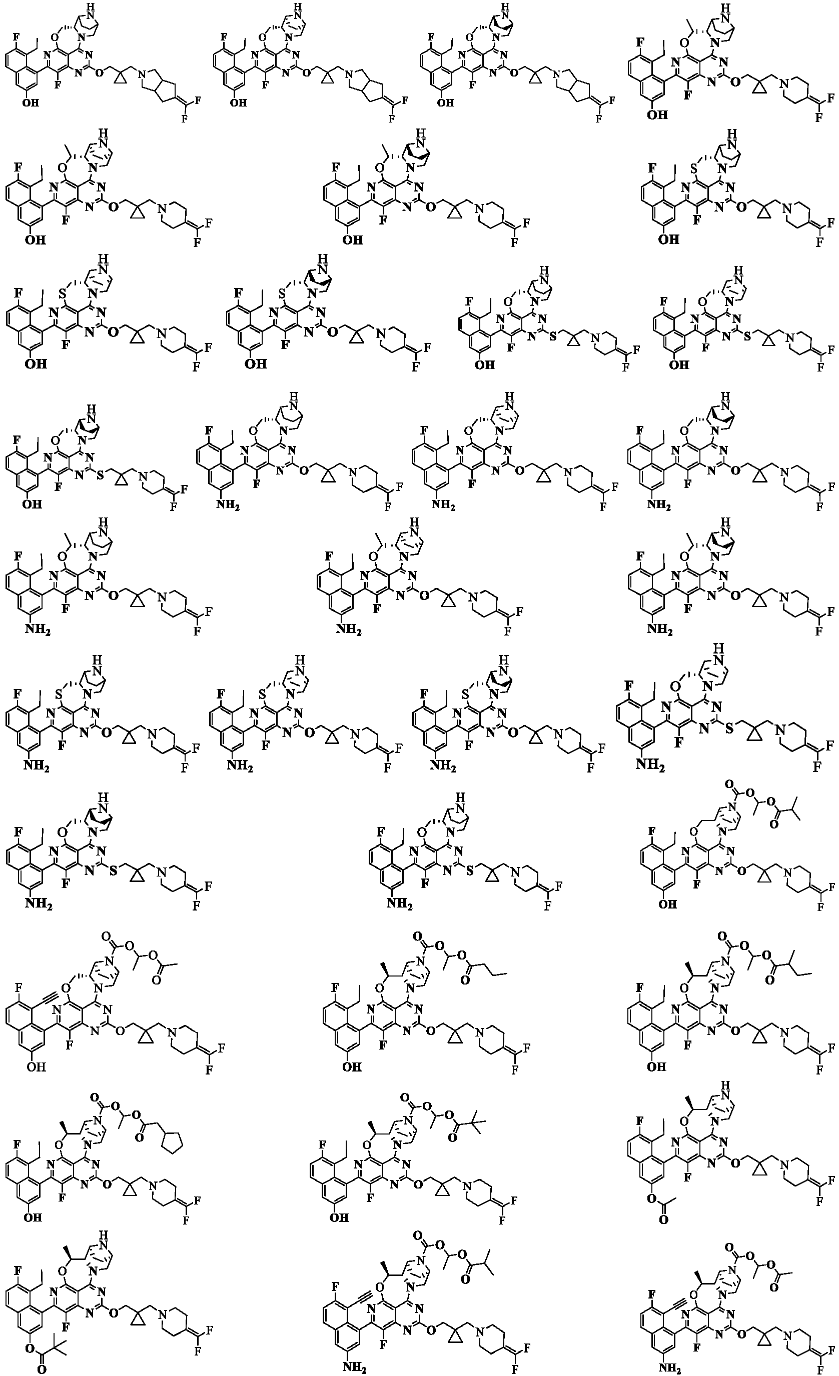

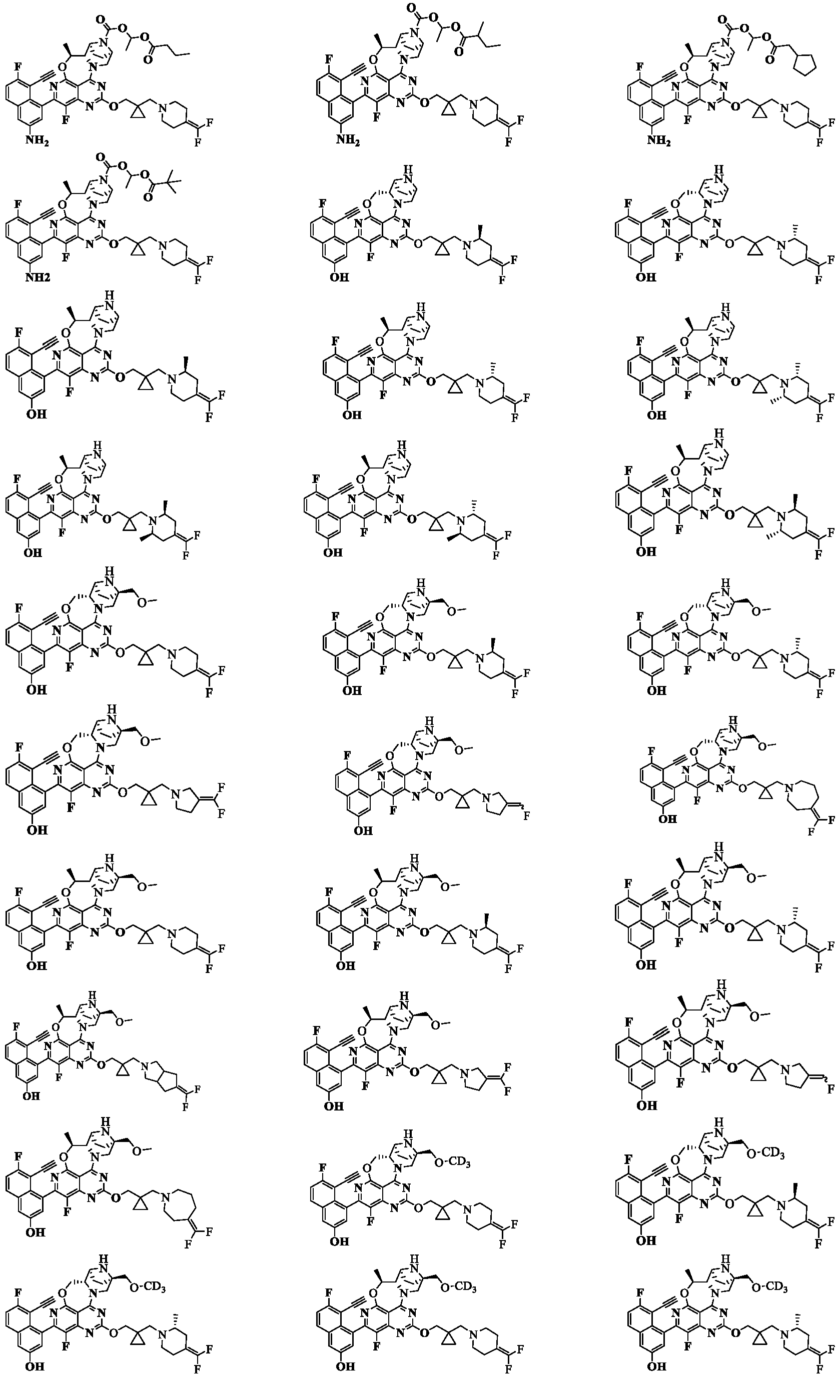

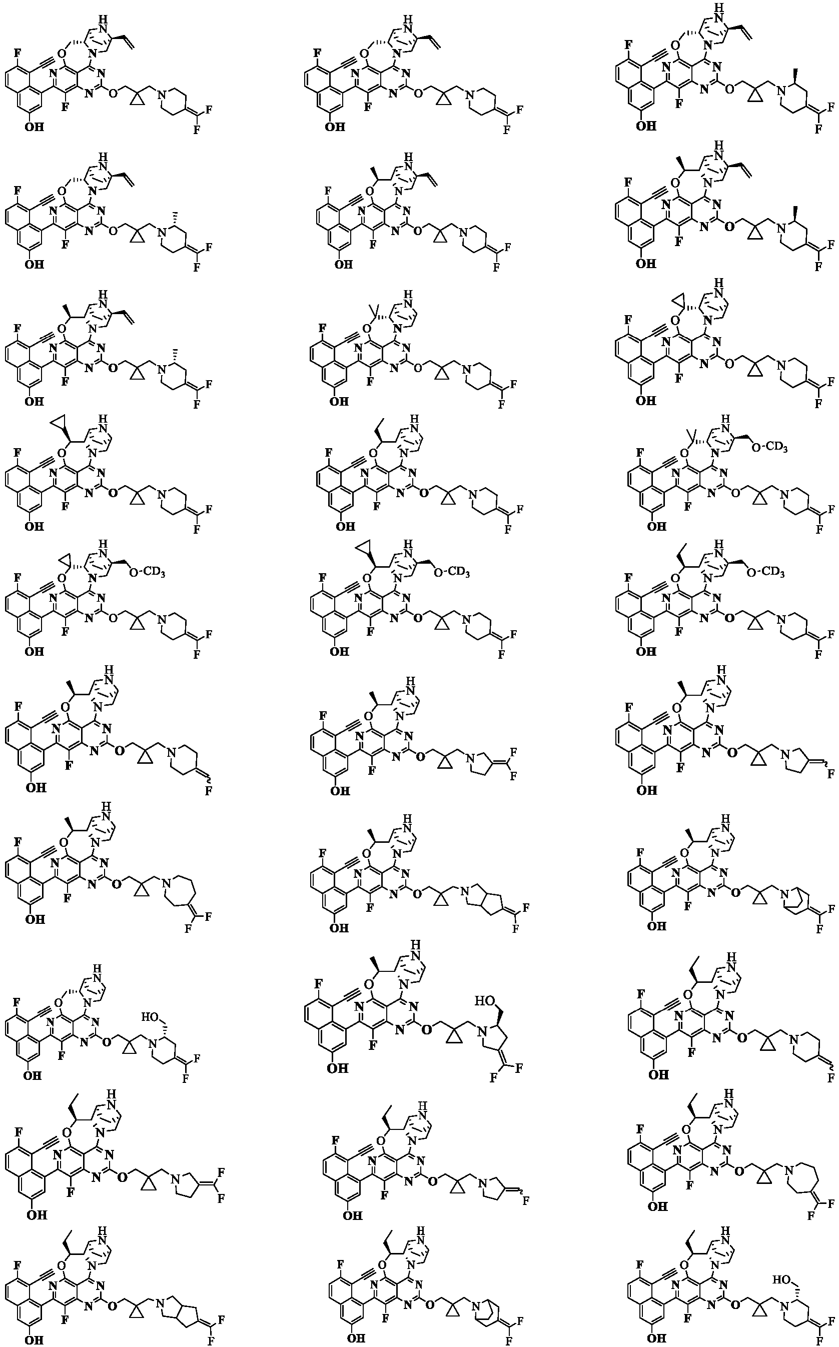

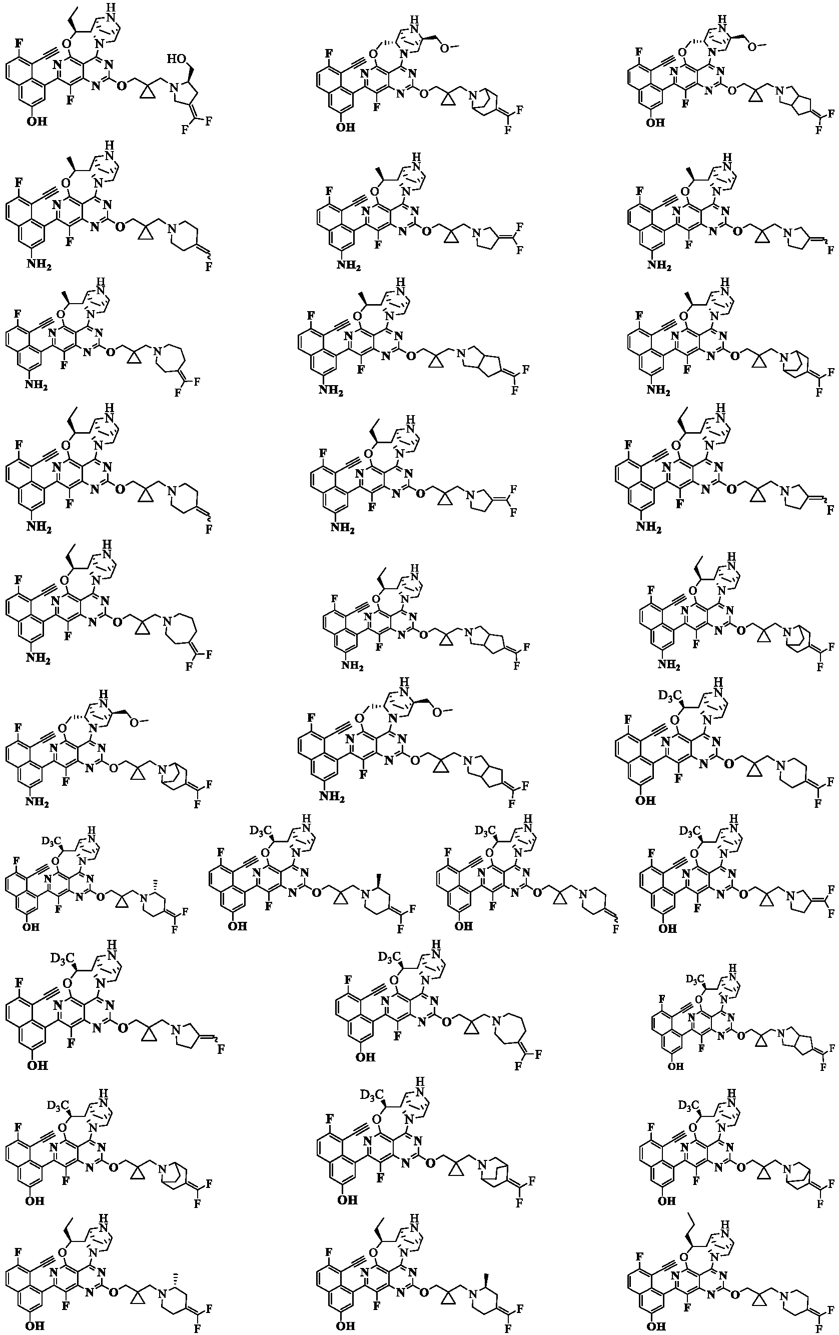

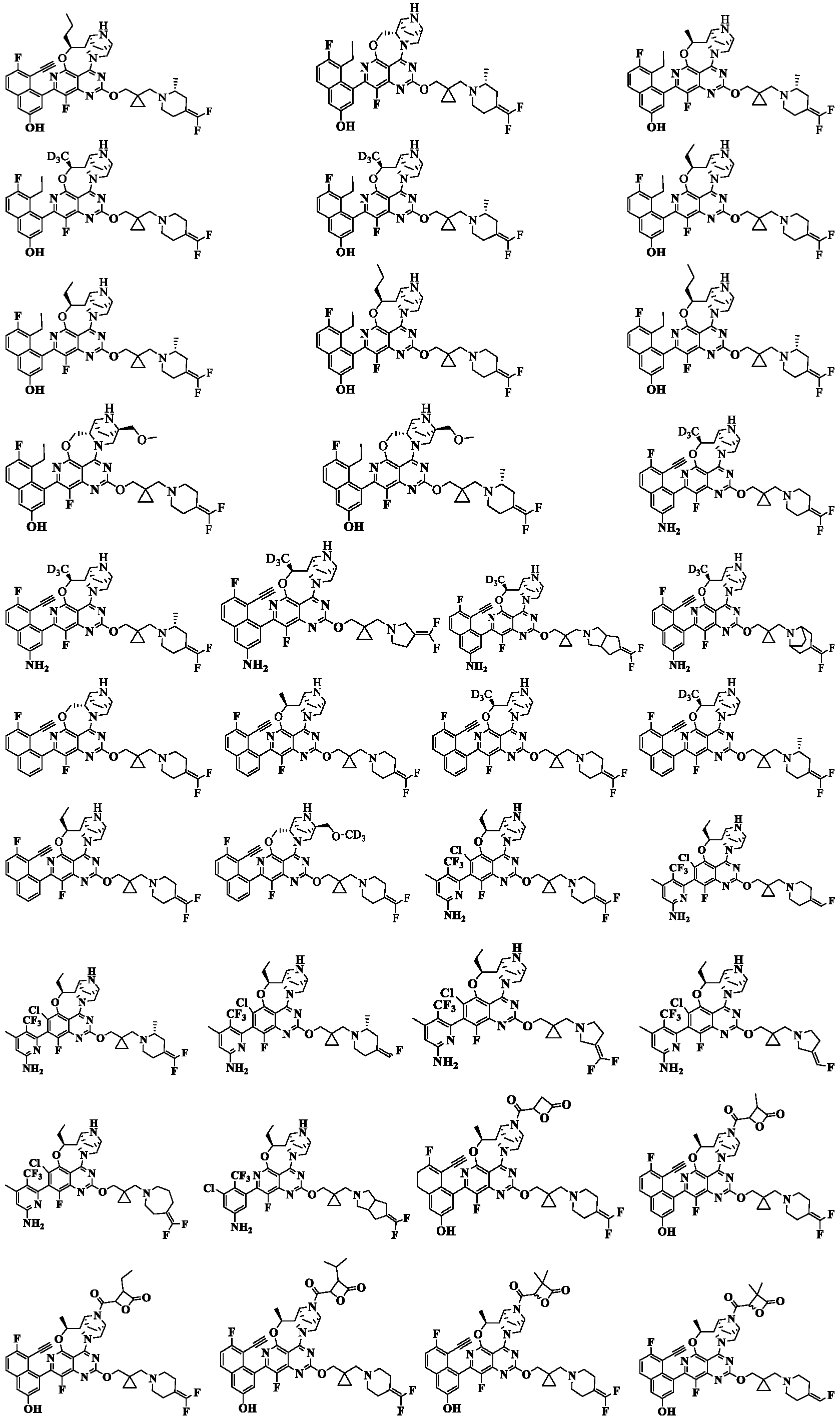

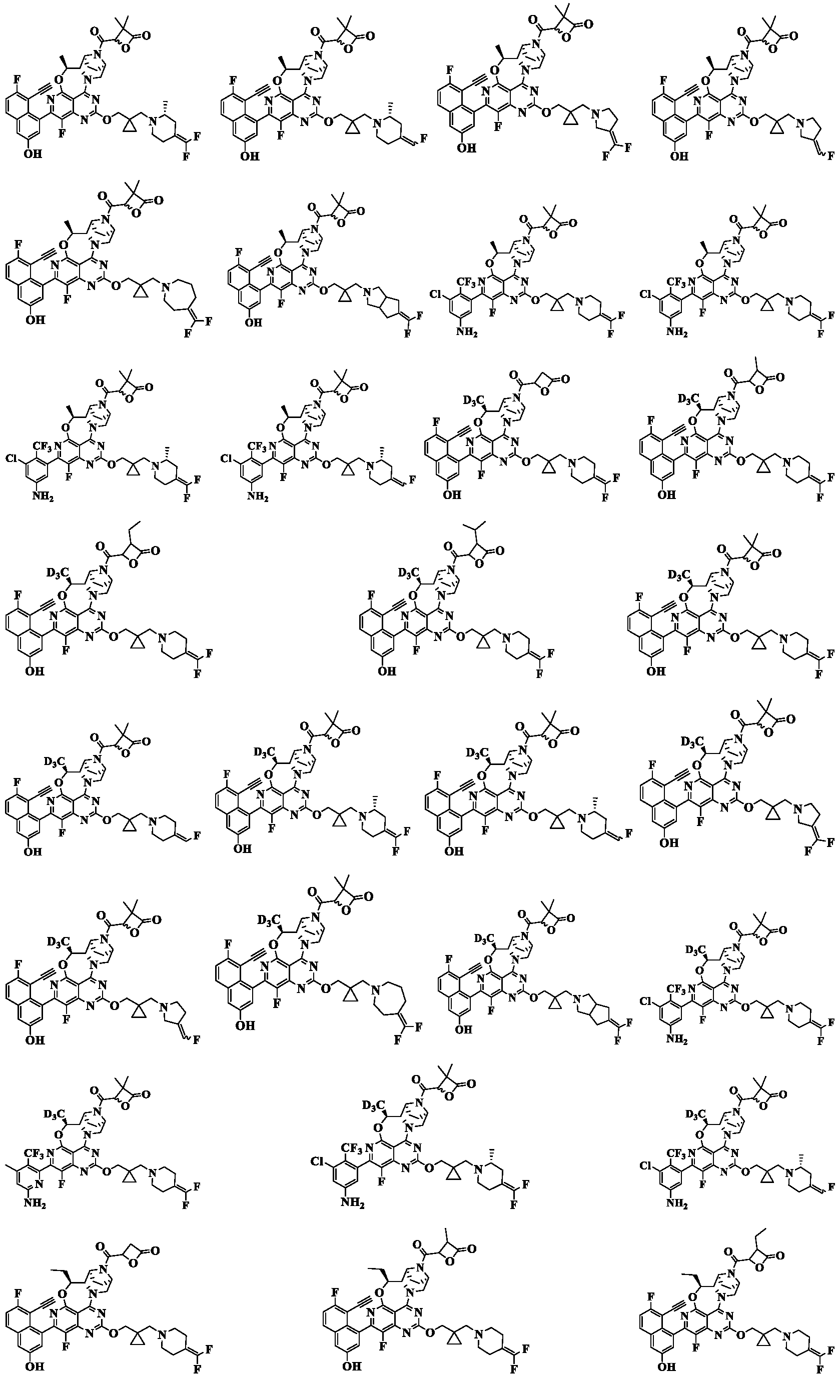

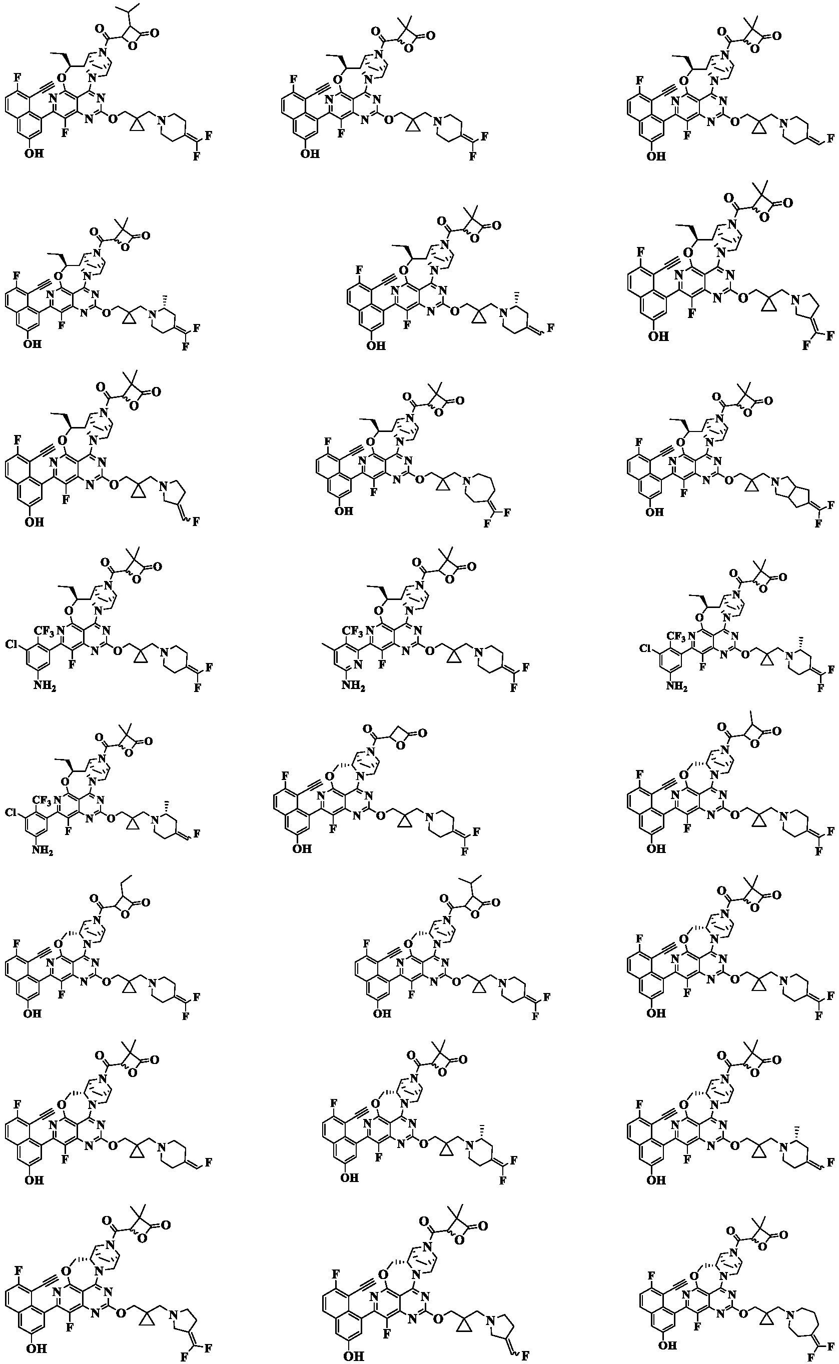

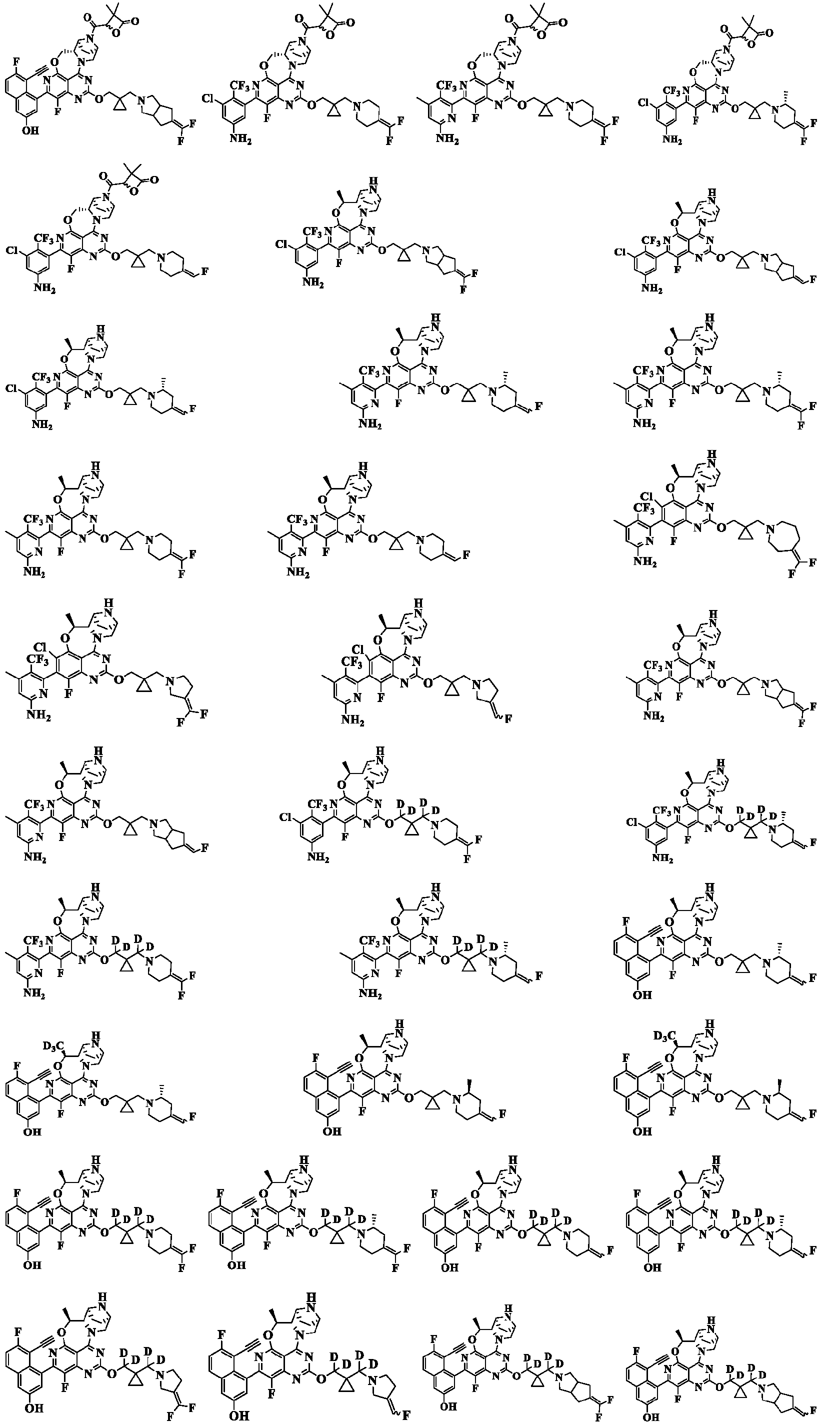

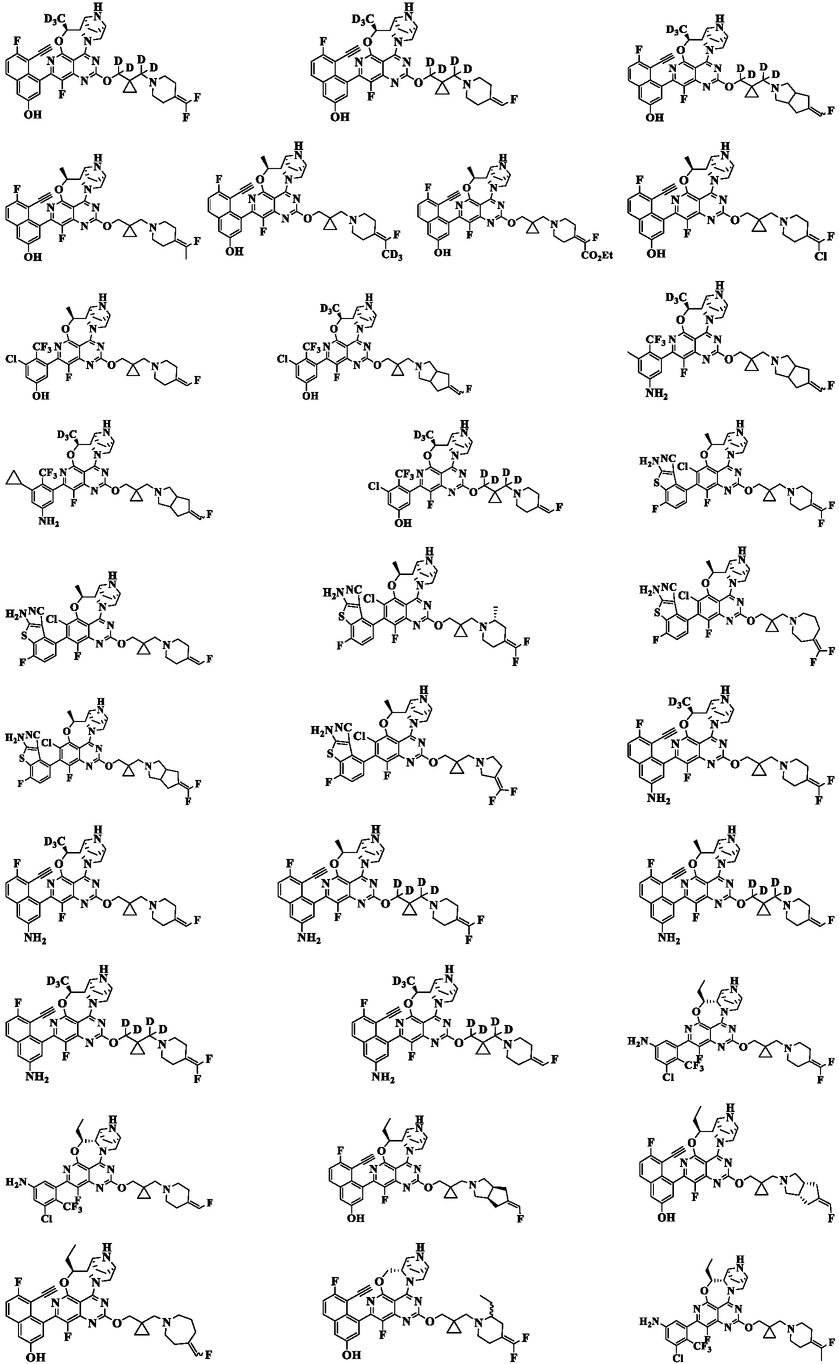

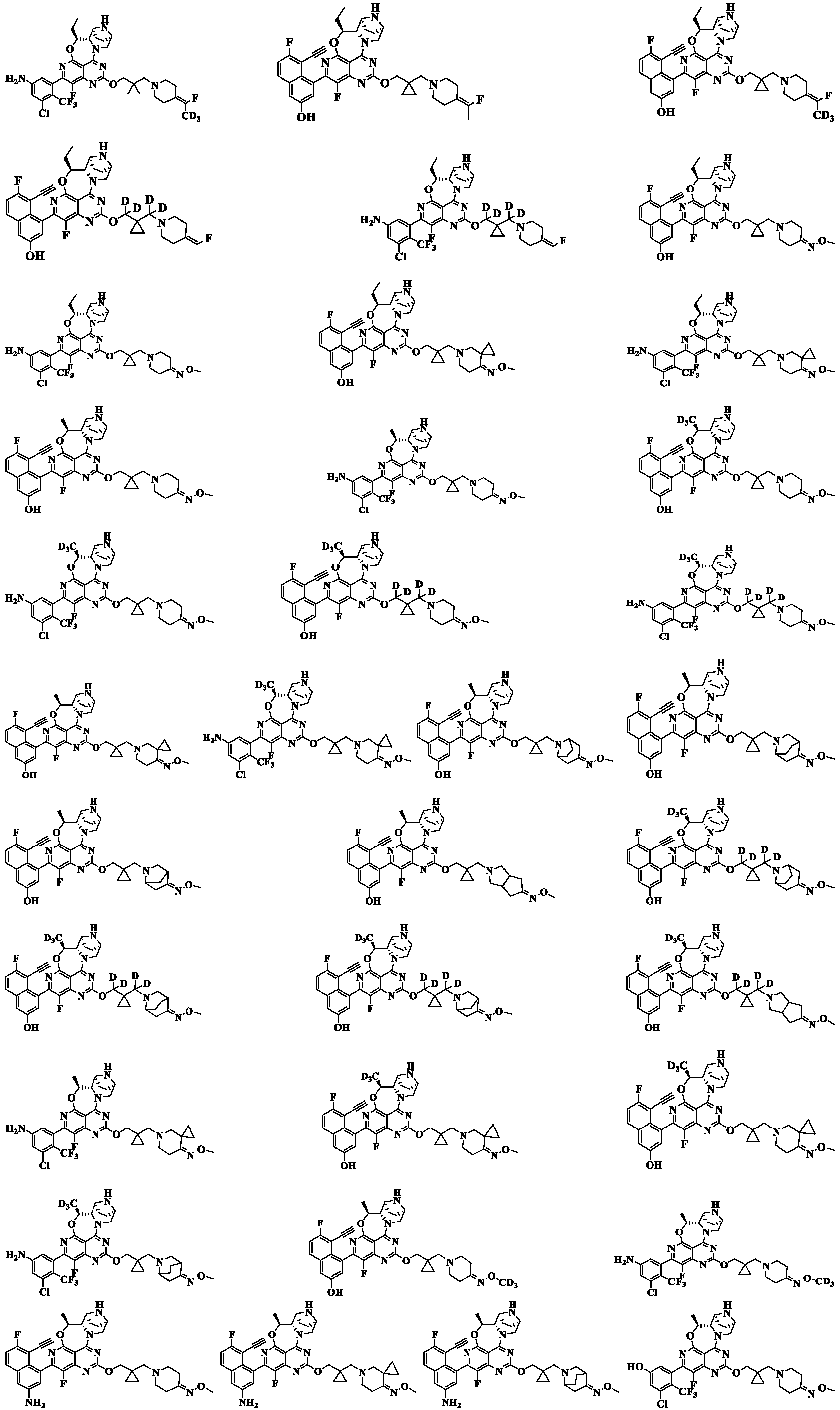

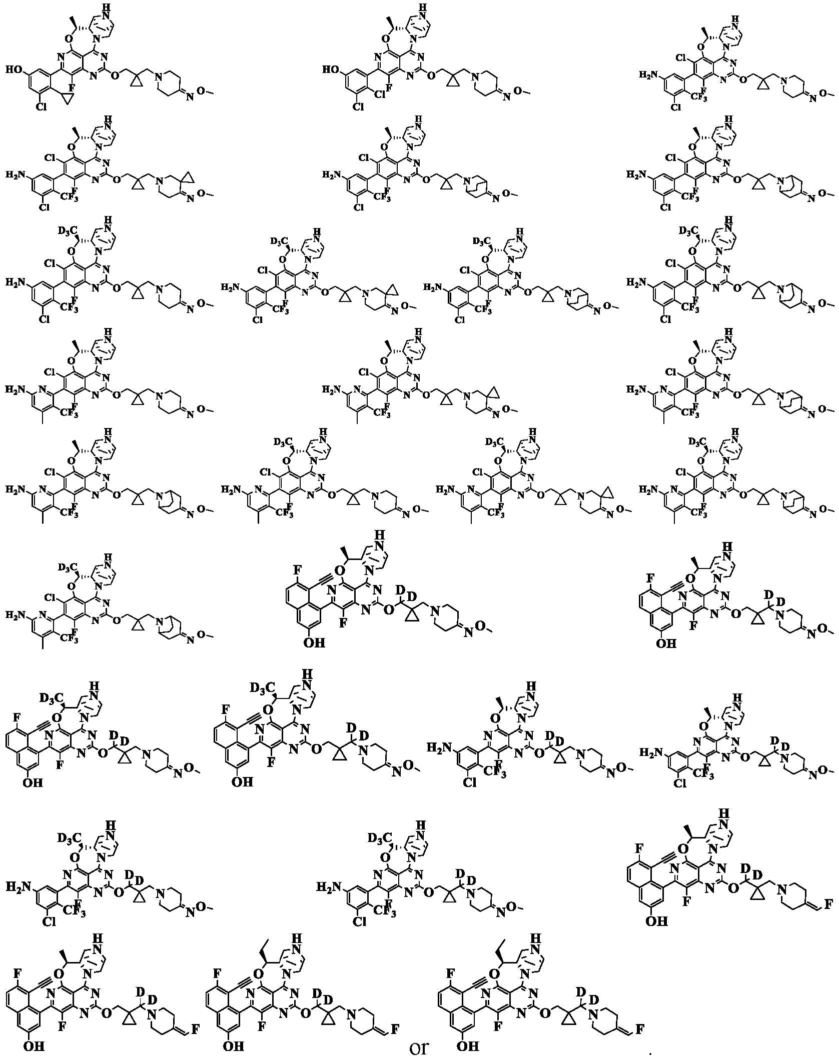

13. A method for preparing a compound represented by general formula (I), or a stereoisomer or pharmaceutically acceptable salt thereof, **characterized in that** the method comprises the following step: 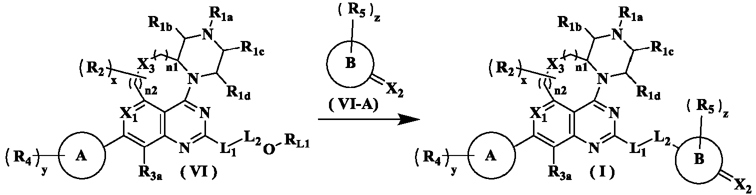
a compound represented by general formula (VI) is reacted with a compound represented by general formula (VI-A) in the presence of a halide salt and a base to obtain the compound represented by general formula (I);
preferably, the method is a method for preparing a compound represented by general formula (II-D), or a stereoisomer or pharmaceutically acceptable salt thereof, comprising the following step: 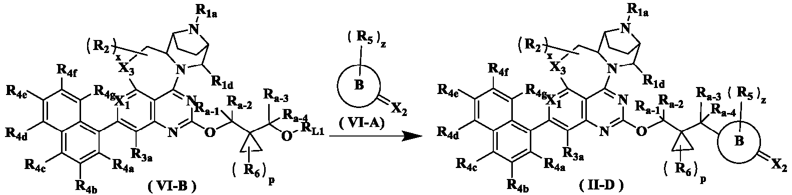
a compound represented by general formula (VI-B) is reacted with the compound represented by general formula (VI-A) in the presence of a halide salt and a base to obtain the compound represented by general formula (II-D);
or preferably, the method is a method for preparing a compound represented by general formula (II-E), or a stereoisomer or pharmaceutically acceptable salt thereof, comprising the following step: 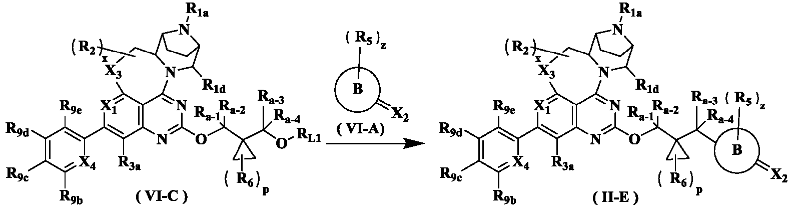
a compound represented by general formula (VI-C) is reacted with the compound represented by general formula (VI-A) in the presence of a halide salt and a base, and a protecting group is further removed to afford the compound represented by general formula (II-E);
R_{L1} is selected from hydrogen or a hydroxyl protecting group;
each of Rₐ₋₁, Rₐ₋₂, Rₐ₋₃ and Rₐ₋₄ is independently selected from hydrogen, deuterium, halogen, amino, hydroxyl, cyano, C₁₋₃ alkyl, C₁₋₃ deuterated alkyl, C₁₋₃ haloalkyl or C₁₋₃ hydroxyalkyl; preferably hydrogen or deuterium;
ring A, X₁, X₂, X₃, L₁, L₂, R₁ₐ, R_{1b}, R_{1c}, R_{1d}, R₂, R₃ₐ, R₄, R₅, x, y, z, n1 and n2 are as defined in claim 1;
ring B is as defined in claim 1 or 11;
R₄ₐ, R_{4b}, R_{4c}, R_{4d}, R₄ₑ, R_{4f} or R_{4g} is as defined in claim 5;
when R_{4g} is alkynyl, R_{4g} can be optionally further substituted with C₁₋₆ silanyl in addition to the above definition;
R₉ₐ, R_{9b}, R_{9c}, R_{9d} or R₉ₑ are as defined in claim 7;
and R₆ and p are as defined in claim 3, 5 or 7.

14. A compound represented by general formula (VII-F), or a stereoisomer or pharmaceutically acceptable salt thereof, **characterized in that** 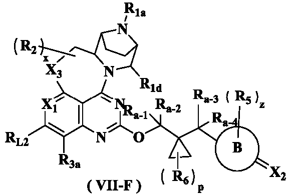
R_{L2} is selected from halogen; preferably chlorine or bromine;
each of Rₐ₋₁, Rₐ₋₂, Rₐ₋₃ and Rₐ₋₄ is independently selected from hydrogen, deuterium, halogen, amino, hydroxyl, cyano, C₁₋₃ alkyl, C₁₋₃ deuterated alkyl, C₁₋₃ haloalkyl or C₁₋₃ hydroxyalkyl; preferably hydrogen or deuterium;
X₁, X₂, X₃, R₁ₐ, R_{1d}, R₂, R₃ₐ, R₅, x and z are as defined in claim 1;
ring B is as defined in claim 1 or 11;
and R₆ and p are as defined in claim 3, 5 or 7.

15. The compound or the stereoisomer or pharmaceutically acceptable salt thereof of claim 14, **characterized in that** the compound has the following structure:

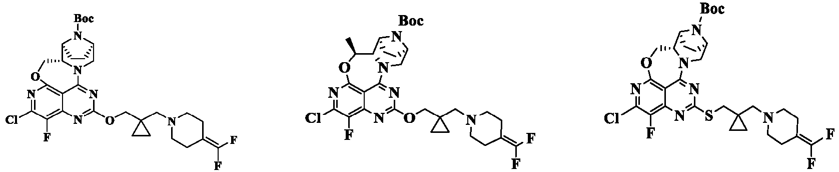

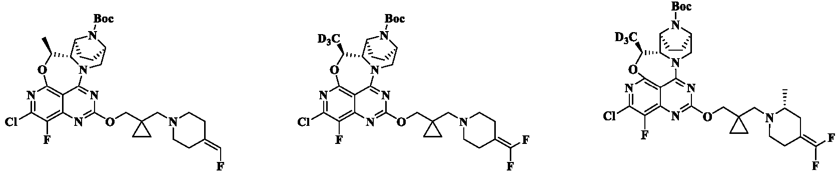

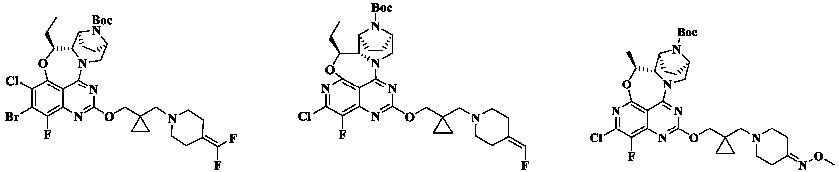

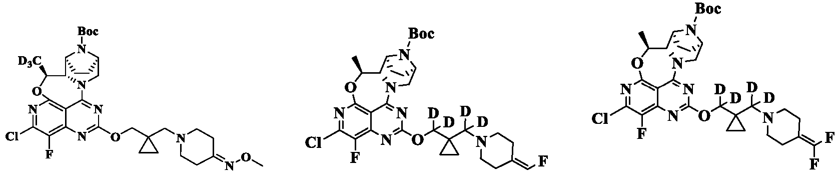

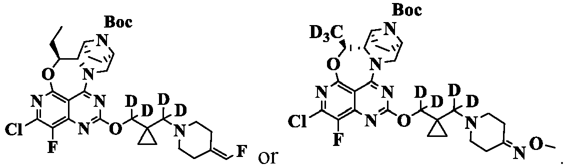

16. A method for preparing a compound represented by general formula (I), or a stereoisomer or pharmaceutically acceptable salt thereof, **characterized in that** the method comprises the following step 1: 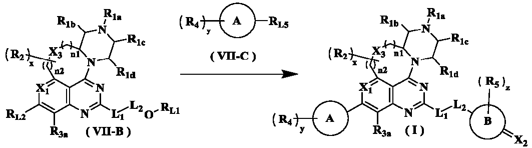
a compound represented by general formula (VII-B) and a compound represented by general formula (VII-C) are subjected to a coupling reaction to afford the compound represented by general formula (I);
optionally, further comprising step 2: 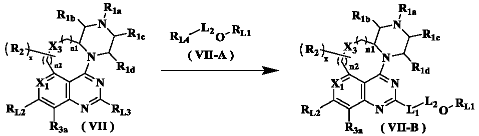
a compound represented by general formula (VII) is reacted with a compound represented by general formula (VII-A) under an alkaline condition to afford the compound represented by general formula (VII-B);
R_{L4} is selected from -OH, or -SH; preferably -OH;
preferably, the method is a method for preparing a compound represented by general formula (II-D), or a stereoisomer or pharmaceutically acceptable salt thereof, comprising the following step 1: 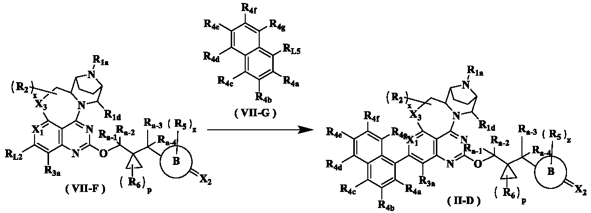
a compound represented by general formula (VII-F) and a compound represented by general formula (VII-G) are subjected to a coupling reaction to afford the compound represented by general formula (II-D);
optionally, further comprising step 2: 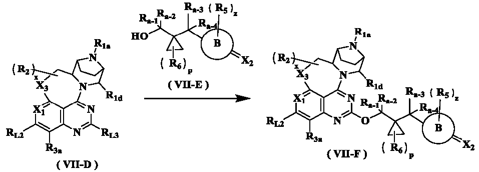
a compound represented by general formula (VII-D) is reacted with a compound represented by general formula (VII-E) under an alkaline condition to afford the compound represented by general formula (VII-F);
or preferably, the method is a method for preparing a compound represented by general formula (II-E), or a stereoisomer or pharmaceutically acceptable salt thereof, comprising the following step 1: 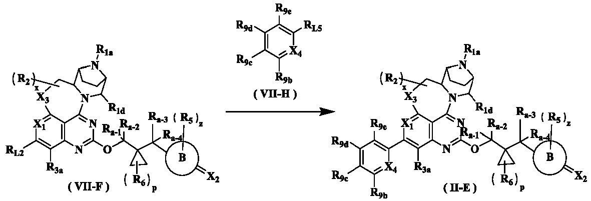
the compound represented by general formula (VII-F) and a compound represented by general formula (VII-H) are subjected to a coupling reaction to afford the compound represented by general formula (II-E);
optionally, further comprising step 2: 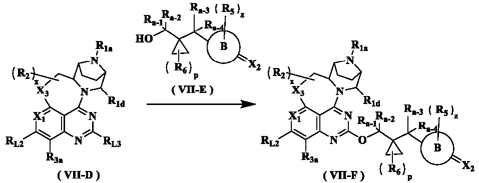
the compound represented by general formula (VII-D) is reacted with the compound represented by general formula (VII-E) under an alkaline condition to afford the compound represented by general formula (VII-F);
each of Rₐ₋₁, Rₐ₋₂, Rₐ₋₃ and Rₐ₋₄ is independently selected from hydrogen, deuterium, halogen, amino, hydroxyl, cyano, C₁₋₃ alkyl, C₁₋₃ deuterated alkyl, C₁₋₃ haloalkyl or C₁₋₃ hydroxyalkyl; preferably hydrogen or deuterium;
R_{L2} is selected from halogen; preferably chlorine or bromine;
R_{L3} is selected from halogen, hydroxyl or -S(O)ₘ₁-C₁₋₃ alkyl; preferably chlorine, bromine, -S(O)-CH₃ or -S(O)₂-CH₃;
R_{L5} is selected from boronic acid group, borate salt group, chain borate ester group or cyclic borate ester group;
m1 is selected from 0, 1 or 2;
ring A, X₁, X₂, X₃, L₁, L₂, R₁ₐ, R_{1b}, R_{1c}, R_{1d}, R₂, R₃ₐ, R₄, R₅, x, y, z, n1 and n2 are as defined in claim 1;
ring B is as defined in claim 1 or 11;
R₄ₐ, R_{4b}, R_{4c}, R_{4d}, R₄ₑ, R_{4f} and R_{4g} are as defined in claim 5;
when R_{4g} is alkynyl, R_{4g} can be optionally further substituted with C₁₋₆ silanyl in addition to the above definition;
R₉ₐ, R_{9b}, R_{9c}, R_{9d} and R₉ₑ are as defined in claim 7;
and R₆ and p are as defined in claim 3, 5 or 7.

17. A pharmaceutical composition, **characterized in that** the pharmaceutical composition comprises a therapeutically effective dose of the compound, or the stereoisomer or pharmaceutically acceptable salt thereof of any one of claims 1 to 12, and one or more pharmaceutically acceptable carriers, diluents or excipients.

18. The compound, or the stereoisomer or pharmaceutically acceptable salt thereof of any one of claims 1 to 12 or the pharmaceutical composition of claim 17 for use in the preparation of a KRAS inhibitor drug; preferably a drug for KRAS G12D, KRAS G12V or KRAS G13D mutations.

19. The compound, or the stereoisomer or pharmaceutically acceptable salt thereof of any one of claims 1 to 12 or the pharmaceutical composition of claim 17 for use in the preparation of a drug for treating diseases or conditions such as Noonan syndrome, Leopard syndrome, leukemia, neuroblastoma, melanoma, esophageal cancer, head and neck tumors, breast cancer, lung cancer and colon cancer; preferably in the preparation of a drug for treating non-small cell lung cancer, colon cancer, esophageal cancer and head and neck tumors.
